# EUROPEAN PATENT SPECIFICATION

(11) **EP 2 150 534 B1**
(45) Date of publication and mention of the grant of the patent: **11.01.2012**
(21) Application number: 08734547.6
(22) Date of filing: 24.04.2008
(51) Int. Cl.: C07D 217/26, C07D 401/06, C07D 403/06, A61K 31/472, A61K 31/4725, A61P 25/18

(54) **ISOQUINOLINONE DERIVATIVES AS NK3 ANTAGONISTS**
ISOCHINOLINONDERIVATE ALS NK3-ANTAGONISTEN
DÉRIVÉS D'ISOQUINOLINONE UTILISÉS EN TANT QU'ANTAGONISTES DE NK3

(30) Priority: 26.04.2007 DK 200700620
(43) Date of publication of application: 10.02.2010
(73) Proprietor: H. Lundbeck A/S, 2500 Valby (DK)
(72) Inventor: SIMONSEN, Klaus, Bæk, DK-5230 Odense M (DK); KEHLER, Jan, DK-2800 Kgs. Lyngby (DK); JUHL, Karsten, DK-2670 Greve (DK); KHANZHIN, Nikolay, DK-3050 Humlebæk (DK); NIELSEN, Søren, Møller, DK-3400 Hillerød (DK)
(86) International application number: PCT/DK2008/050092
(87) International publication number: WO 2008/131779

(56) References cited:
- WO-A-95/32948
- WO-A-2005/014575
- ALBERT, JEFFREY S. ET AL: "Neurokinin -3 receptor antagonists in schizophrenia" EXPERT OPINION ON THERAPEUTIC PATENTS , 16(7), 925-937 CODEN: EOTPEG; ISSN: 1354-3776, 2006, XP002489687

## Description

### Field of the invention

The present invention relates to compounds useful in therapy, in particular in the treatment of psychosis, to compositions comprising said compounds, and to methods of treating diseases comprising the administration of said compounds.

### Background of the invention

The currently approved antipsychotic drugs share the common feature of reducing the dopamine signalling in the brain. This is achieved through either a dopamine D2 receptor antagonistic or partial agonistic effect. The first generation antipsychotics (also referred to as "typical") are often associated with extra-pyrimidal side effects wherefore the use of these agents have diminished. Second generation or "atypical" antipsychotics in addition to the D2 receptor affinity have affinity to the serotonin receptor 2A (5-HT_{2A}). Some atypical antipsychotics in addition have affinity for the 5-HT_{2C}, 5-HT₆, or 5-HT₇ receptors. Atypical antipsychotics give rise to fewer extra-pyrimidal side effects, but are still hampered by weight gain and QT_{C} effects. Examples of atypicals are clozapine, olanzapine and risperidone.

More recently, neurokinin receptors have been suggested as targets for CNS diseases [Albert, Expert Opin. Ther. Patents, 14, 1421-1433, 2004]. Neurokinins (or tachykinins) are a family of neuropeptides, which include substance P (SP), neurokinin A (NKA), and neurokinin B (NKB). The biological effects of these substances are primarily effected through binding to and activation of the three neurokinin receptors NK1, NK2, and NK3. Although some cross reactivity probably exists, SP has the highest affinity and is believed to be the endogenous ligand for NK1, and likewise for NKA and NK2, and for NKB and NK3.

NK3 is primarily expressed centrally in regions including cortical regions, such as frontal, parietal and cingulated cortex; nuclei of the amygdale, such as the basal, central and lateral nuclei; the hippocampus; and mesencephalon structures, such as ventral tegmental area, substantia nigra pars compacta, and dorsal raphe nuclei [Spooren et al, Nature Reviews, 4, 967-975, 2005]. The NK3 receptor is expressed on dopaminergic neurons, and Spooren et al has suggested that the antipsychotic effects of NK3 antagonists are mediated by an inhibition of the dopamine tone, particularly at the D2 receptor combined with a reduction of the serotonergic tone, particularly at the 5-HT_{2A} receptor.

Two structurally distinct NK3 antagonists, namely talnetant and osanetant, have been clinically tested for antipsychotic, and in particular antischizophrenic effects.

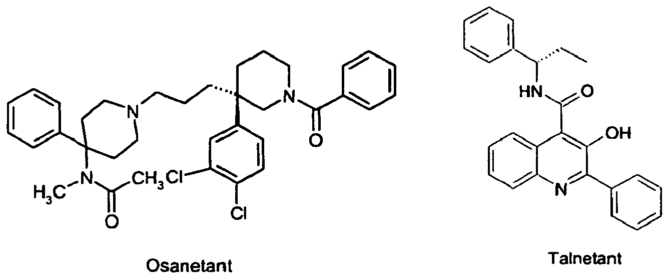

Osanetant proved superior to placebo in clinical trials, in particular on positive symptoms of psychosis, i.e. delusions, hallucinations and paranoia [Am.JPsychiatry, 161, 2004, 975-984]. Similarly, talnetant has been shown in clinical trials to ameliorate the cognitive behaviour of schizophrenics [Curr.Opion.Invest.Drug, 6, 717-721, 2005]. Nevertheless, both compounds are hampered by poor pharmacokinetic and pharmacodynamic properties including poor solubility, poor bioavailability, relatively high clearance, and poor blood-brain barrier penetration [Nature reviews, 4, 967-975, 2005]. These results lend support to the notion that the NK3 receptor is a promising target for the treatment of e.g. psychosis, however emphasizing the need for identifying compounds with adequate pharmacokinetic and pharmacodynamic properties.

WO95/32948 discloses a range of quinoline derivatives, including talnetant as NK3 antagonists.

More recently, WO 2006/130080 discloses compounds having the core structure 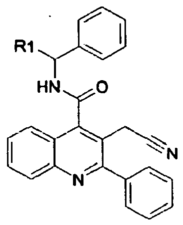 which compounds are said to be NK3 antagonists; and WO 2006/050991 and WO 2006/050992 disclose further quinolinecarboxamides derivatives, which derivatives are said to be NK3 antagonists.

WO 2005/014575 discloses compounds of the formula 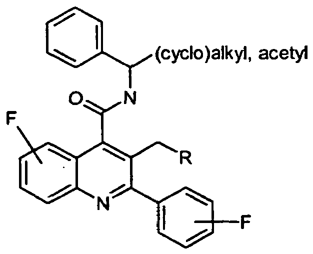 wherein R represents N-containing heterocycles, i.e. pyrazolyl, triazolyl and tetrazolyl.

Finally, Ind.J.Chem. Section B, 18B, 304-306, 1979 discloses a study on the synthesis of compounds with the following core structure

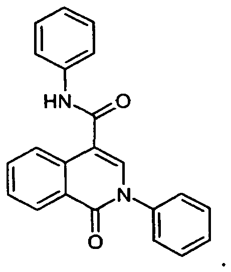

### Summary of the invention

The present inventors have surprisingly found that certain isoquinolinone derivatives are potent NK3 antagonists, which may as such be used in the treatment of e.g. psychosis. Accordingly, in one embodiment the invention relates to compounds of formula I 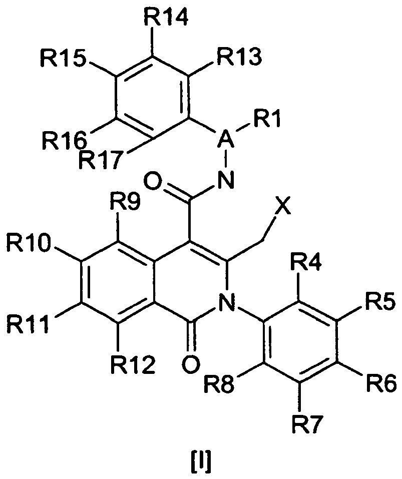 wherein A represents N, CH or CR¹;
wherein each R¹ independently represents hydrogen, C₁₋₆alkyl, C₂₋₆alkenyl, C₂-₆ alkynyl, -C(O)-C₁₋₆alkyl, -C(O)-C₂₋₆alkenyl, -C(O)-C₂₋₆alkynyl, -C(O)-O-C₁₋₆alkyl, - C(O)-O-C₂₋₆alkenyl, -C(O)-O-C₂₋₆alkynyl or phenyl, wherein said phenyl, C₁₋₆alkyl, C₂₋₆alkenyl or C₂₋₆alkynyl is optionally substituted with one or more substituents selected from halogen, hydroxyl, haloC₁₋₆alkyl, nitro, C₁₋₆alkoxy, and NR²R³; wherein X represents hydrogen, C₁₋₆alkyl, C₂₋₆alkenyl, C₂₋₆alkynyl, cyano, -OR², -O-C(O)R², -OC(O)NR²R³, -C(O)-NR²R³, -N(R²)C(O)R³, -N(R²)-C(O)NR²R³ or NR²R³, or X represents a mono-cyclic, bi-cyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₂₋₆alkenyl, C₂₋₆alkynyl, C₁₋₆alkoxy, -C(O)-C₁₋₆alkyl, -C(O)-C₂₋₆alkenyl, -C(O)-C₂₋₆alkynyl, - C(O)-O-C₁₋₆alkyl, -C(O)-O-C₂₋₆alkenyl, -C(O)-O-C₂-₆alkynyl, -O-C(O)-C₁₋₆alkyl, -O-C(O)-C₂₋₆alkenyl, -O-C(O)-C₂₋₆alkynyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-,-C(O)-NH-, or S(O)₂;
wherein Z represents hydrogen, C₁₋₆alkyl, NR²R³, cyano or a mono-cyclic or fused bicyclic moiety comprising 4 tol2 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein each of R⁴-R⁸, R⁹-R¹², and R¹³-R¹⁷ independently represent hydrogen, C₁₋₆ alkyl, C₂₋₆alkenyl, C₂₋₆alkynyl, halogen, NR²R³, hydroxy, cyano, nitro, C₁₋₆alkoxy, haloC₁₋₆alkyl or hydroxyC₁₋₆alkyl;
wherein R² and R³ independently represent hydrogen, C₁₋₆alkyl, C₂₋₆alkenyl, C₂₋₆ alkynyl, hydroxyC₁₋₆alkyl, haloC₁₋₆alkyl or phenyl;
and pharmaceutically acceptable salts thereof.

In one embodiment, the invention relates to compounds of formula I and pharmaceutically acceptable salts thereof for use in therapy.

In one embodiment, the invention relates to pharmaceutical compositions comprising compounds of formula 1 and pharmaceutically acceptable salts thereof.

In one embodiment, the invention relates to methods of treatment, which methods comprise the administration of therapeutically effective amounts of a compound of formula I and pharmaceutically acceptable salts thereof to a patient in need thereof.

In one embodiment, the invention relates to the use of a compound of formula I and pharmaceutically acceptable salts thereof in the manufacture of a medicament.

In one embodiment, the invention relates to compounds of formula I and pharmaceutically acceptable salts thereof for use in the treatment of diseases.

### Definitions

In the present context, "alkyl" is intended to indicate a straight, branched and/or cyclic saturated hydrocarbon. In particular "C₁₋₆alkyl" is intended to indicate such hydrocarbon having 1, 2, 3, 4, 5, or 6 carbon atoms. Examples of C₁₋₆alkyl include methyl, ethyl, propyl, butyl, pentyl, hexyl, cyclopropyl, cyclobutyl, cyclopentyl, cyclohexyl, 2-methyl-propyl, tert.-butyl, and cyclopropylmethyl.

In the present context, "alkenyl" is intended to indicate a straight, branched and/or cyclic hydrocarbon comprising at least one carbon-carbon double bond. In particular "C₂₋₆alkenyl" is intended to indicate such hydrocarbon having 2, 3, 4, 5, or 6 carbon atoms. Examples of C₂₋₆alkenyl include ethenyl, 1-propenyl, 2-propenyl, 1-butenyl, 2-butenyl, 3-butenyl, and cyclohexenyl.

In the present context, "alkynyl" is intended to indicate a straight, branched and/or cyclic hydrocarbon comprising at least one carbon-carbon triple bond and optionally also one or more carbon-carbon double bonds. In particular "C₂₋₆alkynyl" is intended to indicate such hydrocarbon having 2, 3, 4, 5, or 6 carbon atoms. Examples of C₂₋₆alkynyl include ethynyl, 1-propynyl, 2-propynyl, 1-butynyl, 2-butynyl, 3-butynyl and 5-but-1-en-3-ynyl.

In the present context "halogen" is intended to indicate members of the 7^{th} group of the periodic system, e.g. flouro, chloro, bromo, and iodo.

In the present context, "alkoxy" is intended to indicate a moiety of the formula -OR', wherein R' indicates alkyl as defined above. In particular "C₁₋₆alkoxy" is intended to indicate such moiety wherein the alkyl part has 1, 2, 3, 4 5, or 6 carbon atoms.

In the present context, haloalkyl is intended to indicate an alkyl as defined above substituted with one or more halogens. In particular, haloC₁₋₆alkyl is intended to indicate a moiety wherein the alkyl part has 1, 2, 3, 4, 5 or 6 carbon atoms. One example of haloalkyl is trifluoromehtyl.

In the present context, pharmaceutically acceptable salts include pharmaceutical acceptable acid addition salts, pharmaceutically acceptable metal salts, ammonium and alkylated ammonium salts. Acid addition salts include salts of inorganic acids as well as organic acids.

Examples of suitable inorganic acids include hydrochloric, hydrobromic, hydroiodic, phosphoric, sulfuric, sulfamic, nitric acids and the like.

Examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-halotheophyllines, for example 8-bromotheophylline and the like. Further examples of pharmaceutical acceptable inorganic or organic acid addition salts include the pharmaceutically acceptable salts listed in J. Pharm. Sci. 1977,66,3, which is incorporated herein by reference.

Examples of metal salts include lithium, sodium, potassium, magnesium salts and the like.

Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

In the present context, a "ring atom" is intended to indicate the atoms constituting a ring, and ring atoms are selected from C, N, O and S. As an example, benzene and toluene both have 6 carbons as ring atoms whereas pyridine has 5 carbons and I nitrogen as ring atoms.

In the present context, a "mono-cyclic moiety" is intended to indicate a ring formed structure comprising only one ring. Similarly, a "bi-cyclic moiety" is intended to indicate a structure comprised of two joined rings. The two rings may be joined at two adjacent ring atoms in each ring in which case said bi-cyclic moiety is said to be fused. Naphthalene is an example of a fused bi-cyclic ring moiety. Alternatively, the two rings arc joined at a shingle ring atom in which case said bi-cyclic moiety is said to be spiro. 1,4-Dioxa-8-aza-spiro[4.5]decane is an example of a bi-cyclic ring moiety in spiro form. Alternatively, the two rings may be joined at two non-adjacent ring atoms in each ring in which case said bi-cyclic ring moiety is said to be caged. 3-Aza-bicyclo[3.2.2]nonane is an example of a caged bi-cyclic moiety. A "tri-cyclic moiety" is intended to indicate a structure comprised of three joined rings. As discussed for the bi-ctyclic moieties above, tri-cyclic moieties may be fused, spiro or caged, or in deed, a combination thereof.

In the present context, the term "therapeutically effective amount" of a compound means an amount sufficient to cure, alleviate or partially arrest the clinical manifestations of a given disease and its complications in a therapeutic intervention comprising the administration of said compound. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

In the present context, the term "treatment" and "treating" means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatments are two separate aspects of the invention. The patient to be treated is preferably a mammal, in particular a human being.

### Detailed description of the invention

The compounds of the present invention are defined by formula I below 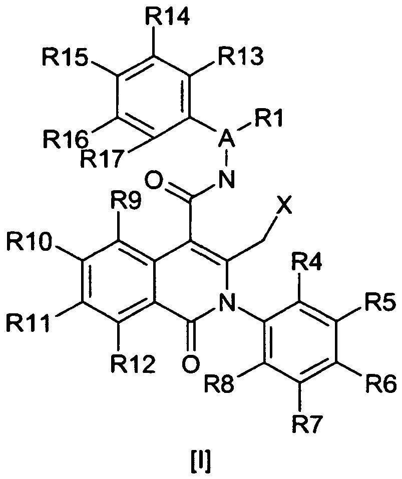 wherein A represents N, CH or CR¹;
wherein each R¹ independently represents hydrogen, C₁₋₆alkyl, C₂₋₆alkenyl, C₂₋₆ alkynyl, -C(O)-C₁₋₆alkyl, -C(O)-C₂₋₆alkenyl, -C(O)-C₂₋₆alkynyl, -C(O)-O-C₁₋₆alkyl, - C(O)-O-C₂₋₆alkenyl, -C(O)-O-C₂₋₆alkynyl or phenyl, wherein said phenyl, C₁₋₆alkyl, C₂₋₆alkenyl or C₂₋₆alkynyl is optionally substituted with one or more substituents selected from halogen, hydroxyl, haloC₁₋₆alkyl, nitro, C₁₋₆alkoxy, and NR²R³; wherein X represents hydrogen, C₁₋₆alkyl, C₂₋₆alkenyl, C₂₋₆alkynyl, cyano, -OR², -O-C(O)R², -OC(O)NR²R³, -C(O)-NR²R³, -N(R²)C(O)R³, -N(R²)-C(O)NR²R³ or NR²R³, or X represents a mono-cyclic, bi-cyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₂₋₆alkenyl, C₂₋₆alkynyl, C₁₋₆alkoxy, -C(O)-C₁₋₆alkyl, -C(O)-C₂₋₆alkenyl, -C(O)-C₂₋₆alkynyl, - C(O)-O-C₁₋₆alkyl, -C(O)-O-C₂₋₆alkenyl, -C(O)-O-C₂₋₆alkynyl, -O-C(O)-C₁₋₆alkyl, -O-C(O)-C₂₋₆alkenyl, -O-C(O)-C₂₋₆alkynyl, -C(O)H, COOH; or wherein W represents a moiety of the formula -(CH₂)ₐ-Y-(CH₂)_{b}-Z;
wherein a and b independently represent an integer selected from 0, 1, 2 and 3;
wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein each of R⁴-R⁸, R⁹-R¹², and R¹³-R¹⁷ independently represent hydrogen, C₁₋₆ alkyl, C₂₋₆alkenyl, C₂₋₆alkynyl, halogen, NR²R³, hydroxy, cyano, nitro, C₁₋₆alkoxy, haloC₁₋₆alkyl or hydroxyC₁₋₆alkyl;
wherein R² and R³ independently represent hydrogen, C₁₋₆alkyl, C₂₋₆alkenyl, C₂₋₆ alkynyl, hydroxyC₁₋₆alkyl, haloC₁₋₆alkyl or phenyl;
and pharmaceutically acceptable salts thereof.

In one embodiment, R⁴-R⁸ represent hydrogen.

In one embodiment, R⁹-R¹² represent hydrogen.

In one embodiment, R¹³-R¹⁷ represent hydrogen.

In one embodiment, the compounds of the present invention are defined by formula Ia. 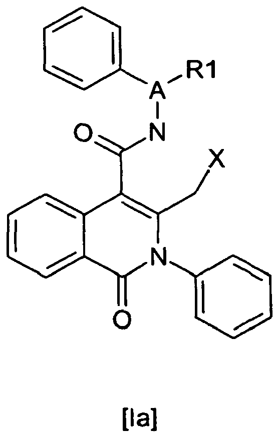 wherein A represents N, CH or CR¹;
wherein each R¹ independently represents hydrogen, C₁₋₆alkyl, -C(O)-C₁₋₆alkyl, -C(O)-O-C₁₋₆alkyl, or phenyl, wherein said phenyl or C₁₋₆alkyl, is optionally substituted with one or more substituents selected from halogen, hydroxyl, haloC₁₋₆ alkyl, nitro, C₁₋₆alkoxy, and NR²R³;
wherein X represents hydrogen, C₁₋₆alkyl, cyano, -OR², -O-C(O)R², -OC(O)NR²R³, - C(O)-NR²R³, -N(R²)C(O)R³, -N(R²)-C(O)NR²R³ or NR²R³, or X represents a mono-cyclic, bicyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein each of R⁴-R⁸, R⁹R¹², and R¹³-R¹⁷ independently represent hydrogen, C₁₋₆ alkyl, C₂₋₆alkenyl, C₂₋₆alkynyl, halogen, NR²R³, hydroxy, cyano, nitro, C₁₋₆alkoxy, haloC₁₋₆alkyl or hydroxyC₁₋₆alkyl;
wherein R² and R³ independently represent hydrogen, C₁₋₆alkyl, C₂₋₆alkenyl, C₂₋₆ alkynyl, hydroxyC₁₋₆alkyl, haloC₁₋₆alkyl or phenyl; and pharmaceutically acceptable salts thereof. In particular, A represents CH and R¹ represents C₁₋₆alkyl, such as ethyl or cyclopropyl.

In one embodiment wherein the compounds of the present invention are defined by formula Iₐ, X represents hydrogen, C₁₋₆alkyl, cyano, -OR², -O-C(O)R², - OC(O)NR²R³, -C(O)-NR²R³-, -N(R²)C(O)R³, -N(R²)-C(O)NR²R³ or NR²R³, wherein R² and R³ independently represent hydrogen, C₁₋₆alkyl, hydroxyC₁₋₆alkyl, haloC₁₋₆ alkyl or phenyl. In particular, X represents hydrogen, methyl, or NR²R³, wherein R² and R³ independently represent hydrogen, C₁₋₆alkyl, or hydroxyC₁₋₆alkyl, and particular mentioning is made of R² and R³ independently representing hydrogen, cyclopropylmethyl, methyl, ethyl or cyclopropyl.

In one embodiment wherein the compounds of the present invention are defined by formula Iₐ, X represents a mono-cyclic, bi-cyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moicty may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -O-C(O)-, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂.
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen. C₁₋₆alkyl, hydroxyC₁₋₆alkyl, haloC₁₋₆alkyl or phenyl.

In one embodiment wherein the compounds of the present invention are defined by formula Iₐ, X represents a mono-cyclic moiety having 5 ring atoms one of which is nitrogen, and wherein one, two or three additional ring atoms may be a hetero atom selected from N, O and S, and wherein said mono-cyclic moiety may optionally be substituted with one or more substituents W. wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -O-C(O)-, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl. Examples of such mono-cyclic moiety having 5 ring atoms include pyrrole, 2H-pyrrole, pyrazole, isothiazole, pyrrolidine, pyrroline, tetrazole, imidazolidine, imidazoline, pyrazolidine and pyrazoline.

In one embodiment wherein the compounds of the present invention are defiled by formula Iₐ, X represents a mono-cyclic moiety having 6 ring atoms one of which is nitrogen, and wherein one, two or three additional ring atoms may be a hetero atom selected from N, O and S, and wherein said mono-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -O-C(O)-, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moicty comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl. Examples of such mono-cyclic moiety having 6 ring atoms include pyridine, pyrazine, pyrimidine, pyridazine, piperidine, piperazine dihydropyridine, tetrahydropyridine, and morpholine.

In one embodiment, wherein the compounds of the present invention are defined by formula Iₐ, X represents a mono-cyclic moiety having 7 ring atoms one of which is nitrogen, and wherein one, two or three additional ring atoms may be a hetero atom selected from N, O and S, and wherein said mono-cyclic moiety may optionally be substituted with one or more substituents W, wherein W is selected from hydrogen, hydroxy, halogen, cyano. (=O), -O-(CH₂)_{c}-O-, wherein c is 2 or 3 (spiro), (CH₂)_{d}, wherein d is 5 or 6 (spiro), C₁₋₆alkyl, C₂₋₆alkenyl, C₂₋₆alkynyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N. O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and Halogen substituted phenyl;
wherein R² and R³ independently represent hydrogen, C₁₋₆alkyl, hydroxyC₁₋₆alkyl, haloC₁₋₆alkyl or phenyl. Examples of such mono-cyclic moiety having 7 ring atoms include azepanc, [1,4]diazepane and 2,3,4,5-tetrahydro-1H-[1,4]diazepane.

In one embodiment wherein compounds of the present invention are defined by formula Iₐ, X represents a mono-cyclic or bi-cyclic moiety having 8 ring atoms one of which is nitrogen, and wherein one, two or three additional ring atoms may be a hetero atom selected from N, O and S, and wherein said cyclic or bicyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl. Examples of such mono-cyclic or bi-cyclic moiety having 8 ring atoms include quinnuclidine, 8-aza-bicyclo[3.2.1]octane and 2-aza-bicyclo[2.2.2]octane.

In one embodiment wherein the compounds of the present invention are defined by formula Iₐ, X represents a mono-cyclic or bi-cyclic moiety having 9 ring atoms one of which is nitrogen, and wherein one, two or three additional ring atoms may be a hetero atom selected from N, O and S, and wherein said mono-cyclic or bicyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆ alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, C₂₋₆alkenyl, C₂-₆ alkynyl, hydroxyC₁₋₆alkyl, haloC₁₋₆alkyl or phenyl. Examples of such mono-cyclic or bi-cyclic moiety having 9 ring atoms include indolizine, isoindole, 3H-indole, indole, 1H-indazole, purine, indoline, and isoindoline.

In one embodiment wherein the compounds of the present invention are defined by formula Iₐ, X represents a mono-cyclic or bi-cyclic moiety having 10 ring atoms one of which is nitrogen, and wherein one, two or three additional ring atoms may be a hetero atom selected from N, O and S, and wherein said mono-cyclic or bicyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆ alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl. Examples of such mono-cyclic or bi-cyclic moiety having 10 ring atoms include 4H-quinolizine, isoquinoline, quinoline, phthalazine, naphthyridine, quinoxaline, cinnoline, pterridine, 1,2,3,4-tetrahydro-isoquinolone, 1,4,8-triaza-spiro[4.5]decane and 1,2,3,4-tetrahydro-quinoline.

In one embodiment, the compounds of the present invention are defined by formula I_{b} 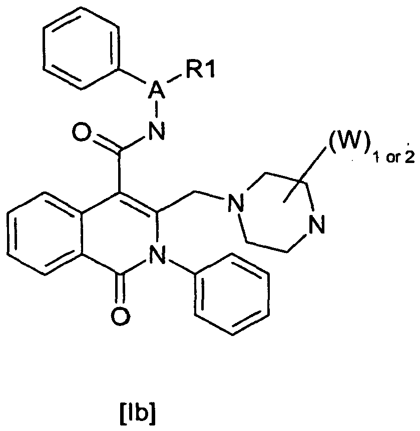 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A represent CH, and R¹ represents C₁₋₆alkyl, such as ethyl or cyclopropyl.

In one embodiment wherein the compounds of the present invention are defined by formula I_{b}, W represents a moiety of the formula -(CH₂)ₐ-Y-(CH₂)_{b}-Z; wherein a and b independently represent an integer selected from 0, 1, 2 and 3; wherein Y represents a bond, C(O), O, S, -O-C(O), -C(O)-O-, -NR²-, -NR²-C(O)-,-C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl. In a further embodiment, Y represents a bond, C(O) or S(O)₂; wherein a+b is 0, 1, 2, 3, or 4; and wherein Z represents mono-cyclic or fused bicyclic moiety comprising 5 to 12 carbon ring atoms and optionally one, two or three hetero atoms selected from amongst N, O, and S, and wherein said cyclic or bicyclic moiety is optionally substituted with one or more substituents selected from the group consisting of halogen, cyano, C₁₋₆alkyl, hydroxyl, and C₁₋₆alkoxy, phenyl, pyrazinyl, pyridyl, and halogen substituted phenyl. Particular mentioning is made of the embodiment, wherein Y represents a bond or C(O), and a+b is 0, 1, 2, or 3. In particular, Z comprises 5-9 ring atoms, such as e.g. 5, 6, or 9 ring atoms. Examples of Z with 5 ring atoms include pyrrolide and cyclopentyl; examples of Z with 6 ring atoms include phenyl, pyrimidinyl, morpholinyl and pyridyl; examples of Z with 9 ring atoms include furo [3.2-c]pyridyl. A particular example of Z is morpholinyl, e.g. attached to the rest of the W moiety via the nitrogen, and optionally substituted with one or more substituents selected from the group consisting of halogen, cyano, C₁₋₆ alkyl, hydroxyl, and C₁₋₆alkoxy, phenyl, pyrazinyl, pyridyl, and halogen substituted phenyl. Another particular example of Z is piperidyl, e.g. attached to the rest of the W moiety via the nitrogen, and optionally substituted with one or more substituents selected from the group consisting of halogen, cyano, C₁₋₆alkyl, hydroxyl, and C₁₋₆ alkoxy, phenyl, pyrazinyl, pyridyl, and halogen substituted phenyl. Another particular example of Z is pyridyl, e.g. attached to the rest of the W moiety via the nitrogen. Another particular example of Z is pyridyl, e.g. attached to the rest of the W moiety at the 2, 3 or 4 position, and optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl. Another particular example of Z is phenyl, optionally substituted with one or more substituents selected from the group consisting of halogen, cyano, C₁₋₆alkyl, hydroxyl, and C₁₋₆ alkoxy, phenyl, pyrazinyl, pyridyl, and halogen substituted phenyl. Another particular example of Z is pyrrolidinyl, optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl. Another particular example of Z is indolyl, e.g. attached to the rest of the W moiety at the 4, 5, 6, or 7 position, and optionally substituted with one or more substituents selected from the group consisting of halogen, cyano, C₁₋₆alkyl, hydroxyl, and C₁₋₆alkoxy, phenyl, pyrazinyl, pyridyl, and halogen substituted phenyl. Other particular examples ofZ include cyclopentyl, furopyridyl, tetrahydrofuranyl, benzo[1.4]oxazinyl, benzo[1.4]dioxinyl, benzo[1.2.5]thiadiazol, and quinazolinyl.

In one embodiment wherein the compounds of the present invention arc defined by formula I_{b}. Y represents a bond. C(O). -C(O)-O-, C(O)-NH-, -O-, or S(O)₂; a+b is 0.1, 2, 3 or 4; and wherein Z represents hydrogen, C₁₋₆alkyl, such as methyl, iso-propyl, iso-butyl, or tert.-butyl, NR²R³ or cyano.

In one embodiment wherein the compounds of the present invention arc defined by formula I_{b}, A represents CH: R¹ represents C₁₋₆alkyl, such as ethyl or cyclopropyl, W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆ alkoxy, -C(O)-C₁₋₆alkyl, -C(O)-O-C₁₋₆alkyl, -O-C(O)-C₁₋₆alkyl, -C(O)H, COOH.

In one embodiment the compounds of the present invention are defined by formula I_{c} 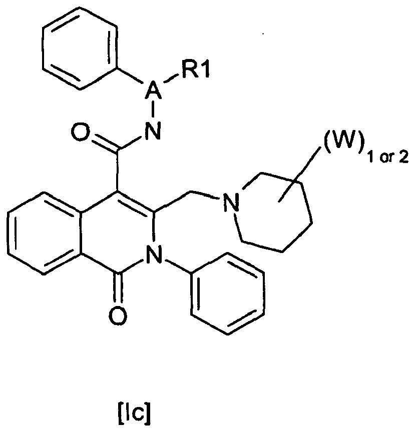 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A represent CH, and R¹ represents C₁₋₆alkyl, such as ethyl or cyclopropyl.

In one embodiment wherein the compounds of the present invention are defined by formula I_{c}, W independently represents hydrogen, hydroxyl, phenyl, phenyl substituted with C₁₋₆alkoxy, piperidyl, pyridyl, -O-(CH₂)_{c}-O-, wherein c is 2 or 3 (spiro), N(CH₃)₂, C₁₋₆alkyl, -(CH₂)ₐ-C(O)-O-(CH₂)_{b}-H, wherein a+b is 1, 2, or 3, or 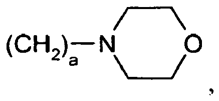 wherein a represents 1, 2, or 3.

In one embodiment, the compounds of the present invention are defined by formula I_{d} 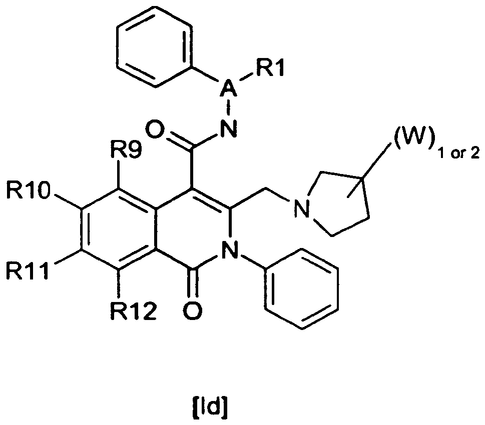 wherein A represents N, CH or CR¹:
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)₃-O-,
wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH;
wherein R² and R³ independently represent hydrogen, C₁₋₆alkyl, hydroxyC₁₋₆alkyl, haloC₁₋₆alkyl or phenyl; wherein R⁹-R¹² independently represent hydrogen or halogen; and pharmaceutically acceptable salts thereof. In particular, A is CH; R¹ represents C₁₋₆alkyl, such as ethyl or cyclopropyl; and each of R⁹-R¹² independently represent hydrogen or halogen.

In one embodiment, the compounds of the present invention are defined by formula Iₑ 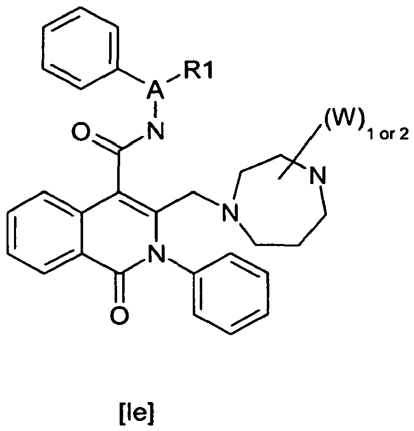 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-,
wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3;
wherein Y represents a bond, C(O), O, S, -O-C(O), -C(O)-O-, NR², -NR²-C(O)-, -C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A is CH, and R¹ represents C₁₋₆alkyl, such as ethyl or cyclopropyl. In particular, Z comprises 5-9 ring atoms, such as e.g. 5, 6, or 9 ring atoms. Examples of Z with 5 ring atoms include pyrrolide and cyclopentyl; examples of Z with 6 ring atoms include phenyl, pyrimidinyl, morpholinyl and pyridyl; examples of Z with 9 ring atoms include furo [3.2-c]pyridyl.

In one embodiment, the compounds of the present invention are defined by formula I_{f} 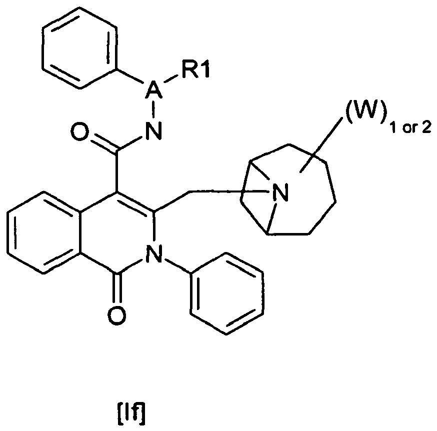 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A is CH, and R¹ represents C₁₋₆alkyl, such as ethyl or cyclopropyl. In particular, Z comprises 5-9 ring atoms, such as e.g. 5, 6, or 9 ring atoms. Examples of Z with 5 ring atoms include pyrrolide and cyclopentyl; examples of Z with 6 ring atoms include phenyl, pyrimidinyl, morpholinyl and pyridyl; examples of Z with 9 ring atoms include furo [3.2-c]pyridyl.

In one embodiment, the compounds of the present invention are defined by formula I_{g} 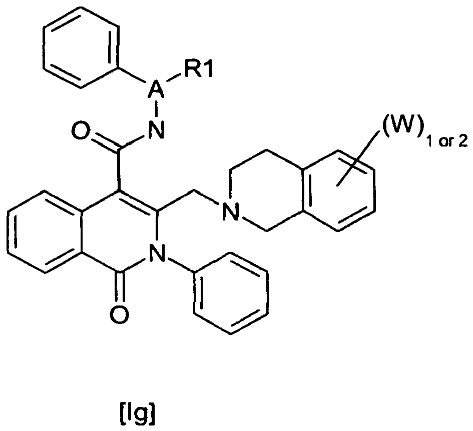 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -N(R²)-C(O)-R³, -C(O)H, COOH; or wherein W represents a moiety of the formula -(CH₂)ₐ-Y-(CH₂)_{b}-Z; wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A is CH, and R¹ represents C₁₋₆alkyl, such as ethyl or cyclopropyl. In particular, Z comprises 5-9 ring atoms, such as e.g. 5, 6, or 9 ring atoms. Examples of Z with 5 ring atoms include pyrrolide and cyclopentyl; examples of Z with 6 ring atoms include phenyl, pyrimidinyl, morpholinyl and pyridyl; examples of Z with 9 ring atoms include furo [3.2-c]pyridyl.

In one embodiment, the compounds of the present invention are defined by formula Iₕ 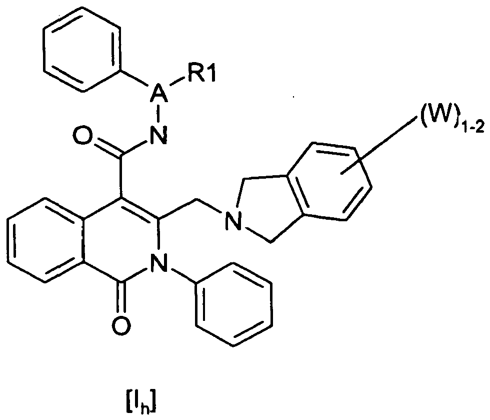 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three arc hetero atoms selected from amongst N, O, and S, and wherein said mono-cyclic or fused bi-cyclic moiety is optionally substituted with one or more substituents selected from the group consisting of halogen, cyano. C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A is CH, and R¹ represents C₁₋₆alkyl, such as ethyl or cyclopropyl. In particular, Z comprises 5-9 ring atoms, such as e.g. 5, 6, or 9 ring atoms. Examples of Z with 5 ring atoms include pyrrolide and cyclopentyl; examples of Z with 6 ring atoms include phenyl, pyrimidinyl, morpholinyl and pyridyl; examples of Z with 9 ring atoms include furo [3.2-c]pyridyl.

In one embodiment, the compounds of the present invention are defined by formula Iᵢ 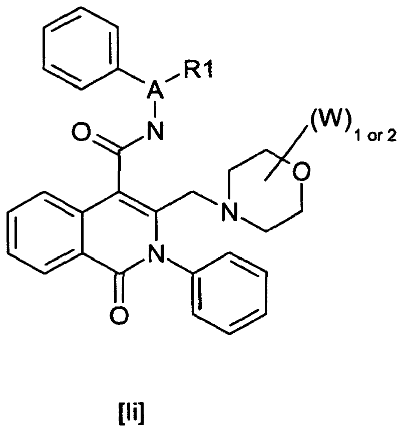 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein X represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A is CH, and R¹ represents C₁₋₆alkyl, such as ethyl or cyclopropyl. In particular, Z comprises 5-9 ring atoms, such as e.g. 5, 6, or 9 ring atoms. Examples of Z with 5 ring atoms include pyrrolide and cyclopentyl: examples of Z with 6 ring atoms include phenyl, pyrimidinyl, morpholinyl and pyridyl; examples of Z with 9 ring atoms include furo [3.2-c]pyridyl.

In one embodiment, the compounds of the present invention are defined by formula Ij 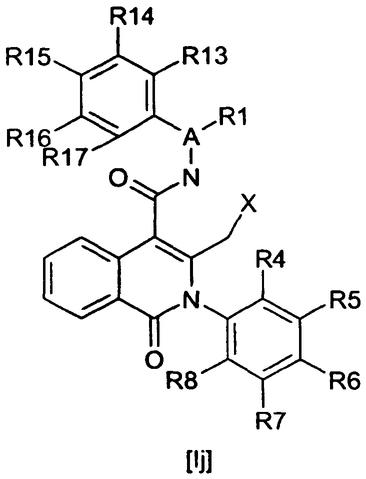 wherein A represents N, CH or CR¹:
wherein each R¹ independently represents hydrogen, C₁₋₆alkyl, -C(O)-C₁₋₆alkyl, -C(O)-O-C₁₋₆alkyl, or phenyl, wherein said phenyl or C₁₋₆alkyl is optionally substituted with one or more substituents selected from halogen, hydroxyl, haloC₁₋₆ alkyl, nitro, C₁₋₆alkoxy, and NR²R³:
wherein X represents hydrogen, C₁₋₄alkyl, cyano, -OR², -O-C(O)R², -OC(O)NR²R³, - C(O)-NR²R³, -N(R²)C(O)R³, -N(R²)-C(O)NR²R³ or NR²R³, or X represents a mono-cyclic, bicyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, phenyl, pyridyl, and halogen substituted phenyl;
wherein one of R⁴-R⁸ represents halogen and the other represent hydrogen; wherein one of R¹³-R¹⁷ represents halogen and the other represent hydrogen;
wherein R² and R³ independently represent hydrogen, C₁₋₆alkyl, hydroxyC₁₋₆alkyl, haloC₁₋₆alkyl or phenyl; and pharmaceutically acceptable salts thereof. In particular, A represents CH; R¹ represents C₁₋₆alkyl, such as ethyl or cyclopropyl; R¹⁴ represents halogen; and R⁵ or R⁸ represents halogen. Particular mentioning is made of the embodiment wherein X represents piperazinyl or 1-piperidyl substituted with one or two substituents W, wherein said W is a moiety of the formula -(CH₂)ₐ-Y-(CH₂)_{b}-Z, wherein a and b independently represent 0, 1, 2 or 3; Y represents a bond, O, -NR²-C(O)-; and wherein Z represents hydrogen, C₁₋₆alkyl, piperidyl, morpholinyl, pyridyl, phenyl, phenyl substituted with C₁₋₆alkoxy, or pyrrolidinyl.

In one embodiment, the compounds of the invention are defined by formula Iₖ 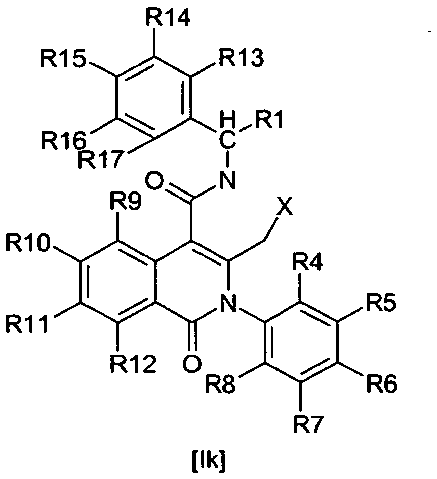 wherein R¹ represents hydrogen, C₁₋₆alkyl, C₂₋₆alkenyl or C₂₋₆alkynyl;
wherein X represents hydrogen, C₁₋₆alkyl, C₂₋₆alkenyl, C₂₋₆alkynyl or NR²R³, or X represents a mono-cyclic or bi-cyclic moiety having 5-9 ring atoms one of which is nitrogen, and wherein one, two or three additional ring atoms may be a hetero atom selected from N, O and S, and wherein said mono-cyclic or bi-cyclic moiety may optionally be substituted with one or more substituents W, wherein W is selected from hydrogen, halogen, hydroxy, (=O), C₁₋₆alkyl, C₂₋₆alkenyl, C₂₋₆alkynyl, C₁₋₆alkoxy, or wherein W represents a moiety of the formula -(CH₂)ₐ-Y-(CH₂)_{b}-Z;
wherein a and b independently represent an integer selected from 0, 1, 2 and 3; wherein Y represents a bond, and wherein Z represents a mono-cyclic moiety comprising 5 to 6 ring atoms of which optionally one, two or three are hetero atoms selected from amongst N, O, and S, and wherein said mono-cyclic moiety is optionally substituted with one or more substituents selected from the group consisting of halogen, C₁₋₆alkyl, hydroxyl, and C₁₋₆alkoxy;
wherein each of R⁴-R⁸ independently represents hydrogen or halogen;
wherein each of R⁹-R¹² independently represents hydrogen or halogen provided that at least one of R⁹-R¹² represents halogen;
wherein each of R¹³-R¹⁷ independently represent hydrogen or halogen; wherein R² and R³ independently represent hydrogen, C₁₋₆alkyl, C₂₋₆alkenyl, C₂₋₆ alkynyl; and pharmaceutically acceptable salts thereof. In particular, the compounds defined by formula Iₖ may be further defined by formula Iₖ' 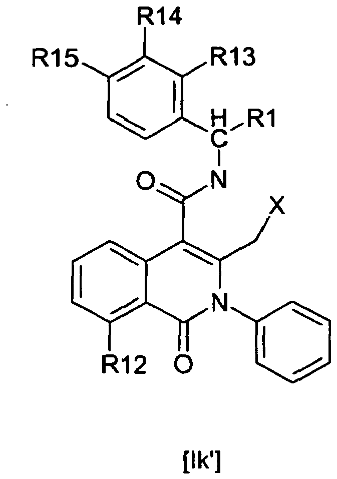 wherein R¹ is selected from C₁₋₆alkyl;
wherein X is selected from hydrogen, C₁₋₆alkyl or NR²R³, or X represents a mono-cyclic or bi-cyclic moiety having 5-9 ring atoms one of which is nitrogen, and wherein one additional ring atoms may be a hetero atom selected from N, and wherein said mono-cyclic or bi-cyclic moiety may optionally be substituted with one or more substituents W, wherein W is selected from hydrogen, hydroxy, (=O), C₁₋₆alkyl or wherein W represents a moiety of the formula -(CH₂)ₐ-Y-(CH₂)_{b}-Z;
wherein a and b independently represent an integer selected from 0, 1, 2 and 3; wherein Y represents a bond, and wherein Z represents a mono-cyclic moiety comprising 5 to 6 ring atoms of which optionally one is a hetero atom selected from amongst N, O, and S, and wherein said mono-cyclic moiety is optionally substituted with one or more substituents selected from the group consisting of halogen, C₁₋₆ alkyl, hydroxyl, and C₁₋₆alkoxy;
wherein R¹² represents halogen;
R¹³-R¹⁵ each independently represents hydrogen or halogen;
and pharmaceutically acceptable salts thereof. In particular, R¹ represents methyl, ethyl, cyclopropyl, cyclobutyl, cyclopentyl or cyclohexyl, and X represents

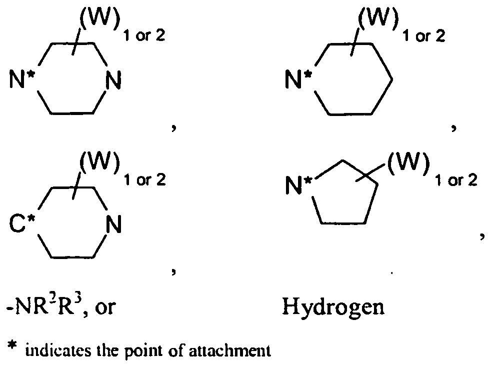

In one embodiment, wherein the compounds of the invention are defined by formula Iₖ' and R¹ represents methyl, ethyl, cyclopropyl, cyclobutyl, cyclopentyl or cyclohexyl, X represents 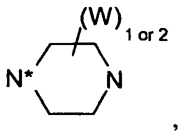 and wherein W represents hydrogen, (=O), C₁₋₆-alkyl, or -(CH₂)ₐ-Y-(CH₂)_{b}-Z. In particular, W represents hydrogen, methyl, cyclopropylmethyl, isopropyl, isobutyl, tert-butyl, (=O), or or -(CH₂)ₐ-Y-(CH₂)ₐ-Z, wherein a+b is 2 and Z is selected from 4-morpholinyl, phenyl, 1-piperidine or 1-pyrrolidinyl.

In one embodiment, wherein the compounds of the invention are defined by formula Iₖ' and R¹ represents methyl, ethyl, cyclopropyl, cyclobutyl, cyclopentyl or cyclohexyl, X represents 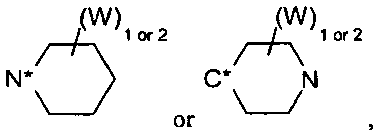 wherein W represents hydrogen, hydroxyl, C₁₋₆-alkyl or -(CH₂)ₐ-Y-(CH₂)ₐ-Z. In particular, W represents hydrogen, hydroxyl, tert-butyl, or a+b is 0 or 2 and Z represents 4-morpholinyl, phenyl, optionally substituted with C₁₋₆alkoxy, or 4-piperidyl.

In one embodiment, wherein the compounds of the invention are defined by formula Iₖ' and R¹ represents methyl, ethyl, cyclopropyl, cyclobutyl, cyclopentyl or cyclohexyl, X represent 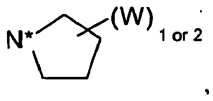 and W represents (=O), and in particular, X represents

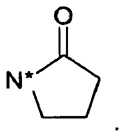

In one embodiment, wherein the compounds of the invention are defined by formula Iₖ' and R¹ represents methyl, ethyl, cyclopropyl, cyclobutyl, cyclopentyl or cyclohexyl, X represents -NR²R³. In particular, R² and R³ independently represents hydrogen or C₁₋₆-alkyl, and special mention is made of the compounds wherein R² represents hydrogen, and R³ represents cyclopropyl, isobutyl or tert-butyl.

In one embodiment, wherein the compounds of the invention are defined by formula Iₖ' and R¹ represents methyl, ethyl, cyclopropyl, cyclobutyl, cyclopentyl or cyclohexyl, X is hydrogen.

In one embodiment, the compounds of the invention are defined by formula Iₖ" 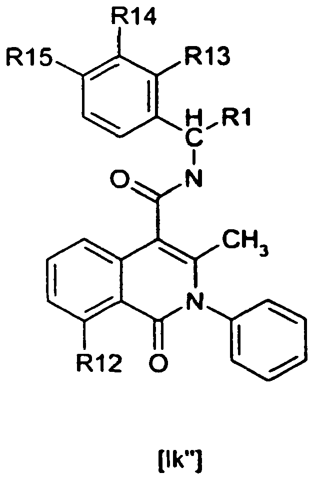 wherein R¹ represents ethyl, cylopropyl or cyclobutyl:
wherein R¹² represents chloro; and
R¹³, R¹⁴ and R¹⁵ each individually represent hydrogen, fluoro or chloro, wherein two of R¹³, R¹⁴ and R¹⁵ represent hydrogen, and pharmaceutically acceptable salts thereof.

In particular, said compound is essentially the S enantiomers as depicted in formula Iₖ"' 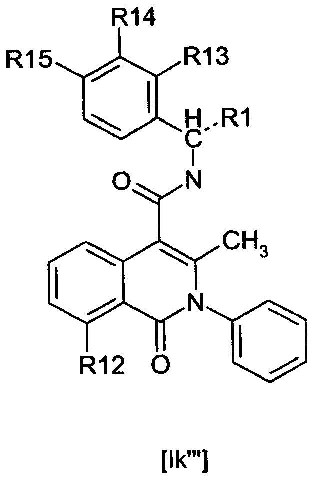 wherein R¹ represents ethyl or cylopropyl;
wherein R¹² represents chloro; and
R¹³, R¹⁴ and R¹⁵ each individually represent hydrogen, fluoro or chloro, wherein two of R¹³, R¹⁴ and R¹⁵ represent hydrogen.

In one embodiment, the compounds of the inventions are defined by formula I_{d}' 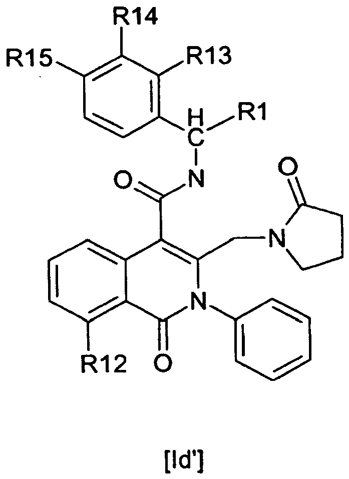 wherein R¹ represents ethyl or cyclopropyl;
R¹² represent halogen;
R¹³, R¹⁴ and R¹⁵ each independently represents hydrogen, or halogen;
and pharmaceutically acceptable salts thereof.

In one embodiment, the compounds of the invention are defined by formula I_{d}" 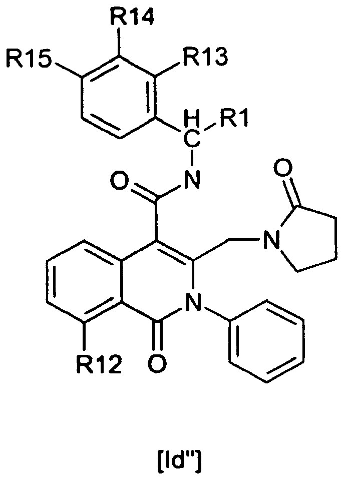 wherein R¹ represents ethyl or cyclopropyl:
R¹² represent chloro or fluoro:
R¹², R¹⁴ and R¹⁵ each independently represent hydrogen, chloro or fluoro, wherein two of R¹², R¹⁴ and R¹⁵ represent hydrogen;
and pharmaceutically acceptable salts thereof. In particular, said compound is essentially the S enantiomer as depicted in formula Id"' 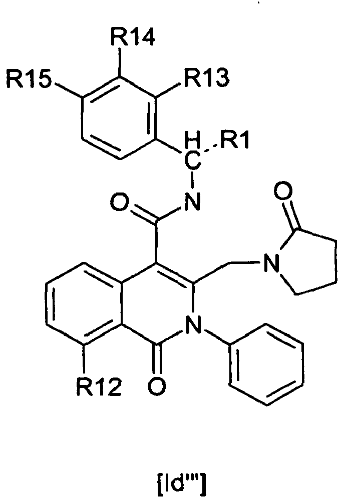 wherein R¹ represents ethyl or cyclopropyl;
R¹² represent chloro or fluoro;
R¹², R¹⁴ and R¹⁵ each independently represent hydrogen, chloro or fluoro, wherein two of R¹², R¹⁴ and R¹⁵ represent hydrogen;
and pharmaceutically acceptable salts thereof.

In one embodiment the compounds of the present invention are selected from the below list. For convenience, the number indicated in bold in front of the compound name refers to the corresponding example number.
**1a** 3-Methyl-1-oxo-2-phenyl-1,2-dihydro-isoquinoline-4-carboxylic acid ((S)-1-phenyl-propyl)-amide
**1b** 3,6-Dimethyl-1-oxo-2-phenyl-1,2-dihydro-isoquinoline-4-carboxylic acid ((S)-1-phenyl-propyl)-amide
**1c** 7-Chloro-3-methyl-1-oxo-2-phenyl-1,2-dihydro-isoquinoline-4-carboxylic acid ((S)-1-phenyl-propyl)-amide
**1d** 7-Bromo-3-methyl-1-oxo-2-phenyl-1,2-dihydro-isoquinoline-4-carboxylic acid ((S)-1-phenyl-propyl)-amide
**1e** 6-Fluoro-3-methyl-1-oxo-2-phenyl-1,2-dihydro-isoquinoline-4-carboxylic acid ((S)-1-phenyl-propyl)-amide
**1f** 3,5-Dimethyl-1-oxo-2-phenyl-1,2-dihydro-isoquinoline-4-carboxylic acid ((S)-1-phenyl-propyl)-amide
**1g** 7-Fluoro-3-methyl-1-oxo-2-phenyl-1,2-dihydro-isoquinoline-4-carboxylic acid ((S)-1-phenyl-propyl)-amide
**1h** 3,7-Dimethyl-1-oxo-2-phenyl-1,2-dihydro-isoquinoline-4-carboxylic acid ((S)-1-phenyl-propyl)-amide
1i 8-Chloro-3-methyl-1-oxo-2-phenyl-1,2-dihydro-isoquinoline-4-carboxylic acid ((S)-1-phenyl-propyl)-amide
**1r** 3-Methyl-l-oxo-2-phenyl-1,2-dihydro-isoquinoline-4-carboxylic acid ((S)-cyclopropyl-phenyl-methyl)-amide
**1s** 3-Methyl-1-oxo-2-phenyl-1,2-dihydro-isoquinoline-4-carboxylic acid [(S)-cyclopropyl-(3-fluoro-phenyl)-methyl]-amide
**1j** 3,8-Dimethyl-1-oxo-2-phenyl-1,2-dihydro-isoquinoline-4-carboxylic acid ((S)-1-phenyl-propyl)-amide
**1k** 2-(2-Fluoro-phenyl)-3-methyl-1-oxo-1,2-dihydro-isoquinoline-4-carboxylic acid ((S)-1-phenyl-propyl)-amide
**1l** 3-Methyl-1-oxo-2-o-tolyl-1,2-dihydro-isoquinoline-4-carboxylic acid ((S)-1-phenyl-propyl)-amide
**1m** 2-(2-Chloro-phenyl)-3-methyl-l-oxo-1,2-dihydro-isoquinoline-4-carboxylic acid ((S)-1-phenyl-propyl)-amide
**1n** 2-(2,6-Difluoro-phenyl)-3-methyl-1-oxo-1,2-dihydro-isoquinoline-4-carboxylic acid ((S)-1-phenyl-propyl)-amide
**1o** 3-(3-Fluoro-phenyl)-3-methyl-1-oxo-1,2-dihydro-isoquinoline-4-carboxylic acid ((S)-1-phenyl-propyl)-amide
**1p** 3-Methyl-1-oxo-2-m-tolyl-1,2-dihydro-isoquinoline-4-carboxylic acid ((S)-1-phenyl-propyl)-amide
**1q** 2-(3-Chloro-phenyl)-3-methyl-1-oxo-1,2-dihydro-isoquinoline-4-carboxylic acid ((S)-1-phenyl-propyl)-amide
**1t** 8-Chloro-3-methyl-1-oxo-2-phenyl-1,2-dihydro-isoquinoline-4-carboxylic acid [(S)-cyclopropyl-(3-fluoro-phenyl)-methyl]-amide
**2a** 3-Dimethylaminomethyl-1-oxo-2-phenyl-1,2-dihydro-isoquinoline-4-carboxylic acid ((S)-1-phenyl-propyl)-amide
**2b** 3-Methylaminomethyl-1-oxo-2-phenyl-1,2-dihydro-isoquinoline-4-carboxylic acid ((S)-1-phenyl-propyl)-amide
**2c** 3-Ethylaminomethyl-1-oxo-2-phenyl-1,2-dihydro-isoquinoline-4-carboxylic acid ((S)-1-phenyl-propyl)-amide
**2d** 3-Cyclopropylaminomethyl-1-oxo-2-phenyl-1,2-dihydro-isoquinoline-4-carboxylic acid ((S)-1-phenyl-propyl)-amide
**2e** 3-[(Cyclopropylmethyl-amino)-methyl]-1-oxo-2-phenyl-1,2-dihydro-isoquinoline-4-carboxylic acid ((S)-1-phenyl-propyl)-amide
**2f** 3-(3,6-Dihydro-2H-pyridin-1-ylmethyl)-1-oxo-2-phenyl-1,2-dihydro-isoquinoline-4-carboxylic acid ((S)-1-phenyl-propyl)-amide
**2g** 3-[4-(2-Methoxy-phenyl)-piperazin-1-ylmethyl]-1-oxo-2-phenyl-1,2-dihydro-isoquinoline-4-carboxylic acid ((S)-1-phenyl-propyl)-amide
**2h** 3-[4-(4-Fluoro-phenyl)-piperazin-1-ylmethyl]-1-oxo-2-phenyl-1,2-dihydro-isoquinolinc-4-carboxylic acid ((S)-1-phenyl-propyl)-amide
**2i** 3-(4-Formyl-piperazin-1-ylmethyl)-1-oxo-2-phenyl-1,2-dihydro-isoquinoline-4-carboxylic acid ((S)-1-phenyl-propyl)-amide
**2j** 4-[1-Oxo-2-phenyl-4-(1-phenyl-propylcarbamoyl)-1,2-dihydro-isoquinolin-3-ylmethyl]-piperazine-1-carboxylic acid ethyl ester
**2k** 3-(4-Methyl-piperazin-1-ylmethyl)-1-oxo-2-phenyl-1,2-dihydro-isoquinoline-4-carboxylic acid ((S)-1-phenyl-propyl)-amide
**2l** 1-Oxo-2-phenyl-3-(1,3,4,9-tetrahydro-beta-carbolin-2-ylmethyl)-1,2-dihydro-isoquinolinc-4-carboxylic acid ((S)-1-phenyl-propyl)-amide
**2m** 1-Oxo-2-phenyl-3-piperazin-1-ylmethyl-1,2-dihydro-isoquinoline-4-carboxylic acid ((S)-1-phenyl-propyl)-amide
**2n** 3-(3-Methyl-piperazin-1-ylmethyl)-1-oxo-2-phenyl-1,2-dihydro-isoquinoline-4-carboxylic acid ((S)-1-phenyl-propyl)-amide
**2o** 3-(3,5-Dimethyl-piperazin-1-ylmethyl)-1-oxo-2-phenyl-1,2-dihydro-isoquinoline-4-carboxylic acid ((S)-1-phenyl-propyl)-amide
**2p** 3-(4-Benzyl-piperazin-1-ylmethyl)-1-oxo-2-phenyl-1,2-dihydro-isoquinoline-4-carboxylic acid ((S)-1-phenyl-propyl)-amide
**2q** 1-Oxo-3-[4-(2-oxo-2-pyrrolidin-1-yl-ethyl)-piperazin-1-ylmethyl]-2-phenyl-1,2-dihydro-isoquinoline-4-carboxylic acid ((S)-1-phenyl-propyl)-amide
**2r** 3-Morpholin-4-ylmethyl-1-oxo-2-phenyl-1,2-dihydro-isoquinoline-4-carboxylic acid ((S)-1-phenyl-propyl)-amide
**2s** 3-(2,6-Dimethyl-morpholin-4-ylmethyl)-1-oxo-2-phenyl-1,2-dihydro-isoquinoline-4-carboxylic acid ((S)-1-phenyl-propyl)-amide
**2t** 1-Oxo-2-phenyl-3-thiomorpholin-4-ylmethyl-1,2-dihydro-isoquinoline-4-carboxylic acid ((S)-1-phenyl-propyl)-amide
**2u** 3-(1,4-Dioxa-8-aza-spiro[4.5]dec-8-ylmethyl)-1-oxo-2-phenyl-1,2-dihydro-isoquinoline-4-carboxylic acid ((S)-1-phenyl-propyl)-amide
**2v** 1-Oxo-2-phenyl-3-piperidin-1-ylmethyl-1,2-dihydro-isoquinoline-4-carboxylic acid ((S)-1-phenyl-propyl)-amide
**2w** 3-(2-Methyl-piperidin-1-ylmethyl)-1-oxo-2-phenyl-1,2-dihydro-isoquinoline-4-carboxylic acid ((S)-1-phenyl-propyl)-amide
**2x** 3-(2,6-Dimethyl-piperidin-1-ymethyl)-1-oxo-2-phenyl-1,2-dihydro-isoquinoline-4-carboxylic acid ((S)-1-phenyl-propyl)-amide
**2y** 3-(2-Hydroxymethyl-piperidin-1-ylmethyl)-1-oxo-2-phenyl-1,2-dihydro-isoquinoline-4-carboxylic acid ((S)-1-phenyl-propyl)-amide
**2z** 1-[1-Oxo-2-phenyl-4-(1-phenyl-propylcarbamoyl)-1,2-dihydro-isoquinolin-3-ylmethyl]-piperidine-3-carboxylic acid ethyl ester
**2aa** 3-(3-Methyl-piperidin-1-ylmethyl)-1-oxo-2-phenyl-1,2-dihydro-isoquinoline-4-carboxylic acid ((S)-1-phenyl-propyl)-amide
**2ab** 3-(4-Hydroxy-piperidin-1-ylmethyl)-1-oxo-2-phenyl-1,2-dihydro-isoquinoline-4-carboxylic acid ((S)-1-phenyl-propyl)-amide
**2ac** 1-Oxo-2-phenyl-3-(4-phenyl-piperidin-1-ylmethyl)-1,2-dihydro-isoquinoline-4-carboxylic acid ((S)-1-phenyl-propyl)-amide
**2ad** 1-[1-Oxo-2-phenyl-4-(1-phenyl-propylcarbamoyl)-1,2-dihydro-isoquinolin-3-ylmethyl]-piperidinc-4-carboxylic acid ethyl ester
**2ae** 3-(4-Methyl-piperidin-1-ylmethyl)-1-oxo-2-phenyl-1,2-dihydro-isoquinoline-4-carboxylic acid ((S)-1-phenyl-propyl)-amide
**2af** 1-Oxo-2-phenyl-3-(4-pyridin-2-yl-piperazin-1-ylmethyl)-1,2-dihydro-isoquinoline-4-carboxylic acid ((S)-1-phenyl-propyl)-amide
**2ag** 3-(Octahydro-quinolin-1-ylmethyl)-1-oxo-2-phenyl-1,2-dihydro-isoquinoline-4-carboxylic acid ((S)-1-phenyl-propyl)-amide
**2ah** 3-Azepan-1-ylmethyl-1-oxo-2-phenyl-1,2-dihydro-isoquinoline-4-carboxylic acid ((S)-1-phenyl-propyl)-amide
**2ai** 3-(3-Hydroxy-piperidin-1-ylmethyl)-1-oxo-2-phenyl-1,2-dihydro-isoquinoline-4-carboxylic acid ((S)-1-phenyl-propyl)-amide
**2aj** 3-[4-(2,4-Dimethyl-phenyl)-piperazin-1-ylmethyl]-1-oxo-2-phenyl-1,2-dihydro-isoquinoline-4-carboxylic acid ((S)-1-phenyl-propyl)-amide
**2ak** 3-[4-(3,4-Dimethyl-phenyl)-piperazin-1-ylmethyl]-1-oxo-2-phenyl-1,2-dihydro-isoquinoline-4-carboxylic acid ((S)-1-phenyl-propyl)-amide
**2al** 3-(4-Dimethylamino-piperidin-1-ylmethyl)-1-oxo-2-phenyl-1,2-dihydro-isoquinoline-4-carboxylic acid ((S)-1-phenyl-propyl)-amide
**2am** 3-[4-(2,5-Dimethyl-phenyl)-piperazin-1-ylmethyl]-1-oxo-2-phenyl-1,2-dihydro-isoquinoline-4-carboxylic acid ((S)-1-phenyl-propyl)-amide
**2an** 3-[4-(2-Fluoro-phenyl)-piperazin-1-ylmethyl]-1-oxo-2-phenyl-1,2-dihydro-isoquinolinc-4-carboxylic acid ((S)-1-phenyl-propyl)-amide
**2ao** 3-[4-(3-Methoxy-phenyl)-piperazin-1-ylmehyl]-1-oxo-2-phenyl-1,2-dihydro-isoquinoline-4-carboxylic acid ((S)-1-phenyl-propyl)-amide
**2ap** 1-Oxo-2-phenyl-3-(4-m-tolyl-piperazin-1-ylmethyl)-1,2-dihydro-isoquinoline-4-carboxylic acid ((S)-1-phenyl-propyl)-amide
**2aq** 3-[4-(4-Methoxy-phenyl)-pipcrazin-1-ylmethyl]-1-oxo-2-phenyl-1,2-dihydro-isoquinolinc-4-carboxylic acid ((S)-1-phenyl-propyl)-amide
**2ar** 1-Oxo-3-(4-phenethyl-piperazin-1-ylmethyl)-2-phenyl-1,2-dihydro-isoquinoline-4-carboxylic acid ((S)-1-phenyl-propyl)-amide
**2as** 1-Oxo-3-phenyl-3-(4-pyrimidin-3-yl-piperazin-1-ylmethyl)-1,2-dihydro-isoquinoline-4-carboxylic acid ((S)-1-phenyl-propyl)-amide
**2at** 3-[4-(2-Cyano-phenyl)-piperazin-1-ylmethyl]-1-oxo-2-phenyl-1,2-dihydro-isoquinolinc-4-carboxylic acid ((S)-1-phenyl-propyl)-amide
**2au** 3-[4-(4-Chloro-phenyl)-piperazin-1-ylmethyl]-1-oxo-2-phenyl-1,2-dihydro-isoquinolinc-4-carboxylic acid ((S)-1-phenyl-propyl)-amide
**2av** 3-((1S,3R,5R)-3-Hydroxy-8-aza-bicyclo[3.2.1]oct-8-ylmethyl)-1-oxo-2-phenyl-1,2-dihydro-isoquinoline-4-carboxylic acid ((S)-1-phenyl-propyl)-amide
**2aw** 3-(4-Acetyl-piperazin-1-ylmethyl)-1-oxo-2-phenyl-1,2-dihydro-isoquinoline-4-carboxylic acid ((S)-1-phenyl-propyl)-amide
**2ax** 3-(4-Methyl-[1,4]diazepan-1-ylmethyl)-1-oxo-2-phenyl-1,2-dihydro-isoquinoline-4-carboxylic acid ((S)-1-phenyl-propyl)-amide
**2ay** 3-(4-Ethyl-piperazin-1-ylmethyl)-1-oxo-2-phenyl-1,2-dihydro-isoquinoline-4-carboxylic acid ((S)-1-phenyl-propyl)-amide
**2az** 3-((2S,6R)-2,6-Dimethyl-morpholin-4-ylmethyl)-1-oxo-2-phenyl-1,2-dihydro-isoquinoline-4-carboxylic acid ((S)-1-phenyl-propyl)-amide
**2ba** 3-[4-(2,4-Difluoro-phenyl)-piperazin-1-ylmethyl]-1-oxo-2-phenyl-1,2-dihydro-isoquinoline-4-carboxylic acid ((S)-1-phenyl-propyl)-amide
**2bb** 3-[4-(3-Dimethylamino-propyl)-piperazin-1-ylmethyl]-1-oxo-2-phenyl-1,2-dihydro-isoquinoline-4-carboxylic acid ((S)-1-phenyl-propyl)-amide
**2bc** 3-(4-Isopropyl-piperazin-1-ylmethyl)-1-oxo-2-phenyl-1,2-dihydro-isoquinoline-4-carboxylic acid ((S)-1-phenyl-propyl)-amide
**2bd** 3-(3-Aza-bicyclo[3.2.2]non-3-ylmethyl)-1-oxo-2-phenyl-1,2-dihydro-isoquinoline-4-carboxylic acid ((S)-1-phenyl-propyl)-amide
**2be** 3-(4-Cyclopentyl-piperazin-1-ylmethyl)-1-oxo-2-phenyl-1,2-dihydro-isoquinoline-4-carboxylic acid ((S)-1-phenyl-propyl)-amide
**2bf** 3-[1,4']Bipiperidinyl-1'-ylmethyl-1-oxo-2-phenyl-1,2-dihydro-isoquinoline-4-carboxylic acid ((S)-1-phenyl-propyl)-amide
**2bg** 3-(3,4-Dihydro-1H-isoquinolin-2-ylmethyl)-1-oxo-2-phenyl-1,2-dihydro-isoquinoline-4-carboxylic acid ((S)-1-phenyl-propyl)-amide
**2bh** 3-[4-(2-Dimethylamino-ethyl)-piperazin-1-ylmethyl]-1-oxo-2-phenyl-1,2-dihydro-isoquinoline-4-carboxylic acid ((S)-1-phenyl-propyl)-amide **2bi** 3-(4-Hydroxymethyl-piperidin-1-ylmethyl)-1-oxo-2-phenyl-1,2-dihydro-isoquinoline-4-carboxylic acid ((S)-1-phenyl-propyl)-amide
**2bj** 1-Oxo-2-phenyl-3-[4-(tetrahydro-furan-2-carbonyl)-piperazin-1-ylmethyl]-1,2-dihydro-isoquinoline-4-carboxylic acid ((S)-1-phenyl-propyl)-amide
**2bk** 3-(4-Isobutyl-piperazin-1-ylmethyl)-1-oxo-2-phenyl-1 1,2-dihydro-isoquinoline-4-carboxylic acid ((S)-1-phenyl-propyl)-amide
**2bl** 3-[4-(2-Methoxy-ethyl)-piperazin-1-ylmethyl]-1-oxo-2-phenyl-1,2-dihydro-isoquinoline-4-carboxylic acid ((S)-1-phenyl-propyl)-amide
**2bm** 3-[4-(2-Morpholin-4-yl-ethyl)-piperazin-1-ylmethyl]-1-oxo-2-phenyl-1,2-dihydro-isoquinoline-4-carboxylic acid ((S)-1-phenyl-propyl)-amide
**2bn** 3-(1,3-Dihydro-isoindol-2-ylmethyl)-1-oxo-2-phenyl-1,2-dihydro-isoquinoline-4-carboxylic acid ((S)-1-phenyl-propyl)-amide
**2bo** 3-[4-(1-Methyl-piperidin-4-yl)-piperazin-1-ylmethyl]-1-oxo-2-phenyl-1,2-dihydro-isoquinoline-4-carboxylic acid ((S)-1-phenyl-propyl)-amide
**2bp** 1-Oxo-2-phenyl-3-[4-(2-piperidin-1-yl-ethyl)-piperazin-1-ylmethyl]-1,2-dihydro-isoquinoline-4-carboxylic acid ((S)-1-phenyl-propyl)-amide
**2bq** 1-Oxo-2-phenyl-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2br** 3-(4-Dimethylcarbamoylmethyl-piperazin-1-ylmethyl)-1-oxo-2-phenyl-1,2-dihydro-isoquinoline-4-carboxylic acid ((S)-1-phenyl-propyl)-amide
**2bs** 3-(Octahydro-pyrido[1,2-a]pyrazin-2-ylmethyl)-1-oxo-2-phenyl-1,2-dihydro-isoquinoline-4-carboxylic acid ((S)-1-phenyl-propyl)-amide
**2bt** 3-(4-Formyl-[1,4]diazepan-1-ylmethyl-1-oxo-2-phenyl-1,2-dihydro-isoquinoline-4-carboxylic acid ((S)-1-phenyl-propyl)-amide
**2bu** 3-[4-(4-Cyano-phenyl)-pipcrazin-1-ylmethyl]-1-oxo-2-phenyl-1,2-dihydro-isoquinoline-4-carboxylic acid ((S)-1-phenyl-propyl)-amide
**2bv** 1-Oxo-2-phenyl-3-(4-pyridin-4-ylmethyl-piperazin-1-ylmethyl)-1,2-dihydro-isoquinoline-4-carboxylic acid ((S)-1-phenyl-propyl)-amide
**2bw** 1-Oxo-2-phenyl-3-[4-(3-pyrrolidin-1-yl-propyl)-piperazin-1-ylmethyl]-1,2-dihydro-isoquinoline-4-carboxylic acid ((S)-1-phenyl-propyl)-amide
**2bx** 1-Oxo-2-phenyl-3-(4-pyridin-2-ylmethyl-piperazin-1-ylmethyl)-1,2-dihydro-isoquinoline-4-carboxylic acid ((S)-1-phenyl-propyl)-amide
**2by** 3-(4-Ethanesulfonyl-piperacin-1-ylmethyl)-1-oxo-2-phenyl-1,2-dihydro-isoquinoline-4-carboxylic acid ((S)-1-phenyl-propyl)-amide
**2bz** 3-(4-sec-Butyl-piperazin-1-ylmethyl)-1-oxo-2-phenyl-1,2-dihydro-isoquinoline-4-carboxylic acid ((S)-1-phenyl-propyl)-amide
**2ca** 3-[4-(1-Ethyl-propyl)-piperazin-1-ylmethyl]-1-oxo-2-phenyl-1,2-dihydro-isoquinoline-4-carboxylic acid ((S)-1-phenyl-propyl)-amide
**2cb** 3-[4-(2-Cyano-ethyl)-piperazin-1-ylmethyl]-1-oxo-2-phenyl-1,2-dihydro-isoquinoline-4-carboxylic acid ((S)-1-phenyl-propyl)-amide
**2cc** 3-(4-Methanesulfonyl-piperazin-1-ylmethyl)-1-oxo-2-phenyl-1,2-dihydro-isoquinoline-4-carboxylic acid ((S)-1-phenyl-propyl)-amide
**2cd** {1-[1-Oxo-2-phenyl-4-(1-phenyl-propylcarbamoyl)-1,2-dihydro-isoquinolin-3-ylmethyl]-piperidin-4-yl}-acetic acid ethyl ester
**2ce** 3-[4-(3-Fluoro-phenyl)-piperazin-1-ylmethyl]-1-oxo-2-phenyl-1,2-dihydro-isoquinoline-4-carboxylic acid ((S)-1-phenyl-propyl)-amide
**2cf** 1-Oxo-2-phenyl-3-((S)-3-phenyl-piperidin-1-ylmethyl)-1,2-dihydro-isoquinoline-4-carboxylic acid ((S)-1-phenyl-propyl)-amide
**2cg** 1-Oxo-2-phenyl-3-[4-(pyrrolidine-1-carbonyl)-piperazin-1-ylmethyl]-1,2-dihydro-isoquinoline-4-carboxylic acid ((S)-1-phenyl-propyl)-amide
**2ch** 3-[4-(Morpholine-4-carbonyl)-piperazin-1-ylmethyl]-1-oxo-2-phenyl-1,2-dihydro-isoquinoline-4-carboxylic acid ((S)-1-phenyl-propyl)-amide
**2ci** 3-(4-Methoxy-piperidin-1-ylmethyl)-1-oxo-2-phenyl-1,2-dihydro-isoquinoline-4-carboxylic acid ((S)-1-phenyl-propyl)-amide
**2cj** 3-(4'-Hydroxy-3',4',5',6'-tetrahydro-2'H-[3,4']bipyridinyl-1'-ylmethyl)-1-oxo-2-phenyl-1,2-dihydro-isoquinoline-4-carboxylic acid ((S)-1-phenyl-propyl)-amide
**2ck** 3-(4-Hydroxy-3,4,5,6-tetrahydro-2H-[4,4']bipyridinyl-l-ylmethyl)-1-oxo-2-phenyl-1,2-dihydro-isoquinoline-4-carboxylic acid ((S)-1-phenyl-propyl)-amide
**2cl** 3-(3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide **2cm** 3-(4-Hydroxy-4-methyl-piperidin-1-ylmethyl)-1-oxo-2-phenyl-1,2-dihydro-isoquinoline-4-carboxylic acid ((S)-1-phenyl-propyl)-amide
**2cn** 3-(hexahydro-spiro[benzo[1,3]dioxole-2,4'-piperidin]-1'-ylmethyl)-1-oxo-2-phenyl-1,2-dihydro-isoquinoline-4-carboxyline acid ((S)-1-phenyl-propyl)-amide
**2co** 1-Oxo-2-phenyl-3-(3,4,5,6-tetrahydro-2H-[4,4']bipyridinyl-1-ylmethyl)-1,2-dihydro-isoquinoline-4-carboxylic acid ((S)-1-phenyl-propyl)-amide
**2cp** 3-[4-(2-Dimethylamino-ethyl)-piperidin-1-ylmethyl]-1-oxo-2-phenyl-1,2-dihydro-isoquinoline-4-carboxylic acid ((S)-1-phenyl-propyl)-amide
**2cq** 3-(4-Dimethylsulfamoyl-piperazin-1-ylmethyl)-1-oxo-2-phenyl-1,2-dihydro-isoquinoline-4-carboxylic acid ((S)-1-phenyl-propyl)-amide
**2cr** 3-(1,1-Dioxo-thiomorpholin-4-ylmethyl)--oxo-2-phenyl-1,2-dihydro-isoquinoline-4-carboxylic acid ((S)-1-phenyl-propyl)-amide
**2cs** 1-Oxo-2-phenyl-3-(2-pyridin-2-ylmethyl-piperidin-1-ylmethyl)-1,2-dihydro-isoquinoline-4-carboxylic acid ((S)-1-phenyl-propyl)-amide
**2ct** 3-(2-Morpholin-4-ylmethyl-piperidin-1-ylmethyl)-1-oxo-2-phenyl-1,2-dihydro-isoquinoline-4-carboxylic acid ((S)-1-phenyl-propyl)-amide
**2cu** 3-(4-Furo[3,2-c]pyridin-4-yl-piperazin-1-ylmethyl)-1-oxo-2-phenyl-1,2-dihydro-isoquinoline-4-carboxylic acid ((S)-1-phenyl-propyl)-amide
**2cv** 3-(4-Cyclopropylmethyl-piperazin-1-ylmethyl)-1-oxo-2-phenyl-1,2-dihydro-isoquinoline-4-carboxylic acid ((S)-1-phenyl-propyl)-amide
**2cw** 3-[4-(2-Morpholin-4-yl-ethyl)-piperidin-1-ylmethyl]-1-oxo-2-phenyl-1,2-dihydro-isoquinoline-4-carboxylic acid ((S)-1-phenyl-propyl)-amide
**2cx** 1-Oxo-2-phenyl-3-(4-pyrimidin-2-yl-[1,4]diazepan-1-ylmethyl)-1,2-dihydro-isoquino line-4-carboxylic acid ((S)-1-phenyl-propyl)-amide
**2cy** 3-(4-Methyl-6,7-dihydro-4H-thieno[3,2-c]pyridin-5-ylmethyl)-1-oxo-2-phenyl-1,2-dihydro-isoquinoline-4-carboxylic acid ((S)-1-phenyl-propyl)-amide
**2cz** 3-[1,4]Diazepan-1-ylmethyl-1-oxo-2-phenyl-1,2-dihydro-isoquinoline-4-carboxylic acid ((S)-1-phenyl-propyl)-amide
**2da** 3-((2S,5R)-2,5-Dimethyl-piperazin-1-ylmethyl)-1-oxo-2-phenyl-1,2-dihydro-isoquinoline-4-carboxylic acid ((S)-1-phenyl-propyl)-amide
**2db** 3-((S)-3-Methyl-piperazin-1-ylmethyl)-1-oxo-2-phenyl-1,2-dihydro-isoquinoline-4-carboxylic acid ((S)-1-phenyl-propyl)-amide
**2dc** 3-((R)-3-Methyl-piperazin-1-ylmethyl)-1-oxo-2-phenyl-1,2-dihydro-isoquinoline-4-carboxylic acid ((S)-1-phenyl-propyl)-amide
**2dd** 3-[3-(3-Chloro-phenyl)-piperazin-1-ylmethyl]-1-oxo-2-phenyl-1,2-dihydro-isoquinoline-4-carboxylic acid ((S)-1-phenyl-propyl)-amide
**2de** 3-[4-(1H-Indol-4-yl)-piperazin-1-ylmethyl]-1-oxo-2-phenyl-1,2-dihydro-isoquinoline-4-carboxylic acid ((S)-1-phenyl-propyl)-amide
**2df** 1-Oxo-3-(3-oxo-piperazin-1-ylmethyl)-2-phenyl-1,2-dihydro-isoquinoline-4-carboxylic acid ((S)-1-phenyl-propyl)-amide
**2dg** 3-[4-(1H-Indol-5-yl)-piperazin-1-ylmethyl]-1-oxo-2-phenyl-1,2-dihydro-isoquinoline-4-carboxylic acid ((S)-1-phenyl-propyl)-amide
**2dh** 3-(6,9-Diaza-spiro[4.5]dec-9-ylmethyl)-1-oxo-2-phenyl-1,2-dihydro-isoquinoline-4-carboxylic acid ((S)-1-phenyl-propyl)-amide
**2di** 3-(1,4-Diaza-spiro[5.5]undec-4-ylmethyl)-1-oxo-2-phenyl-1,2-dihydro-isoquinoline-4-carboxylic acid ((S)-1-phenyl-propyl)-amide
**2dj** 3-(3-Isopropyl-piperazin-1-ylmethyl)-1-oxo-2-phenyl-1,2-dihydro-isoquinoline-4-carboxylic acid ((S)-1-phenyl-propyl)-amide
**2dk** 3-(3,3-Dimethyl-piperazin-1-ylmethyl)-1-oxo-2-phenyl-1,2-dihydro-isoquinoline-4-carboxylic acid ((S)-1-phenyl-propyl)-amide
**2dl** 3-[3-(4-Fluoro-phenyl)-piperazin-1-ylmethyl]-1-oxo-2-phenyl-1,2-dihydro-isoquinoline-4-carboxylic acid ((S)-1-phenyl-propyl)-amide
**2dm** 1-Oxo-2-phenyl-3-(3-p-tolyl-piperazin-1-ylmethyl)-1,2-dihydro-isoquinoline-4-carboxylic acid ((S)-1-phenyl-propyl)-amide
**2dn** 4-[1-Oxo-2-phenyl-4-(1-phenyl-propylcarbamoyl)-1,2-dihydro-isoquinolin-3-ylmethyl]-piperazine-1-carboxylic acid tert-butyl ester
**2do** 3-(4-Methylcarbamoylmethyl-piperazin-1-ylmethyl)-1-oxo-2-phenyl-1,2-dihydro-isoquinoline-4-carboxylic acid ((S)-1-phenyl-propyl)-amide
**2dp** 8-Chloro-3-dimethylaminomethyl-1-oxo-2-phenyl-1,2-dihydro-isoquinoline-4-carboxylic acid ((S)-1-phenyl-propyl)-amide
**2dr** 3-Cyclopentylaminomethyl-1-oxo-2-phenyl-1,2-dihydro-isoquinoline-4-carboxylic acid ((S)-1-phenyl-propyl)-amide
**2ds** 3-Cyclohcxylaminomethyl-1-oxo-2-phenyl-1,2-dihydro-isoquinoline-4-carboxylic acid ((S)-1-phenyl-propyl)-amide
**2dt** 3-{[(2-Hydroxy-ethyl)-methyl-amino]-methyl}-1-oxo-2-phenyl-1,2-dihydro-isoquinoline-4-carboxylic acid ((S)-1-phenyl-propyl)-amide
**2du** 3-Imidazol-1-ylmethyl-1-oxo-2-phenyl-1,2-dihydro-isoquinoline-4-carboxylic acid ((S)-1-phenyl-propyl)-amide
**2dv** 3-(3-Methyl-imidazol-1-ylmethyl)-1-oxo-2-phenyl-1,2-dihydro-isoquinoline-4-carboxylic acid ((S)-1-phenyl-propyl)-amide
**2dw** 3-(4-Methyl-imidazol-1-ylmethyl)-1-oxo-2-phenyl-1,2-dihydro-isoquinoline-4-carboxylic acid ((S)-1-phenyl-propyl)-amide
**2dx** 3-(2,5-Dihydro-pyrrol-1-ylmethyl)-1-oxo-2-phenyl-1,2-dihydro-isoquinoline-4-carboxylic acid ((S)-1-phenyl-propyl)-amide
**2dy** 3-(2.5-Dimethyl-2,5-dihydro-pyrrol-1-ylmethyl)-1-oxo-2-phenyl-1,2-dihydro-isoquinoline-4-carboxylic acid ((S)-1-phenyl-propyl)-amide
**2dz** 1-Oxo-2-phenyl-3-pyrrolidin-1-ylmethyl-1,2-dihydro-isoquinoline-4-carboxylic acid ((S)-1-phenyl-propyl)-amide
**2ea** I-Oxo-2-phcnyl-3-(thiazol-2-ylaminomethyl)-1,2-dihydro-isoquinoline-4-carboxylic acid ((S)-1-phenyl-propyl)-amide
**2eb** 1-Oxo-2-phenyl-3-(pyrimidin-4-ylaminomethyl)-1,2-dihydro-isoquinoline-4-carboxylic acid ((S)-1-phenyl-propyl)-amide
**2ec** 3-(tert-Butylamino-methyl)-1-oxo-2-phenyl-1,2-dihydro-isoquinoline-4-carboxylic acid ((S)-1-phenyl-propyl)-amide
**2ed** 3-[(2-Hydroxy-1,1-dimethyl-ethylamino)-methyl]-1-oxo-2-phenyl-1,2-dihydro-isoquinoline-4-carboxylic acid ((S)-1-phenyl-propyl)-amide
**2ee** 3-(Isopropylamino-methyl)-1-oxo-2-phenyl-1,2-dihydro-isoquinoline-4-carboxylic acid ((S)-1-phenyl-propyl)-amide
**2ef** 3-[(2-Hydroxy-1-methyl-ethylamino)-methyl]-1-oxo-2-phenyl-1,2-dihydro-isoquinoline-4-carboxylic acid ((S)-1-phenyl-propyl)-amide
**2eg** 3-[(1-Hydroxymethyl-propylamino)-methyl]-1-oxo-2-phenyl-1,2-dihydro-isoquinoline-4-carboxylic acid ((S)-1-phenyl-propyl)-amide
**2eh** 3-[(2,2-Dimethyl-propylamino)-methyl]-1-oxo-2-phenyl-1,2-dihydro-isoquinoline-4-carboxylic acid ((S)-1-phenyl-propyl)-amide
**2ei** 1-Oxo-2-phenyl-3-prop-2-ynylaminomethyl-1,2-dihydro-isoquinoline-4-carboxylic acid ((S)-1-phenyl-propyl)-amide
**2ej** 3-Allylaminomethyl-1-oxo-2-phenyl-1,2-dihydro-isoquinoline-4-carboxylic acid ((S)-1-phenyl-propyl)-amide
**2ek** 3-[(Methyl-prop-2-ynyl-amino)-methyl]-1-oxo-2-phenyl-1,2-dihydro-isoquinoline-4-carboxylic acid ((S)-1-phenyl-propyl)-amide
**2el** 3-Diallylaminomethyl-1-oxo-2-phenyl-1,2-dihydro-isoquinoline-4-carboxylic acid ((S)-1-phenyl-propyl)-amide
**2em** 3-Diethylaminomethyl-1-oxo-2-phenyl-1,2-dihydro-isoquinoline-4-carboxylic acid ((S)-1-phenyl-propyl)-amide
**2en** 3-[(Isopropyl-methyl-amino)-methyl]-1-oxo-2-phenyl-1,2-dihydro-isoquinoline-4-carboxylic acid ((S)-1-phenyl-propyl)-amide
**2eo** 3-[((S)-2-Hydroxy-1-methyl-ethylamino)-methyl]-1-oxo-2-phenyl-1,2-dihydro-isoquinoline-4-carboxylic acid ((S)-1-phenyl-propyl)-amide
**2ep** 3-[((R)-2-Hydroxy-1-methyl-ethylamino)-methyl]-1-oxo-2-phenyl-1,2-dihydro-isoquinoline-4-carboxylic acid ((S)-1-phenyl-propyl)-amide
**2eq** 3-{[(2-Methoxy-ethyl)-methyl-amino]-methyl}-1-oxo-2-phenyl-1,2-dihydro-isoquinoline-4-carboxylic acid ((S)-1-phenyl-propyl)-amide
**2er** 3-((R)-3-Hydroxy-pyrrolidin-1-ylmethyl)-1-oxo-2-phenyl-1,2-dihydro-isoquinoline-4-carboxylic acid ((S)-1-phenyl-propyl)-amide
**2es** 3-((S)-3-Hydroxy-pyrrolidin-1-ylmethyl)-1-oxo-2-phenyl-1,2-dihydro-isoquinoline-4-carboxylic acid ((S)-1-phenyl-propyl)-amide
**2et** 3-[(Cyclopentyl-methyl-amino)-methyl]-1-oxo-2-phenyl-1,2-dihydro-isoquinoline-4-carboxylic acid ((S)-1-phenyl-propyl)-amide
**2eu** 3-{[(2-Hydroxy-1-methyl-ethyl)-methyl-amino]-methyl}-1-oxo-2-phenyl-1,2-dihydro-isoquinolinc-4-carboxylic acid ((S)-1-phenyl-propyl)-amide
**2ev** 3-{[Ethyl-(2-hydroxy-ethyl)-amino]-methyl}-1-oxo-2-phenyl-1,2-dihydro-isoquinoline-4-carboxylic acid ((S)-1-phenyl-propyl)-amide
**2ew** 3-[(Ethyl-methyl-amino)-methyl]-1-oxo-2-phenyl-1,2-dihydro-isoquinoline-4-carboxylic acid ((S)-1-phenyl-propyl)-amide
**2ex** 3-Cyclobutylaminomethyl-1-oxo-2-phenyl-1,2-dihydro-isoquinoline-4-carboxylic acid ((S)-1-phenyl-propyl)-amide
**2ey** 3-Azetidin-1-ylmethyl-1-oxo-2-phenyl-1,2-dihydro-isoquinoline-4-carboxylic acid ((S)-1-phenyl-propyl)-amide
**2ez** 3-(4-tert-Butyl-piperazin-1-ylmethyl)-1-oxo-2-phenyl-1,2-dihydro-isoquinoline-4-carboxylic acid ((S)-1-phenyl-propyl)-amide
**2fa** 3-[4-(2-Hydroxy-ethyl)-piperazin-1-ylmethyl]-1-oxo-2-phenyl-1,2-dihydro-isoquinoline-4-carboxylic acid ((S)-1-phenyl-propyl)-amide
**2fb** 3-{4-[2-(2-Hydroxy-ethoxy)-ethyl]-piperazin-1-ylmethyl}-1-oxo-2-phenyl-1,2-dihydro-isoquinoline-4-carboxylic acid ((S)-1-phenyl-propyl)-amide
**2fc** 3-[4-(3-Chloro-5-trifluoromethyl-pyridin-2-yl)-piperazin-1-ylmethyl]-1-oxo-2-phenyl-1,2-dihydro-isoquinoline-4-carboxylic acid ((S)-1-phenyl-propyl)-amide
**2fd** 3-[4-(3,5-Dichloro-pyridin-4-yl)-piperazin-1-ylmethyl]-1-oxo-2-phenyl-1,2-dihydro-isoquinoline-4-carboxylic acid ((S)-1-phenyl-propyl)-amide
**2fe** 4-[1-Oxo-2-phenyl-4-((S)-1-phenyl-proprylcarbamoyl)-1,2-dihydro-isoquinolin-3-ylmethyl]-piperazine-1-carboxylic acid benzyl ester
**2ff** 3-[4-(3-Morpholin-4-yl-propyl)-piperazin-1-ylmethyl]-1-oxo-2-phenyl-1,2-dihydro-isoquinolinc-4-carboxylic acid ((S)-1-phenyl-propyl)-amide
**2fg** 1-Oxo-2-phenyl-3-[4-(3-piperidin-1-yl-propyl)-piperazin-1-ylmethyl]-1,2-dihydro-isoquinolinc-4-carboxylic acid ((S)-1-phenyl-propyl)-amide
**2fh** 3-[4-(4,6-Dimethoxy-pyrimidin-2-ylmethyl)-piperazin-1-ylmethyl]-1-oxo-2-phenyl-1,2-dihydro-isoquinoline-4-carboxylic acid ((S)-1-phenyl-propyl)-amide
**2fi** 3-[4-(3-Hydroxy-propyl)-piperazin-1-ylmethyl]-1-oxo-2-phenyl-1,2-dihydro-isoquinoline-4-carboxylic acid ((S)-1-phenyl-propyl)-amide
**2fj** 3-[4-(2,3-Dihydroxy-propyl)-piperazin-1-ylmethyl]-1-oxo-2-phenyl-1,2-dihydro-isoquinoline-4-carboxylic acid ((S)-1-phenyl-propyl)-amide
**2fk** (2-Oxo-2-{4-[1-oxo-2-phenyl-4-(1-phenyl-propylcarbamoyl)-1,2-dihydro-isoquinolin-3-ylmethyl]-piperazin-1-yl}-ethyl)-carbamic acid tert-butyl ester
**2fl** 3-[4-(1H-Indazol-5-yl)-piperazin-1-ylmethyl]-1-oxo-2-phenyl-1,2-dihydro-isoquinoline-4-carboxylic acid ((S)-1-phenyl-propyl)-amide
**2fm** 1-Oxo-2-phenyl-3-(4-quinolin-6-yl-piperazin-1-ylmethyl)-1,2-dihydro-isoquinoline-4-carboxylic acid ((S)-1-phenyl-propyl)-amide
**2fn** 3-[4-(6,7-Dimethoxy-quinazolin-4-yl)-piperazin-1-ylmethyl]-1-oxo-2-phenyl-1,2-dihydro-isoquinoline-4-carboxylic acid ((S)-1-phenyl-propyl)-amide
**2fo** 4-{4-[1-Oxo-2-phenyl-4-(1-phenyl-propylcarbamoyl)-1,2-dihydro-isoquinolin-3-ylmethyl]-piperazin-1-yl}-piperidine-1-carboxylic acid tert-butyl ester
**2fp** 3-{4-[2-(4-Chloro-phenoxy)-ethyl]-piperazin-1-ylmethyl}-1-oxo-2-phenyl-1,2-dihydro-isoquinoline-4-carboxylic acid ((S)-1-phenyl-propyl)-amide
**2fq** {4-[1-Oxo-2-phenyl-4-(1-phenyl-propylcarbamoyl)-1,2-dihydro-isoquinolin-3-ylmethyl]-piperazin-1-yl}-acetic acid tert-butyl ester
**2fr** 1-Oxo-2-phenyl-3-[4-(3,3,3-trifluoro-2-hydroxy-propyl)-piperazin-1-ylmethyl]-1,2-dihydro-isoquinoline-4-carboxylic acid ((S)-1-phenyl-propyl)-amide
**2fs** 3-[4-(2-Hydroxy-propyl)-piperazin-1-ylmethyl]-1-oxo-2-phenyl-1,2-dihydro-isoquinoline-4-carboxylic acid ((S)-1-phenyl-propyl)-amide
**2fu** 3-[4-(4-Amino-6,7-dimethoxy-quinazolin-2-yl)-piperazin-1-ylmethyl]-1-oxo-2-phenyl-1,2-dihydro-isoquinoline-4-carboxylic acid ((S)-1-phenyl-propyl)-amide
**2fv** (2-{4-[1-Oxo-2-phenyl-4-(1-phenyl-propylcarbamoyl)-1,2-dihydro-isoquinolin-3-ylmethyl]-piperazin-1-yl}-ethyl)-carbamic acid tert-butyl ester
**2fw** 1-Oxo-3-{4-[2-(2-oxo-imidazolidin-1-yl)-ethyl]-piperazin-1-ylmethyl}-2-phenyl-1,2-dihydro-isoquinoline-4-carboxylic acid ((S)-1-phenyl-propyl)-amide
**2fx** 3-{4-[(4,6-Dimethoxy-pyrimidin-2-yl)-phenyl-methyl]-piperazin-1-ylmethyl}-1-oxo-2-phenyl-1,2-dihydro-isoquinoline-4-carboxylic acid ((S)-1-phenyl-propyl)-amide
**2fy** 3-(4-Benzo[1,2,5]thiadiazol-4-yl-piperazin-1-ylmethyl)-1-oxo-2-phenyl-1,2-dihydro-isoquinoline-4-carboxylic acid ((S)-1-phenyl-propyl)-amide
**2fz** 3-[4-(2,3-Dihydro-benzo[1,4]dioxin-5-yl)-piperazin-1-ylmethyl]-1-oxo-2-phenyl-1,2-dihydro-isoquinoline-4-carboxylic acid ((S)-1-phenyl-propyl)-amide
**2ga** 3-[4-(4-Methyl-quinolin-2-yl)-piperazin-1-ylmethyl]-1-oxo-2-phenyl-1,2-dihydro-isoquinoline-4-carboxylic acid ((S)-1-phenyl-propyl)-amide
**2gb** 1-Oxo-2-phenyl-3-[4-(pyridin-2-ylcarbamoylmethyl)-piperazin-1-ylmethyl]-1,2-dihydro-isoquinoline-4-carboxylic acid ((S)-1-phenyl-propyl)-amide
**2gc** 3-[4-(6-Chloro-3-oxo-3,4-dihydro-2H-benzo[1,4]oxazin-8-yl)-piperazin-1-ylmethyl]-1-oxo-2-phenyl-1,2-dihydro-isoquinoline-4-carboxylic acid ((S)-1-phenyl-propyl)-amide
**2gd** 3-(4-Carbamoylmethyl-piperazin-1-ylmethyl)-1-oxo-2-phenyl-1,2-dihydro-isoquinoline-4-carboxylic acid ((S)-1-phenyl-propyl)-amide
**2ge** 3-(4-Hydroxy-4-phenyl-piperidin-1-ylmethyl)-1-oxo-2-phenyl-1,2-dihydro-isoquinolinc-4-carboxylic acid ((S)-1-phenyl-propyl)-amide
**2gf** 3-[4-(4-Chloro-phenyl)-4-hydroxy-piperidin-1-ylmethyl]-1-oxo-2-phenyl-1,2-dihydro-isoquinolinc-4-carboxylic acid ((S)-1-phenyl-propyl)-amide
**2gg** 1-[1-Oxo-2-phenyl-4-(1-phenyl-propylcarbamoyl)-1,2-dihydro-isoquinolin-3-ylmethyl]-piperidine-4-carboxylic acid
**2gh** 3-(4-Cyano-4-phenyl-piperidin-1-ylmethyl)-1-oxo-2-phenyl-1,2-dihydro-isoquinoline-4-carboxylic acid ((S)-1-phenyl-propyl)-amide
**2gi** 3-(4-Benzyl-4-hydroxy-piperidin-1-ylmethyl)-1-oxo-2-phenyl-1,2-dihydro-isoquinoline-4-carboxylic acid ((S)-1-phenyl-propyl)-amide
**2gj** 1-[1-Oxo-2-phenyl-4-(1-phenyl-propylcarbamoyl)-1,2-dihydro-isoquinolin-3-ylmethyl]-4-phenyl-piperidine-4-carboxylic acid ethyl ester
**2gk** 1-Oxo-2-phenyl-3-[4-(phenyl-propionyl-amino)-piperidin-1-ylmethyl]-1,2-dihydro-isoquinoline-4-carboxylic acid ((S)-1-phenyl-propyl)-amide
**2g1** Methyl-{1-[1-oxo-2-phenyl-4-(1-phenyl-propylcarbamoyl)-1,2-dihydro-isoquinolin-3-ylmethyl]-piperidin-4-yl}-carbamic acid tert-butyl ester
**2gm** 1-Oxo-2-phenyl-3-[4-(2-pyrrolidin-1-yl-ethyl)-piperidin-1-ylmethyl]-1,2-dihydro-isoquinoline-4-carboxylic acid ((S)-1-phenyl-propyl)-amide
**2gn** 3-{4-[5-(4-Fluoro-phenyl)-[1,3,4]oxadiazol-2-yl]-piperidin-1-ylmethyl}-1-oxo-2-phenyl-1,2-dihydro-isoquinoline-4-carboxylic acid ((S)-1-phenyl-propyl)-amide
**2go** 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide
**2gp** 1-Oxo-2-phenyl-3-[4-(3-pyrarin-2-yl-[1,2,4]oxadiazol-5-yl)-piperidin-1-ylmethyl]-1,2-dihydro-isoquinoline-4-carboxylic acid ((S)-1-phenyl-propyl)-amide
**2gq** 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide
**2gr** 3-(4'-Hydroxy-3',4',5',6'-tetrahydro-2'H-[2,4']bipyridinyl-1'-ylmethyl)-1-oxo-2-phenyl-1,2-dihydro-isoquinoline-4-carboxylic acid ((S)-1-phenyl-propyl)-amide
**2gs** 3-(spiro[isochroman-1,4'-piperidin]-1'-ylmethyl)-1-oxo-2-phenyl-1,2-dihydro-isoquinoline-4-carboxylic acid ((S)-1-phenyl-propyl)-amide
**2gt** 3-[4-Hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide
**2gu** 3-[4-(3-Chloro-phenyl)-4-hydroxy-piperidin-1-ylmethyl]-1-oxo-2-phenyl-1,2-dihydro-isoquinoline-4-carboxylic acid ((S)-1-phenyl-propyl)-amide
**2gv** 3-(6-chloro-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide
**2gw** 3-(4-{[4-Chloro-3-(4-fluoro-phenyl)-indan-1-yl]-methyl-amino}-piperidin-1-ylmethyl)-1-oxo-2-phenyl-1,2-dihydro-isoquinoline-4-carboxylic acid ((S)-1-phenyl-propyl)-amide
**2gx** 3-(1-acetyl-spiro[indoline-3,4'-piperidin]-1'-ylmethyl)-1-oxo-2-phenyl-1,2-dihydro-isoquinoline-4-carboxylic acid ((S)-1-phenyl-propyl)-amide
**2gy** 3-(1-acetyl-5-flluoro-spiro[indoline-3,4'-piperidin]-1'-ylmethyl)-1-oxo-2-phenyl-1,2-dihydro-isoquinoline-4-carboxylic acid ((S)-1-phenyl-propyl)-amide
**2gz** 1-Oxo-3-(4-oxo-1-phenyl-1,3,8-triaza-spiro[4.5]dec-8-ylmethyl)-2-phenyl-1,2-dihydro-isoquinoline-4-carboxylic acid ((S)-1-phenyl-propyl)-amide
**2ha** 3-(4-Acetylamino-piperidin-1-ylmethyl)-1-oxo-2-phenyl-1,2-dihydro-isoquinoline-4-carboxylic acid ((S)-1-phenyl-propyl)-amide
**2hb** 1-Oxo-3-(1-oxo-2,8-diaza-spiro[4.5]dec-8-ylmethyl)-2-phenyl-1,2-dihydro-isoquinoline-4-carboxylic acid ((S)-1-phenyl-propyl)-amide
**2hc** 3-[4-Hydroxy-4-(3-trifluoromethyl-phenyl)-piperidin-1-ylmethyl]-1-oxo-2-phenyl-1,2-dihydro-isoquinoline-4-carboxylic acid ((S)-1-phenyl-propyl)-amide
**2hd** 1-Oxo-2-phenyl-3-(4-trifluoromethyl-piperidin-1-ylmethyl)-1,2-dihydro-isoquinoline-4-carboxylic acid ((S)-1-phenyl-propyl)-amide
**2he** 3-[4-(4-Methyl-piperazine-1-carbonyl)-piperidin-1-ylmethyl]-1-oxo-2-phenyl-1, ,2-dihydro-isoquinoline-4-carboxylic acid ((S)-1-phenyl-propyl)-amide
**2hf** 3-(5-isopropyl-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide
**2hg** 3-[4-(Acetyl-methyl-amino)-4-phenyl-piperidin-1-ylmethyl]-1-oxo-2-phenyl-1,2-dihydro-isoquinoline-4-carboxylic acid ((S)-1-phenyl-propyl)-amide
**2hh** 4-{1-[1-Oxo-2-phenyl-4-(1-phenyl-propylcarbamoyl)-1,2-dihydro-isoquinolin-3-ylmethyl]-piperidin-4-yl}-piperazine-1-carboxylic acid tert-butyl ester
**2hi** (2-{1-[1-Oxo-2-phenyl-4-(1-phenyl-propylcarbamoyl)-1,2-dihydro-isoquinolin-3-ylmethyl]-piperidin-4-yl}-ethyl)-carbamic acid tert-butyl ester
**2hj** 3-(4-Methylamino-4-phenyl-piperidin-1-ylmethyl)-1-oxo-2-phenyl-1,2-dihydro-isoquinolinc-4-carboxylic acid ((S)-1-pheny(-propyl)-amide
**2hk** 3-(4-Acetylamino-4-phenyl-piperidin-1-ylmethyl)-1-oxo-2-phenyl-1,2-dihydro-isoquinoline-4-carboxylic acid ((S)-1-phenyl-propyl)-amide
**2hl** 3-[4-Acetylamino-4-(3-fluoro-phenyl)-piperidin-1-ylmethyl]-1-oxo-2-phenyl-1,2-dihydro-isoquinoline-4-carboxylic acid ((S)-1-phenyl-propyl)-amide
**2hm** 1-Oxo-3-[4-(4-oxo-piperidine-1-carbonyl)-piperidin-1-ylmethyl]-2-phenyl-1,2-dihydro-isoquinoline-4-carboxylic acid ((S)-1-phenyl-propyl)-amide
**2hn** 3-[4,4']Bipiperldinyl-1-ylmethyl-1-oxo-2-phenyl-1,2-dihydro-isoquinoline-4-carboxylic acid ((S)-1-phenyl-propyl)-amide
**2ho** {1-[1-Oxo-2-phenyl-4-(1-phenyl-propylcarbamoyl)-1,2-dihydro-isoquinolin-3-ylmethyl]-piperidin-4-yl}-carbamic acid tert-butyl ester
**2hp** 3-[1,4']Bipiperidinyl-1'-ylmethyl-1-oxo-2-phenyl-1,2-dihydro-isoquinoline-4-carboxylic acid [cyclopropyl-(3-fluoro-phenyl)-methyl]-amide
**2hq** 1-oxo-3-(4-phenethyl-piperazin-1-ylmethyl)-2-phenyl-1,2-dihydro-isoquinoline-4-carboxylic acid [cyclopropyl-(3-fluoro-phenyl)-methyl]-amide
**2hr** 3-(4-Isopropyl-piperazin-1-ylmethyl)-1-oxo-2-phenyl-1,2-dihydro-isoquinoline-4-carboxylic acid [cyclopropyl-(3-fluoro-phenyl)-methyl]-amide
**2hs** 3-[4-(2-Morpholin-4-yl-ethyl)-piperazin-1-ylmethyl]-1-oxo-2-phenyl-1,2-dihydro-isoquinoline-4-carboxylic acid [cyclopropyl-(3-fluoro-phenyl)-methyl]-amide
**2ht** 3-[4-(1-Methyl-piperidin-4-yl)-piperazin-1-ylmethyl]-1-oxo-2-phenyl-1,2-dihydro-isoquinoline-4-carboxylic acid [cyclopropyl-(3-fluoro-phenyl)-methyl]-amide
**2hu** 1-Oxo-2-phenyl-3-[4-(2-piperidin-1-yl-ethyl)-piperazin-1-ylmethyl]-1,2-dihydro-isoquinoline-4-carboxylic acid [cyclopropyl-(3-fluoro-phenyl)-methyl]-amide
**2hv** 1-Oxo-2-phenyl-3-[4-(2-pyrrolidin-1-yl-ethyl)-piperazin-1-ylmethyl]-1,2-dihydro-isoquinoline-4-carboxylic acid [cyclopropyl-(3-fluoro-phenyl)-methyl]-amide
**2hw** 1-Oxo-2-phenyl-3-(4-pyridin-4-ylmethyl-piperazin-1-ylmethyl)-1,2-dihydro-isoquinoline-4-carboxylic acid [cyclopropyl-(3-fluoro-phenyl)-methyl]-amide
**2hx** 3-(4-Hydroxy-3,4,5,6-tetrahydro-2H-[4,4']bipyridinyl-1-ylmethyl)-1-oxo-2-phenyl-1,2-dihydro-isoquinoline-4-carboxylic acid [cyclopropyl-(3-fluoro-phenyl)-mcthyl]-amidc
**2hy** 3-[4-(2-Morpholin-4-yl-ethyl)-piperidin-1-ylmethyl]-1-oxo-2-phenyl-1,2-dihydro-isoquinoline-4-carboxylic acid [cyclopropyl-(3-fluoro-phenyl)-methyl]-amide
**2hz** 3-[4-Hydroxy-4-(3-methoxy-phenyl)-piperidin-1-ylmethyl]-1-oxo-2-phenyl-1,2-dihydro-isoquinoline-4-carboxylic acid [cyclopropyl-(3-fluoro-phenyl)-methyl]-amide
**2ia** 3-[4-(Acetyl-methyl-amino)-4-phenyl-piperidin-1-ylmethyl]-1-oxo-2-phenyl-1,2-dihydro-isoquinoline-4-carboxylic acid [cyclopropyl-(3-fluoro-phenyl)-methyl]-amide
**2ib** 2-(2-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2ic** 2-(2-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2id** 3-[1,4']Bipiperidinyl-1'-ylmethyl-2-(2-fluoro-phenyl)-1-oxo-1,2-dihydro-isoquinoline-4-carboxylic acid ((S)-1-phenyl-propyl)-amide
**2ie** 2-(2-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2if** 2-(2-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2ig** 2-(2-Fluoro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2ih** 2-(2-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2ii** 2-(2-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ij** 2-(2-Fluoro-phenyl)-3-(4-hydroxy-3,4,5,6-tetrahydro-2H-[4,4']bipyridinyl-1-ylmethyl)-1-oxo-1,2-dihydro-isoquinoline-4-carboxylic acid ((S)-1-phenyl-propyl)-amide
**2ik** 2-(2-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide
**2il** 2-(2-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide

**2im** 3-[4-(2-Morpholin-4-yl-ethyl)-piperazin-1-ylmethyl]-1-oxo-2-o-tolyl-1,2-dihydro-isoquinoline-4-carboxylic acid ((S)-1-phenyl-propyl)-amide **2in** 1-Oxo-3-[4-(2-piperidin-1-yl-ethyl)-piperazin-1-ylmethyl]-2-o-tolyl-1,2-dihydro-isoquinoline-4-carboxylic acid ((S)-1-phenyl-propyl)-amide
**2io** 1-Oxo-3-(4-pyridin-4-ylmethyl-piperazin-1-ylmethyl)-2-o-tolyl-1,2-dihydro-isoquinoline-4-carboxylic acid ((S)-1-phenyl-propyl)-amide
**2ip** 2-(3-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2iq** 2-(3-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2ir** 3-[1,4']Bipiperidinyl-1'-ylmethyl-2-(3-fluoro-phenyl)-1-oxo-1,2-dihydro-isoquinoline-4-carboxylic acid ((S)-1-phenyl-propyl)-amide
**2is** 2-(3-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2it** 2-(3-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2iu** 2-(3-Fluoro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2iv** 2-(3-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2iw** 2-(3-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ix** 2-(3-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide
**2iy** 2-(3-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2iz** 3-[4-(Acetyl-methyl-amino)-4-phenyl-piperidin-1-ylmethyl]-2-(3-fluoro-phenyl)-1-oxo-1,2-dihydro-isoquinoline-4-carboxylic acid ((S)-1-phenyl-propyl)-amide
**2ja** 2-(3-Chloro-phenyl)-1-oxo-3-(4-phenethyl-piperazin-1-ylmethyl)-1,2-dihydro-isoquinoline-4-carboxylic acid ((S)-1-phenyl-propyl)-amide
**2jb** 2-(3-Chloro-phenyl)-3-(4-isopropyl-piperazin-1-ylmethyl)-1-oxo-1,2-dihydro-isoquinolinc-4-carboxylic acid ((S)-1-phenyl-propyl)-amide
**2jc** 3-[1,4']Bipiperidinyl-1'-ylmethyl-2-(3-chloro-phenyl)-1-oxo-1,2-dihydro-isoquinoline-4-carboxylic acid ((S)-1-phenyl-propyl)-amide
**2jd** 2-(3-Chloro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2je** 2-(3-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2jf** 2-(3-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2jg** 2-(3-Chloro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2jh** 2-(3-Chloro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ji** 2-(3-Chloro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide
**2jj** 2-(3-Chloro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2jk** 3-[4-(Acetyl-methyl-amino)-4-phenyl-piperidin-1-ylmethyl]-2-(3-chloro-phenyl)-1-oxo-1,2-dihydro-isoquinoline-4-carboxylic acid ((S)-1-phenyl-propyl)-amide
**2ji** 1-Oxo-3-(4-phenethyl-piperazin-1-ylmethyl)-2-m-tolyl-1,2-dihydro-isoquinoline-4-carboxylic acid ((S)-1-phenyl-propyl)-amide
**2jm** 3-[1,4']Bipiperidinyl-1'-ylmethyl-1-oxo-2-m-tolyl-1,2-dihydro-isoquinoline-4-carboxylic acid ((S)-1-phenyl-propyl)-amide
**2jn** 3-[4-(2-Morpholin-4-yl-ethyl)-piperazin-1-ylmethyl]-1-oxo-2-m-tolyl-1,2-dihydro-isoquinoline-4-carboxylic acid ((S)-1-phenyl-propyl)-amide
**2jo** 3-[4-(1-Methyl-piperidin-4-yl)-piperazin-1-ylmethyl]-1-oxo-2-m-tolyl-1,2-dihydro-isoquinoline-4-carboxylic acid ((S)-1-phenyl-propyl)-amide
**2jp** 1-Oxo-3-[4-(2-piperidin-1-yl-ethyl)-piperazin-1-ylmethyl]-2-m-tolyl-1,2-dihydro-isoquinoline-4-carboxylic acid ((S)-1-phenyl-propyl)-amide
**2jq** 1-Oxo-3-[4-(2-pyrrolidin-1-yl-ethyl)-piperazin-1-ylmethyl]-2-m-tolyl-1,2-dihydro-isoquinoline-4-carboxylic acid ((S)-1-phenyl-propyl)-amide
**2jr** 1-Oxo-3-(4-pyridin-4-ylmethyl-piperazin-1-ylmethyl)-2-m-tolyl-1,2-dihydro-isoquinoline-4-carboxylic acid ((S)-1-phenyl-propyl)-amide
**2js** 3-[4-(2-Morpholin-4-yl-ethyl)-piperidin-1-ylmethyl]-1-oxo-2-m-tolyl-1,2-dihydro-isoquino line-4-carboxylic acid ((S)-1-phenyl-propyl)-amide
**2jt** 3-[4-Hydroxy-4-(3-methoxy-phenyl)-piperidin-1-ylmethyl]-1-oxo-2-m-tolyl-1,2-dihydro-isoquinoline-4-carboxylic acid ((S)-1-phenyl-propyl)-amide
**2ju** 3-[4-(Acetyl-methyl-amino)-4-phenyl-piperidin-1-ylmethyl]-1-oxo-2-m-tolyl-1,2-dihydro-isoquinoline-4-carboxylic acid ((S)-1-phenyl-propyl)-amide
**3a** 1-Oxo-3-(2-oxo-pyrrolidin-1-ylmethyl)-2-phenyl-1,2-dihydro-isoquinoline-4-carboxylic acid ((S)-1-phenyl-propyl)-amide
**3b** 8-Chloro-1-oxo-3-(2-oxo-pyrrolidin-1-ylmethyl)-2-phenyl-1,2-dihydro-isoquinolinc-4-carboxylic acid ((S)-1-phenyl-propyl)-amide
**4a** 3-Cyanomethyl-1-oxo-2-phenyl-1,2-dihydro-isoquinoline-4-carboxylic acid ((S)-1-phenyl-propyl)-amide
**5a** 3-Methyl-1-oxo-2-phenyl-1,2-dihydro-isoquinoline-4-carboxylic acid N,N-diphenyl-hydrazide
**5b** N'-(3-Methyl-1-oxo-2-phenyl-1,2-dihydro-isoquinoline-4-carbonyl)-N-phenyl-hydrazinecarboxylic acid methyl ester
**1aa** 2-(3-Methoxy-phenyl)-3-methyl-1-oxo-1,2-dihydro-isoquinoline-4-carboxylic acid ((S)-1-phenyl-propyl)-amide
**1ab** 2-(4-Methoxy-phenyl)-3-methyl-1-oxo-1,2-dihydro-isoquinoline-4-carboxylic acid ((S)-1-phenyl-propyl)-amide
**1ac** 2-(4-Fluoro-phenyl)-3-methyl-1-oxo-1,2-dihydro-isoquinoline-4-carboxylic acid ((S)-1-phenyl-propyl)-amide
**1ad** 2-(4-Chloro-phenyl)-3-methyl-1-oxo-1,2-dihydro-isoquinoline-4-carboxylic acid ((S)-1-phenyl-propyl)-amide
**1ae** 3-Methyl-1-oxo-2-p-tolyl-1,2-dihydro-isoquinoline-4-carboxylic acid ((S)-1-phenyl-propyl)-amide
**1af** 2-(3-Methoxy-phenyl)-3-methyl-1-oxo-1,2-dihydro-isoquinoline-4-carboxylic acid [(S)-cyclopropyl-(3-fluoro-phenyl)-methyl]-amide
**1ag** 2-(4-Methoxy-phenyl)-3-methyl-1-oxo-1,2-dihydro-isoquinoline-4-carboxylic acid [(S)-cyclopropyl-(3-fluoro-phenyl)-methyl]-amide
**1ah** 2-(4-Fluoro-phenyl)-3-methyl-1-oxo-1,2-dihydro-isoquinoline-4-carboxylic acid [(S)-cyclopropyl-(3-fluoro-phenyl)-methyl]-amide
**1ai** 2-(4-Chloro-phenyl)-3-methyl-1-oxo-1,2-dihydro-isoquinoline-4-carboxylic acid [(S)-cyclopropyl-(3-fluoro-phenyl)-methyl]-amide
**1aj** 3-Methyl-1-oxo-2-p-tolyl-1,2-dihydro-isoquinoline-4-carboxylic acid [(S)-cyclopropyl-(3-fluoro-phenyl)-methyl]-amide
**1ak** 2-(2-Methoxy-phenyl)-3-methyl-1-oxo-1,2-dihydro-isoquinoline-4-carboxylic acid ((S)-1-phenyl-propyl)-amide
**1al** 2-(2-Methoxy-phenyl)-3-methyl-1-oxo-1,2-dihydro-isoquinoline-4-carboxylic acid [(S)-cyclopropyl-(3-fluoro-phenyl)-methyl]-amide
**1am** 3-Methyl-8-nitro-1-oxo-2-phenyl-1,2-dihydro-isoquinoline-4-carboxylic acid ((S)-1-phenyl-propyl)-amide
**1an** 3-Methyl-8-nitro-1-oxo-2-phenyl-1,2-dihydro-isoquinoline-4-carboxylic acid [(S)-cyclopropyl-(3-fluoro-phenyl)-methyl]-amide
**1ao** 8-Methoxy-3-methyl-1-oxo-2-phenyl-1,2-dihydro-isoquinoline-4-carboxylic acid ((S)-1-phenyl-propyl)-amide
**1ap** 8-Methoxy-3-methyl-1-oxo-2-phenyl-1,2-dihydro-isoquinoline-4-carboxylic acid [(S)-cyclopropyl-(3-fluoro-phenyl)-methyl]-amide
**1aq** 8-Amino-3-methyl-1-oxo-2-phenyl-1,2-dihydro-isoquinoline-4-carboxylic acid ((S)-1-phenyl-propyl)-amide
**1ar** 8-Cyano-3-methyl-1-oxo-2-phenyl-1,2-dihydro-isoquinoline-4-carboxylic acid ((S)-1-phenyl-propyl)-amide
**1as** 8-Chloro-3-methyl-1-oxo-2-phenyl-1,2-dihydro-isoquinoline-4-carboxylic acid [(S)-1-(4-chloro-phenyl)-propyl]-amide
**1at** 8-Chloro-3-methyl-1-oxo-2-phenyl-1,2-dihydro-isoquinoline-4-carboxylic acid [(S)-1-(4-fluoro-phenyl)-propyl]-amide
**1au** 8-Chloro-3-methyl-1-oxo-2-phenyl-1,2-dihydro-isoquinoline-4-carboxylic acid [(S)-1-(2-fluoro-phenyl)-propyl]-amide
**1av** 8-Chloro-3-methyl-1-oxo-2-phenyl-1,2-dihydro-isoquinoline4-carboxylic acid [(S)-1-(3-chloro-phenyl)-propyl]-amide
**1aw** 8-Cloro-3-methyl-1-oxo-2-phenyl-1,2-dihydro-isoquinoline-4-carboxylic acid [(S)-(3-chloro-phenyl)-cyclopropyl-methyl]-amide
**1ax** 8-Chloro-3-methyl-1-oxo-2-phenyl-1,2-dihydro-isoquinoline-4-carboxylic acid [(S)-(4-chloro-phenyl)-cyclopropyl-methyl]-amide
**1ay** 8-Chloro-3-methyl-1-oxo-2-phenyl-1,2-dihydro-isoquinoline-4-carboxylic acid [(S)-1-(3-fluoro-phenyl)-propyl]-amide
**1az** 8-Chloro-3-methyl-1-oxo-2-phenyl-1,2-dihydro-isoquinoline-4-carboxylic acid ((S)-2-methyl-1-phenyl-propyl)-amide
**1ba** 8-Chloro-3-methyl-1-oxo-2-phenyl-1,2-dihydro-isoquinoline-4-carboxylic acid [(S)-cyclopropyl-(4-fluoro-phenyl)-methyl]-amide
**1bb** 8-Chloro-3-methyl-1-oxo-2-phenyl-1,2-dihydro-isoquinoline-4-carboxylic acid [(S)-1-(2-chloro-phenyl)-propyl]-amide
**1bc** 8-Chloro-3-methyl-1-oxo-2-phenyl-1,2-dihydro-isoquinoline-4-carboxylic acid ((S)-cyclopentyl-phenyl-methyl)-amide
**1bd** 8-Chloro-3-methyl-1-oxo-2-phenyl-1,2-dihydro-isoquinoline-4-carboxylic acid [(S)-(2-chloro-phenyl)-cyclopropyl-methyl]-amide
**1be** 8-Chloro-3-methyl-1-oxo-2-phenyl-1,2-dihydro-isoquinoline-4-carboxylic acid [(S)-cyclopropyl-(2-fluoro-phenyl)-methyl]-amide
**1bf** 8-Chloro-3-methyl-1-oxo-2-phenyl-1,2-dihydro-isoquinoline-4-carboxylic acid ((S)-cyclohexyl-phenyl-methyl)-amide
**1bg** 8-Chloro-3-methyl-1-oxo-2-phenyl-1,2-dihydro-isoquinoline-4-carboxylic acid ((S)-cyclopropyl-phenyl-methyl)-amide
**1bh** 8-Chloro-3-methyl-1-oxo-2-phenyl-1,2-dihydro-isoquinoline-4-carboxylic acid [(S)-cyclobutyl-(3-fluoro-phenyl)-methyl]-amide
**1bi** 8-Chloro-3-methyl-1-oxo-2-phenyl-1,2-dihydro-isoquinoline-4-carboxylic acid [(S)-cyclobutyl-(4-fluoro-phenyl)-methyl]-amide
**1bl** 8-Chloro-3-methyl-1-oxo-2-phenyl-1,2-dihydro-isoquinoline-4-carboxylic acid [(R)-cyclopropyl-(3-fluoro-phenyl)-methyl]-amide
**1bn** 8-Chloro-3-methyl-1-oxo-2-phenyl-1,2-dihydro-isoquinoline-4-carboxylic acid ((S)cyclobutyl-phenyl-methyl)-amide
**1bo** 8-Chloro-3-methyl-1-oxo-2-phenyl-1,2-dihydro-isoquinoline-4-carboxylic acid [cyclobutyl-(2-fluoro-phenyl)-methyl]-amide
**2ka** 3-(4-tert-Butyl-piperazin-1-ylmethyl)-8-chloro-1-oxo-2-phenyl-1,2-dihydro-isoquinoline-4-carboxylic acid [(S)-cyclopropyl-(3-fluoro-phenyl)-methyl]-amide
**2kb** 8-Chloro-3-(4-isopropyl-piperazin-1-ylmethyl)-1-oxo-2-phenyl-1,2-dihydro-isoquinoline-4-carboxylic acid ((S)-1-phenyl-propyl)-amide
**2kc** 8-Chloro-3-(4-isobutyl-piperazin-1-ylmethyl)-1-oxo-2-phenyl-1,2-dihydro-isoquinoline-4-carboxylic acid ((S)-1-phenyl-propyl)-amide
**2kd** 8-Chloro-3-(octahydro-pyrido[1,2-a]pyrazin-2-ylmethyl)-1-oxo-2-phenyl-1,2-dihydro-isoquinoline-4-carboxylic acid ((S)-1-phenyl-propyl)-amide
**2ke** 8-Chloro-3-(4-hydroxy-3,4,5,6-tetrahydro-2H-[4,4']bipyridinyl-1-ylmethyl)-1-oxo-2-phenyl-1,2-dihydro-isoquinoline-4-carboxylic acid ((S)-1-phenyl-propyl)-amidc
**2kf** 8-Chloro-3-(4-cyclopropylmethyl-piperazin-1-ylmethyl)-1-oxo-2-phenyl-1,2-dihydro-isoquinoline-4-carboxylic acid ((S)-1-phenyl-propyl)-amide
**2kg** 8-Chloro-3-[4-(2-morpholin-4-yl-ethyl)-piperidin-1-ylmethyl]-1-oxo-2-phenyl-1,2-dihydro-isoquinoline-4-carboxylic acid ((S)-1-phenyl-propyl)-amide
**2kh** 8-Chloro-3-[1,4]diazepan-1-ylmethyl-1-oxo-2-phenyl-1,2-dihydro-isoquinoline-4-carboxylic acid ((S)-1-phenyl-propyl)-amide
**2ki** 8-Chloro-3-((S)-3-methyl-piperazin-1-ylmethyl)-1-oxo-2-phenyl-1,2-dihydro-isoquinoline-4-carboxylic acid ((S)-1-phenyl-propyl)-amide
**2kj** 8-Chloro-3-imidazol-1-ylmethyl-1-oxo-2-phenyl-1,2-dihydro-isoquinoline-4-carboxylic acid ((S)-1-phenyl-propyl)-amide
**2kk** 8-Chloro-3-(4-methyl-imidazol-1-ylmethyl)-1-oxo-2-phenyl-1,2-dihydro-isoquinoline-4-carboxylic acid ((S)-1-phenyl-propyl)-amide
**2kl** 3-(tert-Butylamino-methyl)-8-chloro-1-oxo-2-phenyl-1,2-dihydro-isoquinoline-4-carboxylic acid ((S)-1-phenyl-propyl)-amide
**2km** 8-Chloro-3-(isopropylamino-methyl)-1-oxo-2-phenyl-1,2-dihydro-isoquinoline-4-carboxylic acid ((S)-1-phenyl-propyl)-amide
**2kn** 8-Chloro-3-[4-hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide
**2ko** 3-(4-tert-Butyl-piperazin-1-ylmethyl)-8-chloro-1-oxo-2-phenyl-1,2-dihydro-isoquinoline-4-carboxylic acid ((S)-1-phenyl-propyl)-amide
**2kp** 3-Benzoimidazol-1-ylmethyl-1-oxo-2-phenyl-1,2-dihydro-isoquinoline-4-carboxylic acid ((S)-1-phenyl-propyl)-amide
**2kq** 1-Oxo-2-phenyl-3-pyrazol-1-ylmethyl-1,2-dihydro-isoquinoline-4-carboxylic acid ((S)-1-phenyl-propyl)-amide
**2kr** 3-(4-Methyl-pyrazol-1-ylmethyl)-1-oxo-2-phenyl-1,2-dihydro-isoquinoline-4-carboxylic acid ((S)-1-phenyl-propyl)-amide
**2ks** 1-Oxo-2-phenyl-3-[1,2,3]triazol-1-ylmethyl-1,2-dihydro-isoquinoline-4-carboxylic acid ((S)-1-phenyl-propyl)-amide
**2kt** 2-(3-Methoxy-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ku** 2-(4-Methoxy-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2kv** 2-(4-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2kw** 2-(4-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2kx** 3-[1,4']Bipiperidinyl-1'-ylmethyl-2-(4-fluoro-phenyl)-1-oxo-1,2-dihydro-isoquinoline-4-carboxylic acid ((S)-1-phenyl-propyl)-amide
**2ky** 2-(4-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2kz** 2-(4-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**21a** 2-(4-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**21b** 2-(4-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**21c** 2-(4-Chloro-phenyl)-1-oxo-3-(4-phenethyl-piperazin-1-ylmethyl)-1,2-dihydro-isoquino line-4-carboxylic acid ((S)-1-phenyl-propyl)-amide
**21d** 2-(4-Chloro-phenyl)-3-(4-isopropyl-piperazin-1-ylmethyl)-1-oxo-1,2-dihydro-isoquinoline-4-carboxylic acid ((S)-1-phenyl-propyl)-amide
**21e** 3-[1,4']Bipiperidinyl-1'-ylmethyl-2-(4-chloro-phenyl)-1-oxo-1,2-dihydro-isoquinoline-4-carboxylic acid ((S)-1-phenyl-propyl)-amide
**21f** 2-(4-Chloro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**21g** 2-(4-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**21h** 2-(4-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**21i** 2-(4-Chloro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**21j** 3-(4-tert-Butyl-piperazin-1-ylmethyl)-8-fluoro-1-oxo-2-phenyl-1,2-dihydro-isoquinoline-4-carboxylic acid ((S)-1-phenyl-propyl)-amide
**21k** 8-Chloro-3-cyclopropylaminomethyl-1-oxo-2-phenyl-1,2-dihydro-isoquinoline-4-carboxylic acid ((S)-1-phenyl-propyl)-amide
**21l** 3-(4-tert-Butyl-piperazin-1-ylmethyl)-8-fluoro-1-oxo-2-phenyl-1,2-dihydro-isoquinoline-4-carboxylic acid [(S)-cyclopropyl-(3-fluoro-phenyl)-methyl]-amide
**21m** 8-Fluoro-1-oxo-2-phenyl-3-piperazin-1-ylmethyl-1,2-dihydro-isoquinoline-4-carboxylic acid [(S)-cyclopropyl-(3-fluoro-phenyl)-methyl]-amide
**21n** 8-Fluoro-1-oxo-3-(3-oxo-pyrazolidin-1-ylmethyl)-2-phenyl-1,2-dihydro-isoquinoline-4-carboxylic acid [(S)-cyclopropyl-(3-fluoro-phenyl)-methyl]-amide
**210** 8-Fluoro-1-oxo-3-(3-oxo-piperazin-1-ylmethyl)-2-phenyl-1,2-dihydro-isoquinoline-4-carboxylic acid [(S)-cyclopropyl-(3-fluoro-phenyl)-methyl]-amide
**3c** 1-Oxo-2-phenyl-3-(1H-[1,2,4]triazol-3-ylsulfanylmethyl)-1,2-dihydro-isoquinoline-4-carboxylic acid ((S)-1-phenyl-propyl)-amide
**3d** 8-Chloro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide

**3e** 8-Fluoro-1-oxo-3-(2-oxo-pyrrolidin-1-ylmethyl)-2-phenyl-1,2-dihydro-isoquinoline-4-carboxylic acid ((S)-1-phenyl-propyl)-amide
**3f** 8-Fluoro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide
**3g** 8-Fluoro-1-oxo-3-(5-oxo-pyrazolidin-1-ylmethyl)-2-phenyl-1,2-dihydro-isoquinoline-4-carboxylic acid [(S)-cyclopropyl-(3-fluoro-phenyl)-methyl]-amide
**3h** 8-Fluoro-1-oxo-3-(2-oxo-piperazin-1-ylmethyl)-2-phenyl-1,2-dihydro-isoquinoline-4-carboxylic acid [(S)-cyclopropyl-(3-fluoro-phenyl)-methyl]-amide
**3i** 8-Fluoro-1-oxo-3-(2-oxo-piperidin-1-ylmethyl)-2-phenyl-1,2-dihydro-isoquinoline-4-carboxylic acid [(S)-cyclopropyl-(3-fluoro-phenyl)-methyl]-amide
**4b** 8-Chloro-3-cyanomethyl-1-oxo-2-phenyl-1,2-dihydro-isoquinoline-4-carboxylic acid [(S)-cyclopropyl-(3-fluoro-phenyl)-methyl]-amide
**6a** 2-(3,4-Dichloro-phenyl)-3-methyl-1-oxo-1,2-dihydro-isoquinoline-4-carboxylic acid ((S)-1-phenyl-propyl)-amide
**6b** 8-Fluoro-1-oxo-3-(2-oxo-pyrrolidin-1-ylmethyl)-2-phenyl-1,2-dihydro-isoquinoline-4-carboxylic acid ((S)-cyclopropyl-phenyl-methyl)-amide **6c** 8-Fluoro-3-methyl-1-oxo-2-phenyl-1,2-dihydro-isoquinoline-4-carboxylic-4-carboxylic acid ((S)-cyclopropyl-phenyl-methyl)-amide
**7a** 3-Ethyl-1-oxo-3-phenyl-1,2-dihydro-isoquinoline-4-carboxylic acid ((S)-1-phenyl-propyl)-amide
**7b** 8-Fluoro-3-methyl-1-oxo-2-phenyl-1,2-dihydro-isquinoline-4-carboxylic acid ((S)-1-phenyl-propyl)-amide
**7c** 8-Fluoro-2-(3-fluoro-phenyl)-3-methyl-1-oxo-1,2-dihydro-isoquinoline-4-carboxylic acid ((S)-1-phenyl-propyl)-amide
**7d** 8-Fluoro-2-(4-fluoro-phenyl)-3-methyl-1-oxo-1,2-dihydro-isoquinoline-4-carboxylic acid ((S)-1-phenyl-propyl)-amide
**7f** 8-Fluoro-2-(3-fluoro-phenyl)-1-oxo-3-(2-oxo-pyrrolidin-1-ylmethyl)-1,2-dihydro-isoquinoline-4-carboxylic acid [(S)-cyclopropyl-(3-fluoro-phenyl)-methyl]-amide **7g** 8-Fluoro-2-(4-fluoro-phenyl)-1-oxo-3-(2-oxo-pyrrolidin-1-ylmethyl)-1,2-dihydro-isoquinoline-4-carboxylic acid [(S)-cyclopropyl-(3-fluoro-phenyl)-methyl]-amide
**7i** 8-Fluoro-2-(2-fluoro-phenyl)-3-methyl-1-oxo-1,2-dihydro-isoquinoline-4-carboxylic acid [(S)-cyclopropyl-(3-fluoro-phenyl)-methyl]-amide
**7j** 8-Fluoro-2-(3-fluoro-phenyl)-3-methyl-1-oxo-1,2-dihydro-isoquinoline-4-carboxylic acid [(S)-cyclopropyl-(3-fluoro-phenyl)-methyl]-amide
**7k** 8-Fluoro-2-(4-fluoro-phenyl)-3-methyl-1-oxo-1,2-dihydro-isoquinoline-4-carboxylic acid [(S)-cyclopropyl-(3-fluoro-phenyl)-methyl]-amide
**7l** 6,8-Difluoro-3-methyl-1-oxo-2-phenyl-1,2-dihydro-isoquinoline-4-carboxylic acid [(S)-cyclopropyl-(3-fluoro-phenyl)-methyl]-amide
**7m** 5,8-Difluoro-3-methyl-1-oxo-2-phenyl-1,2-dihydro-isoquinoline-4-carboxylic acid [(S)-cyclopropyl-(3-fluoro-phenyl)-methyl]-amide
**7n** 3-Methyl-1-oxo-2-phenyl-8-trifluoromethyl-1,2-dihydro-isoquinoline-4-carboxylic acid ((S)-1-phenyl-propyl)-amide
**8a** 3-(1-tert-Butyl-piperidin-4-ylmethyl)-8-chloro-1-oxo-2-phenyl-1,2-dihydro-isoquinoline-4-carboxylic acid [(S)-cyclopropyl-(3-fluoro-phenyl)-methyl]-amide and pharmaceutically acceptable salts thereof.

Further, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

The compounds of the present invention may have one or more asymmetric centres and it is intended that any optical isomers (i.e. enantiomers or diastereomers), as separated, pure or partially purified optical isomers and any mixtures thereof including racemic mixtures, i.e. a mixture of stereoisomeres, are included within the scope of the invention. In particular, when A represents CH or CR¹, A may be an optical centre giving rise to two optical isomers, an R form and an S form. In one embodiment, the compounds of the present invention have the S form.

In a particular embodiment, the compounds of the present invention have the following absolute configuration around A, A being CH

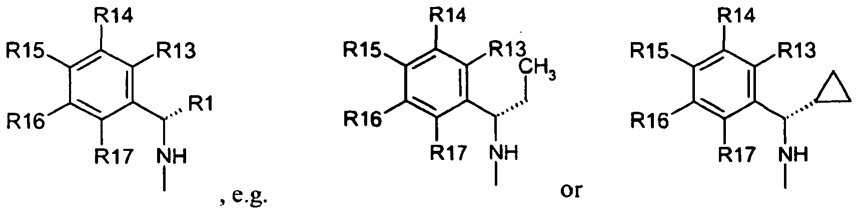

In this context is understood that when specifying the enantiomeric form, then the compound is in enantiomeric excess, c.g. essentially in a pure form. Accordingly, one embodiment of the invention relates to a compound of the invention having an enantiomeric excess of at least 60%, at least 70%, at least 80%, at least 85%, at least 90%, at least 96%, preferably at least 98%.

Racemic forms can be resolved into the optical antipodes by known methods, for example by separation of diastereomeric salts thereof with an optically active acid, and liberating the optically active amine compound by treatment with a base. Another method for resolving racemates into the optical antipodes is based upon chromatography of an optically active matrix. The compounds of the present invention may also be resolved by the formation of diastereomeric derivatives. Additional methods for the revolution of optical isomers, known to those skilled in the art, may be used. Such methods include those discussed by J. Jaques, A. Collet and S. Wilcn in "Enantiomers, Racemates, and Resolutions", John Wiley and Sons, New York (1981). Optically active compounds can also be prepared from optically active starting materials.

Furthermore, when a double bond or a fully or partially saturated ring system is present in the molecule geometric isomers may be formed. It is intended that any geometric isomers, as separated, pure or partially purified geometric isomers or mixtures thereof are included within the scope of the invention. Likewise, molecules having a bond with restricted rotation may form geometric isomers. These are also intended to be included within the scope of the present invention.

Furthermore, some of the compounds of the present invention may exist in different tautomeric forms and it is intended that any tautomeric forms that the compounds are able to form are included within the scope of the present invention.

NK3 receptor antagonists have been implicated in various diseases in addition to psychosis and schizophrenia discussed above. Langlois et al in J.Pharm.Exp.Ther., 299, 712-717, 2001, concludes that NK3 antagonists may be applicable in CNS diseases in general, and in anxiety and depression in particular. Yip et al in Br.J.Phar., 122, 715-722, 1997 further implicates NK3 antagonists in diverse brain functions, such as cortical processing, learning and memory, neuroendocrine and behavioral regulation. Additional studies have shown that NKB and NK3 receptors are involved in pain, and that NK3 antagonists have an antinociceptive and analgesic effect [Fioramonti, Neurogastroenterol.Motil., 15, 363-369, 2003]. Mazelin et al in Life Sci., 63, 293-304, 1998 show that NK3 antagonists have an effect in gut inflammation and concludes that such antagonists may be used in the treatment of irritable bowel syndrome (IBS). In addition, NK3 antagonists have in *in vivo* models been demonstrated to be useful in the treatment of airway related diseases, such as asthma, airway hyperresponsiveness, cough, and bronchorestriction [Daoui, Am.J.Respir.Crit.Care Med., 158, 42-48, 1998]. Maubach et al in Neurosci., 83, 1047-1062, 1998 show that NKB and the NK3 agonist senktide increase the frequency and duration of epileptiform discharges, and thus by inference that NK3 antagonists have a anticonvulsive potential. Finally, Kemel et al in J.Neurosci., 22, 1929-1936, 2002, suggests the use of NK3 antagonists in the treatment of Parkinson's Disease.

Accordingly, clinical, pre-clinical, *in vivo* and *in vitro* studies support that NK3 receptor antagonists are of relevance for the treatment or prevention of various disorders including psychosis, schizophrenia, depression, anxiety, cognitive impairment, obesity, Alzheimer's disease, Parkinson's disease, pain, convulsions, cough, asthma, airway hyperresponsiveness, microvascular hypersensitivity, bronchoconstriction, gut inflammation, and inflammatory bowel syndrome.

Schizophrenia is classified into subgroups. The paranoid type is characterised by delusions and hallucinations and absence of thought disorder, disorganized behavior, and affective flattening. The disorganized type, which is also named 'hebephrenic schizophrenia' in the ICD, in which thought disorder and flat affect are present together. The cataconic type, in which prominent psychomotor disturbances are evident, and symptoms may include catatonic stupor and waxy flexibility. The undifferentiated type in which psychotic symptoms are present but the criteria for paranoid, disorganized, or catatonic types have not been met. The symptoms of schizophrenia normally manifest themselves in three broad categories, i.e. positive, negative and cognitive symptoms. Positive symptoms are those, which represent an "excess" of normal experiences, such as hallucinations and delusions. Negative symptoms are those where the patient suffers from a lack of normal experiences, such as anhedonia and lack of social interaction. The cognitive symptoms relate to cognitive impairment in schizophrenics, such as lack of sustained attention and deficits in decision making. The current antipsychotics are fairly successful in treating the positive symptoms but fare less well for the negative and cognitive symptoms. Contrary to that, NK3 antagonists have been shown clinically to improve on both positive and negative symptoms in schizophrenics [Am.J.Psychiatry, 161, 975-984, 204], and according to the above discussion they are also expected to deliver an effect on the cognitive symptoms.

Cognitive impairment include a decline in cognitive functions or cognitive domains, e.g. working memory, attention and vigilance, verbal learning and memory, visual learning and memory, reasoning and problem solving e.g. executive function, speed of processing and/or social cognition. In particular, cognitive impairment may indicate deficits in attention, disorganized thinking, slow thinking, difficulty in understanding, poor concentration, impairment of problem solving, poor memory, difficulties in expressing thoughts and/or difficulties in integrating thoughts, feelings and behaviour, or difficulties in extinction of irrelevant thoughts.

In one embodiment, the present invention relates to the compounds of the present invention for use in therapy.

In one embodiment, the present invention relates to a compound for use in a method of treating a disease selected from psychosis; schizophrenia; schizophrenoform disorder; schizoaffective disorder; delusional disorder; brief psychotic disorder; shared psychotic disorder; psychotic disorder due to a general medical condition; substance or drug induced psychotic disorder (cocaine, alcohol, amphetamine etc); schizoid personality disorder; schizoptypal personality disorder; psychosis or schizophrenia associated with major depression, bipolar disorder, Alzheimer's disease or Parkinson's disease; major depression; general anxiety disorder; bipolar disorder (maintenance treatment, recurrence prevention and stabilization); mania; hypomania; cognitive impairment; ADHD; obesity; appetite reduction; Alzheimer's disease; Parkinson's disease; pain; convulsions; cough; asthma; airway hyperresponsiveness; microvascular hypersensitivity; bronchoconstriction; chronic obstructive pulmonary disease; urinary incontinence; gut inflammation; and inflammatory bowel syndrome the method comprising the administration of a therapeutically effective amount of a compound of the present invention to a patient in need thereof.

In one embodiment, the present invention relates to a compound for use in a method for the treatment of schizophrenia, the method comprising the administration of a therapeutically effective amount of a compound of the present invention to a patient in need thereof. In particular, said treatment includes the treatment of the positive, negative and/or cognitive symptoms of schizophrenia.

In one embodiment, the present invention relates to a compound for use in a method of treating cognitive impairment, the method comprising the administration of a therapeutically effective amount of a compound of the present invention to a patient in need thereof. In particular, said cognitive impairment is manifested as a decline in working memory, attention and vigilance, verbal learning and memory, visual learning and memory, reasoning and problem solving e.g. executive function, speed of processing and/or social cognition.

The antipsychotic effect of typical and atypical anti-psychotics, in particular D2 antagonists is exerted via an inhibition of the post-synaptic D2 receptors. Pre-synaptic D2 auto-receptors, however, are also affected by the administration of these compounds giving rise to an increase in the dopamine neuron firing rate, which, in fact, counteracts the antipsychotic effects. The increased firing rate continues until the effect of the pre-synaptic auto-receptors is blocked (the depolarization block), typically after approximately 3 weeks of chronic treatment with typical or atypical anti-psychotics. This model explains the up to 3 weeks delay of clinical effect normally seen when D2 antagonist treatment is initiated. NK3 antagonists seem to inhibit the increase in the dopamine neuron firing mediated by the pre-synaptic D2 auto-receptors brought about by D2 antagonists, wherefore the combined administration of NK3 antagonists and D2 antagonists is expected to give rise to a faster onset of the clinical effect. Moreover, D2 antagonists are known to increase prolactin levels, which may give rise to serious side effects, such as osteoporosis. It is known that NK3 agonists give rise to an increase in prolactin from which it may be deduced that a NK3 antagonist will lower an increased, i.e. normalise the prolactin level. A combined use of NK3 antagonists and D2 antagonists may thus address some of the safety aspects associated with D2 antagonists administration. Similarly, NK3 antagonists may be administered together with antagonists/inverse agonists/negative modulators/partial agonists of one or more of the targets dopamine D2 receptor, dopamine D3 receptor, dopamine D4 receptor, phosphodiesterase PDE10, serotonin 5-HT_{1A} receptor, serotonin 5-HT_{2A} receptor, serotonin 5-HT₆ receptor, adrenergic alpha 2 receptor, cannabinoid type 1 receptor, histamine H3 receptor, cyclooxygenases, sodium channels or glycine transporter GlyT1; or with agonists/positive modulators/partial agonists of one or more of the targets serotonin 5-HT_{2C} receptor, KCNQ channels, NMDA receptor, AMPA receptor, nicotinic alpha-7 receptor, muscarinic M I receptor, muscarinic M4 receptor, metabotropic glutamate receptor mGluR2, metabotropic glutamate receptor mGluR5, dopamine D1 receptor or dopamine D5 receptor.

Such combined administration of compounds of the present invention and other anti-psychotic compounds, such as D2 antagonists, D2 partial agonists, PDE10 antagonists, 5-HT_{2A} antagonists, 5-HT₆ antagonists or KCNQ4 antagonists may be sequential or concomitant. Examples of D2 antagonists or partial agonists include haloperidol, chlorpromazine, sulpirid, risperidone, ziprasidon, olanzapine, quetiapin, and clozapine.

In one embodiment, the compound of the present invention is administered in an amount from about 0.001 mg/kg body weight to about 100 mg/kg body weight per day. In particular, daily dosages may be in the range of 0.01 mg/kg body weight to about 50 mg/kg body weight per day. The exact dosages will depend upon the frequency and mode of administration, the sex, the age the weight, and the general condition of the subject to be treated, the nature and the severity of the condition to be treated, any concomitant diseases to be treated, the desired effect of the treatment and other factors known to those skilled in the art.

A typical oral dosage for adults will be in the range of 1-1000 mg/day of a compound of the present invention, such as 1-500 mg/day.

In one embodiment, the present invention relates to the use of the compounds of the present invention in the manufacture of a medicament for the treatment of a disease selected from psychosis; schizophrenia: schizophrenoform disorder; schizoaffective disorder; delusional disorder; brief psychotic disorder; shared psychotic disorder; psychotic disorder due to a general medical condition; substance or drug induced psychotic disorder (cocaine, alcohol, amphetamine etc); schizoid personality disorder; schizoptypal personality disorder: psychosis or schizophrenia associated with major depression, bipolar disorder. Alzheimer's disease or Parkinson's discase; major depression; general anxiety disorder; bipolar disorder (maintenance treatment, recurrence prevention and stabilization); mania; hypomania; cognitive impairment; ADHD: obesity; appetite reduction; Alzheimer's disease; Parkinson's disease; pain; convulsions; cough; asthma: airway hyperresponsiveness; microvascular hypersensitivity: bronchoconstriction: chronic obstructive pulmonary disease; urinary incontinence; gut inflammation; and inflammatory bowel syndrome.

In one embodiment, the present invention relates to the use of a compound of the present invention in the manufacture of a medicament for the treatment of schizophrenia. In particular, said treatment includes the treatment of the positive, negative and/or cognitive symptoms of schizophrenia.

In one embodiment, the present invention relates to the use of a compound of the present invention in the manufacture of a medicament for the treatment of cognitive impairment. In particular, said cognitive impairment is manifested as a decline in working memory, attention and vigilance, verbal learning and memory, visual learning and memory, reasoning and problem solving e.g. executive function, speed of processing and/or social cognition.

In one embodiment, the present invention relates to a compound of the present invention for use in the treatment of a disease selected from psychosis; schizophrenia; schizophrenoform disorder; schizoaffective disorder; delusional disorder; brief psychotic disorder; shared psychotic disorder; psychotic disorder due to a general medical condition; substance or drug induced psychotic disorder (cocaine, alcohol, amphetamine etc); schizoid personality disorder; schizoptypal personality disorder; psychosis or schizophrenia associated with major depression, bipolar disorder, Alzheimer's disease or Parkinson's disease; major depression; general anxiety disorder; bipolar disorder (maintenance treatment, recurrence prevention and stabilization); mania; hypomania; cognitive impairment; ADHD; obesity; appetite reduction; Alzheimer's disease; Parkinson's disease; pain; convulsions; cough; asthma; airway hyperresponsiveness; microvascular hypersensitivity; bronchoconstriction; chronic obstructive pulmonary disease; urinary incontinence; gut inflammation; and inflammatory bowel syndrome.

In one embodiment, the present invention relates to a compound of the present invention for use in the treatment of schizophrenia. In particular, said treatment includes the treatment of the positive, negative and/or cognitive symptoms of schizophrenia.

In one embodiment, the present invention relates to a compound of the present invention for use in the treatment of cognitive impairment. In particular, said cognitive impairment is manifested as a decline in working memory, attention and vigilance, verbal learning and memory, visual learning and memory, reasoning and problem solving e.g. executive function, speed of processing and/or social cognition.

The compounds of the present invention may be administered alone as a pure compound or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for moral administration include solid dosage forms such as capsules, tablets, dragees, pills, lozenges, powders and granules. Where appropriate, they can be prepared with coatings.

Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use.

Other suitable administration forms include suppositories, sprays, ointments, cremes, gels, inhalants, dermal patches, implants, etc.

Conveniently, the compounds of the invention are administered in a unit dosage form containing said compounds in an amount of about 0.1 to 500 mg, such as 10 mg, 50 mg 100 mg, 150 mg, 200 mg or 250 mg of a compound of the present invention.

For parenteral routes such as intravenous, intrathecal, intramuscular and similar administration, typically doses are in the order of about half the dose employed for oral administration.

For parenteral administration, solutions of the compound of the invention in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous medina employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospho lipids, fatty acids, fatty acid amines, polyoxyethylene and water. The pharmaceutical compositions formed by combining the compound of the invention and the pharmaceutical acceptable carriers are then readily administered in a variety of dosage forms suitable for the disclosed routes of administration.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be tablet, e.g. placed in a hard gelatine capsule in powder or pellet form or in the form of a troche or lozenge. The amount of solid carrier may vary but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

Tablets may be prepared by mixing the active ingredient with ordinary adjuvants and/or diluents followed by the compression of the mixture in a conventional tabletting machine. Examples of adjuvants or diluents comprise: Corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.

In one embodiment, the invention relates to a pharmaceutical composition comprising a compound of the present invention together with a second anti-psychotic agent. In one embodiment, said second anti-psychotic agent is selected from antagonists/inverse agonists/negative modulators/partial agonists of the targets dopamine D2 receptor, dopamine D3 receptor, dopamine D4 receptor, phosphodiesterase PDE10, serotonin 5-HT_{1A} receptor, serotonin 5-HT_{2A} receptor, serotonin 5-HT₆ receptor, adrenergic alpha 2 receptor, cannabinoid type 1 receptor, histamine H3 receptor, cyclooxygenases, sodium channels or glycine transporter GlyT1; or from agonists/positive modulators/partial agonists of the targets serotonin 5-HT_{2C} receptor, KCNQ channels, NMDA receptor, AMPA receptor, nicotinic alpha-7 receptor, muscarinic M1 receptor, muscarinic M4 receptor, metabotropic glutamate receptor mGluR2, metabotropic glutamate receptor mGluR5, dopamine D1 receptor or dopamine D5 receptor. In one embodiment, said second anti-psychotic agent is selected from typical anti-psychotics, atypical anti-psychotics, D2 antagonists, partial D2 agonists, PDE10 antagonists, 5-HT_{2A} antagonists, 5-HT₆ antagonists and KCNQ4 antagonists, and in particular atypical anti-psychotics, D2 antagonists, partial D2 agonists. Particular examples of such anti-psychotics include haloperidol, chlorpromazine, sulpirid, risperidone, ziprasidon, olanzapine, quetiapine, and clozapine.

In one embodiment, the invention relates to a pharmaceutical kit comprising a container comprising a compound of the present invention and a separate container comprising an anti-psychotic drug. Typical anti-psychotics, atypical anti-psychotics, D2 antagonists, partial D2 agonists, PDE10 antagonists, 5-HT_{2A} antagonists, 5-HT₆ antagonists and KCNQ4 antagonists, and in particular atypical anti-psychotics, D2 antagonists, partial D2 agonists. Particular examples of such anti-psychotics include halopcridol, chlorpromazine, sulpirid, risperidone, ziprasidon, olanzapine, quetiapine, and clozapine.

All references, including publications, patent applications, and patents, cited herein arc hereby incorporated by reference in their entirety and to the same extent as if each reference were individually and specifically indicated to be incorporated by reference and were set forth in its entirety herein (to the maximum extent permitted by law), regardless of any separately provided incorporation of particular documents made elsewhere herein.

The use of the terms "a" and "an" and "the" and similar referents in the context of describing the invention are to be construed to cover both the singular and the plural, unless otherwise indicated herein or clearly contradicted by context. For example, the phrase "the compound" is to be understood as referring to various "compounds" of the invention or particular described aspect, unless otherwise indicated.

Unless otherwise indicated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or aspect of the invention using terms such as "comprising", "having," "including," or "containing" with reference to an element or elements is intended to provide support for a similar aspect or aspect of the invention that "consists of", "consists essentially or, or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

### SYNTHETIC ROUTES

The compounds of the present invention of the general formula I, wherein R¹-R¹⁷, A, and X arc as defined above can be prepared by the methods outlined in the following reaction schemes and examples. In the described methods it is possible to make use of variants or modifications, which are themselves known to chemists skilled in the art or could be apparent to the person of ordinary skill in this art. Furthermore, other methods for preparing compounds of the invention will be readily apparent to the person skilled in the art in light of the following reaction schemes and examples.

In the intermediate compounds of the general formulae I - XX, R1-R17, A, and X are as defined under formula I.

For compounds, which can exist as a mixture or equilibrium between two or more tautomers, only one tautomer is represented in the schemes, although it may not be the most stable tautomer. For compounds, which can exist in enantiomeric, stereoisomeric or geometric isomeric forms their geometric configuration is specified; otherwise the structure represents a mixture of stereoisomers. Such compounds include, but not limited to 1,3-ketoesters or enamines of the general formula IV and VII, which can exist in equilibrium between keto or enol forms and the latter may also exist in isomeric Z- and E-forms as well-known to chemists skilled in the art. Such compounds also include compounds of the present invention of the general formula I, which may exist as a mixture of atropisomers due to restricted rotation around carbon-carbon single bonds similar to atropisomerism in ortho, ortho'-disubstituted biaryl compounds also well-known to the person skilled in the art.

Starting materials of the general formulae III, VI, and XI are either obtained from commercial sources as summarized in the Table 2 or they can be readily prepared by standard methods or their modifications described in the literature.

2-Bromobenzoic acids of the general formula II are coupled with keto-esters of the general formula III in the presence of a strong base such as sodium hydride and copper or copper salts such as copper (I) bromide in a suitable solvent such as 1,4-dioxane, acetonitrile or an excess of the above keto-esters at suitable temperature such as reflex or at 70°C with the formation of compounds of the general formula IV. Such arylation reaction is well-known in general as copper-catalyzed Ullmann-type coupling reaction (review: S.V. Ley, A.W. Thomas Angew. Chem. Int. Ed. 2003, 42, 5400). Also, in this particular case when the coupling reaction involves activated methylene compounds such as compounds of the general formula IV in the presence of copper or copper salts the reaction is known as the Hurtley reaction (W.R.H. Hurtley J. Chem. Soc. 1929, 1870).

The obtained compounds of the general formula IV are then reacted with anilines of the general formula VI with or without appropriate solvent under the heating conditions with the formation of isoquinolones of the general formula VIII via intermediate formation of enamines of the general formula VII which are usually not separated from the reaction mixture. Alternatively, the enamines of the general formula VII can be obtained from anilines of the general VI in the presence of the appropriate dehydrating agent such as tetraethoxysilane under heating conditions or at ambient temperature in the presence of a catalytic amount of acid such as acetic acid. Then, the cyclisation reaction with the formation of isoquinolones of the general formula VIII can be carried out under the heating conditions as mentioned above or at ambient temperature in the presence of an appropriate coupling reagent such as EDC/HOBT.

Furthermore, isoquinolinones of the general formula VIII can be obtained directly from the starting 2-bromobenzoic acids of the general formula II and deprotonated enamines of the general formula V in a modified one-pot procedure involving Hurtley reaction performed at 70°C with the formation of the compounds of the general formula VII and subsequent cyclisation performed at higher temperature. Enamines of the general formula V are readily available from ketoesters III and anilines VI under conditions described above for preparation of enamines of the general formula VII.

Compounds of the general formula VIII are readily hydrolyzed to acids of the general formula IX under conditions for ester hydrolysis well-known to chemists skilled in the art. Finally, the subsequent coupling with amines (A = C) or hydrazines (A = N) of the general formula XI leads to the formation of the compounds of the invention of the general formula I. Such coupling reactions usually performed via activation of the acid with an appropriate coupling or activation reagent such as but not limited to thionyl chloride with the formation of corresponding acid chloride. Hydrazines of the general formula I where R1 = H can be converted to disubstituted hydrazides of the same general formula where R1 is not hydrogen by acylation, alkylation or arylation reactions with appropriate acylation or alkylation reagents such as but not limited to acid chlorides, carbamoyl chlorides, chloroformates or alkylhalogenides.

Compounds of the general formula I where X is hydrogen can be converted to the compounds of the general formula XII by regioselective bromination reaction in the presence of bromination reagent such as bromine. Then the bromine atom can be substituted by various nucleophiles of the general formula X-H or X⁻ with the formation of the compounds of the invention of the general formula I. Those skilled in the art will readily appreciate that many nitrogen, carbon and sulphur nucleophiles such as but not limited to amines, aromatic amines, amides, heterocycles, alcohols, phenols, cyanides or thiols are commercially or readily available in the neutral or in the deprotonated anionic form required for such transformation.

If necessary, further derivatisation or transformation can be performed in the substituents R4-R12 and X using standard methods of organic synthesis known to the person of ordinary skill in the art.

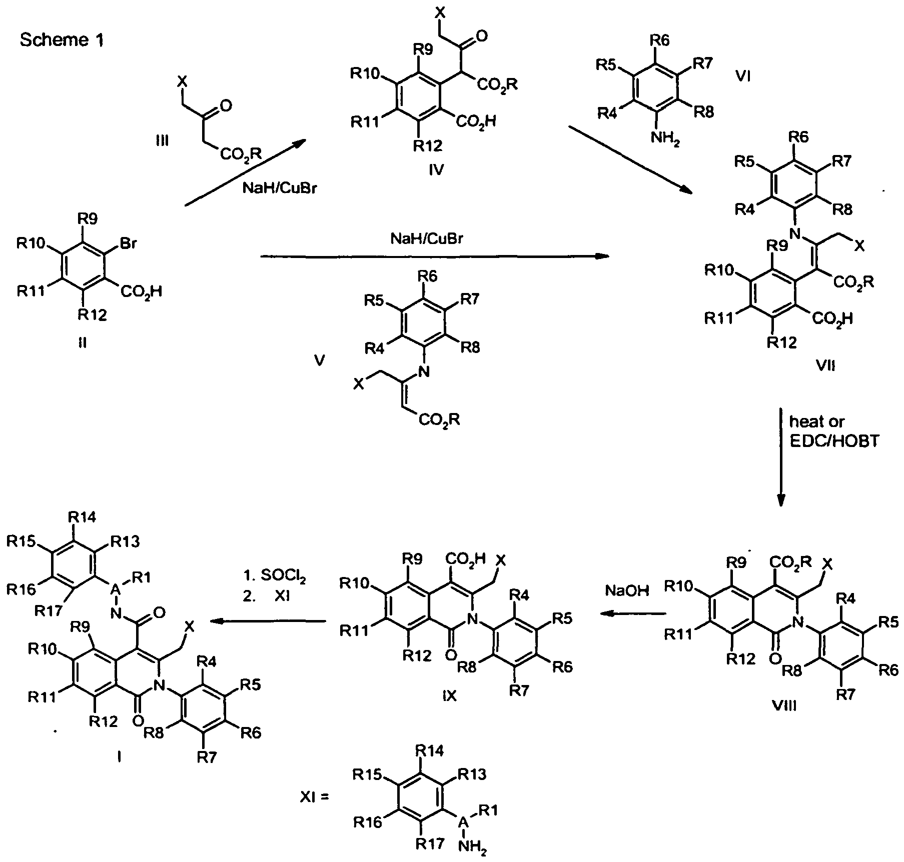

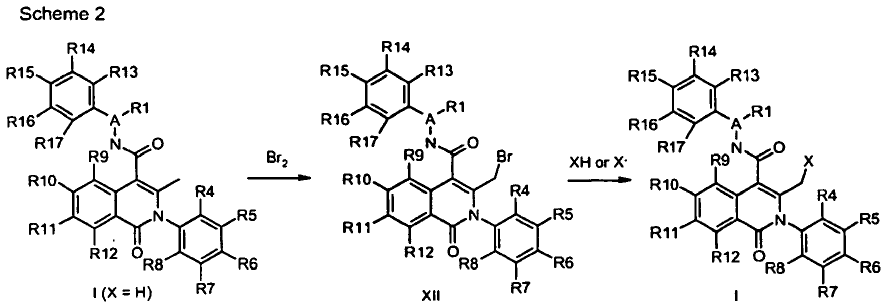

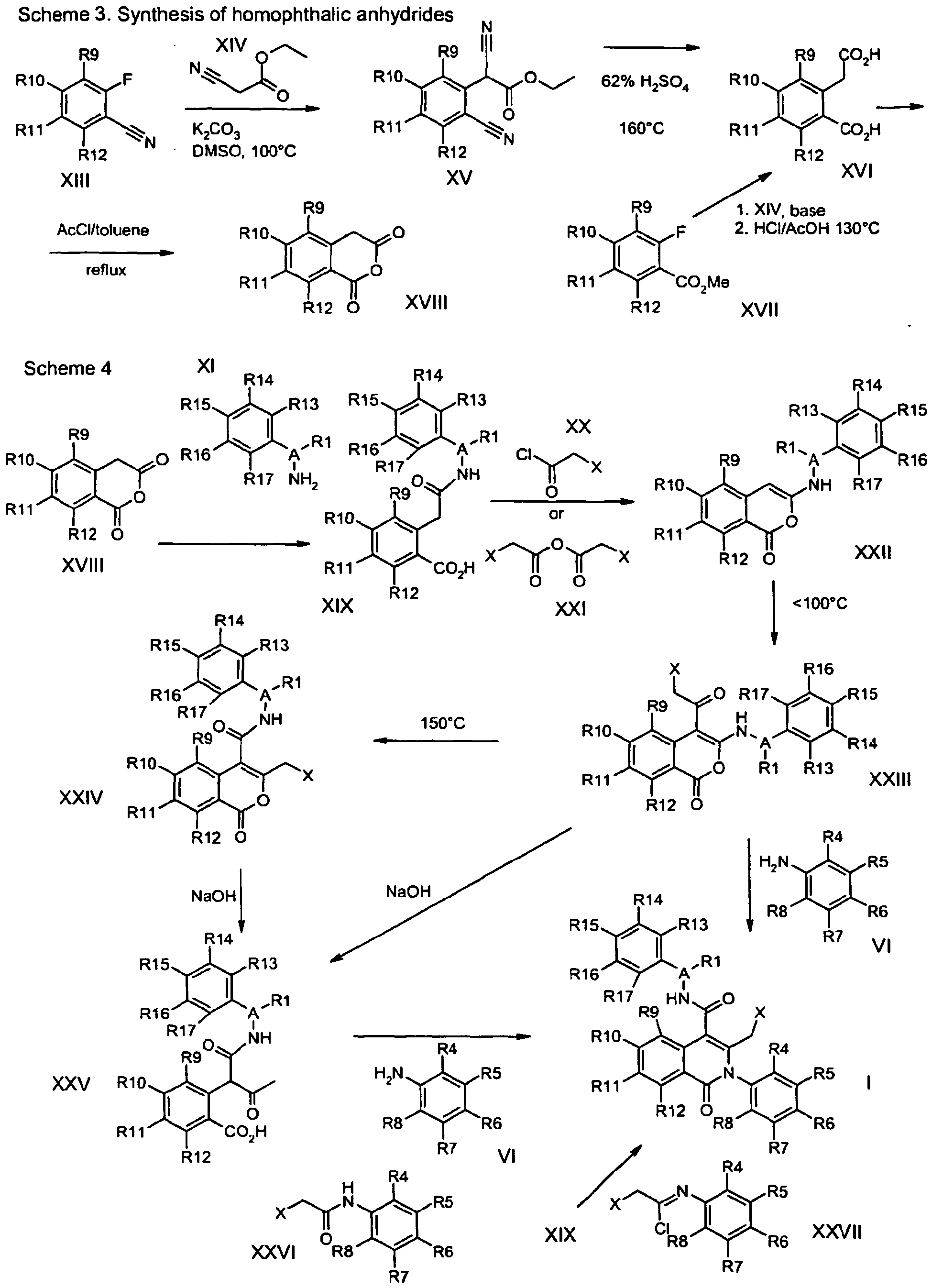

Alternatively, compounds of the general formula I can be prepared starting from substituted homophthalic anhydrides of the general formula XVIII as shown in Scheme 4. Homophthalic anhydrides are either commercially available or can be prepared as shown in scheme 3 starting from corresponding fluorobenzonitriles or fluorobenzoates of the general formula XIII and XVII, respectively. They undergo aromatic nucleophilic substitution reaction with ethyl cyanoacetate XIV in the presence of base such as potassium carbonate or cesium carbonate under heating conditions in appropriate solvent such as dimethylsulfoxide. The coupling products are hydrolyzed in the presence of the strong acids such as sulphuric or hydrochloric acids in water under heating conditions with the formation of diacids of the general formula XVI. Finally, the diacids are converted into the homophthalic anhydrides of the general formule XVIII in the presence of dehydrating agent such as but not limited to acetyl chloride without a solvent or in the appropriate solvent such as toluene under heating conditions such as reflux.

Homophthalic anhydrides of the general formula XVIII can be regioselectively converted into the acid-amides of the general formula XIX at room temperature or under heating conditions in the appropriate solvent such as acetonitrile. Then they are treated with appropriate acid anhydrides or acid chlorides of the general formula XX and XXI, respectively, in the absence or presence of base such as triethyl amine and DMAP in the appropriated solvent, usually in acetonitrile, at room temperature or under mild heating conditions (T < +100°C). This transformation first provides intermediate ketene-aminals of the general formula XXII, which undergo further acylation reaction with the formation of compounds of the general formula XXIII. Further heating at higher temperature such as at +150°C leads to the rearrangement with the formation of amides of the general formula XIV. Both compounds of the general formula XXIII and XXIV can be readily hydrolyzed at ambient temperature with an appropriate base such as sodium hydroxide in aqueous methanol or aqueous tetrahydrofurane as a solvent. The obtained keto-acids of the general formula XXV or ketene-aminals of the general formula XXIII are converted to the final compounds of the invention of the general formula I by condensation with anilines of the general formula VI under the same conditions as described above for condensation with keto-acids of the general formula IV.

Alternatively, acid-amides of the general formula XIX can be converted directly to the compounds of the invention of the general formula I by condensation with appropriate imidoyl chlorides of the general formula XXVII which are readily available from corresponding amide of the general formula XXVI, which are easily prepared by well-known coupling between aniline of the general formula VI and corresponding carboxylic acids or their anhydrides or acid chlorides of the general formula XX and XXI, respectively.

### Examples

Analytical LC-MS, method A (used in most cases unless noted otherwise): data were obtained on a Sciex API 150EX analytical LC/MS system equipped with Applied Biosystems AP1150EX single qaudrupole mass spectrometer and atmospheric pressure photo ionisation (APPI) ion source, Shimadzu LC10ADvp LC pumps (3X), Shimadzu SPD-M20A photodiode array detector, SEDERE Sedex 85 - low temperature Evaporative Light Scattering Detector (ELSD), Shimadzu CBM-20A system controller, Gilson 215 autosampler and Gilson 864 degasser controlled by Analyst Software. Column: 30 X 4.6 mm Waters Symmetry C18 column with 3.5 µm particle size: Injection Volume: 15 µL; Column temperature: 60°C; Solventsystem: A = water/trifluoroacetic acid (100:0.05) and B = water/acetonitrile/trifluoroacetic acid (5:95:0.035); Method: Linear gradient elution with 10% B to 100% B in 2.4 minutes then with 10% B in 0.4 minutes and with a flow rate of 3.3 mL/minute. The retention times (t_{R}) are expressed in minutes based on UV-trace at 254 nm.

Analytical LC-MS, method B: data were obtained on a Sciex API300 analytical LC/MS system equipped with Applied Biosystems API300 triple qaudrupole mass spectrometer with atmospheric pressure photo ionisation (APPI) ion source. Shimadzu LC10ADvp LC pumps (3X), Shimadzu SPD-M20A photodiode array detector, Polymer Labs PL-ELS 2100 - low temperature Evaporative Light Scattering Detector (ELSD), Shimadzu SCL10A VP system controller, Gilson 215 autosampler and Gilson 864 degasser controlled by Analyst Software. Column: Symmetry C18 3.5 µm, 4.6 x 30 mm 30 X 4.6 mm; Injection Volume: 5 µL; Column temperature: 60°C; Solventsystem: A = water/trifluoroacetic acid (100:0.05) and B = water/acetonitrile/trifluoroacetic acid (5:95:0.035); Method: Linear gradient elution with 10% B to 100% B in 1.45 minutes then with 10% B in 0.55 minutes and with a flow rate of 5.5 mL/minute:

| Time, min. | %B |
|---|---|
| 0.00 | 10.0 |
| 1.45 | 100.0 |
| 1.55 | 10.0 |
| 2.0 | 10.0 |

The retention times (t_{R}) are expressed in minutes based on UV-trace at 254 nm.

Preparative LC-MS purification was performed on the same Sciex API 150EX system equipped with Gilson 333 and 334 pumps, Shimadzu LC10ADvp pump, Gilson UV/VIS 155 UV detector, Gilson 233XL autosampler, Gilson FC204 fraction, Gilson 506C system interface, Gilson 864 degasser, DIY flowsplitter (approx. 1:1000), and LC Packings Accurate flowsplitter (1:10.000 @ 140 ml/min). The MS and fraction collector was controlled by Masschrom software (Macintosh PC), the LC system was controlled by Unipoint software. For a small scale (<20 mg) purification fractions were collected in 4 ml vials using Symmetry G18 5 µm, 10 x 50 mm column, injection volume of 0-300 µL, flow rate of 5.7 ml/min and duration of 8 min. Gradient:

| Time, min. | %B |
|---|---|
| 0.00 | 10.0-50.0 (variable, depending on the sample) |
| 7.00 | 100.0 |
| 7.10 | 10.0-50.0 |
| 8.00 | 10.0-50.0 |

¹H N M R spectra were recorded at 500.13 MHz on a Bruker Avance DRX-500 instrument at T=303.3 K. Variable temperature ¹H NMR spectra were recorded at 250 MHz on a Bruker Avance DPX-250 instrument. Deuterated dimethyl sulfoxide (DMSO-d₆, 99.8%D) was used as solvent unless noted otherwise. Tetramethylsilane was used as internal reference standard. Chemical shift values are expressed in ppm-values relative to tetramethylsilane. The following abbreviations or their combinations are used for multiplicity of NMR signals: s = singlet, d = doublet, t = triplet, q = quartet, qui = quintet, h = heptet, dd = double doublet, ddd = double double doublet, dt = double triplet, dq = double quartet, tt = triplet of triplets, m = multiplet and br = broad or broad singlet.

Microwave experiments were performed in scaled process vials or reactors using an Emrys Synthesizer or Emrys Optimizer EXP from Personal Chemistry or a Milestone Microsynth instrument from Milestone. Before sealing the process vial it was flashed with argon. When a reaction was heated in a microwave instrument, it was cooled to 25°C before the next process step.

### Preparation of intermediates

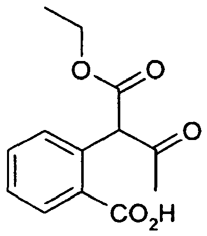

### 2-(1-Ethoxycarbonyl-2-oxo-propyl)-benzoic acid.

To a cold (ice/water bath) stirred mixture of 2-bromobenzoic acid (20.1g, 0.1 mol) and ethyl acetoacetate (13.01 g, 0.1 mol) in 1,4-dioxane (200 ml) sodium hydride (8 g, 0.2 mol, 60% dispersion in mineral oil) was added in small portions. After addition the cold bath was removed and the reaction mixture was allowed to stir at ambient temperature for 10 min. Copper (I) bromide (15.2 g. 0.106 mol) was added in portions and the obtained mixture was heated at reflux for 1 h. It was allowed to cool and stirred overnight at ambient temperature. The obtained green suspension was quenched with ethyl acetate (200 ml) and ice (300 g) and acidified with concentrated HCl (50 ml). The organic phase was washed with aq. 2 M HCl (2x 100 ml) and brine, dried (Na₂SO₄) and evaporated in vacuo. The remaining mineral oil was removed by decantation after shaking with heptane (3x100 ml) and evaporation. Yield ca. 20g, pale-yellow oil. The crude product was used in the next step without further purification. LC-MS (m/z) 205 ([M-CO₂]⁺); t_{R} = 0.91. ¹H NMR (500 MHz, DMSO-d₆): mixture of at least 3 tautomers.

The following compounds were prepared analogously from corresponding 2-bromobenzoic acids and used in the next step without further purification and characterisation unless noted otherwise: 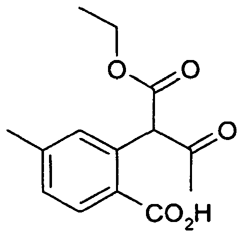 *2-(1-Ethoxycarbonyl-2-oxo-propyl)-4-methyl-benzoic acid.*

LC-MS (m/z) 247.2 ([MH-H₂O]⁺); t_{R} = 1.05. 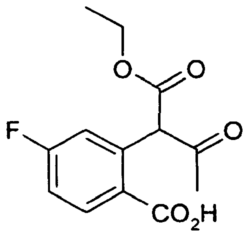 *2-(1-Ethoxycarbonyl-2-oxo-propyl)-4-fluoro-benzoic acid.* 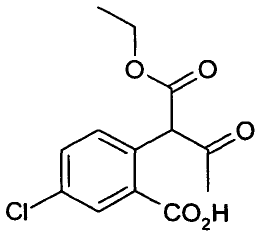 *5-Chloro-2-(1-ethoxycarbonyl-2-oxo-propyl)-benzoic acid.* 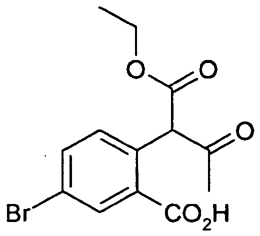 *5-Bromo-2-(1-ethoxycarbonyl-2-oxo-propyl)-benzoic acid.* 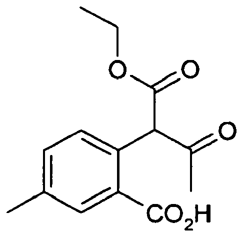 *2-(1-Ethoxycarbonyl-2-oxo-propyl)-5-methyl-benzoic acid.* 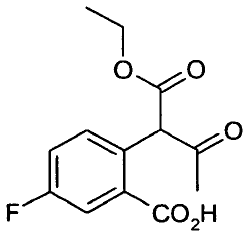 *2-(1-Ethoxycarbonyl-2-oxo-propyl)-5-fluoro-benzoic acid acid.* 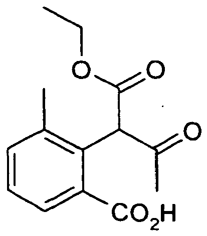 *2-(1-Ethoxycarbonyl-2-oxo-propyl)-3-methyl-benzoic acid.* 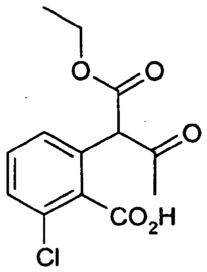 *2-Chloro-6-(1-ethoxycarbonyl-2-oxo-propyl)-benzoic acid.* 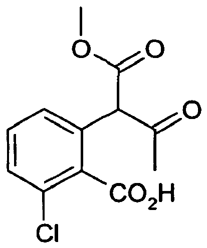 *2-Chloro-6-(1-methoxycarbonyl-2-oxo-propyl)-benzoic acid.*

To a stirred mixture of 2-bromo-6-chlorobenzoic acid (20 g, 85 mmol), methylacetoacetate (310 ml, excess), and copper (I) bromide 12.2 g, 85 mmol) sodium hydride (8.5 g, 212 mmol, 60% in oil) was added by portions and then heated at 70°C for 16 hours. The resulting mixture was diluted with water (600 ml) and extracted with diethyl ether (3 x 500 ml). Aqueous phase was acidified with 2M HCl and extracted with ethyl acetate (2 x 800 ml). The combined organic solution was washed with brine (100 ml), dried (Na₂SO₄) and evaporated. The obtained oil was treated with a hot mixture of ethyl acetate (20 ml) and heptane (200 ml) and decanted for 2 times, dried in vacuo to give 8 g of yellow-brown solid, yield 34%. ¹H NMR (500 MHz, DMSO-d₆): 1.73 (s, 3H), 3.6 (s, 3H), 7.24 (d, 1H), 7.44 (t, 2H), 7.5 (d, 1H), 12.85 (s, 1H, OH of enol form), 13.4 (br, 1H, CO₂H). 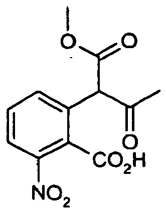 *2-(1-Methoxycarbonyl-2-oxo-propyl)-6-nitro-benzoic acid.* 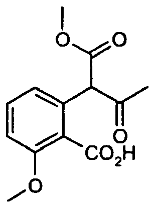 2-Methoxy-6-(1-methoxycarbonyl-2-oxo-propyl)-benzoic acid. 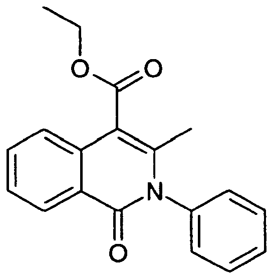 *3-Methyl-1-oxo-2-phenyl-1,2-dihydro-isoquinoline-4-carboxylic acid ethyl ester.*

A mixture of 2-(1-ethoxycarbonyl-2-oxo-propyl)-benzoic acid (7.6 g, 30.4 mmol) and aniline (10 ml, 108 mmol) was flushed with argon, sealed in the Emrys process vial and heated at 150°C for 15 min under microwave irradiation. The volatiles were removed in vacuo and the obtained residue was partitioned between ethyl acetate (100 ml) and 2M HCl (100 ml). The organic layer was washed with brine (2 x 20 ml), dried (Na₂SO₄) and evaporated to give 8.7 g of brown oil that solidified. The obtained crude product was of ca. 90% purity according to ¹H NMR and can be used in the next step without further purification. Alternatively, the crude product was dissolved in hot ethyl acetate (20 ml), precipitated with heptane (60 ml), allowed to cool, filtered and washed with diethyl ether to give 4.2 g of a pale-brown crystalline solid, yield 45%. LC-MS (m/z) 308.2 (MH⁺); t_{R} = 1.43. ¹H NMR (500 MHz, DMSO-d₆): 1.34 (t, 3H), 1.97 (s, 3H), 4.41 (q, 2H), 7.38 (d, 2H), 7.51 (t, 1H), 7.56 (t (overlapping m), 3H), 7.62 (d, I H), 7.8 (t, 1H), 8.22 (d, 1H).

The following compounds were prepared analogously from corresponding crude 2-(1-ethoxycarbonyl-2-oxo-propyl)-4-methylbenzoic acids and corresponding anilines. 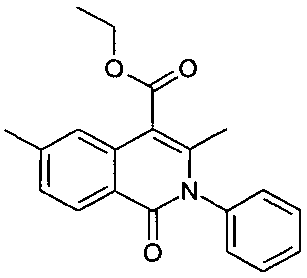 *3,6-Dimethyl-1-oxo-2-phenyl-1,2-dihydro-isoquinoline-4-carboxylic acid ethyl ester.*

The crude product was used in the next step without crystallization. LC-MS (m/z) 322.1 (MH⁻); t_{R} = 1.53. ¹H NMR (500 MHz, DMSO-d₆): 1.34 (t, 3H), 1.96 (s, 3H), 2.47 (s, 3H), 4.41 (q, 2H), 7.28 (t, 1H), 7.37 (m, 3H), 7.50 (d, 1H), 7.56 (m, 2H), 8.11 (d, 1H). 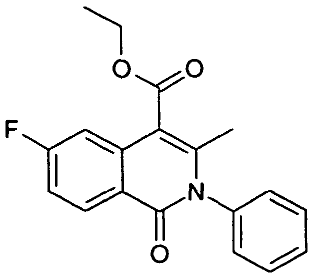 *6-Fluoro-3-methyl-1-oxo-2-phenyl-1,2-dihydro-isoquinoline-4-carboxylic acid ethyl ester.*

The crude product was used in the next step without crystallization. LC-MS (m/z) 326.3 (MH⁻); t_{R} = 1.54. ¹H NMR (500 MHz, DMSO-d₆): 1.33 (t, 3H), 2.0 (s, 3H), 4.41 (q, 2H), 7.28 (t, 1H), 7.39 (d, 2H), 7.42 (m, 1H), 7.51 (t, 1H), 7.57 (t, 2H), 8.29 (dd, 1H). 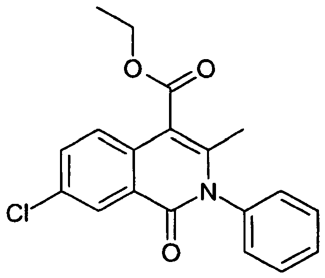 *7-Chloro-3-metl-1-oxo-2-phenyl-1,2-dihydro-isoquinoline-4-carboxylic acid ethyl ester.*

The crude product was used in the next step without crystallization. LC-MS (m/z) 342.0 (MH⁺); t_{R} = 1.67. ¹H NMR (500 MHz, DMSO-d₆): 1.33 (t, 3H), 1.98 (s, 3H), 4.41 (q, 2H), 7.4 (d, 2H), 7.51 (t, 1H), 7.57 (t, 2H), 7.7 (d, 1H), 7.85 (dd, 1H), 8.15 (d, 1H). 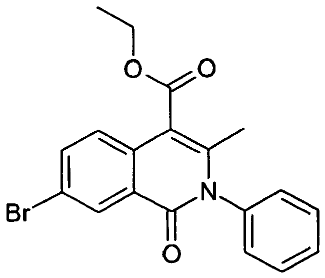 *7-Bromo-3-methyl-1-oxo-2-phenyl-1,2-dihydro-isoquinoline-4-carboxylic acid ethyl ester.*

The crude product was used in the next step without crystallization. LC-MS (m/z) 386.1 (MH⁺, ⁷⁹Sr); t_{R} =1.71. ¹H NMR (500 MHz, DMSO-d₆): 1.33 (t, 3H), 1.98 (s, 3H), 4.4 (q, 2H), 7.39 (d, 2H), 7.51 (t, 1H), 7.57 (t, 2H), 7.62 (d, 1H), 7.96 (dd, 1H), 8.29 (d, I H). 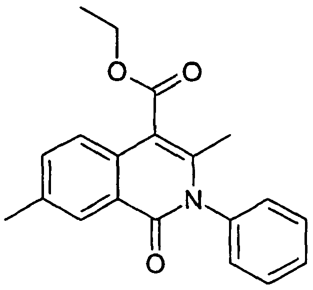 *3,7-Dimethyl-1-oxo-2-phenyl-1,2-dihydro-isoquinoline-4-carboxylic acid ethyl ester.*

The crude product as a brown oil was used in the next step without purification. LC-MS (m/z) 322.2 (MH⁺); t_{R} = 1.59. 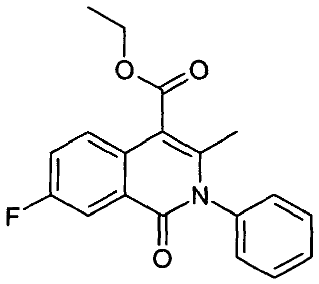 *7-Fluoro-3-methyl-1-oxo-2-phenyl-1,2-dihydro-isoquinoline-4-carboxylic acid ethyl ester.*

The crude product as a brown oil was used in the next step without purification. LC-MS (m/z) 326.1 (MH⁻): t_{R} = 0.79 (method B). 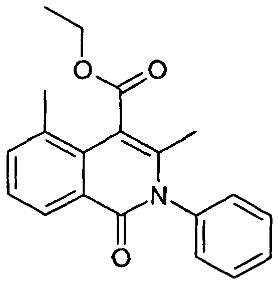 *3,5-Dimethyl-1-oxo-2-phenyl-1,2-dihydro-isoquinoline-4-carboxylic acid ethyl ester.*

The crude product was used in the next step without purification. LC-MS (m/z) 322.1 (MH⁺); t_{R} = 1.63. 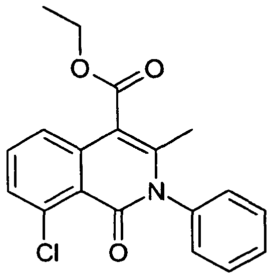 *8-Chloro-3-methyl-1-oxo-2-phenyl-1,2-dihydro-isoquinoline-4-carboxylic acid ethyl ester.*

The crude product was used in the next step without purification. LC-MS (m/z) 342.0 (MH⁺); t_{R} = 1.55. 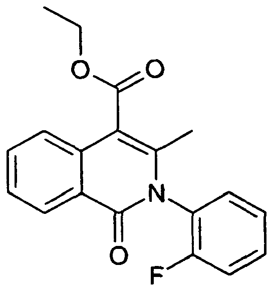 *2-(2-Fluoro-phenyl)-3-methyl-1-oxo-1,2-dihydro-isoquinoline-4-carboxylic acid ethyl ester.*

LC-MS (m/z) 326.5 (MH⁺); t_{R} = 1.46. 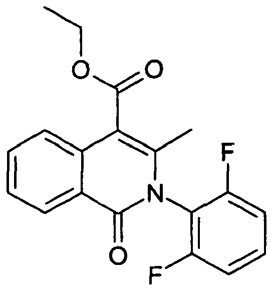 *2-(2,6-Difluoro-phenyl)-3-methyl-1-oxo-1,2-dihydro-isoquinoline-4-carboxylic acid ethyl ester.*

The crude product was used in the next step without purification and characterization. 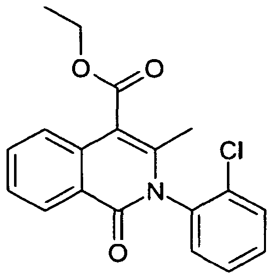 *2-(2-Chloro-phenyl)-3-methyl-1-oxo-1,2-dihydro-isoquinoline-4-carboxylic acid ethyl ester.*

The crude product was used in the next step without purification and characterization. 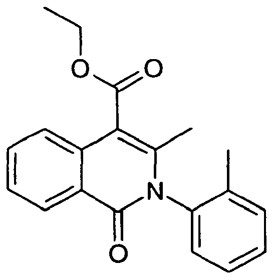 *3-Methyl-1-oxo-2-o-tolyl-1,2-dihydro-isoquinoline-4-carboxylic acid ethyl ester.*

The crude product was used in the next step without purification and characterization. 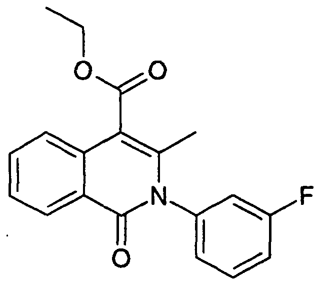 *2-(3-Fluoro-phenyl)-3-methyl-1-oxo-1,2-dihydro-isoquinoline-4-carboxylic acid ethyl ester.*

LC-MS (m/z) 326.3 (MH⁺); t_{R} = 1.47. 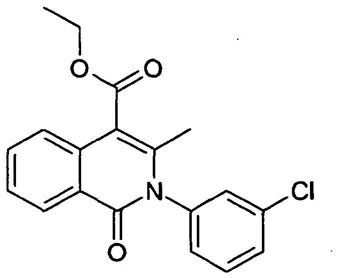 *2-(3-Chloro-phenyl)-3-methyl-1-oxo-1,2-dihydro-isoquinoline-4-carboxylic acid ethyl ester.*

LC-MS (m/z) 342.1 (MH⁺); t_{R} = 1.61. 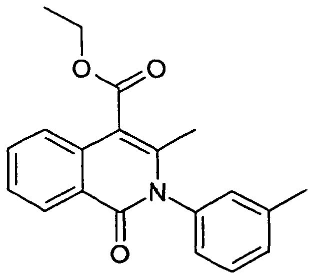 *3-Methyl-1-oxo-2-m-tolyl-1,2-dihydro-isoquinoline-4-carboxylic acid ethyl ester.*

LC-MS (m/z) 322.1 (MH⁺); t_{R} = 1.57. 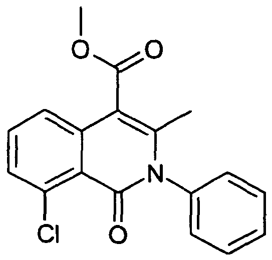 *8-Chloro-3-methyl-1-oxo-2-phenyl-1,2-dihydro-isoquinoline-4-carboxylic acid methyl ester.*

A mixture of 2-chloro-6-(1-methoxycarbonyl-2-oxo-propyl)-benzoic acid (4 g, 15 mmol), aniline (1.369 ml, 15 mmol) and tetraethoxysilane (6.65 ml, 30 mmol) in methanol (20 ml) was sealed and heated under microwave irradiation at +150°C for 20 min. The obtained solution was diluted with water (150 ml) and extracted with ethyl acetate (2x300 ml). The combined organic solution was dried (MgSO₄) and evaporated. The obtained crude enamine (2-chloro-6-(1-methoxycarbonyl-2-phcnylamino-propenyl)-benzoic acid) was dissolved in dimethylformamide (80 ml) followed by addition of 1-ethyl-3-(3-dimethylaminopropyl)carbodiimide hydrochloride (EDC, 4.3 g, 22.5 mmol) and 1-hydroxybenzotriazole (HOBT, 3.04 g, 22.5 mmol). It was stirred for 16 hours and partitioned between water (250 ml), diethyl ether (250 ml) and ethyl acetate (150 ml). The organic phase was washed with 0.5 M NaOH (2x 100 ml), brine (2x200 ml), dried (MgSO₄) and evaporated to give 4.25 g of brown oil, which was used in the next step without further purification. Yield 87%. LC-MS (m/z) 328.0 (MH⁺); t_{R} = 1.36. ¹H NMR (500 MHz, CDCl₃): 2.02 (s, 3H), 3.98 (s, 3H), 7.23 (d, 2H), 7.44-7.55 (m, 6H). 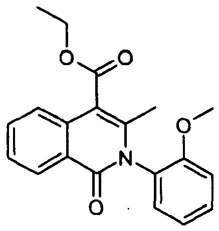 *2-(2-Methoxy-phenyl)-3-methyl-1-oxo-1,2-dihydro-isoquinoline-4-carboxylic acid ethyl ester.*

The title compound was used in the next step without purification. LC-MS (m/z) 338.3 (MH⁺); t_{R} = 1.38. 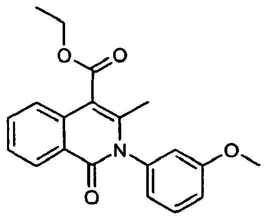 *2-(3-Methoxy-phenyl)-3--methyl-1-oxo-1,2-dihydro-isoquinoline-4-carboxylic acid ethyl ester.*

The title compound was used in the next step without purification. LC-MS (m/z) 338.5 (MH⁻): t_{R} = 1.41. 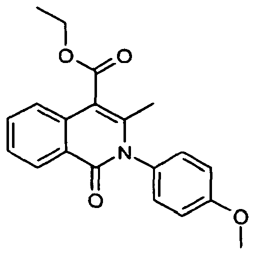 *2-(4-Methoxy-phenyl)-3-methyl-1-oxo-1,2-dihydro-isoquinoline-4-carboxylic acid ethyl ester.*

The title compound was used in the next step without purification. LC-MS (m/z) 338.5 (MH⁺); t_{R} = 1.4. 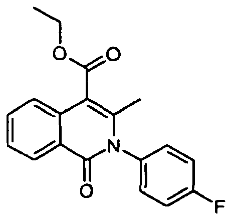 *2-(4-Fluoro-phenyl)-3-methyl-l-oxo-1,2-dihydro-isoquinoline-4-carboxylic acid ethyl ester.*

The title compound was used in the next step without purification. LC-MS (m/z) 326.2 (MH⁺); t_{R} = 1.41. 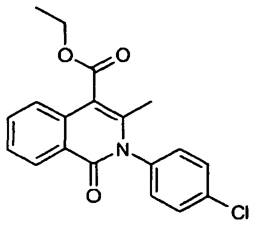 *2-(4-Chloro-phenyl)-3-methyl-1-oxo-1,2-dihydro-isoquinoline-4-carboxylic acid ethyl ester.*

The title compound was used in the next step without purification. LC-MS (m/z) 342.2 (MH⁺); t_{R} = 1.53. 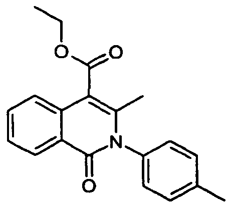 *3-Methyl-1-oxo-2-p-tolyl-1,2-dihydro-isoquinoline-4-carboxylic acid ethyl ester.*

The title compound was used in the next step without purification. LC-MS (m/z) 321.7 (MH⁺); t_{R} = 1.51. 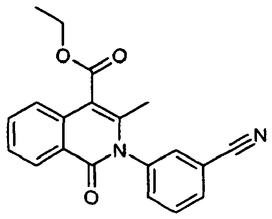 *2-(3-Cyano-phenyl)-3-methyl-1-oxo-1,2-dihydro-isoquinoline-4-carboxylic acid ethyl ester.*

The title compound was used in the next step without purification. LC-MS (m/z) 333.0 (MH⁺); t_{R} = 1.3. 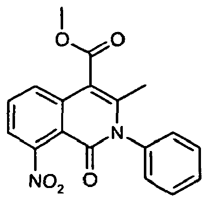 *3-Methyl-8-nitro-1-oxo-2-phenyl-1,2-dihydro-isoquinoline-4-carboxylic acid methyl ester.*

The title compound was used in the next step without purification. LC-MS (m/z) 339.3 (MH⁺); t_{R} = 1.26. 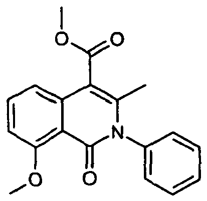 *8-Methoxy-3-methyl-1-oxo-2-phenyl-1,2-dihydro-isoquinoline-4-carboxylic acid methyl ester.*

The title compound was used in the next step without purification. LC-MS (m/z) 324.3 (MH⁺); t_{R} = 1.12. 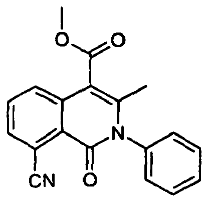 *8-Cyano-3-methyl-1-oxo-2-phenyl-1,2-dihydro-isoquinoline-4-carboxylic acid methyl ester.*

A suspension of 3-methyl-8-nitro-1-oxo-2-phenyl-1,2-dihydro-isoquinoline-4-carboxylic acid methyl ester (320 mg, 0.95 mmol) and Zn (898 mg, 13.8 mmol) in THF (4.5 ml) and 2M aq. HCl (4.5 ml) was stirred for 10 min, filtered and partitioned between ethyl acetate (3x 150 ml) and sat. aq. NaHCO₃ (100 ml). The combined organic solution was dried (MgSO₄) and evaporated to give 8-amino-3-methyl-1-oxo-2-phenyl-1,2-dihydro-isoquinoline-4-carboxylic acid methyl ester as a dark oil (200 mg, 0.645 mmol, 69% yield. LC-MS (m/z) 309.4 (MH⁺); t_{R} = 1.2. It was dissolved in conc. aq. HCl (0.4 ml) and H₂O (3.25 ml). To the obtained solution NaNO₂ (46.3 mg, 0.645 mmol, in 0.12 ml H₂O) was added dropwise at 0°C. After 15 min it was neutralized (pH = 6-7) with sat. aq. NaHCO₃ to give diazonium salt solution. To a solution of KCN (202 mg, 3 mmol) in water (0.4 ml) solution of CuSO₄x5H₂O (197 mg, 0.774 mmol) in water (0.8 ml) was added dropwise at 0°C. After addition completion benzene (2 ml) was added and the mixture was warmed to +60°C. To this mixture diazonium salt solution was added dropwise during 15 min then kept at 70°C for 85 min. It was allowed to cool to r.t., diluted with ethyl acetate (10 ml) and filtered via plug of cellite. The organic layer was washed with brine (10 ml), dried (MgSO₄) and evaporated to give 97.9 mg of the title compound (48% yield). LC-MS (m/z) 319.2 (MH⁺); t_{R} = 1.15. 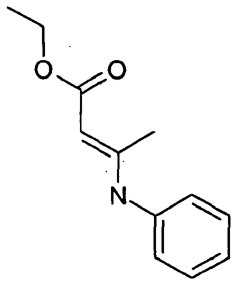 *3-Phenylamino-but-2-enoic acid ethyl ester.*

The compound was prepared according to the described procedure (Q. Dai, W. Yang, and X. Zhang Org. Letters 2005, 5343).

¹H NMR (500 MHz, DMSO-d₆): 1.19 (t, 3H), 2.01 (s, 3H), 4.06 (q, 2H), 4.7 (s, 1H), 7.17 (overlapping t, 1H), 7.18 (overlapping d, 2H), 7.36 (t, 2H), 10.36 (s, 1H). 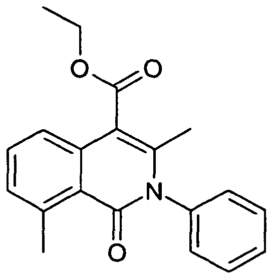 *3,8-Dimethyl-1-oxo-2-phenyl-1,2-dihydro-isoquinoline-4-carboxylic acid ethyl ester.*

To a cold (icc/water bath) solution of 2-bromo-6-methyl-benzoic acid (650 mg, 3.023 mmol) and 3-phenylamino-but-2-enoic acid ethyl ester (750 mg, 3.65 mmol) in acetonitrile (10 ml), sodium hydride (267 mg, 6.67 mmol, 60% dispersion in mineral oil) was added in portions. The cold bath was removed and the mixture was sonicated at ambient temperature until gas evolution ceased (10 min). Copper (I) bromide was added (957 mg, 6.67 mmol) and the obtained suspension was flushed with argon, sealed and heated under microwave irradiation at +80°C for 30 min. LC-MS indicated a full conversion to 2-(1-ethoxycarbonyl-2-phenylamino-propenyl)-6-methyl-benzoic acid (LC-MS (m/z) 340.4 (MH⁻): t_{R} = 1.59. The obtained suspension was heated at +160°C under microwave irradiation for 30 min. It was diluted with ethyl acetate (50 ml) and filtered. The obtained organic solution was washed with 1M HCl (3 x 50 ml), water (2 x 10 ml) and sat. aq. NaHCO₃ solution (30 ml), absorbed on SiO₂ (3 g) and flash chromatographed on SiO₂ (50 g, gradient heptane - 30% ethyl acetate in heptane) to give 217 mg of yellow oil. Yield 22%. LC-MS (m/z) 322.2 (MH⁺); t_{R} = 1.62. ¹H NMR (500 MHz, DMSO-d₆): 1.32 (t, 3H), 1.91 (s, 3H), 2.74 (s, 3H), 4.39 (q, 2H), 7.3 (d, 1H), 7.36 (overlapping m, 3H), 7.49 (m, 1H), 7.55 (t, 2H), 7.61 (t, 1H). 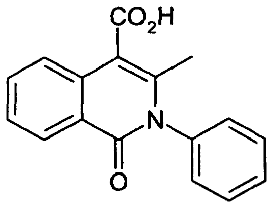 *3-Methyl-1-oxo-2-phenyl-1,2-dihydro-isoquinoline-4-carboxylic acid.*
3-Methyl-1-oxo-2-phenyl-1,2-dihydro-isoquinoline-4-carboxylic acid ethyl ester (4.2 g, 13.7 mmol) was dissolved in 100 ml of hot methanol and a solution of NaOH (6 g) in water (20 ml) was added. The reaction mixture was heated at reflux for 2 hrs, diluted with water (200 ml) and stirred overnight at ambient temperature. Organic volatiles were removed under reduced pressure and the obtained aqueous solution was extracted with ethyl acetate (2 x 50 ml). The aqueous solution was poured into ice/2M HCl (150 ml). The obtained suspension was sonicated, and the product was separated by filtration, washed with water and dried in vacuo to give 2.9 g of a colourless solid, yield 76%. LC-MS (m/z) 280.2 (MH⁺); t_{R} = 0.9. ¹H NMR (500 MHz, DMSO-d₆): 2.01 (s, 3H), 7.36 (d, 2H), 7.50 (t, 1H), 7.56 (q (overlapping m), 3H), 7.72 (d, 1H), 7.81 (t, 1H), 8.22 (d, 1H), 13.5 (br, 1H).

The following acids were prepared analogously from corresponding ethyl esters: 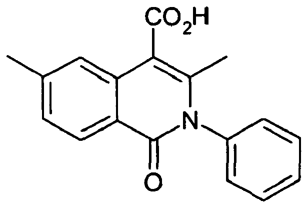 *3,6-Dimethyl-1-oxo-2-phenyl-1,2-dihydro-isoquinoline-4-carboxylic acid.*

LC-MS (m/z) 293.9 (MH⁺); t_{R} = 0.99. ¹H NMR (500 MHz, DMSO-d₆): 2.0 (s, 3H), 2.47 (s, 3H), 7.34 (d, 2H), 7.38 (d, 1H), 7.47 (s, 1H), 7.50 (d, 1H), 7.56 (t, 2H), 8.11 (d, 1H), 13.4 (br, 1H). 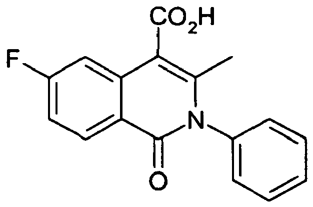 *6-Fluoro-3-methyl-1-oxo-2-phenyl-1,2-dihydro-isoquinoline-4-carboxylic acid.*

LC-MS (m/z) 298.4 (MH⁺); t_{R} = 0.99. ¹H NMR (500 MHz, DMSO-d₆): 2.05 (s, 3H), 7.37 (d, 2H), 7.41 (dt, 1H), 7.48 (dd, 1H), 7.51 (t, 1H), 7.57 (t, 2H), 8.28 (dd, 1H), 13.6 (b, 1H). 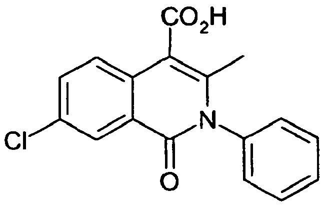 *7-Chloro-3-methyl-1-oxo-2-phenyl-1,2-dihydro-isoquinoline-4-carboxylic acid.*

The crude product was used in the next step without crystallization. LC-MS (m/z) 314.2 (MH⁻): t_{R} = 1.13. ¹H NMR (500 MHz, DMSO-d₆): ¹H NMR (500 MHz, DMSO-d₆): 2.03 (s, 3H), 7.38 (d, 2H), 7.51 (t, 1H), 7.57 (t, 2H), 7.79 (d, 1H), 7.86 (dd, 1H), 8.15(d, 1H), 13.55 (b, 1H). 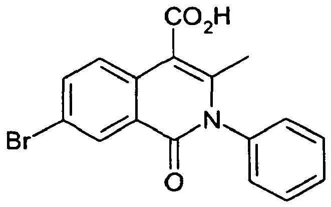 *7-Bromo-3-methyl-1-oxo-2-phenyl-1,2-dihydro-isoquinoline-4-carboxylic acid.*

LC-MS (m/z) 358.1 (MH⁺, ⁷⁹Br); t_{R} = 1.2. ¹H NMR (500 MHz, DMSO-d₆): 2.02 (s, 3H), 7.38 (d, 2H), 7.51 (t, 1H), 7.57 (t, 2H), 7.72 (d, 1H), 7.97 (dd, 1H), 8.29 (d, 1H), 13.55 (b, I H). 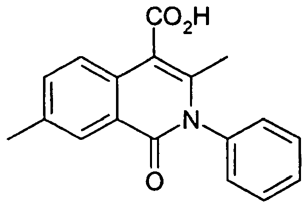 *3,7-Dimethyl-1-oxo-2-phenyl-1,2-dihydro-isoquinoline-4-carboxylic acid.*

LC-MS (m/z) 293.9 (MH⁺); t_{R} = 1.02. ¹H NMR (500 MHz, DMSO-d₆): 2.0 (s, 3H), 2.45 (s, 3H), 7.34 (d, 2H), 7.5 (t, 1H), 7.56 (t, 2H), 7.63 (m, 2H), 8.02 (s, 1H), 13.38 (br, I H). 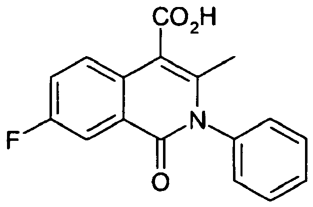 *7-Fluoro-3-methyl-1-oxo-2 phenyl-1,2-dihydro-isoquinoline-4-carboxylic acid.*

LC-MS (m/z) 298.2 (MH⁺); t_{R} = 1.01. ¹H NMR (500 MHz, DMSO-d₆): 2.02 (s, 3H), 7.37 (d, 2H), 7.51 (t, 1H), 7.57 (t, 2H), 7.71 (dt, 1H), 7.83 (dd, 1H), 7.88 (dd, 1H), 13.54 (br. 1H). 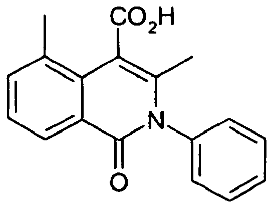 *3,5-Dimethyl-1-oxo-2-phenyl-1,2-dihydro-isoquinoline-4-carboxylic acid.*

LC-MS (m/z) 294.1 (MH⁺); t_{R} = 1.09. ¹H NMR (500 MHz, DMSO-d₆): 1.95 (s, 3H), 2.74 (s, 3H), 7.27-7.64 (m, 8H), 13.35 (b, 1H). 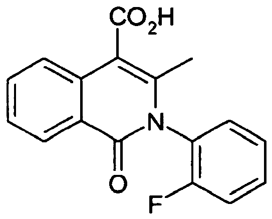 *2-(2-Fluoro-phenyl)-3-methyl-1-oxo-1,2-dihydro-isoquinoline-4-carboxylic acid.*

LC-MS (m/z) 298.4 (MH⁺); t_{R} = 0.83. 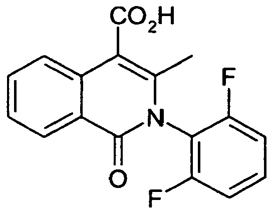 *2-(2,6-Difluoro-phenyl)-3-methyl-1-oxo-1,2-dihydro-isoquinoline-4-carboxylic acid.*

LC-MS (m/z) 316.2 (MH⁺); t_{R} = 1.03. 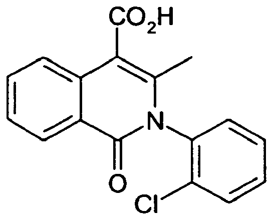 *2-(2-Chloro-phenyl)-3-metlryl-1-oxo-1,2-dilydro-isoquinolirre-4-carboxylic acid.*

LC-MS (m/z) 314.2 (MH⁺); t_{R} = 1.0. 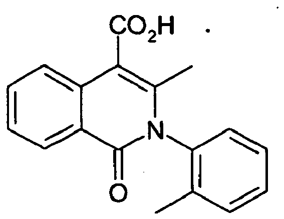 *3-Methyl-1-oxo-2-o-tolyl-1,2-dihydro-isoquinoline-4-carboxylic acid.*

LC-MS (m/z) 294.1 (MH⁺); t_{R} = 0.99. 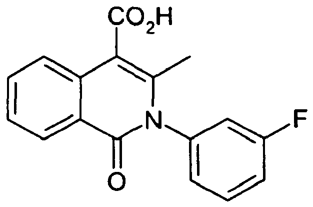 *2-(3-Fluoro-phenyl)-3-methyl-1-oxo-1,2-dihydro-isoquinoline-4-carboxylic acid.*

LC-MS (m/z) 298.4 (MH⁺); t_{R} = 0.96. ¹H NMR (250 MHz, DMSO-d₆): 2.02 (s, 3H), 7.24 (d, 1H), 7.32-7.42 (m, 2H), 7.5-7.65 (m, 2H), 7.71 (d, 1H), 7.81 (m, 1H), 8.21 (d, 1H), 13.49 (br, 1H). 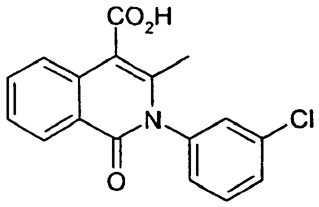 *2-(3-Chloro-phenyl)-3-methyl-1-oxo-1,2-dihydro-isoquinoline-4-carboxylic acid.*

LC-MS (m/z) 314.2 (MH⁻); t_{R} = 1.09. ¹H NMR (500 MHz, DMSO-d₆): 2.04 (s, 3H), 7.39(t, 1H), 7.56(t, 1H), 7.58-7.62 (m, 3H), 7.72 (d, 1H), 7.81 (t, 1H), 8.21 (d, I H), 13.5 (br, 1H). 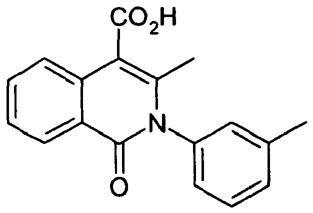 *3-Methyl-1-oxo-2-m-tolyl-1,2-dihydro-isoquinoline-4-carboxylic acid.*

LC-MS (m/z) 293.9 (MH⁺); t_{R} = 1.03. ¹H NMR (250 MHz, DMSO-d₆): 2.02 (s, 3H), 2.38 (s, 3H), 7.14 (overlapping d, 1H), 7.16 (overlapping s, 1H), 7.31 (d, 1H), 7.44 (t, 1H), 7.54 (t, 1H), 7.71 (d, 1H), 7.8 (t, 1H), 8.21 (d, 1H), 13.46 (br, 1H). 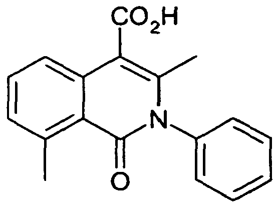 *3,8-Dimethyl-1-oxo-2 phenyl-1,2-dihydro-isoquinoline-4-carboxylic acid.*

A solution of 3,8-dimethyl-1-oxo-2-phenyl-1,2-dihydro-isoquinoline-4-carboxylic acid ethyl ester (200 mg, 0.62 mmol) in DMSO (3 ml) and aqueous IN NaOH (2 ml, 2 mmol) was heated under microwave irradiation at +120°C for 20 min and diluted with water (30 ml). Uncharged impurities were extracted with diethyl ether (30 ml) and the aqueous solution was poured into ice (50 g) with aqueous 2M HCl (10 ml). The obtained precipitate was filtered, washed with water and dried in vacuo to give 75 mg of yellow solid. Yield 41%. LC-MS (m/z) 293.9 (MH⁺); t_{R} = 1.09. ¹H NMR (500 MHz, DMSO-d₆): 1.95 (s, 3H), 2.74 (s, 3H), 7.3 (d, 1H), 7.32 (d, 2H), 7.48 (two overlapping m, 2H), 7.55 (t, 2H), 7.62 (t, 1H), 13.42 (b, 1H). 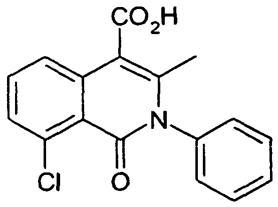 *8-Chloro-3-methyl-1-oxo-2-phenyl-1,2-dihydro-isoquinoline-4-carboxylic acid.*

The title compound was prepared analogously by hydrolysis of the corresponding methyl or ethyl ester at 100°C. LC-MS (m/z) 314.1 (MH⁺); t_{R} = 1.03. 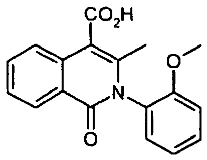 *2-(2-Methoxy-phenyl)-3-methyl-1-oxo-1,2-dihydro-isoquinoline-4-carboxylic acid.*

The title compound was used in the next step without purification. LC-MS (m/z) 310.3 (MH⁺); t_{R} = 0.87. 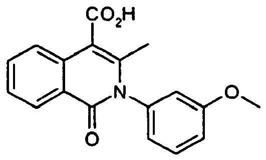 *2-(3-Methoxy-phenyl)-3-methyl-1-oxo-1,2-dihydro-isoquinoline-4-carboxylic acid.*

The title compound was used in the next step without purification. LC-MS (m/z) 310.4(MH⁻); t_{R} = 0.9. 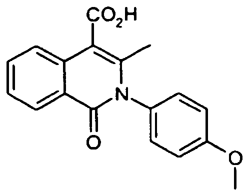 *2-(4-Methoxy-phenyl)-3-methyl-1-oxo-1,2-dihydro-isoquinoline-4-carboxylic acid.*

The title compound was used in the next step without purification. LC-MS (m/z) 310.4 (MH⁻); t_{R} = 0.89. 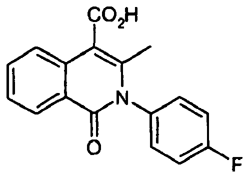 *2-(4-Fluoro-phenyl)-3-methyl-1-oxo-1,2-dihydro-isoquinoline-4-carboxylic acid.*

The title compound was used in the next step without purification. LC-MS (m/z) 298.2 (MH⁺); t_{R} = 0.9. 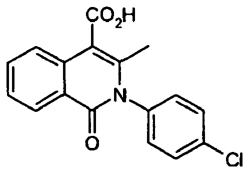 *2-(4-Chloro phenyl)-3-methyl-1-oxo-1,2-dihydro-isoquinoline-4-carboxylic acid.*

The title compound was used in the next step without purification. LC-MS (m/z) 314.2 (MH⁺); t_{R} = 1.53. 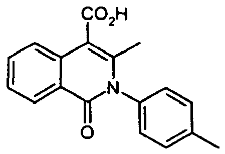 *3-Methyl-1-oxo-2-p-tolyl-1,2-dihydro-isoquinoline-4-carboxylic acid.*

The title compound was used in the next step without purification. LC-MS (m/z) 293.9 (MH⁺); t_{R} = 0.99. 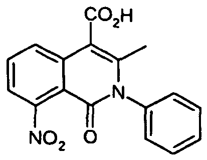 *3-Methyl-8-nitro-1-oxo-2-phenyl-1,2-dihydro-isoquinoline-4-carboxylic acid.*

The title compound was used in the next step without purification. LC-MS (m/z) 325.5 (MH⁺); t_{R} = 0.94. 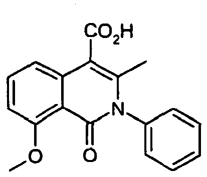 *8-Methoxy-3-methyl-1-oxo-2 phenyl-1,2-dihydro-isoquinoline-4-carboxylic acid.*

The title compound was used in the next step without purification. LC-MS (m/z) 310.6 (MH⁺); t_{R} = 0.73. 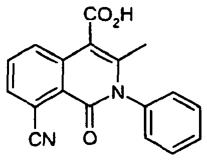 *8-Cyano-3-methyl-1-oxo-2-phenyl-1,2-dihydro-isoquinoline-4-carboxylic acid.*

LC-MS (m/z) 305.0 (MH⁺); t_{R} = 0.82.

Synthesis of homophthalic anhydrides of the general formula XVIII: 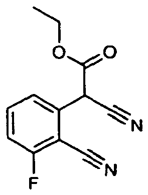 *Cyano-(2-cyano-3-fluoro-phenyl)-acetic acid ethyl ester.*

A mixture of cyanoacetic acid ethyl ester (26.7 mL, 251 mmol), 2,6-difluorobenzonitrile (33.2 g, 239 mmol) and potassium carbonate (82.5 g, 597 mmol) in dimethyl sulfoxide (120 mL) was stirred at +55°C for 16 hours and poured into ice-water mixture (ca. 400 mL). It was acidified with conc. aq. HCl with caution (CO₂ evolution) and extracted with ethyl acetate (600 ml). The organic phase was washed with brine (100 mL) and evaporated to give 55.1 g of a pale yellow solid that was used in the next step without further purification. ¹H NMR (500 MHz, CDCl₃): 1.35 (t, J=7.0 Hz, 3H), 4.34 (m, 2H), 5.13 (s, 1H), 7.33 (t, J=8.4 Hz, 1H), 7.57 (d, J=7.9 Hz, 1H), 7.33 (dd, J=7.9 Hz, J=13.9 Hz, 1H). 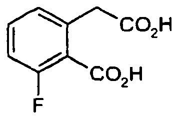 *2-Carboxymethyl-6-fluoro-benzoic acid.*

A mixture of 62% sulfuric acid (2:1 conc. H₂SO₄ in water, 400 ml) and cyano-(2-cyano-3-fluoro-phenyl)-acetic acid ethyl ester (52.0 g, 224 mmol) was stirred at +150°C overnight (16 hours). The reaction mixture was poured into ice (ca. 500 g) and neutralised with 10.8 N aq. NaOH (500 mL) with cooling. The mixture was extracted with ethyl acetate (3 x 500mL) and the combined organic solution was washed with brine, dried over MgSO₄ and evaporated to give 40.28 g of crude product that was used in the next step without further purification. The analytical sample was prepared by recrystallisation from toluene - ethyl acetate. ¹H NMR (500 MHz, DMSO-d₆): 3.4 (br, CO₂H + H₂O), 3.77 (s, 2H), 7.17 (d, J=7.9 Hz, 1H), 7.2 (overlapping t (unres. dd), 1H), 7.45 (dd, J=7.9 Hz, J=13.9 Hz, 1H), 12.95 (br, CO₂H). 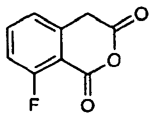 *8-Fluoro-isochroman- 1,3-dione.*
2-Carboxymethyl-6-fluoro-benzoic acid (130 mg, 0.65 mmol) in acetyl chloride (2 ml) was heated under microwave irradiation at 150°C for 10 min then concentrated in vacuo to give the title product (120 mg, 100% yield). The compound is hydroscopic and slowly decomposes back to the starting diacid in wet solvents or under moisturized atmosphere. ¹H NMR (500 MHz, DMSO-d₆): 4.29 (s, 2H), 7.27 (d, J=7.8 Hz, 1H), 7.34 (dd, J=8.5 Hz, J=11 Hz, 1H), 7.76 (dt, J=5.3 Hz, J=8 Hz, 1H).

The following homophthalic anhydrides were obtained analogously by the described above 3-step procedure from corresponding fluorobenzonitriles and ethyl cyanoacetate: 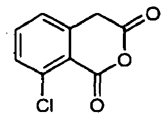 8-Chloro-isochroman-1,3-dione. 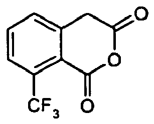 8-Trifluoromethyl-isochroman-1,3-dione. 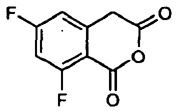 6,8-Difluoro-isochroman-1,3-dione. 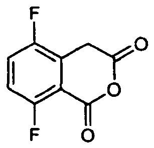 5,8-Difluoro-isochroman-1,3-dione.

Synthesis of chiral and racemic amines of the general formula XI: 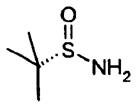 *(S)-(-)-2-Methyl-2-propanesulfinamide.*

The title chiral auxiliary was prepared according to a described procedure for the (R)-(+)-enantiomer by D.J. Weix and J.A. Ellman *Organic Syntheses* **2005**, *82*, 157. 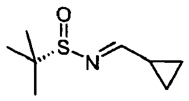 *(S)-2-Methyl-2-propanesulfinic acid 1-cyclopropyl-methylideneamide.*

The title compound was prepared according to a general procedure described by G. Liu, D.A. Cogan, T.D. Owens, T.P. Tang, and J.A. Ellman J. Org. Chem. 1999, 64, 1278: A mixture of cyclopropanecarboxaldehyde (35.0 g, 0.5 mol), 2-methyl-2-propanesulfinic acid 1-cyclopropyl-methylideneamide (30 g, 0.25 mol) and anhydrous CuSO₄ (120 g, 0.75 mol) in CH₂Cl₂ (1500 mL) was stirred at room temperature overnight. The reaction mixture was filtered and evaporated to give the title compound (39 g, yield 95%), which was used in the next step without further purification. 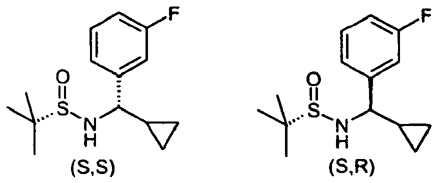 *(S)-2-Methyl-2-propanesulfinic acid [(S)-cyclopropyl-(3-fluoro-phenyl)-methyl]-amide* and *(S)-2-Methyl-2-propanesulfinic acid [(R)-cyclopropyl-(3-fluoro-phenyl)-methyl]-amide.*

The title compounds were obtained according to a general described procedure for 1,2-stereoselective addition of organometallic reagents to sullinyl imines by D.A. Cogan, G. Liu, J.A. Ellman, Tetrahedron 1999, 55, 8883.

Procedure A: To an anhydrous lithium chloride (1.7 g, 40 mmol) THF (20 ml) was added under nitrogen followed by slow addition of i-PrMgCl (22 mL, 2 M in THF) and the obtained mixture was stirred at r.t. overnight. The obtained *i*-PrMgCl·LiCl solution was added dropwise to a stirred solution of 1-bromo-3-fluorobenzene (5.6 g, 33 mmol) in THF (25 ml) at 0°C and stirring continued for 2 hours. The obtained Grignard reagent was added to a solution of (S)-2-methyl-2-propanesulfinic acid 1-cyclopropyl-methylideneamide (2.5 g, 14 mmol) in CH₂Cl₂ (60 mL) at -48°C. The mixture was stirred at -48°C for 5 hours and then at room temperature overnight. The reaction mixture was quenched by addition of aq. sat. NH₄Cl (50 mL) and extracted with CH₂Cl₂ (3x100 mL). The combined organic solution was dried (Na₂SO₄) and evaporated to give a crude mixture, which was purified by column chromatography on silica gel (EtOAc/petroleum ether = 1/10). The obtained mixture of diastereoisomers was resolved by SFC to give the title (S,S)-isomer as the major product (1.5 g, yield 37.5%) and the title (S,R)-isomer (0.16 g, yield: 4.0%).

Procedure B: Alternatively, to a suspension of Mg (13.4 g, 0.55 mol) in 50 mL of anhydrous THF at 50°C a solution of 1-bromo-3-fluorobenzene (89.0 g, 0.50 mol) was added dropwise. The mixture was stirred for 2 hours at +50°C and then it was added dropwise to a solution of (S)-2-methyl-2-propanesulfinic acid 1-cyclopropyl-methylideneamide (78.0 g, 0.46 mol) in 100 mL of THF at 50-60°C and stirred for 2 hours. It was quenched with aq. sat. NH₄Cl (100 ml), water (300 mL), filtered, and the both solid and filtrate were extracted with hot ethyl acetate (600 mL) and evaporated in vacuo. The residue was crystallized from a mixture of ethyl acetate and petroleum ether (1:1, 200 mL) at -20°C to give 80 g of the title (S,S)-isomer as a white powder, 66% yield, de 100% according to chiral HPLC. ¹H NMR (CDCl₃, 400 MHz, TMS=0 ppm): 7.34-7.28 (m, 1H), 7.16-7.12 (m, 2H), 7.00-6.96 (m, 1H), 3.68 (dd, J = 8.8 Hz, 3.2 Hz, 1H), 3.52 (s, 1H), 1.42 (s, 9H), 1.15-1.08 (m, 1H), 0.84-0.75 (m, 1H), 0.69-0.61 (m, 1H), 0.55-0.46 (m, 1H), 0.28-0.21 (m, 1H). 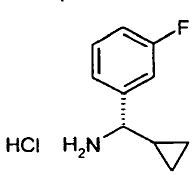 *(S)-(+)-C-[C-Cyclopropyl-C-(3-fluoro-phenyl)]-methylamine hydrochloride*

To a saturated solution of HCl in anhydrous dioxane (400 ml) (S)-2-methyl-2-propanesulfinic acid [(S)-cyclopropyl-(3-fluoro-phenyl)-methyl]-amide (80 g, 0.3 mol) was added at 0°C. After stirring at r.t. for 1 hour, the reaction mixture was evaporated in vacuo. The residue was washed with anhydrous ether (2x100 ml) and dried in vacuo to give 56 g of the title compound as a white solid, yield 93%, ee 100% according to chiral HPLC. [a]²⁰_{D} = + 52.69 (c = 10 mg/mL, CH₃OH). ¹H NMR

(CD₃OD, 400 MHz, TMS=0): 7.44-7.39 (m, 1H), 7.25-7.19 (m, 2H), 7.12-7.07 (m, 1H), 3.56 (d, *J* = 10.0 Hz, 1H), 1.37-1.28 (m, 1H), 0.78-0.75 (m, 1H), 0.61-0.55 (m, 2H), 0.39-0.36 (m, 1H). 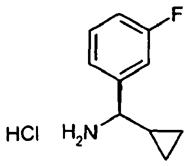 *(R)-(-)-C-[C-Cyclopropyl-C-(3-fluoro-phenyl)]-methylamine hydrochloride.*

The title compound was prepared according to the above identical procedure from (S)-2-methyl-2-propanesulfinic acid [(R)-cyclopropyl-(3-fluoro-phenyl)-methyl]-amidc (0.16g, 0.6 mmol) to give 0.116 g of the title compound as a white solid. [α]²⁰_{D} = - 49.18 (c = 10 mg/mL, CH₃OH), cc 100%. ¹H NMR (CD₃OD, 400 MHz, TMS=0): identical with (S)-enantiomer.

The following enantiomerically pure amine hydrochlorides were obtained analogously in three-step procedure starting from condensation of the corresponding aldehyde with chiral auxiliary, stereoselctive Grignard addition where the mixture of diastereoisomers was resolved either by recrystallisation or by chromatography (SFC or column) and the major (S,S)-diastereoisomer was finally converted to a chiral amine with HCl.

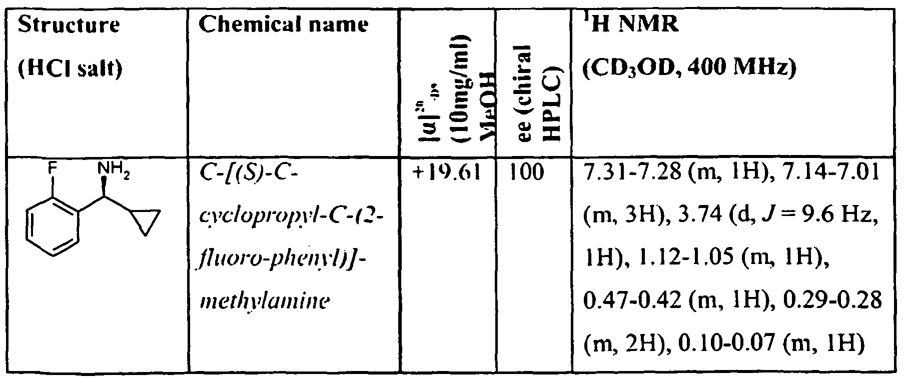

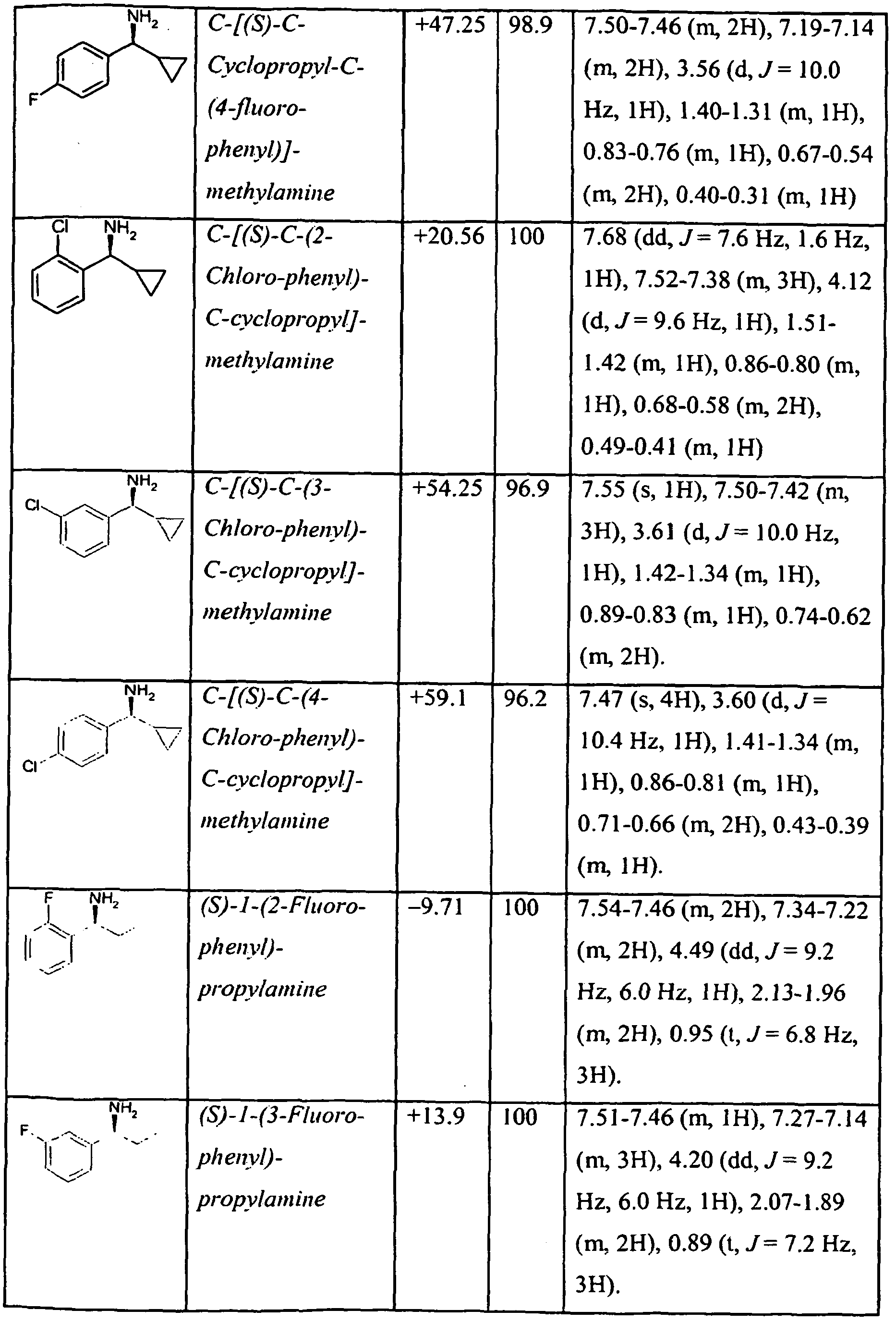

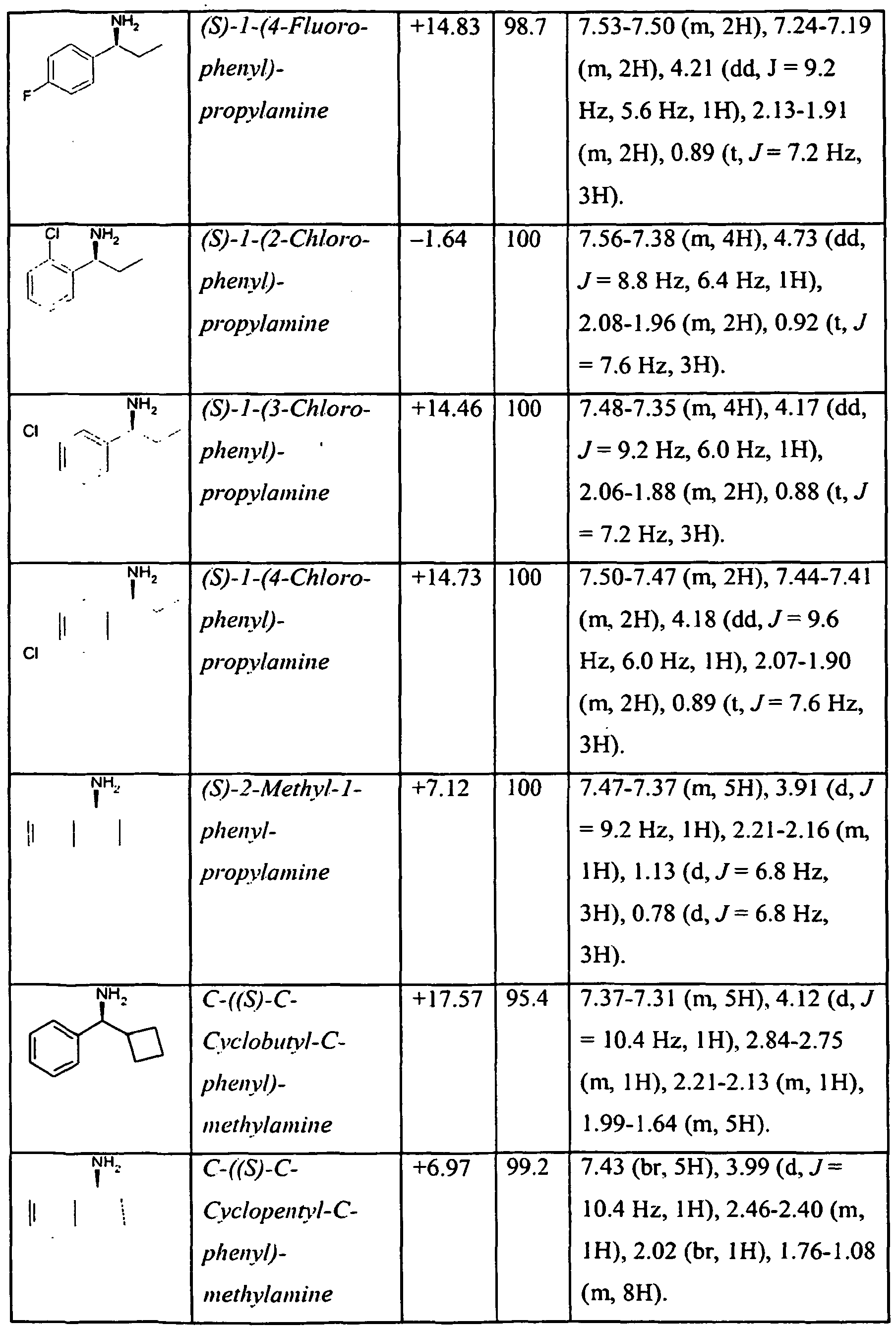

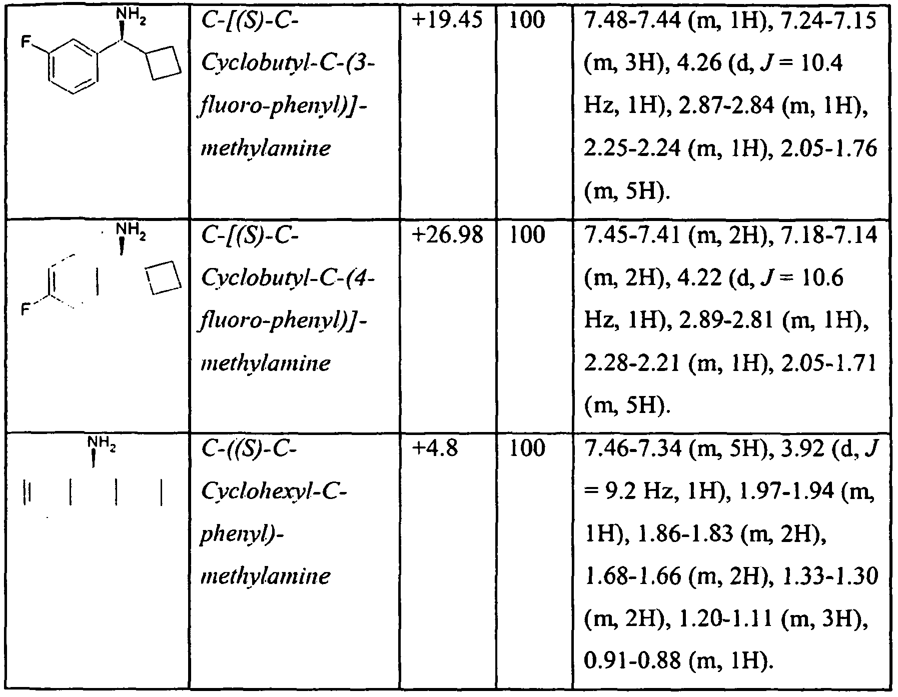

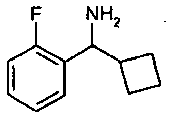

*C-Cyclobutyl-C-(2-fluoro-phenyl)-methylamine.*

One third of a solution of cyclobutyl bromide (5.0 g, 41.3 mmol) in anhydrous tetrahydrofuran (24 mL) was stirred with magnesium (1.11 g, 46.3 mmol) under reflux. The remaining solution was added dropwise over a period of 15 minutes, and the stirring at reflux continued for 30 min. To the obtained solution 2-fluorobenzonitrile (1.2 g) in THF (15 ml) was added dropwise at 0°C. The mixture was stirred for 5.5 h at 0°C followed by addition of methanol (30 ml) and sodium borohydride (1.13 g). The reaction mixture was stirred for 16 hours at ambient temperature and concentrated. The residue was partitioned between chloroform (3x 100 ml) and water, and the pH was adjusted to 1. The mixture was extracted with chloroform. The aqueous phase was adjusted to pH=10, and extracted with chloroform (3 x 100 ml). The combined organic layers were dried, evaporated and purified by column chromatography on silica gel (ethyl acetate/petroleum ether = 1/1) to afford the title amine (0.45 g, yield: 7.9%) ¹H NMR (CD₃OD, 400 MHz) 7.49-7.38 (m, 2H), 7.30-7.15 (m, 2H), 4.50 (d, *J* = 10.4 Hz, 1H), 3.00-2.90 (m, 1H), 2.29-2.21 (m, 1H), 2.09-1.71 (m, 5H).

Synthesis of acid-amides of the general formula XIX: 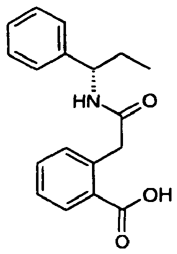 *2-[((S)-1-Phenyl-propylcarbamoyl)-methyl]-benzoic acid.*

A mixture of homophthalic anhydride (810 mg, 5 mmol) and (S)-(-)-1-phenylpropylamine (676 mg, 5 mmol) in acetonitrile (15 ml) was heated under microwave irradiation at +150°C for 15 min. White precipitate was collected by filtration, washed with heptane and dried in vacuo to give pure title compound in 72% yield (1.065g). Alternatively, to a stirred solution of homophthalic anhydride (16.214 g, 0.1 mol) in acetonitrile (100 ml) (S)-(-)-1-phenylpropylamine (13.83 g, 0.102 mol) was added dropwise (exothermic reaction) and the obtained reaction mixture was refluxed for 5 min. It was allowed to cool and the product was isolated by filtration as above to give 23.4 g of colourless solid, 79% yield. LC-MS (m/z) 298.5 (MH⁺); t_{R} = 1.11. ¹H NMR (500 MHz, DMSO-d₆): 0.84 (t, J=7.3 Hz, 3H), 1.67 (quintet, J=7.3 Hz, 2H), 3.85 (d of AB system, J=15.1 Hz, 1H), 3.95 (d of AB system, J=15.1 Hz, 1H), 4.66 (q, J=7.6 Hz, I H), 7.2 (unres. m, 1H), 7.25-7.34 (m, 5H), 7.45 (t, J=7.3 Hz, 1H), 7.8 (d, J=7.8 Hz, 1H), 8.39 (br. d, J=7.7 Hz, 1H, NH).

The following compounds were obtained analogously from corresponding homophthalic anhydrides of general formula and amines of the general formula XVIII and XI, respectively. The reactions were usually run at room temperature and the products were isolated by extraction or filtration and used in the next steps without further purification. 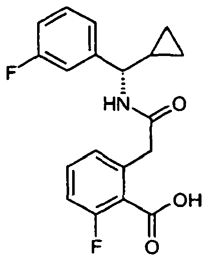 *2-({[(S)-Cyclopropyl-(3-fluoro-phenyl)-methyl]-carbamoyl}-methyl)-6-fluoro-benzoic acid.*

LC-MS (m/z) 346.2 (MH⁺); t_{R} = 1.14. ¹H NMR (500 MHz, DMSO-d₆): 0.35 (m, 1H), 0.39 (m, I H), 0.5 (m, 2H), 1.12 (m, I H), 3.68 & 3.74 (two d of AB system, J= 15.2 Hz, 2H, CH₂), 4.25 (t, J= 8.5 Hz, 1H), 7.05 (dt, J=2.2, 8.05 Hz, 1H), 7.13 (d, J= 8.1 Hz, 1H), 7.16-7.21 (m, 2H), 7.35 (m, 1H), 7.42 (m, 1H), 8.66 (d, J= 8.2 Hz, 1H, NH), 13.44 (br., CO₂H). 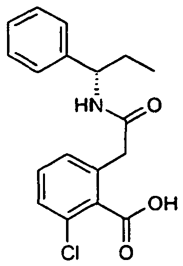 *2-Chloro-6-[((S)-1-phenyl-propylcarbamoyl)-methyl]-benzoic acid.*

LC-MS (m/z) 332.2 (MH⁻): t_{R} = 1.19. ¹H NMR (500 MHz DMSO-d₆):0.84 (t, J=7.3 Hz, 3H), 1.68 (quintet, J=7.3 Hz, 2H), 3.54 & 3.61 (two d of AB system, J=15.3 Hz, 2H, CH₂), 4.67 (q, J=7.5 Hz, 1H), 7.19-7.4 (m, 8H), 8.48 (d, J=8.3 Hz, 1H, NH), 13.64 (br., CO₂H). 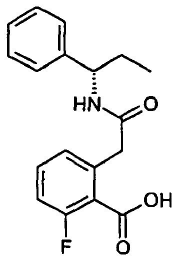 *2-Fluoro-6-[((S)-1-phenyl-propylcarbamoyl)-methyl]benzoic acid.*

LC-MS (m/z) 316.3 (MH⁺); t_{R} = 1.13. ¹H NMR (500 MHz, CDCl₃): 0.79 (t, J= 7.4 Hz, 3H), 1.76 (d of quintet, 2H, diastereotopic CH₂ ofEt), 3.64 (s, 2H, CH₂), 4.75 (q, J= 7.7 Hz, 1H), 7.04 (t, J=8.5 Hz, 1H), 7.08 (d, J=7.7 Hz, 1H), 7.17-7.23 (overlapping m, 3H), 7.24-7.28 (overlapping m, 2H), 7.32 (m, 1H), 7.45 (br d, J= 8.1 Hz, 1H, NH). 8.48 (d, J=8.3 Hz, 1H, NH), 11.14 (br., CO₂H). 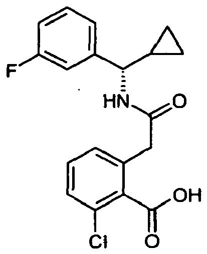 *2-Chloro-6-({[(S)-cyc*/*opropyl-(3-fluoro-phenyl)-methyl]-carbomoyl}-methyl)-benzoic acid.* 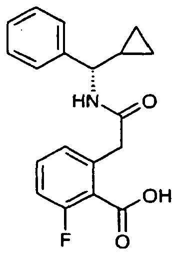 *2-{[((S)-Cyclopropyl-phenyl-methyl)-carbamoyl]-methyl}-6-fluoro-benzoic acid.*

LC-MS (m/z) 328.4 (MH⁺); t_{R} = 1.12. 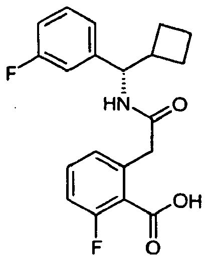 *2-({[(S)-Cyclobutyl-(3-fluoro-phenyl)-methyl]-carbamoyl}-methyl)-6-fluoro-benzoic acid.*

LC-MS (m/z) 360.2 (MH⁺); t_{R} = 1.31. 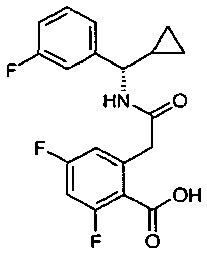 *2-({[(S)-Cyclopropyl-(3-fluoro-phenyl)-methyl]-carbamoyl}-methyl)-4,6-difluorobenzoic acid.* 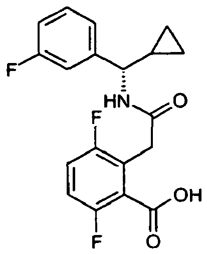 *2-({[(S)-Cy*/*opropyl-(3-fluoro-phenyl)-methyl]-carbamoyl}-methyl)-3,6-difluorobenzoic acid.* 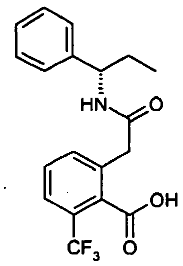 *2-[((S)-1-Phenyl-propylcarbamoyl)-methyl]-6-trifluoromethyl-benzoic acid.*

LC-MS (m/z) 366.4 (MH⁺); t_{R} = 1.24.

Synthesis of compounds of the general formula XXIII: 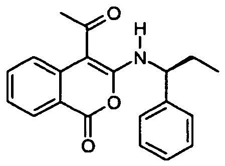 *4-Acetyl-3-((S)-1-phenyl-propylamino)-isochromen-1-one.*

A mixture of 2-[((S)-1-phenyl-propylcarbamoyl)-methyl]-benzoic acid (10 g), acetic anhydride (50 ml), and N,N-dimethylaminopyridine (100 mg) was heated at gentle reflux (Tₘₐₓ = +124°C) for 7 min and evaporated in vacuo at +50°C to give the title compound as a yellow-brown solid (11.1 g, purity 98% by NMR). LC-MS (m/z) 322.3 (MH⁺); t_{R} = 1.72. ¹H NMR (500 MHz, DMSO-d₆): 0.89 (t, J=7.2 Hz, 3H), 1.86-1.97 (m, 2H), 2.56 (s, 3H), 4.95 (q, J= 7.1 Hz, 1H, CH-NH), 7.26 (t, J=7.5 Hz, I H), 7.29 (unres. m, 1H), 7.36-7.4 (unres m., 3H), 7.71 (t, J=7.5 Hz, 1H), 7.75 (d, J=8.3 Hz, I H), 7.98 (d, J=7.8 Hz, 1H), 11.53 (d, J=7.6 Hz, 1H, NH). ¹³C APT NMR(125 MHz, DMSO-d₆, δ(DMSO-d₆)=39.87 ppm): 10.68 (CH₃), 30.0 (CH₂), 31.57 (CH₃), 57.01 (CH), 92.44 (C), 114.88 (C), 124.2 (CH), (CH), 124.26 (CH), 126.65 (CH), 127.8 (CH), 129.05 (CH), 129.93 (CH), 135.71 (CH), 138.68 (C), 141.86 (C), 158.51 (C), 160.55 (C), 194.82 (C, MeCO). 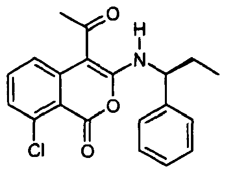 *4-Acetyl-8-chloro-3-((S)-1-phenyl-propylamino)-isochromen-1-one.*
2-Chloro-6-[((S)-1-phenyl-propylcarbamoyl)-methyl]-benzoic acid (11.4 g) and acetic anhydride (50 ml) were heated at 103°C for 60 min and evaporated to give 12.45 g of brown oil (purity ca 95% according to ¹H NMR). LC-MS (m/z) 355.2 (MH⁺); t_{R} = 1.82. Analytically pure sample was prepared by recrystallisation (9 g from 30 ml of hot MeCN) to give the title compound (4.41 g) as a pale yellow solid after cooling (dry ice - EtOH bath) and filtration. LC-MS (m/z) 355.2 (MH⁺); t_{R} = 1.82. ¹H NMR (500 MHz, DMSO-d₆): 0.88 (t, J=7.4 Hz, 3H), 1.92 (m, 2H), 2.5 (s, 3H), 4.92 (q, J=7.3 Hz, 1H, CH-NH), 7.27-7.33 (m, 2H), 7.39 (d (unres. m), J= 4.3 Hz, 4H), 7.59-7.66 (m, 2H), 11.11 (d, J=7.8 Hz, 1H, NH). 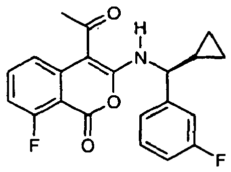 *4-Acetyl-3-{[(S)-cyclopropyl-(3-fluoro-phenyl)-methyl]-amino}-8-fluoro-isochromen-1-one.*

A mixture of 2-({[(S)-cyclopropyl-(3-fluoro-phenyl)-methyl)-carbamoyl}-methyl)-6-fluoro-benzoic acid (12.92 g, 37.41 mmol) and acetic anhydride (100 mL, 1 mol) was stirred at +65°C for 20 hours and evaporated in vacuo (65°C, 10 mbar, 2 hours) to give the title compound as a thick brown oil, which was used in the next step without purification (14.30 g, yield 103.5%, purity 95% according to ¹H NMR). LC-MS (m/z) 370.1 (MH⁻): t_{R} = 1.65. ¹H NMR (500 MHz, DMSO-d₆): 0.45-0.59 (m, 3H), 0.64 (m, I H), 1.41 (m, 1H), 2.53 (s, 3H), 4.36 (t (unres. dd), 1H), 7.03 (dd, J = 8.3, 10.7 Hz, 1H), 7.12 (dt, J = 1.9, 8.5Hz, 1H), 7.27 (d, J=10.2 Hz, 1H), 7.3 (d, J=7.8 Hz, 1H), 7.42 (q, J=7.8 Hz, 1H), 7.53 (d, 8.5 Hz, 1H), 7.7 (m, 1H), 11.3 (d, J=7.3 Hz, 1H, NH). 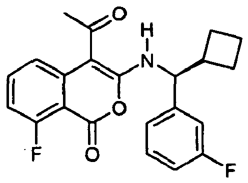 *4-Acetyl-3-{[(S)-cyclobutyl-(3-fluoro-phenyl)-methyl]-amino}-8-fluoro-isochromen-1-one.*

LC-MS (m/z) 384.4 (MH⁺); t_{R} = 1.82. 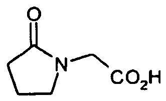 *(2-Oxo-pyrrolidin-1-yl)-acetic acid.*

A mixture of 2-pyrrolidone (2.66 ml, 34 mmol) and NaH (60% in oil, 1.25 g, 31.3 mmol) in THF (75 ml) was stirred until gas evolution ceased (30 min). tert-Butyl 2-bromoacetate (4.45 ml, 30 mmol) was added and stirred at r.t. overnight then partitioned between water (200 ml) and ethyl acetate (200 ml) to give intermediate 2-(oxo-pyrrolidin-1-yl)-acetic acid tert-butyl ester (5.8 g). It was dissolved in acetic acid (40 ml) and conc. aq. HCl (6 ml) with gas evolution observed. After stirring at r.t. for 2 hours it was evaporated and recrystallized from toluene/ethanol to give 2.58 g of the title compound (60% yield). ¹H NMR (500 MHz, DMSO-d₆): 1.95 (quintet, J= 7.7 Hc, 2H), 2.24 (t, J= 8.1 Hz, 2H), 3.38 (m, overalapping with H₂O (3.35 ppm), 2H), 3.91 (s, 2H), 12.77 (br., 1H). 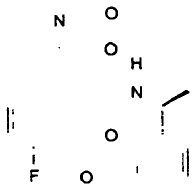 *1-{2-[8-Fluoro-1-oxo-3-((S)-1-phenyl-propylamino)-1H-isochromen-4-yl]-2-oxoethyl}-pyrrolidin-2-one.*

To a solution of 2-oxo-pyrrolidin-1-yl-acetic acid (1.56 g, 10.9 mmol) in 1,2-dichloroethane (40 ml) oxalyl chloride (0.95 ml, 10.9 mmol) and DMF (1 drop) were added with gas evolution observed. After stirring at r.t. for 1 hour, 2-fluoro-6-[((S)-1-phenyl-propylcarbamoyl)-methyl]-benzoic acid (1.56 g, 4.95 mmol), triethylamine (2.1 ml, 14.9 mmol) and DMAP (85 mg, 0.1 eq.) were added and stirred at r.t. overnight. It was partitioned between mixture of 0.2 M aq. HCl (100 ml) - brine (100 ml) and ethyl acetate (250 ml), washed with water and evaporated to give 1.89 g of crude title compound used in the next step without further purification.

¹H NMR (500 MHz, DMSO-d₆, selected resonances): 4.51 & 4.58 (two d of AB system, J=16.3 Hz, N-CH₂-CO), 4.99 (q, J= 7.5 Hz, 1H, CH-NH), 11.31 (d, J= 7.9 Hz, 1H, NH). 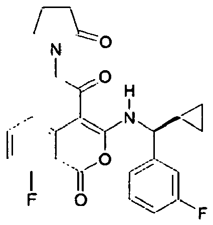 *1-[2-(3-{[(S)-Cyclopropyl-(3-fluoro-phenyl)-methyl]-amino}-8-fluoro-1-oxo-1H-isochromen-4-yl)-2-oxo-ethyl]-pyrrolidin-2-one.*

The title compound was prepared analogously and purified by flash chromatography on SiO₂. LC-MS (m/z) 453.3 (MH⁺); t_{R} = 1.48. 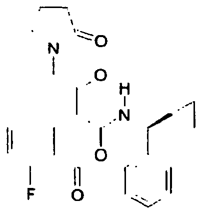 *1-(2-{3-[((S)-Cyclopropyl-phenyl-methyl)-amino]-8-fluoro-1-oxo-1H-isochromen-4-yl}*-2*-oxo-ethyl)-pyrrolidin-*2*-one.*

The title compound was prepared analogously. LC-MS (m/z) 435.3 (MH⁺); t_{R} = 1.43. 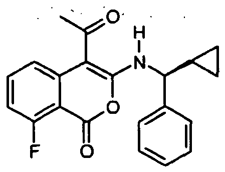 *4-Acetyl-3-[((S)-cyclopropyl-phenyl-methyl)-amino]-8-fluoro-isochromen-1-one.*

LC-MS (m/z) 352.6 (MH⁺); t_{R} = 1.62. ¹H NMR (500 MHz, DMSO-d₆): 0.48 (m, 2H), 0.54 (m, 1H), 0.64 (m, 1H), 1.39 (m, 1H), 2.53 (s, 3H), 4.39 (t (unres. dd), 1H), 7.04 (dd, J = 8.2, 10.8 Hz, 1H), 7.3 (t, J=7.3 Hz, 1H), 7.39 (t, J=7.6 Hz, 2H), 7.43 (d, J=7.3 Hz, 2H), 7.53 (d, 8.5 Hz, 1H), 7.7 (m, 1H), 11.39 (d, J=7.5 Hz, 1H, NH).

Hydrolysis of compounds of the general formula XXIII into compounds of the general formula XXV: 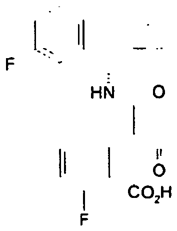 *2-(1-{[(S)-Cyclopropyl-(3-fluoro-phenyl)-methyl]-carbamoyl}-2-oxo-propyl)-6-fluoro-benzoic acid.*
4-Acetyl-3-{[(S)-cyclopropyl-(3-fluoro-phenyl)-methyl]-amino}-8-fluoro-2-benzopyran-1-one (14.30 g, 38.72 mmol) was dissolved in a mixture of tetrahydrofuran (50 mL) and methanol (50 mL) and placed on an ice/water bath with stirring. NaOH (1 M in H₂O, 100 ml) was added and stirring continued for I hour. The cold bath removed and the mixture was allowed to warm to r.t. (20°C) during 1 hour. The reaction mixture was poored into ice-water mixture (200 g + 200 ml), followed by slow addition of 2 M aq. HCl (200 mL) and extracted with ethyl acetate (200 mL), washed with sat. aq. NaCl, dried (Na₂SO₄), filtered and evaporated to give the title compound (14.65 g, yield 97.7%) as a pale brown foam. The crude product was used in the next step without further purification. LC-MS (m/z) 388.3 (MH⁺); t_{R} = 1.2. ¹H NMR (500 MHz, DMSO-d₆): a mixture of tautomers and diastereoisomers. The following compounds were prepared analogously: 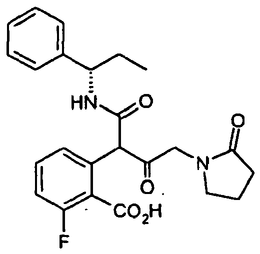 *2-Fluoro-6-[2-oxo-3-(2-oxo-pyrrolidin-1-yl)-1-((S)-1-phenyl-propylcarbamoyl)-propyl]-benzoic acid.* 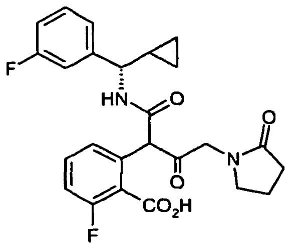 *2-[1-{[(S)-Cyclopropyl-(3-fluoro-phenyl)-methyl]-carbamoyl}-2-oxo-3-(2-oxopyrrolidin-1yl)-propyl]-6-fluoro-benzoic acid.*

LC-MS (m/z) 471.4 (MH⁺); t_{R} = 1.17. 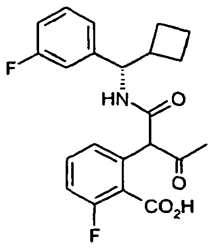 *2-(1-{[(S)-Cyclobutyl-(3-fluoro-phenyl)-methyl]-carbamoyl}-2-oxo-propyl)-6-fluorobenzoic acid.*

LC-MS (m/z) 402.2 (MH⁺; t_{R} = 1.36.

Synthesis of compounds of the general formula XXIV and XXV: Compounds of general formula XXIV obtained analogously as described above for compounds of general formula XXIII but at higher temperature (150°C, 15 min) and they are usually hydrolyzed to compounds of the general formula XXV without isolation and characterization, so only two examples of compounds XXIV are given below. 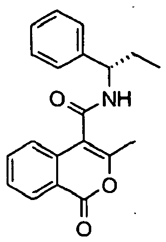 *3-Methyl-1-oxo-1H-isochromene-1-carboxylic acid ((S)-1-phenyl-propyl)-amide.*

The title compound was purified by flash chromatography on SiO₂. LC-MS (m/z) 322.1 (MH⁺); t_{R} = 1.27. ¹H NMR (500 MHz, CDCl₃): 1.03 (t, J=7.3 Hz, 3H), 1.91-2.06 (complex m, 2H, CH₂), 2.19 (s, 3H), 5.11 (q, J=7.8 Hz, 1H), 7.06 (br. d, J=8.3 Hz, 1H, NH), 7.22-7.33 (m, 3H), 7.36-7.43 (m, 4H), 7.54 (t, J= 7.6 Hz, 1H), 7.95 (d, J= 7.8 Hz, 1H). ¹H NMR (500 MHz, DMSO-d₆): 0.92 (t, J= 7.3 Hz, 3H), 1.76 (m, 3H), 2.18 (s, 3H), 4.93 (q, J=8.3 Hz, 1H), 7.21-7.41 (m, 6H), 7.59 (t, J=7.7 Hz, 1H), 7.82 (t, J=7.2 Hz, 1H), 8.17 (d, J=7.8 Hz, I H), 9.1 (br. d, J=8.4 Hz, 1H, NH). 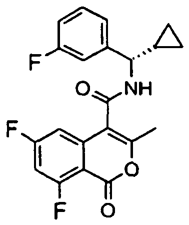 *6,8-Difluoro-3-methyl-1-oxo-111-isochromene-4-carboxylic acid [(S)-cyclopropyl-(3-fluoro-phenyl)-methyl]amide.*

The title compound was purified by flash chromatography on SiO₂. LC-MS (m/z) 388.4 (MH⁻): t_{R} = 1.39. 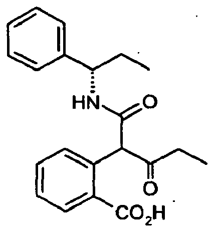 *2-[2-Oxo-1-((S)-1-phenyl-propylcarbamoyl)-butyl]-benzoic acid.*

A sealed mixture of 2-[((S)-1-phenyl-propylcarbamoyl)-methyl]-benzoic acid (409 mg, 1.38 mmol), propionic anhydride (10 ml) and 4-N,N-dimethylaminopyridine (15 mg) was heated under microwave irradiation at 150°C for 20 min and partitioned between 1M HCl (50 ml) and ethyl acetate (100 ml). The organic layer was washed with sat. aq. NaHCO₃ (2x50 ml) and brine and concentrated in vacuo. To the obtained residue methanol (25 ml) tetrahydrofurane (25 ml) and 2M aq. NaOH (50 ml) was added and stirred at r.t. for 1 hour. The organic volatiles were removed in vacuo and the pH was adjusted to 1 with 3M aq. HCl. The crude title product (495 mg) was separated by extraction with ethyl acetate (150 ml) and used in the next step without further purification. ¹H NMR (500 MHz, DMSO-d₆): a mixture of tautomers and diastereoisomers.

The following compounds were obtained analogously: 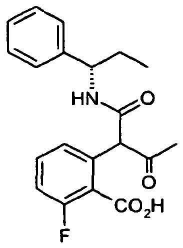 *2-Fluoro-6-[2-oxo-1-((S)-phenyl-propylcarbamoyl)-propyl]-benzoic acid.*

LC-MS (m/z) 358.4 (MH⁺); t_{R} = 1.17. 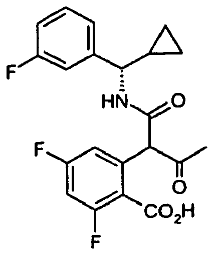 *2-(1-{[(S)-Cyclopropyl-(3-fluoro-phenyl)-methyl]-carbamoyl)-2-oxo-propyl)-4,6-difluoro-benzoic acid.*

LC-MS (m/z) 406.7 (MH⁺); t_{R} = 1.3. 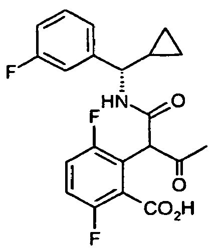 *2-(1-{[(S)-Cyclopropyl-(3-fluoro-phenyl)-methyl]-carbamoyl}-2-oxo-propyl)-3,6-difluoro-benzoic acid.*

LC-MS (m/z) 406.0 (MH⁺); t_{R} = 0.72 (method B). 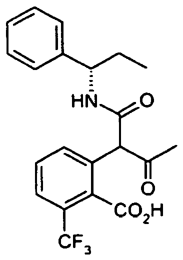 2-[2-Oxo-1-((S)-1-phenyl-propylcarbamoyl)-propyl)-6-trifluoromethyl-benzoic acid.

The title compound was used in the next step directly. 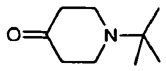 *1-tert-Butyl-piperidin-4-one.*

The title compound was prepared according to the described procedure: J.S. Amato, J.Y.L. Chung, R.J. Cvetovich, X. Gong, M. McLaughlin, R.A. Reamer J. Org. Chem. 2005, 70, 1930. 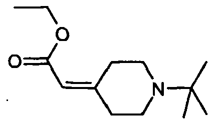 *(1-tert-Buyl-piperidin-4-ylidene)-acetic acid ethyl ester.*

A mixture of 1-tert-Butyl-piperidin-4-one (4.59 g, 23.6 mmol) and ethyl (triphenylphosphorylidene)acetate (10.3 g, 23.6 mmol) in toluene (100 mL) was stirred at reflux for 24 hours under nitrogen and evaporated in vacuo. The residue was purified by column chromatography on silica gel (petroleum ether/triethylamine = 100/l) to afford 5.1 g of the title compound as a pale yellow oil, 76.5% yield. 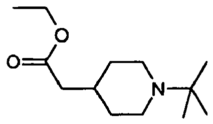 *(1-tert-Butyl-piperidin-4-yl)-acetic acid ethyl ester.*

To a solution of (1-tert-butyl-piperidin-4-ylidene)-acetic acid ethyl ester (5.1 g, 22.7 mmol) in ethanol (50 mL) 10% Pd/C (0.6 g) was added and the mixture was stirred under the hydrogen atmosphere (42 psi) at ambient temperature for 14 hours. The catalyst was removed by filtration, and the filtrate was concentrated to give the title (4.03 g, yield 78%), which was used in the next step directly. 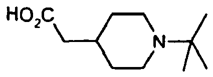 *(1-tert-Butyl-pipereidin-4-yl)-acetic acid.*

To a solution of NaOH in water/ethanol (v/v = 40/10, 50 mL) (1-tert-butyl-piperidin-4-yl)-acetic acid ethyl ester (4.03 g, 17.8 mmol) was added and the mixture was stirred at r.t. overnight. I N aq. HCl was added to adjust the pH to 5. Volotiles were evaporated in vacuo, the residue was diluted with methanol (30 mL), and the insoluble inorganic salts were removed by filtration. The filtrate was concentrated and purified by column chromatography on silica gel (CH₂Cl₂/MeOH = 20/1 to 5/1) to give 0.7 g of title compound as a light grey powder. ¹H NMR (400 MHz, DMSO-d₆): 3.15-3.08 (br m, 2H), 2.30 (br t, *J* = 11.2 Hz, 2H), 2.05 (d, *J* = 6.4 Hz, 2H), 1.71-1.68 (m, 3H), 1.30-1.27 (m, 2H), 1.10 (s, 9H). 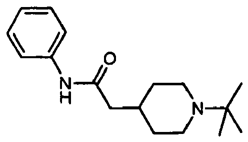 *2-(1-tert-Butyl-piperidin-4-yl)-N-phenyl-acetamide.*

To a mixture of (1-tert-butyl-piperidin-4-yl)-acetic acid (672 mg, 3.37 mmol), aniline (0.32 ml, 3.5 mmol), and HOBt (568 mg, 4.2 mmol) in DMF (20 ml), EDC (805 mg, 4.2 mmol) was added. It was stirred at r.t. for 2 hours and partitioned between water (200 ml) and ethyl acetate (200 ml). The organic phase was washed with 0.5 N aq. NaOH (2x50 ml) and brine (3x50 ml), dried (MgSO₄) and concentrated in vacuo. The residue was purified by flash chromatography on (SiO₂, gradient heptane - ethyl acetate) to give 250 mg of the title compound. ¹H NMR (500 MHz, DMSO-d₆): 1.07 (s, 9H), 1.32 (br. m, 2H), 1.81 (br. d, 2H), 1.89 (m, 1H), 2.11 (br. t, 2H), 2.25 (d, 2H), 3.03 (br. d, 2H), 7.1 (t, 1H), 7.24 (br., 1H), 7.31 (t, 2H), 7.52 (d, 2H).

### Compounds of the invention

### Example 1

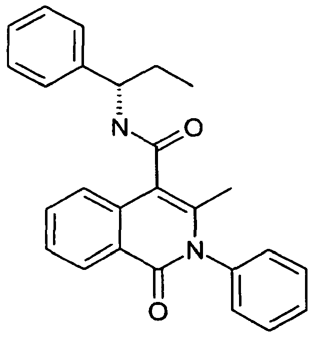

***1a** 3-Methyl-1-oxo-2-phenyl-1,2-dihydro-isoquinoline-4-carboxylic acid ((S)-1-phenyl-propyl)-amide.*

A solution of 3-methyl-1-oxo-2-phenyl-1,2-dihydro-isoquinoline-4-carboxylic acid (212 mg, 0.76 mmol) in SOCl₂ (1.5 ml) and dimethylformamide (ca. 5 µL) was heated at reflux for 5 min. Volatiles were removed in vacuo to give 228 mg of the corresponding acid chloride as a pale brown solid. A portion of the acid chloride (110 mg, 0.37 mmol) was dissolved in 1,2-dichloroethane (2 ml) and (S)-(-)-1-phenylpropylamine (150 µL, 1.1 mmol) was added. The obtained suspension was shaked for 5 min, diluted with 1,2-dichloroethane (10 ml), washed with 1M aqueous HCl (3 x 5 ml) and water (5 ml). The organic phase was poured into SiO₂ (5 g), eluted with 1,2-dichloroethane (50 ml) and the product was eluted with 1:1 ethyl acetate - heptane to give 140 mg of colourless crystalline solid. Yield 95%. Alternatively, in another run the reaction mixture was partitioned between 2M HCl and heptane (10 ml) and the product was separated by filtration from the obtained biphasic system. LC-MS (m/z) 397.1 (MH⁺); t_{R} = 1.44. ¹H NMR (500 MHz, CDCl₃): 1.0 (t, 3H), 1.93 (m, 5H), 5.16 (q, 1H), 6.7 (d, 1H), 7.07 (d, 2H), 7.31 (m, I H), 7.36 (d, 4H), 7.4-7.5 (m, 5H), 7.63 (unres. t, I H), 8.31 (d, 1H).

The following compounds were obtained analogously from the corresponding acid and amine and purified by preparative LC-MS: 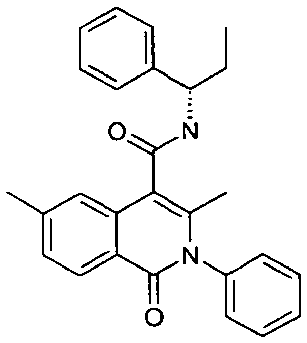 ***1b** 3,6-Dimethyl-1-oxo-2-phenyl-1,2-dihydro-isoquinoline-4-carboxylic acid ((S)-1-phenyl-propyl)-amide.*

LC-MS (m/z) 411.3 (MH⁺); t_{R} = 0.8 (method B). 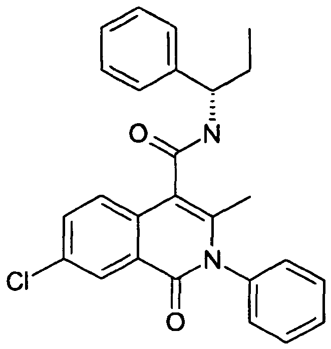 ***1c** 7-Chloro-3-methyl-1-oxo-2-phenyl-1,2-dihydro-isoquinoline-4-carboxylic acid ((S)-1-phenyl-propyl)-amide.*

LC-MS (m/z) 431.3 (MH⁺); t_{R} = 0.86 (method B). 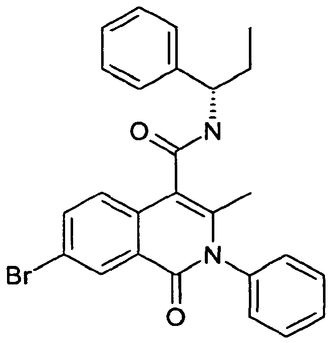 ***1d** 7-Bromo-3-methyl-1-oxo-2-phenyl-1,2-dihydro-isoquinoline-4-carboxylic acid ((S)-1-phenyl-propyl)-amide.*

LC-MS (m/z) 475.1 (MH⁺, ⁷⁹Br); t_{R} = 0.87 (method B). 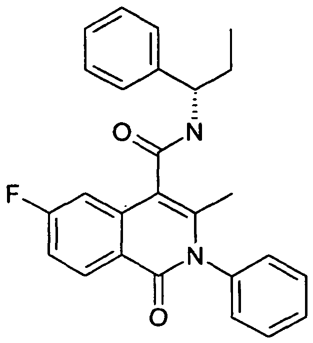 ***1e** 6-Fluoro-3-methyl-1-oxo-2-phenyl-1,2-dihydro-isoquinoline-4-carboxylic acid ((S)-1-phenyl-propyl)-amide.*

LC-MS (m/z) 415.5 (MH⁺); t_{R} = 0.80 (method B). 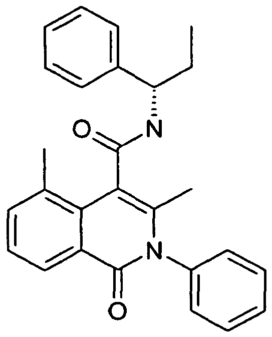 ***1f** 3.5-Dimethyl-1-oxo-2-phenyl-1,2-dihydro-isoquinoline-4-carboxy*/*ic acid ((S)-1-phenyl-propyl)-amide.*

LC-MS (m/z) 411.3 (MH⁺); t_{R} = 0.84 (method B). 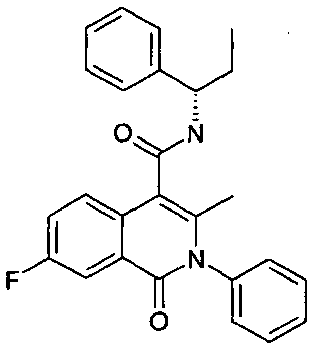 ***1g** 7-Fluoro-3-methyl-1-oxo-2-phenyl-1,2-dihydro-isoquinoline-4-carboxylic acid ((S)-1-phenyl-propyl)-amide.*

LC-MS (m/z) 415.4 (MH⁺); t_{R} = 1.49. 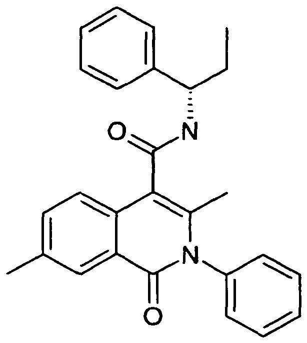 ***1h** 3,7-Dimethyl-1-oxo-2-phenyl-1,2-dihydro-isoquinoline-4-carboxylic acid ((S)-1-phenyl-propyl)-amide.*

LC-MS (m/z) 411.4 (MH⁺); t_{R} = 1.5. 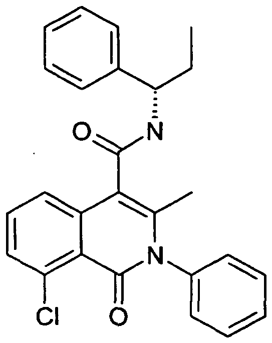 ***1i** 8-Chloro-3-methyl-1-oxo-2-phenyl-1,2-dihydro-isoquinoline-4-carboxylic acid ((S)-1-phenyl-propyl)-amide.*

LC-MS (m/z) 431.4 (MH⁺); t_{R} = 1.54. 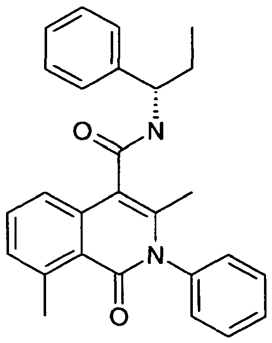 ***1j** 3,8-Dimethyl-1-oxo-2-phenyl-1,2-dihydro-isoquinoline-4-carboxylic acid ((S)-1-phenyl-propyl)-amide.*

LC-MS (m/z) 411.4 (MH⁺); t_{R} = 1.5. ¹H NMR (250 MHz, DMSO-d₆, T=343K): 0.91 (t, 3H), 1.78 (overlapping s, 3H), 1.79 (overlapping m, 1H), 2.75 (s, 3H), 4.94 (q, 1H), 7.15-7.27 (m, 5H), 7.27-7.39 (m, 4H), 7.41-7.58 (m, 4H), 8.67 (d, 1H). 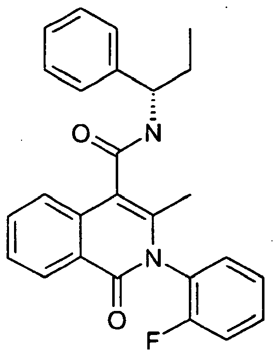 ***1k** 2-(2-Fluoro-phenyl)-3-methyl-1-oxo-1,2-dihydro-isoquinoline-4-carboxylic acid ((S)-1-phenyl-propyl)-amide.*

LC-MS (m/z) 415.5 (MH⁺); t_{R} = 1.49. 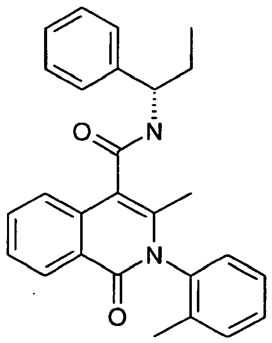 ***1l** 3-Methyl-1-oxo-2-o-tolyl-1,2-dihydro-isoquinolinse-4-carboxylic acid ((S)-1-phenyl-propyl)-amide.*

LC-MS (m/z) 411.5 (MH⁺); t_{R} = 1.5. 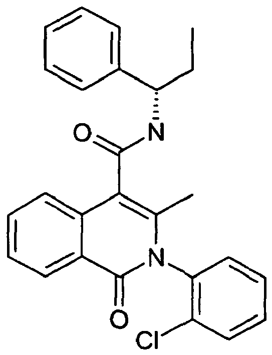 ***1m** 2-(2-Chloro-phenyl)-3-methyl-1-oxo-1,2-dihydro-isoquinoline-4-carboxylic acid ((S)-1-phenyl-propyl)-amide.*

LC-MS (m/z) 431.2 (MH⁺); t_{R} = 1.51. 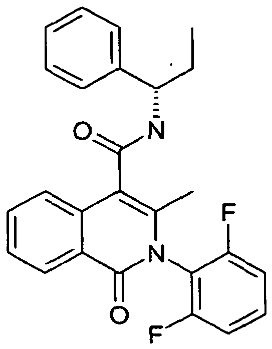 ***1n** 2-(2,6-Difluoro-phenyl)-3-methyl-1-oxo-1,2-dihydro-isoquinoline-4-carboxylic acid ((S)-1-phenyl-propyl)-amide.*

LC-MS (m/z) 433.4 (MH⁺); t_{R} = 1.54. 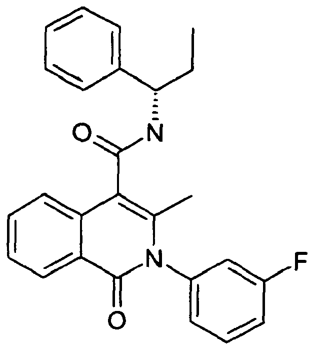 ***1o** 2-(3-Fluror-phenyl)-3-methyl-1-oxo-1,2-dihydro-isoquinoline-4-carboxylic acid ((S)-1-phenyl-propyl)-amide.*

LC-MS (m/z) 415.1 (MH⁺); t_{R} = 1.48. 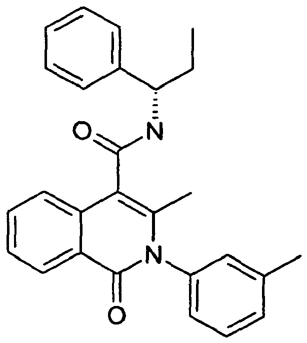 ***1p** 3-Methyl-1-oxo-2-m-tolyl-1,2-dihydro-isoquinoline-4-carboxylic acid ((S)-1-phenyl-propyl)-amide.*

LC-MS(m/z) 411.5 (MH⁻): t_{R} = 1.53. 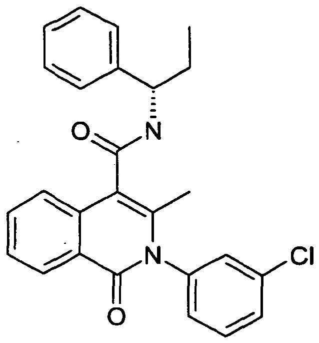 ***1g** 2-(3-Chloro-phenyl)-3-methyl-1-oxo-1,2-dihydro-isoquinoline-4-carboxylic acid ((S)-1-phenyl-propyl)-amide.*

LC-MS (m/z) 431.2 (MH⁺); t_{R} = 1.57. 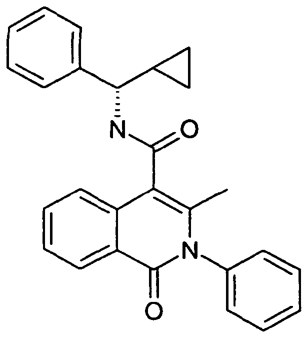 ***1r** 3-Methyl-1-oxo-2-phenyl-1,2-dihydro-isoquinoline-4-carboxylic acid ((S)-cyclopropylphenyl-methyl)-amide.*

LC-MS (m/z) 409.4 (MH⁺); t_{R} = 1.43. ¹H NMR (250 MHz, DMSO-d₆, T=343K): 0.45 (m, 2H), 0.56 (m, 2H), 1.25 (m, 1H), 1.88 (s, 3H), 4.51 (t, 1H), 7.21-7.37 (m, 5H), 7.42-7.59 (m, 7H), 7.7 (dt, 1H), 8.21 (dd, 1H), 8.89 (d, 1H). 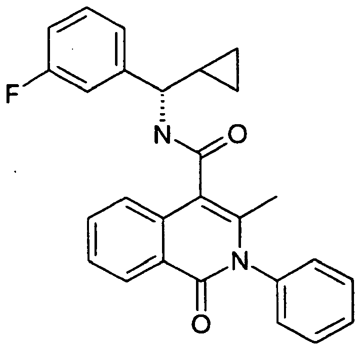 ***1s** 3-Methyl-1-oxo-2-phenyl-1,2-dihydro-isoquinoline-4-carboxylic acid [(S)-cyclopropyl-(3-fluoro-phenyl)-methyl]-amide.*

LC-MS (m/z) 427.1 (MH⁺); t_{R} = 1.48. ¹H NMR (250 MHz, DMSO-d₆, T=343K): 0.48 (m, 2H), 0.57 (m, 2H), 1.24 (m, 1H), 1.89 (s, 3H), 4.51 (t, 1H), 7.05 (m, 1H), 7.21-7.31 (m, 4H), 7.33-7.6 (m, 6H), 7.7 (dt, 1H), 8.21 (dd, 1H), 8.94 (d, 1H). 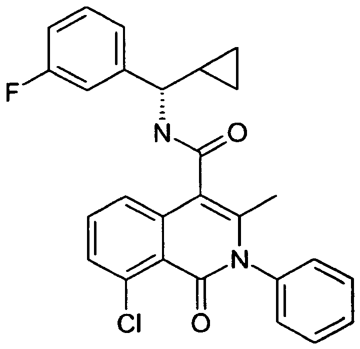 ***1t** 8-Chloro-3-methyl-1-oxo-2-phenyl-1,2-dihydro-isoquinoline-4-carboxylic acid [(S)-cyclopropyl-(3-fluoro-phenyl)-methyl]-amide.*

LC-MS (m/z) 461.6 (MH⁺); t_{R} = 1.51. ¹H NMR (250 MHz, DMSO-d₆, T=343K): 0.48 (m, 2H), 0.58 (m, 2H), 1.27 (m, 1H), 1.87 (s, 3H), 4.5 (t, 1H), 7.06 (m, 1H), 7.21-7.32 (m, 4H), 7.32-7.45 (m, 2H), 7.45-7.66 (m, 5H), 8.95 (d, 1H). 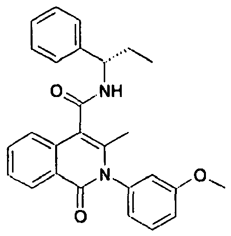 **1aa** 2-(3-Methoxy-phenyl)-3-methyl-1-oxo-1,2-dihydro-isoquinoline-4-carboxylic acid ((S)-1-phenyl-propyl)-amide.

LC-MS (m/z) 426.7 (MH⁺); t_{R} = 1.43. 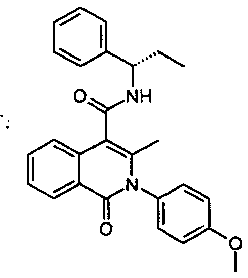 **1ab** 2-(4-Methoxy-phenyl)-3-methyl-1-oxo-1,2-dihydro-isoquinoline-4-carboxylic acid ((S)-1-phenyl-propyl)-amide.

LC-MS (m/z) 427.0 (MH⁺); t_{R} = 1.43. 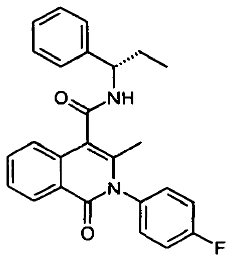 **1ac** 2-(4-Fluoro-phenyl)-3-methyl-1-oxo-1,2-dihydro-isoquinoline-4-carboxylic acid ((S)-1-phenyl-propyl)-amide.

LC-MS (m/z) 415.0 (MH⁺); t_{R} = 1.43. 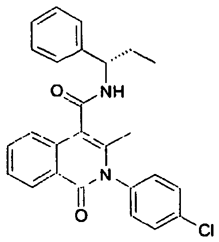 **1ad** 2-(4-Chloro-phenyl)-3-methyl-1-oxo-1,2-dihydro-isoquinoline4-carboxylic acid ((S)-1-phenyl-propyl)-amide.

LC-MS (m/z) 431.1 (MH⁺); t_{R} = 1.54. 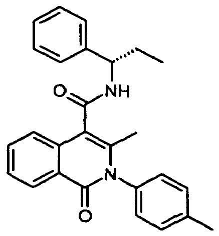 **1ae** 3-Methyl-1-oxo-2-p-tolyl-1,2-dihydro-isoquinoline-4-carboxylic acid ((S)-1-phenyl-propyl)-amide.

LC-MS (m/z) 411.2 (MH⁺); t_{R} = 1.51. 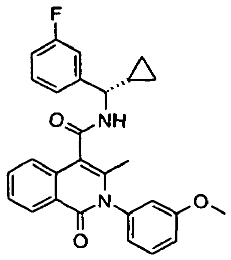 **1af** 2-(3-Methoxy-phenyl)-3-methyl-1-oxo-1,2-dihydro-isoquinoline-4-carboxylic acid [(S)-cyclopropyl-(3-fluoro-phenyl)-methyl]-amide.

LC-MS (m/z) 457.0 (MH⁺); t_{R} = 1.47. 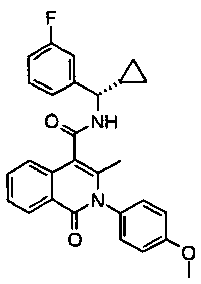 **1ag** 2-(4-Methoxy-phenyl)-3-methyl-1-oxo-1,2-dihydro-isoquinoline-4-carboxylic acid [(S)-cyclopropyl-(3-fluoro-phenyl)-methyl]-amide.

LC-MS (m/z) 457.1 (MH⁺); t_{R} = 1.46. 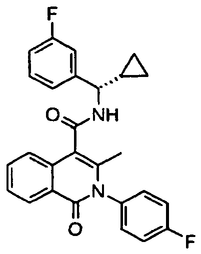 **1ah** 2-(4-Fluoro-phenyl)-3-methyl-1-oxo-1,2-dihydro-isoquinoline-4-carboxylic acid [(S)-cyclopropyl-(3-fluoro-phenyl)-methyl]-amide.

LC-MS (m/z) 445.6 (MH⁺); t_{R} = 1.48. 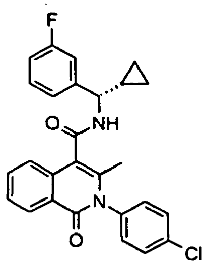 **1ai** 2-(4-Chloro-phenyl)-3-methyl-1-oxo-1,2-dihydro-isoquinoline-4-carboxylic acid [(S)-cyclopropyl-(3-fluoro-phenyl)-methyl]-amide.

LC-MS (m/z) 461.3 (MH⁺); t_{R} = 1.57. 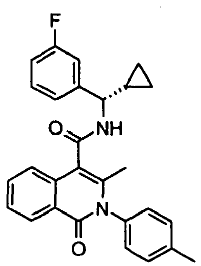 **1aj** 3-Methyl-1-oxo-2-p-tolyl-1,2-dihydro-isoquinoline-4-carboxylic acid [(S)-cyclopropyl-(3-fluoro-phenyl)-methyl]-amide.

LC-MS (m/z) 440.9 (MH⁺); t_{R} = 1.54. 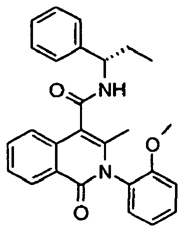 **1ak** 2-(2-Methoxy-phenyl)-3-methyl-1-oxo-1,2-dihydro-isoquinoline-4-carboxylic acid ((S)-1-phenyl-propyl)-amide.

LC-MS (m/z) 427.0 (MH⁺); t_{R} = 1.39. 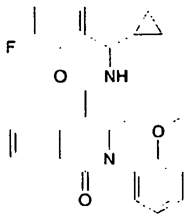 **1al** 2-(2-Methoxy-phenyl)-3-methyl-1-oxo-1,2-dihydro-isoquinoline-4-carboxylic acid [(S)-cyclopropyl-(3-fluoro-phenyl)-methyl]-amide.

*LC-MS (m*/*z) 457.4 (MH⁺); t_{R}* = *1.43.* 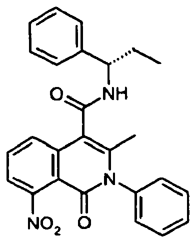 ***1am** 3-Methyl-8-nitro-1-oxo-2-phenyl-1,2-dihydro-isoquinoline-4-carboxylic acid ((S)-1-phenyl-propyl)-amide.*

*LC-MS (m*/*z) 442.4 (MH⁺); t_{R} = 1.46.* 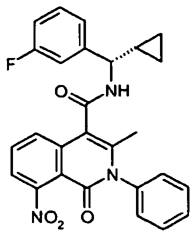 ***1an** 3-Methyl-8-nitro-1-oxo-2-phenyl-1,2-dihydro-isoquinoline-4-carboxylic acid [(S)-cyclopropyl-(3-fluoro-phenyl)-methyl]-amide.*

LC-MS (m/z) 442.4 (MH⁺); t_{R} = 1.46. 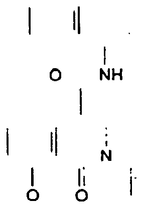 ***1ao** 8-Methoxy-3-methyl-1-oxo-2-phenyl-1,2-dihydro-isoquinoline-4-carboxylic acid ((S)-1-phenyl-propyl)-amide.*

LC-MS (m/z) 437.1 (MH⁺); t_{R} = 1.32. 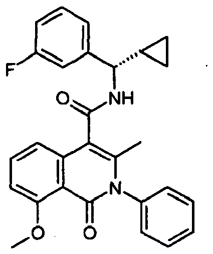 ***1ap** 8-Methoxy-3-methyl-1-oxo-2-phenyl-1,2-dihydro-isoquinoline-4-carboxylic acid [(S)-cyclopropyl-(3-fluoro-phenyl)-methyl]-amide.*

LC-MS (m/z) 457.4 (MH⁺); t_{R} = 1.36. 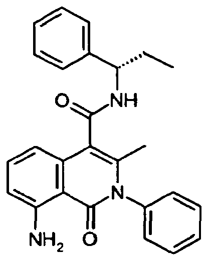 ***1aq** 8-Amino-3-methyl-1-oxo-2-phenyl-1,2-dihydro-isoquinoline-4-carboxylic acid ((S)-1-phenyl-propyl)-amide.*

To a stirred solution of 3-methyl-8-nitro-1-oxo-2-phenyl-1,2-dihydro-isoquinoline-4-carboxylic acid ((S)-1-phenyl-propyl)-amide (**1am**, 10 mg, 0.023 mmol) in tetrahydrofurane (0.15 ml) 2M aq. HCl (0.12 ml, 0.25 mmol) and Zn powder (45 mg, 0.69 mmol) was added. After 30 min the mixture was filtered and partitioned between ethyl acetate (4 ml) and sat. aq. NaHCO₃ (2 ml), then brine (3 ml). The organic solution was dried (MgSO₄) and evaporated to afford 5.9 mg of the title compound (70% yield). LC-MS (m/z) 412.4 (MH⁺); t_{R} = 1.37. 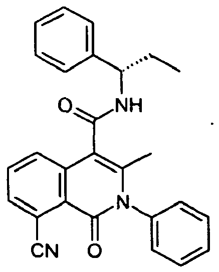 ***1ar** 8-Cyano-3-methyl-1-oxo-2-phenyl-1,2-dihydro-isoquinoline-4-carboxylic acid ((S)-1-phenyl-propyl)-amide.*

LC-MS (m/z) 422.1 (MH⁺); t_{R} = 1.37.

The following compounds were obtained according to the general procedure: the corresponding acid of the general formula IX (0.04 mmol) and amine of the general formula XI (0.06 mmol) in DMF (0.5 ml was stirred in the presence of Et₃N (3 eq.), EDC (1.5 cq.), and HOBT (1.5 cq.) at r.t. overnight followed by partitioning between ethyl acetate (2 ml) and 0.5 M NaOH (1 ml) and flash chromatography on SiO₂.

| | **Chemical name** | **structure** | **t_{R} (min)** | **MW** | **m/z (MH⁺)** |
|---|---|---|---|---|---|
| **1as** | *8-Chloro-3-methyl-1-oxo-2-phenyl-1,2-dihydro-isoquinoline-4-carboxylic acid [(S)-1-(4-chloro-pheny)*/*-propyl]-amide* | 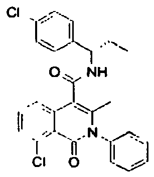 | 1.63 | 465.4 | 465.4 |
| **1at** | *8-Chloro-3-methyl-1-oxo-2-phenyl-1,2-dihydro-isoquinoline-4-carboxylic acid [(S)-1-(4-fluoro-pheny*/*)-propyl]-amide* | 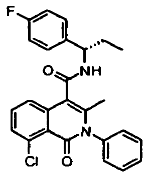 | 1.52 | 448.9 | 449.3 |
| **1au** | *8-Chloro-3-methyl-1-oxo-phenyl-1,2-dihydro-isoquinoline-4-carboxylic acid [(S)-1-(2-fluoro-phenyl)-propyl]-amide* | 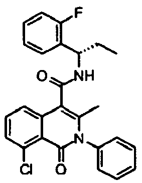 | 1.52 | 448.9 | 449.4 |
| **1av** | *8-Chloro-3-methyl-1-oxo-2-phenyl 1,2-dihydro-isoquinoline-4-carboxylic acid [(S)-1-(3-chloro-phenyl)-propyl]-amide* | 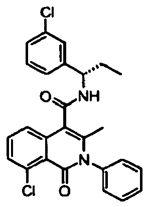 | 1.62 | 465.4 | 465.3 |
| **1aw** | *8-Chloro-3-methyl-1-oxo-2-phenyl-1,2-dihydro-isoquinoline-4-carboxylic acid [(S)-(3-chloro-phenyl)-cyclopropyl-methyl]-amide* | 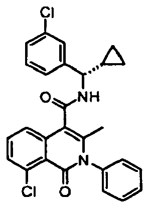 | 1.64 | 477.4 | 477.3 |
| **1ax** | *8-Chloro-3-methyl-1-oxo-2-phenyl-1,2-dihydro-isoquinoline-4-carboxylic acid [(S)-(4-chloro-phenyl)-cyclopropyl-methyl]-amide* | 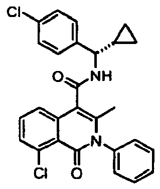 | 1.64 | 477.4 | 477.4 |
| **1ay** | *8-Chloro-3-methyl-1-oxo-2-phenyl-1,2-dihydro-isoquinoline-4-carboxylic acid [(S)-1-(3-fluoro-phenyl)-propyl]-amide* | 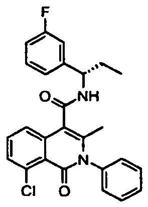 | 1.53 | 448.9 | 449.3 |
| **1az** | *8-Chloro-3-methyl-1-oxo-2-phenyl-1,2-dihydro-isoquinoline-4-carboxylic acid ((S)-2-methyl-1-phenyl-propyl)-amide* | 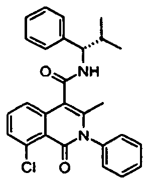 | 1.59 | 444.96 | 445.7 |
| **1ba** | *8-Chloro-3-methyl-1-oxo-2-phenyl-1,2-dihydro-isoquinoline-4-carboxylic acid [(S)-cyclopropyl-(4-fluoro-phenyl)-methyl]-amide* | 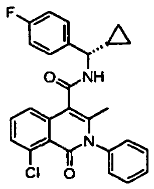 | 1.53 | 460.9 | 461.5 |
| **1bb** | *8-Chloro-3-methyl-1-oxo-2-phenyl-1,2-dihydro-isoquinoline-4-carboxylic acid [(S)-1-(2-chloro-phenyl)-propyl]-amide* | 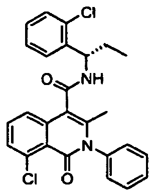 | 1.61 | 465.4 | 465.3 |
| **1bc** | *8-Chloro-3-methyl-1-oxo-2-phenyl-1,2-dihydro-isoquinoline-4-carboxylic acid ((S)-cyclopentyl-phenyl-methyl)-amide* | 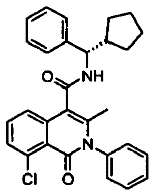 | 1.72 | 470.99 | 471.8 |
| **1bd** | *8-Chloro-3-methyl-1-oxo-2-phenyl-1,2-dihydro-isoquinoline-4-carboxylic acid [(S)-(2-chloro-phenyl)-cyclopropyl-methyl]-amide* | 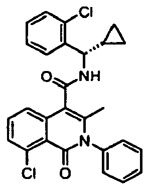 | 1.6 | 477.4 | 477.2 |
| **1be** | *8-Chloro-3-methyl-1-oxo-2-phenyl-1,2-dihydro-isoquinoline-4-carboylic acid [(S)-cyclopropyl-(2fluoro-phenyl)-methyl]-amide* | 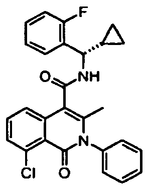 | 1.6 | 460.9 | 461.6 |
| **1bf** | *8-Chloro-3-methyl-1-oxo-2-phenyl-1,2-dihydro-isoquinoline-4-carboxylic acid ((S)-cyclohexyl-phenyl-methyl)-amide* | 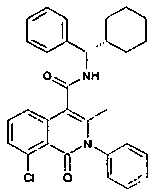 | 1.8 | 485.0 | 485.4 |
| **1bg** | *8-Chloro-3-methyl-1-oxo-2-phenyl-1,2-dihydro-isoquinoline-4-carboxylic acid ((S)-cyclopropyl-phenyl-methyl)-amide* | 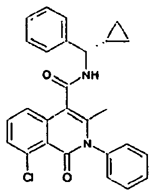 | 1.5 | 442.9 | 443.5 |
| **1bh** | *8-Chloro-3-methyl-1-oxo-2-phenyl-1,2-dihydro-isoquinoline-4-carboxylic acid [(S)-cyclobutyl-(3-fluoro-phenyl)-methyl]-amide* | 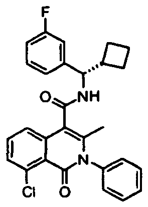 | 1.65 | 474.96 | 475.5 |
| **1bi** | *8-Chloro-3-methyl-1-oxo-2-phenyl-1,2-dihydro-isoquinoline-4-carboxylic acid [(S)-cyclobutyl-(4-fluoro-phenyl)-methyl]-amide* | 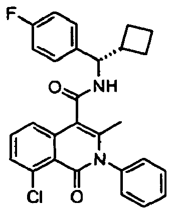 | 1.64 | 474.96 | 475.5 |
| **1bl** | *8-Chloro-3-methyl-1-oxo-2-phenyl-1,2-dihydro-isoquinoline-4-carboylic acid [(R)-cyclopropyl-(3-fluoro-phenyl)-methyl]-amide* | 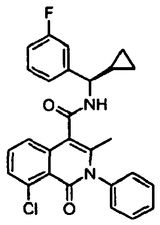 | 1.52 | 460.9 | 461.5 |
| **1bn** | *8-Chloro-3-methyl-1-oxo-2-phenyl-1,2-dihydro-isoquinoline-4-carboxylic acid ((S)cyclobutyl-phenyl-methyl)-amide* | 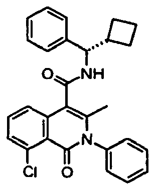 | 1.61 | 456.97 | 457.3 |
| **1bo** | *8-Chloro-3-methyl-1-oxo-2-phenyl-1,2-dihydro-isoquinoline-4-carboxylic acid [cyclobutyl-(2-fluoro-phenyl)methyl]-amide* | 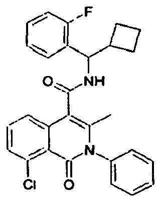 | 1.64 | 474.96 | 475.4 |

### Example 2: Preparation of intermediates

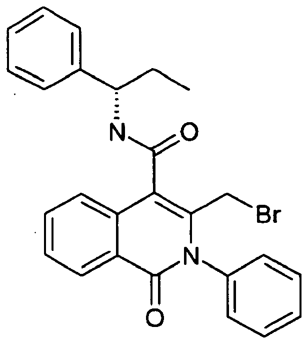

*3-Bromomethyl-1-oxo-2-phenyl-1,2-dihydro-isoquinoline-4-carboxylic acid ((S)-1-phenyl-propyl)-amide.*

To a suspension of CaCO₃ (500 mg, 5 mmol) and 3-methyl-1-oxo-2-phenyl-1,2-dihydro-isoquinoline-4-carboxylic acid ((S)-1-phenyl-propyl)-amide (115 mg, 0.29 mmol) in 1,2-dichloroethane (5 ml) bromine (600 mg, 3.75 mmol) was added. The obtained mixture was sonicated at +33°C for 2 hours, diluted with 1,2-dichloroethane (20 ml) and poured into silica gel column (5 g). The excess of bromine was eluted with 1,2-dichloroethane and the product was eluted with 1:1 ethyl acetate - heptane to give 135 mg of brown solid. It was dissolved in ethyl acetate (0.5 ml) and precipitated with heptane (20 ml) to give 100 mg of yellow-brown solid. Yield 72%. LC-MS (m/z) 475.2 (MH⁺, ⁷⁹Br); t_{R} = 1.59. ¹H NMR (500 MHz, DMSO-d₆, a mixture of two interconverting rotamers): 0.95 (br, 3H, CH₃), 1.74 and 1.82 (two unres. m, 2 x 1H, CH₂), 4.0 (unres. m, 1H, CH₂Br), 4.21 and 4.35 (two unres. m, 2 x 0.5H, CH₂Br), 4.97 (q, 1H, CH), 7.1 (br, 0.5H), 7.24 (br, 0.5H), 7.28-7.21 (br m, 11.5H), 7.89 (br, 0.5H), 8.25 (br, 1H), 9.28 (d, 1H, NH).

The following compounds were obtained analogously: 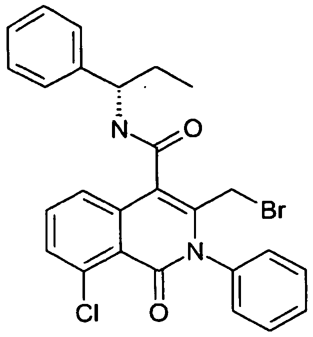 *3-Bromomethyl-8-chloro-1-oxo-2-phenyl-1,2-dihydro-isoquinoline-4-carboxylic acid ((S)-1-phenyl-propyl)-amide.*

The crude product was used in the next step without further purification. LC-MS (m/z) 511.3 (MH⁺, ⁷⁹Br); t_{R} = 1.68. 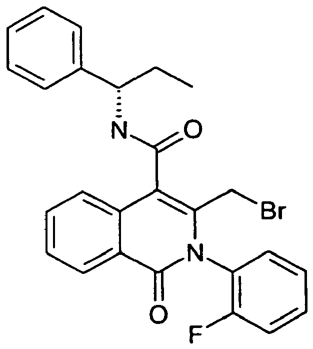 *3-Bromomethyl-2-(2-fluoro-phenyl)-1-oxo-1,2-dihydro-isoquinoline-4-carboxylic acid ((S)-1-phenyl-propyl)-amide.*

The crude product was used in the next step without further purification. LC-MS (m/z) 493.3 (MH⁻, ⁷⁹Br); t_{R} = 1.62. 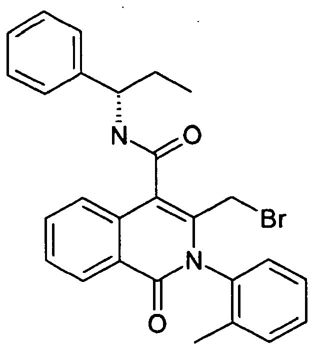 *3-Bromomethyl-1-oxo-2-o-tolyl-1,2-dihydro-isoquinoline-4-carboxylic acid ((S)-1-phenyl-propyl)-amide.*

The crude product was used in the next step without further purification. LC-MS (m/z) 489.3 (MH⁺, ⁷⁹Br); t_{R} = 1.65. 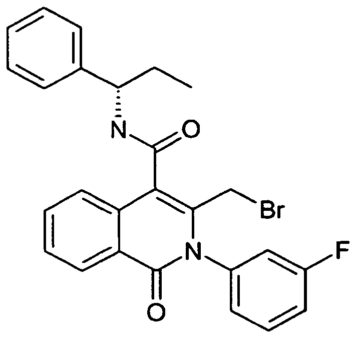 *3-Bromomethyl-2-(3-fluoro-phenyl)-1-oxo-1,2-dihydro-isoquinoline-4-carboxylic acid ((S)-1-phenyl-propyl)-amide.*

LC-MS (m/z) 493.5 (MH⁺, ⁷⁹Br); t_{R} = 0.80 (method B). 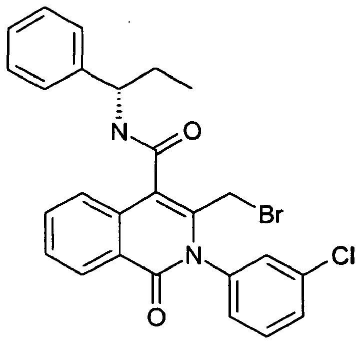 *3-Bromomethyl-1-(3-chloro-phenyl)-1-oxo-1,2-dihydro-isoquinoline-4-carboxylic acid ((S)-1-phenyl-propyl)-amide.*

LC-MS (m/z) 511.5 (MH⁺, ⁸¹Br); t_{R} = 0.84 (method B). 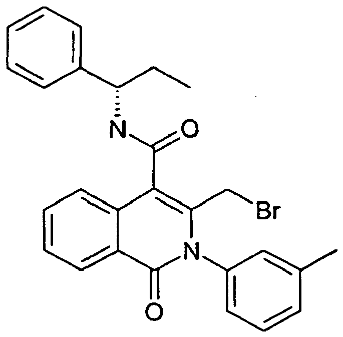 *3-Bromomethyl-1-oxo-2-m-tolyl-1,2-dihydro-isoquinoline-4-carboxylic acid ((S)-1-phenyl-propyl)-amide.*

LC-MS (m/z) 489.2 (MH⁺, ⁷⁹Br): t_{R} = 0.86 (method B). 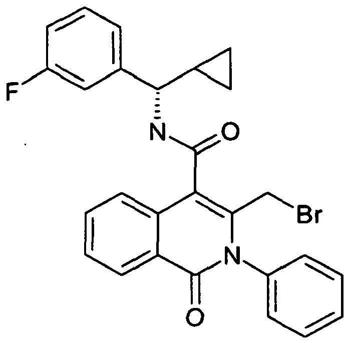 *3-Bromomethyl-1-oxo-1-phenyl-1,2-dihydro-isoquinoline-4-carboxylic acid [(S)-cyclopropyl-(3-fluoro-phenyl)-methyl]-amide.*

LC-MS (m/z) 505.1 (MH⁺); t_{R} = 1.62. 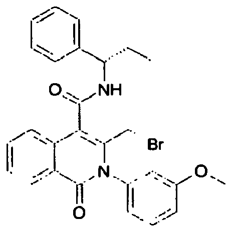 *3-Bromomethyl-2-(3-methoxy-phenyl)-1-oxo-1,2-dihydro-isoquinoline-4-carboxylic acid ((S)-1-phenyl-propyl)-amide.*

The product was purified by flash chromatography on SiO₂ (gradient heptane - ethyl acetate) and used in the next step directly. 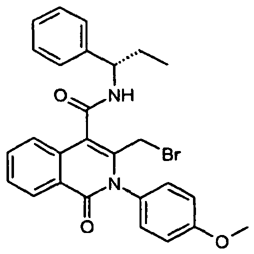 *3-Bromomethyl-2-(4-methoxy-phenyl)-1-oxo-1,2-dihydro-isoquinoline-4-carboxylic acid ((S)-1-phenyl-propyl)-amide.*

The product was purified by flash chromatography on SiO₂ (gradient heptane - ethyl acetate). LC-MS (m/z) 505.1 & 507.2 (MH⁺); t_{R} = 1.52. 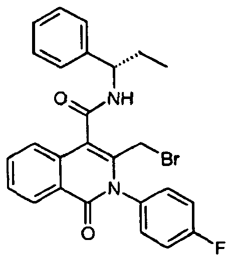 *3-Bromomethyl-2-(4-fluoro-phenyl)-1-oxo-1,2-dihydro-isoquinoline-4-carboxylic acid ((S)-1-phenyl-propyl)-amide.*

The product was purified by flash chromatography on SiO₂ (gradient heptane - ethyl acetate). LC-MS (m/z) 493.3 & 495.3 (MH⁺); t_{R} = 1.54. 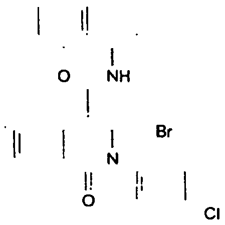 *3-Bromomethyl-2-(4-chloro-phenyl)-1-oxo-1,2-dihydro-isoquinoline-4-carboxylic acid ((S)-1-phenyl-propyl)-amide.*

LC-MS (m/z) 509.2 & 511.1 (MH⁺); t_{R} = 1.63. 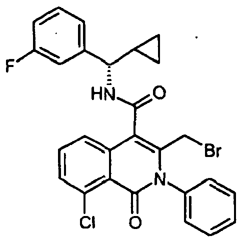 *3-Bromomethyl-8-chloro-1-oxo-2-phenyl-1,2-dihydro-isoquinoline-4-carboxylic acid [(S)-cyclopropyl-(3-fluoro-phenyl)-methyl]-amide.*

LC-MS (m/z) 539.1 & 541.4 (MH⁺); t_{R} = 1.69. 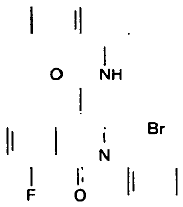 *3-Bromomethyl-8-fluoro-1-oxo-2-phenyl-1,2-dihydro-isoquinoline-4-carboxylic acid ((S)-1-phenyl-propyl)-amide.*

LC-MS (m/z) 493.3 & 495.2 (MH⁺); t_{R} = 1.52. 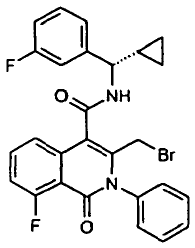 *3-Bromomethyl-fluoro-1-oxo-2-phenyl-1,2-dihydro-isoquinoline-4-carboxylic acid [(S)-cyclopropyl-(3-fluoro-phenyl)-methyl]-amide.*

LC-MS (m/z) 523.5 & 525.6 (MH⁺); t_{R} = 1.56.

### Compounds of the invention

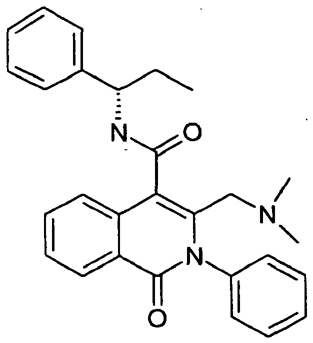

*2a 3-Dimethylarrtinomethyl-1-oxo-2 phenyl-1,2-dihydro-isoquinoline-4-carboxylic acid ((S)-1-phenyl-propyl)-amide.*

A mixture of 3-bromomethyl-1-oxo-2-phenyl-1,2-dihydro-isoquinoline-4-carboxylic acid ((S)-1-phenyl-propyl)-amide (70 mg, 0.147 mmol) and dimethylamine (1 ml of 33% solution in abs. ethanol, excess) was kept at 50°C for 10 min and then evaporated in vacuo. The residue was dissolved in methanol and passed through SGX cation exchange column (1 g, RSO₃H form). Uncharged impurities were eluted with methanol and the product was eluted with 4M NH₃ in methanol to give 53 mg of colourless glass-like solid. It was further purified by flash chromatography on SiO₂ (5 g, gradient 1,2-dichloroethane - 10% ethyl acetate in 1,2-dichloroethane) to give 45 mg of colourless solid. Yield 70%. LC-MS (m/z) 440.1 (MH⁺); t_{R} = 0.88. ¹H NMR (250 MHz, DMSO-d₆, T = 333 K): 0.92 (t, 2H), 1.83 (two overlapping m, 2H), 1.66 (s, 6H), 2.96 and 3.08 (two d of CH_{A}H_{B}N, 2H), 4.96 (q, 1H), 7.19-7.56 (m, 12H), 7.68 (t, 1H), 8.21 (d, 1H), 8.75 (d, 1H). 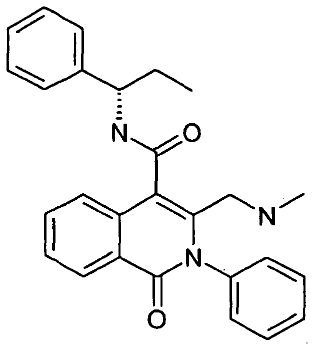 ***2b** 3-Methylaminomethyl-1-oxo-2-phenyl-1,2-dihydro-isoquinoline-4-carboxylic acid ((S)-1-phenyl-propyl)-amide.*

A mixture of 3-bromomethyl-1-oxo-2-phenyl-1,2-dihydro-isoquinoline-4-carboxylic acid ((S)-1-phenyl-propyl)-amide (4.7 mg, 0.01 mmol and methylamine (1 ml of 2M solution in tetrahydrofurane, excess) was kept at ambient temperature for 20 min and then evaporated in vacuo. The product was used for biological testing without further purification. LC-MS (m/z) 426.3 (MH⁺): t_{R} = 0.85.

The following 3-aminomethyl derivatives were prepared analogously from corresponding 3-bromomethyl derivatives and 1.2 - 5 molar equivalents of appropriate aliphatic or aromatic primary or secondary amine in tetrahydrofurane as a solvent at ambient temperature. The reactions were monitored by LC-MS. In several cases longer reaction times were used or the reaction mixture was heated to 50°C and/or diisopropylethylamine (2 eq.) was added as a base. After evaporation of the reaction mixture the compounds were of sufficient purity as detected by analytical LC-MS; otherwise further purification by SCX column or by preparative LC-MS was performed.

### List of compounds from example 2

| | **Chemical name** | **t_{R} (min)** | **MW** | **m/z (MH+)** |
|---|---|---|---|---|
| **2c** | 3-Ethylaminomethyl-1-oxo-2-phenyl-1,2-dihydro-isoquinoline-4-carboxylic acid ((S)-1-phenyl-propyl)-amide | 0.45 (method B) | 439.6 | 440.6 |
| **2d** | 3-Cyclopropylaminomethyl-1-oxo-2-phenyl-1,2-dihydro-isoquinoline-4-carboxylic acid ((S)-1-phenyl-propyl)-amide | 0.49 (method B) | 451.6 | 452.1 |
| **2e** | 3-[(Cyclopropylmethyl-amino)-methyl]-1-oxo-2-phenyl-1,2-dihydro-isoquinoline-4-carboxylic acid ((S)-1-phenyl- propyl)-amide | 0.51 (method B) | 465.6 | 466.4 |
| **2f** | 3-(3,6-Dihydro-2H-pyridin-1-ylmethyl)-1-oxo-2-phenyl-1,2-dihydro-isoquinoline-4-carboxylic acid ((S)-1-phenyl-propyl)-amide | 0.99 | 477.6 | 478.1 |
| **2g** | 3-[4-(2-Methoxy-phenyl)-piperazin-1-ylmethyl]-1-oxo-2-phenyl-1,2-dihydro-isoquinoline-4-carboxylic acid ((S)-1-phenyl-propyl)-amide | 1.14 | 586.7 | 587.5 |
| **2h** | 3-[4-(4-Fluoro-phenyl)-piperazin-1-ylmethyl]-1-oxo-2-phenyl-1,2-dihydro-isoquinoline-4-carboxylic acid ((S)-1-phenyl-propyl)-amide | 1.35 | 574.7 | 575.6 |
| **2i** | 3-(4-Fomyl-piperazin-1-ylmethyl)-1-oxo-2-phenyl-1,2-dihydro-isoquinoline-4-carboxylic acid ((S)-1-phenyl-propyl)-amide | 1.12 | 508.6 | 509.3 |
| **2j** | 4-[1-Oxo-2-phenyl-4-(1-phenyl-propylcarbamoyl)-1,2-dihydro-isoquinolin-3-ylmethyl]-piperazine-1-carboxylic acid ethyl ester | 1.33 | 552.7 | 553.7 |
| **2k** | 3-(4-Methyl-piperazin-1-ylmethyl)-1-oxo-2-phenyl-1,2-dihydro-isoquinoline-4-carboxylic acid ((S)-1-phenyl-propyl)-amide | 0.87 | 494.6 | 495.7 |
| **2l** | 1-Oxo-2-phenyl-3-(1,3,4,9-tetrahydro-beta-carbolin-2-ylmethyl)-1,2-dihydro-isoquinoline-4-carboxylic acid ((S)-1-phenyl-propyl)-amide | 1.27 | 566.7 | 567.7 |
| **2m** | 1-Oxo-2-phenyl-3-piperazin-1-ylmethyl-1,2-dihydro-isoquinoline-4-carboxylic acid ((S)-1-phenyl-propyl)-amide | 0.86 | 480.6 | 481.2 |
| **2n** | 3-(3-Methyl-piperazin-1-ylmethyl)-1-oxo-2-phenyl-1,2-dihydro-isoquinoline-4-carboxylic acid ((S)-1-phenyl-propyl)-amide | 0.88 | 494.6 | 495.7 |
| **2o** | 3-(3,5-Dimethyl-piperazin-1-ylmethyl)-1-oxo-2-phenyl-1,2-dihydro-isoquinoline-4-carboxylic acid ((S)-1-phenyl-propyl)-amide | 0.9 | 508.7 | 509.3 |
| **2p** | 3-(4-Benzyl-piperazin-1-ylmethyl)-1-oxo-2-phenyl-1,2-dihydro-isoquinoline-4-carboxylic acid ((S)-1-phenyl-propyl)-amide | 1.02 | 570.7 | 571.5 |
| **2q** | 1-Oxo-3-[4-(2-oxo-2-pyrrolidin-1-yl-ethyl)-piperazin-1-ylmethyl]-2-phenyl-1,2-dihydro-isoquinoline-4-carboxylic acid ((S)-1-phenyl-propyl)-amide | 0.92 | 591.8 | 592.4 |
| **2r** | 3-Morpholin-4-ylmethyl-1-oxo-2-phenyl-1,2-dihydro-isoquinolinc-4-carboxylic acid ((S)-1-phenyl-propyl)-amide | 1.08 | 481.6 | 482.2 |
| **2s** | 3-(2,6-Dimethyl-morpholin-4-ylmethyl)-1-oxo-2-phenyl-1,3-dihydro-isoquinoline-4-carboxylic acid ((S)-1-phenyl-propyl)-amide | 1.21 | 509.6 | 510.2 |
| **2t** | 1-Oxo-2-phenyl-3-thiomorpholin-4-ylmethyl-1,2-dihydro-isoquinoline-4-carboxylic acid ((S)-1-phenyl-propyl)-amide | 1.22 | 497.7 | 498.8 |
| **2u** | 3-(1,4-Dioxa-8-aza-spiro[4.5]dec-8-ylmethyl)-1-oxo-2-phenyl-1,2-dihydro-isoquinoline-4-carboxylic acid ((S)-1-phenyl-propyl)-amide | 1.01 | 537.7 | 538.4 |
| **2v** | 1-Oxo-2-phenyl-3-piperidin-1-ylmethyl-1,2-dihydro-isoquinoline-4-carboxylic acid ((S)-1-phenyl-propyl)-amide | 1 | 479.6 | 480.3 |
| **2w** | 3-(2-Methyl-piperidin-1-ylmethyl)-1-oxo-2-phenyl-1,2-dihydro-isoquinoline-4-carboxylic acid ((S)-1-phenyl-propyl)-amide | 1.07 | 493.6 | 494.4 |
| **2x** | 3-(2,6-Dimethyl-piperidin-1-ylmethyl)-1-oxo-2-phenyl-1,2-dihydro-isoquinoline-4-carboxylic acid ((S)-1-phenylpropyl)-amide | 1.09 | 507.7 | 508.4 |
| **2y** | 3-(2-Hydroxymethyl-piperidin-1-ylmethyl)-1-oxo-2-phenyl-1,2-dihydro-isoquinoline-4-carboxylic acid ((S)-1-phenyl-propyl)-amide | 0.93 | 509.6 | 510.3 |
| **2z** | 1-[1-Oxo-2-phenyl-4-(1-phenyl-propylcarbamoyl)-1,2-dihydro-isoquinolin-3-ylmethyl]-piperidine-3-carboxylic acid ethyl ester | 1.14 | 551.7 | 552.4 |
| **2aa** | 3-(3-Methyl-piperidin-1-ylmethyl)-1-oxo-2-phenyl-1,2-dihydro-isoquinoline-4-carboxylic acid ((S)-1-phenyl-propyl)-amide | 1.09 | 493.6 | 494.5 |
| **2ab** | 3-(4-Hydroxy-piperidin-1-ylmethyl)-1-oxo-2-phenyl-1,2-dihydro-isoquinoline-4-carboxylic acid ((S)-1-phenyl-propyl)-amide | 0.83 | 495.6 | 496.5 |
| **2ac** | 1-Oxo-2-phenyl-3-(4-phenyl-piperidin-1-ylmethyl)-1,2-dihydro-isoquinoline-4-carboxylic acid ((S)-1-phenyl-propyl)-amide | 1.27 | 555.7 | 556.5 |
| **2ad** | 1-[1-Oxo-2-phenyl-4-(1-phenyl-propylcarbamoyl)-1,2-dihydro-isoquinolin-3-ylmethyl]-piperidine-4-carboxylic acid ethyl ester | 1.11 | 551.7 | 552.5 |
| **2ae** | 3-(4-Methyl-piperidin-1-ylmethyl)-1-oxo-2-phenyl-1,2-dihydro-isoquinoline-4-carboxylic acid ((S)-1-phenyl-propyl)-amide | 1.1 | 493.6 | 494.6 |
| **2af** | 1-oxo-2-phenyl-3-(4-pyridin-2-yl-piperazin-1-ylmethyl)-1,2-dihydro-isoquinoline-4-carboxylic acid ((S)-1-phenyl-propyl)-amide | 0.97 | 557.7 | 558.4 |
| **2ag** | 3-(Octahydro-quinolin-1-ylmethyl)-1-oxo-2-phenyl-1,2-dihydro-isoquinoline-4-carboxylic acid ((S)-1-phenyl-propyl)-amide | 1.18 | 533.7 | 534.4 |
| **2ah** | 3-Azepan-1-ylmethyl-1-oxo-2-phenyl-1,2-dihydro-isoquinoline-4-carboxylic acid ((S)-1-phenyl-propyl)-amide | 1.08 | 493.6 | 494.6 |
| **2ai** | 3-(3-Hydroxy-piperidin-1-ylmethyl)-1-oxo-2-phenyl-1,2-dihydro-isoquinoline-4-carboxylic acid ((S)-1-phenyl-propyl)-amide | 0.88 | 495.6 | 496.5 |
| **2aj** | 3-[4-(2,4-Dimethyl-phenyl)-piperazin-1-ylmethyl]-1-oxo-2-phenyl-1,2-dihydro-isoquinoline-4-carboxylic acid ((S)-1-phenyl-propyl)-amide | 1.45 | 584.8 | 585.5 |
| **2ak** | 3-[4-(3,4-Dimethyl-phenyl)-piperazin-1-ylmethyl]-1-oxo-2-phenyl-1,2-dihydro-isoquinoline-4-carboxylic acid ((S)-1-phenyl-propyl)-amide | 1.29 | 584.8 | 585.6 |
| **2al** | 3-(4-Dimethylamino-piperidin-1-ylmethyl)-1-oxo-2-phenyl-1,2-dihydro-isoquinoline-4-carboxylic acid ((S)-1-phenyl-propyl)-amide | 0.81 | 522.7 | 523.5 |
| **2am** | 3-[4-(2,5-Dimethyl-phenyl)-piperazin-1-ylmethyl]-1-oxo-2-phenyl-1,2-dihydro-isoquinoline-4-carboxylic acid ((S)-1-phenyl-propyl)-amide | 1.47 | 584.8 | 585.6 |
| **2an** | 3-[4-(2-Fluoro-phenyl)-piperazin-1-ylmethyl]-1-oxo-2-phenyl-1,2-dihydro-isoquinoline-4-carboxylic acid ((S)-1-phenyl-propyl)-amide | 1.42 | 574.7 | 575.4 |
| **2ao** | 3-[4-(3-Methoxy-phenyl)-piperazin-1-ylmethyl]-1-oxo-2- phenyl-1,2-dihydro-isoquinoline-4-carboxylic acid ((S)-1-phenyl-propyl)-amide | 1.32 | 586.7 | 587.3 |
| **2ap** | 1-Oxo-2-phenyl-3-(4-m-tolyl-piperazin-1-ylmethyl)-1,2-dihydro-isoquinoline-4-carboxylic acid ((S)-1-phenyl-propyl)-amide | 1.33 | 570.7 | 571.7 |
| **2aq** | 3-[4-(4-Methoxy-phenyl)-piperazin-1-ylmethyl]-1-oxo-2-phenyl-1,2-dihydro-isoquinoline-4-carboxylic acid ((S)-1-phenyl-propyl)-amide | 1.11 | 586.7 | 587.6 |
| **2ar** | 1-Oxo-3-(4-phenethyl-piperazin-1-ylmethyl)-2-phenyl-1,2-dihydro-isoquinoline-4-carboxylic acid ((S)-1-phenyl-propyl)-amide | 1.09 | 584.8 | 585.5 |
| **2as** | 1-Oxo-2-phenyl-3-(4-pyrimidin-2-yl-piperazin-1-ylmethyl)-1,2-dihydro-isoquinoline-4-carboxylic acid ((S)-1-phenyl-propyl)-amide | 1.18 | 558.7 | 559.3 |
| **2at** | 3-[4-(2-Cyano-phenyl)-piperazin-1-ylmethyl]-1-oxo-2-phenyl-1,2-dihydro-isoquinoline-4-carboxylic acid ((S)-1-phenyl-propyl)-amide | 1.43 | 581.7 | 582.7 |
| **2au** | 3-[4-(4-Chloro-phenyl)-piperazin-1-ylmethyl]-1-oxo-2-phenyl-1,2-dihydro-isoquinoline-4-carboxylic acid ((S)-1-phenyl-propyl)-amide | 1.54 | 591.2 | 591.3 |
| **2av** | 3-((1S,3R,5R)-3-Hydroxy-8-aza-bicyclo[3.2.1]oct-8-ylmethyl)-1-oxo-2-phenyl-1,2-dihydro-isoquinoline-4-carboxylic acid ((S)-1-phenyl-propyl)-amide | 0.88 | 521.7 | 522.7 |
| **2aw** | 3-(4-Acetyl-piperazin-1-ylmethyl)-1-oxo-2-phenyl-1,2-dihydro-isoquinoline-4-carboxylic acid ((S)-1-phenyl-propyl)-amide | 1.07 | 522.6 | 523.5 |
| **2ax** | 3-(4-Methyl-[1,4]diazepan-1-ylmethyl)-1-oxo-2-phenyl-1,2-dihydro-isoquinoline-4-carboxylic acid ((S)-1-phenyl-propyl)-amide | 0.89 | 508.7 | 509.2 |
| **2ay** | 3-(4-Ethyl-piperazin-1-ylmethyl)-1-oxo-2-phenyl-1,2-dihydro-isoquinoline-4-carboxylic acid ((S)-1-phenyl-propyl)-amide | 0.89 | 508.7 | 509.2 |
| **2az** | 3-((2S,6R)-2,6-Dimethyl-morpholin-4-ylmethyl)-1-oxo-2-phenyl-1,2-dihydro-isoquinoline-4-carboxylic acid ((S)-1-phenyl-propyl)-amide | 1.21 | 509.6 | 510.2 |
| **2ba** | 3-[4-(2,4-Difluoro-phenyl)-piperazin-1-ylmethyl]-1-oxo-2-phenyl-1,2-dihydro-isoquinoline-4-carboxylic acid ((S)-1-phenyl-propyl)-amide | 1.48 | 592.7 | 593.6 |
| **2bb** | 3-[4-(3-Dimethylamino-propyl)-piperazin-1-ylmethyl]-1-oxo-2-phenyl-1,2-dihydro-isoquinoline-4-carboxylic acid ((S)-1-phenyl-propyl)-amide | 0.76 | 565.8 | 566.7 |
| **2bc** | 3-(4-Isopropyl-piperazin-1-ylmethyl)-1-oxo-2-phenyl-1,2-dihydro-isoquinoline-4-carboxylic acid ((S)-1-phenyl-propyl)-amide | 0.91 | 522.7 | 523.5 |
| **2bd** | 3-(3-Aza-bicyclo[3.2.2]non-3-ylmethyl)-1-oxo-2-phenyl-1,2-dihydro-isoquinoline-4-carboxylic acid ((S)-1-phenyl-propyl)-amide | 1.21 | 519.7 | 520.4 |
| **2be** | 3-(4-Cyclopentyl-piperazin-1-ylmethyl)-1-oxo-2-phenyl-1,2-dihydro-isoquinoline-4-carboxylic acid ((S)-1-phenyl-propyl)-amide | 0.98 | 548.7 | 549.6 |
| **2bf** | 3-[1,4']Bipiperidinyl-1'-ylmethyl-1-oxo-2-phenyl-1,2-dihydro-isoquinoline-4-carboxylic acid ((S)-1-phenyl-propyl)-amide | 0.86 | 562.8 | 563.2 |
| **2bg** | 3-(3,4-Dihydro-1H-isoquinolin-2-ylmethyl)-1-oxo-2-phenyl-1,2-dihydro-isoquinoline-4-carboxylic acid ((S)-1-phenyl-propyl)-amide | 1.22 | 527.7 | 528.7 |
| **2bh** | 3-[4-(2-Dimethylamino-ethyl)-piperazin-1-ylmethyl]-1-oxo-2-phenyl-1,2-dihydro-isoquinoline-4-carboxylic acid ((S)-1-phenyl-propyl)-amide | 0.74 | 551.7 | 552.5 |
| **2bi** | 3-(4-Hydroxymethyl-piperidin-1-ylmethyl)-1-oxo-2-phenyl-1,2-dihydro-isoquinoline-4-carboxylic acid ((S)-1-phenyl-propyl)-amide | 0.86 | 509.6 | 510.3 |
| **2bj** | 1-Oxo-2-phenyl-3-[4-(tetrahydro-furan-2-carbonyl)-piperazin-1-ylmethyl]-1,2-dihydro-isoquinoline-4carboxylic acid ((S)-1-phenyl-propyl)-amide | 1.16 | 578.7 | 579.9 |
| **2bk** | 3-(4-Isobutyl-piperazin-1-ylmethyl)-1-oxo-2-phenyl-1,2-dihydro-isoquinoline-4-carboxylic acid ((S)-1-phenyl-propyl)-amide | 0.98 | 536.7 | 537.4 |
| **2bl** | 3-[4-(2-Methoxy-ethyl)-piperazin-1-ylmethyl]-1-oxo-2-phenyl-1,2-dihydro-isoquinoline-4-carboxylic acid ((S)-1-phenyl-propyl)-amide | 0.91 | 538.7 | 539.6 |
| **2bm** | 3-[4-(2-Morpholin-4-yl-ethyl)-piperazin-1-ylmethyl]-1-oxo-2-phenyl-1,2-dihydro-isoquinoline-4-carboxylic acid ((S)-1-phenyl-propyl)-amide | 0.75 | 593.8 | 594.7 |
| **2bn** | 3-(1,3-Dihydro-isoindol-2-ylmethyl)-1-oxo-2-phenyl-1,2-dihydro-isoquinoline-4-carboxylic acid ((S)-1-phenyl-propyl)-amidc | 1.16 | 513.6 | 514.8 |
| **2bo** | 3-[4-(1-Methyl-piperidin-4-yl)-piperazin-1-ylmethyl]-1-oxo-2-phenyl-1,2-dihydro-isoquinolinc-4-carboxylic acid ((S)-1-phenyl-propyl)-amide | 0.72 | 577.8 | 578.5 |
| **2bp** | 1-Oxo-2-phenyl-3-[4-(2-piperidin-1-yl-ethyl)-piperaz-in-1-ylmethyl]-1,2-dihydro-isoquinoline-4-carboxylic acid ((S)-1-phenyl-propyl)-amide | 0.75 | 591.8 | 592.5 |
| **2bq** | 1-Oxo-2-phenyl-3-[4-(2-pyrrolidin-1-yl-ethyl)-pipcrazin-1-ylmethyl]-1,2-dihydro-isoquinolinc-4-carboxylic acid ((S)-1-phenyl-propyl)-amide | 0.74 | 577.8 | 578.4 |
| **2br** | 3-(4-Dimethylcarbamoylmethyl-piperazin-1-ylmethyl)-1-oxo-2-phenyl-1,2-dihydro-isoquinoline-4-carboxylic acid ((S)-1-phenyl-propyl)-amide | 0.89 | 565.7 | 566.6 |
| **2bs** | 3-(Octahydro-pyrido[1,2-a]pyrazin-2-ylmethyl)-1-oxo-2-phenyl-1,2-dihydro-isoquinoline-4-carboxylic acid ((S)-1-phenyl-propyl)-amide | 0.93 | 534.7 | 535.4 |
| **2bt** | 3-(4-Formyl-[1,4]diazepan-1-ylmethyl)-1-oxo-phenyl-1,2-dihydro-isoquinoline-4-carboxylic acid ((S)-1-phenyl-propyl)-amide | 0.94 | 522.6 | 523.6 |
| **2bu** | 3-[4-(4-Cyano-phenyl)-piperazin-1-ylmethyl]-1-oxo-2-phenyl-1,2-dihydro-isoquinoline-4-carboxylic acid ((S)-1-phenyl-propyl )-amidc | 1.46 | 581.7 | 582.6 |
| **2bv** | 1-Oxo-2-phenyl-3-(4-pyridin-4-ylmethyl-piperazin-1-ylmethyl)-1,2-dihydro-isoquinoline-4-carboxylic acid ((S)-1-phenyl-propyl)-amide | 0.77 | 571.7 | 572.4 |
| **2bw** | 1-Oxo-2-phenyl-3-[4-(3-pyrrolidin-1-yl-propyl)-piperazin-1-ylmethyl]-1,2-dihydro-isoquinoline-4-carboxylic acid ((S)-1-phenyl-propyl)-amide | 0.75 | 591.8 | 592.6 |
| **2bx** | 1-Oxo-2-phenyl-3-(4-pyridin-2-ylmethyl-piperazin-1-ylmethyl)-1,2-dihydro-isoquinoline-4-carboxylic acid ((S)-1-phenyl-propyl)-amide | 0.93 | 571.7 | 572.5 |
| **2by** | 3-(4-Ethanesulfonyl-piperazin-1-ylmethyl)-1-oxo-2-phenyl-1,2-dihydro-isoquinoline-4-carboxylic acid ((S)-1-phenyl-propyl)-amide | 1.38 | 572.7 | 573.7 |
| **2bz** | 3-(4-sec-Butyl-piperazin-1-ylmethyl)-1-oxo-2-phenyl-1,2-dihydro-isoquinoline-4-carboxylic acid ((S)-1-phenyl-propyl)-amide | 0.96 | 536.7 | 537.4 |
| **2ca** | 3-[4-(1-Ethyl-propyl)-piperazin-1-ylmethyl]-1-oxo-2-phenyl-1,2-dihydro-isoquinoline-4-carboxylic acid ((S)-1-phenyl-propyl)-amide | 1.01 | 550.7 | 551.8 |
| **2cb** | 3-[4-(2-Cyano-ethyl)-piperazin-1-ylmethyl]-1-oxo-2-phenyl-1,2-dihydro-isoquinoline-4-carboxylic acid ((S)-1-phenyl-propyl)-amide | 0.9 | 533.7 | 534.4 |
| **2cc** | 3-(4-Methanesulfonyl-piperazin-1-ylmethyl)-1-oxo-2-phenyl-1,2-dihydro-isoquinoline-4-carboxylic acid ((S)-1-phenyl-propyl)-amide | 1.31 | 558.7 | 559.2 |
| **2cd** | {1-[1-Oxo-2-phenyl-4-(1-phenyl-propylcarbamoyl)-1,2-dihydro-isoquinolin-3-ylmethyl]-piperidin-4-yl}-acetic acid ethyl ester | 1.12 | 565.7 | 566.7 |
| **2ce** | 3-[4-(3-Fluoro-phenyl)-piperazin-1-ylmethyl]-1-oxo-2-phenyl-1,2-dihydro-isoquinoline-4-carboxylic acid ((S)-1-phenyl-propyl)-amide | 1.48 | 574.7 | 575.5 |
| **2cf** | 1-Oxo-2-phenyl-3-((S)-3-phenyl-piperidin-1-ylmethyl)-1,2-dihydro-isoquinoline-4-carboxylic acid ((S)-1-phenyl-propyl)-amide | 1.27 | 555.7 | 556.5 |
| **2cg** | 1-Oxo-2-phenyl-3-[4-(pyrrolidine-1-carbonyl)-piperazin-1-ylmethyl]-1,2-dihydro-isoquinoline-4-carboxylic acid ((S)-1-phenyl-propyl)-amide | 1.11 | 577.7 | 578.6 |
| **2ch** | 3-[4-(Morpholine-4-carbonyl)-piperazin-1-ylmethyl]-1-oxo-2-phenyl-1,2-dihydro-isoquinoline-4-carboxylic acid ((S)-1-phenyl-propyl)-amide | 1.04 | 593.7 | 594.7 |
| **2ci** | 3-(4-Methoxy-piperidin-1-ylmethyl)-1-oxo-2-phenyl-1,2-dihydro-isoquinoline-4-carboxylic acid ((S)-1-phenyl-propyl)-amide | 0.98 | 509.6 | 510.2 |
| **2cj** | 3-(4'-Hydroxy-3',4',5',6'-tetrahydro-2'H-[3,4']bipyridinyl-1'-ylmethyl)-1-oxo-2-phenyl-1,2-dihydro-isoquinoline-4-carboxylic acid ((S)-1-phenyl-propyl)-amide | 0.71 | 572.7 | 573.8 |
| **2ck** | 3-(4-Hydroxy-3,4,5,6-tetrahydro-2H-[4,4']bipyridinyl-1-ylmethyl)-1-oxo-2-phenyl-1,2-dihydro-isoquinoline-4-carboxylic acid ((S)-1-phenyl-propyl)-amide | 0.71 | 572.7 | 573.8 |
| **2cl** | 3-(3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide | 1.25 | 583.7 | 584.5 |
| **2cm** | 3-(4-Hydroxy-4-methyl-piperidin-1-ylmethyl)-1-oxo-2-phenyl-1,2-dihydro-isoquinoline-4-carboxylic acid ((S)-1-phenyl-propyl)-amide | 0.91 | 509.6 | 510.1 |
| **2cn** | 3-(hexahydro-spiro[benzo[1,3]dioxole-2,4'-piperidin]-1'-ylmethyl)-1-oxo-2-phenyl-1,2'-dihydro-isoquinoline-4-carboxylic acid ((S)-1-phenyl-propyl)-amide | 1.27 | 591.7 | 592.5 |
| **2co** | 1-Oxo-2-phenyl-3-(3,4,5,6-tetrahydro-2H-[4,4']bipyridinyl-1-ylmethyl)-1,2-dihydro-isoquinoline-4-carboxylic acid ((S)-1-phenyl-propyl)-amide | 0.71 | 556.7 | 557.4 |
| **2cp** | 3-[4-(2-Dimethylamino-ethyl)-piperidin-1-ylmethyl]-1-oxo-2-phenyl-1,2-dihydro-isoquinoline-4-carboxylic acid ((S)-1-phenyl-propyl)-amide | 0.68 | 550.7 | 551.4 |
| **2cq** | 3-(4-Dimethylsulfamoyl-piperazin-1-ylmethyl)-1-oxo-2-phenyl-1,2-dihydro-isoquinoline-4-carboxylic acid ((S)-1-phenyl-propyl)-amide | 1.43 | 587.7 | 588.3 |
| **2cr** | 3-(1,1-Dioxo-thiomorpholin-4-ylmethyl)-1-oxo-2-phenyl-1,2-dihydro-isoquinoline-4-carboxylic acid ((S)-1-phenyl-propyl)-amide | 1.29 | 529.7 | 530.3 |
| **2cs** | 1-Oxo-2-phenyl-3-(2-pyridin-2-ylmethyl-piperidin-1-ylmethyl)-1,2-dihydro-isoquinoline-4-carboxylic acid ((S)-1-phenyl-propyl)-amide | 1.05 | 570.7 | 571.6 |
| **2ct** | 3-(2-Morpholin-4-ylmethyl-piperidin-1-ylmethyl)-1-oxo-2-phenyl-1,2-dihydro-isoquinoline-4-carboxylic acid ((S)-1-phenyl-propyl)-amide | 1.08 | 578.8 | 579.8 |
| **2cu** | 3-(4-Furo[3,2-c]pyridin-4-yl-piperazin-1-ylmethyl)-1-oxo-2-phenyl-1,2-dihydro-isoquinoline-4-carboxylic acid ((S)-1-phenyl-propyl)-amide | 1.01 | 597.7 | 598.5 |
| **2cv** | 3-(4-Cyclopropylmethyl-piperazin-1-ylmethyl)-1-oxo-2-phenyl-1,2-dihydro-isoquinoline-4-carboxylic acid ((S)-1-phenyl-propyl)-amide | 0.95 | 534.7 | 535.3 |
| **2cw** | 3-[4-(2-Morpholin-4-yl-ethyl)-piperidin-1-ylmethyl]-1-oxo-2-phenyl-1,2-dihydro-isoquinoline-4-carboxylic acid ((S)-1-phenyl-propyl)-amide | 0.69 | 592.8 | 593.5 |
| **2cx** | 1-Oxo-2-phenyl-3-(4-pyrimidin-2-yl-[1,4]diazepan-1-ylmethyl)-1,2-dihydro-isoquinoline-4-carboxylic acid ((S)-1-phenyl-propyl)-amide | 1.05 | 572.7 | 573.5 |
| **2cy** | 3-(4-Methyl-6,7-dihydro-4H-thieno[3,2-c]pyridin-5-ylmethyl)-1-oxo-2-phenyl-1,2-dihydro-isoquinoline-4-carboxylic acid ((S)-1-phenyl-propyl)-amide | 1.23 | 547.7 | 548.5 |
| **2cz** | 3-[1,4]Dlazepan-1-ylmethyl-1-oxo-2-phenyl-1,2-dihydro-isoquinoline-4-carboxylic acid ((S)-1-phenyl-propyl)-amide | 0.89 | 494.6 | 495.7 |
| **2da** | 3-((2S,5R)-2,5-Dimethyl-piperazin-1-ylmethyl)-1-oxo-2-phenyl-1,2-dihydro-isoquinoline-4-carboxylic acid ((S)-1-phenyl-propyl)-amide | 0.92 | 508.7 | 509.2 |
| **2db** | 3-((S)-3-Methyl-piperazin-1-ylmethyl)-1-oxo-2-phenyl-1,2-dihydro-isoquinoline-4-carboxylic acid ((S)-1-phenyl-propyl)-amide | 0.88 | 494.6 | 495.7 |
| **2dc** | 3-((R)-3-Methyl-piperazin-1-ylmethyl)-1-oxo-2-phenyl-1,2-dihydro-isoquinoline-4-carboxylic acid ((S)-1-phenyl-propyl)-amide | 0.88 | 494.6 | 495.7 |
| **2dd** | 3-[3-(3-Chloro-phenyl)-piperazin-1-ylmethyl]-1-oxo-2-phenyl-1,2-dihydro-isoquinoline-4-carboxylic acid ((S)-1-phenyl-propyl)-amide | 1.11 | 591.2 | 591.4 |
| **2de** | 3-[4-(1H-Indol-4-yl)-piperazin-1-ylmethyl]-1-oxo-2-phenyl-1,2-dihydro-isoquinoline-4-carboxylic acid ((S)-1-phenyl-propyl)-amide | 1.1 | 595.7 | 596.6 |
| **2df** | 1-Oxo-3-(3-oxo-piperazin-1-ylmethyl)-2-phenyl-1,2-dihydro-isoquinoline-4-carboxylic acid ((S)-1-phenyl-propyl)-amide | 1.09 | 494.6 | 495.7 |
| **2dg** | 3-[4-(1H-Indol-5-yl)-piperazin-1-ylmethyl]-1-oxo-2-phenyl-1,2-dihydro-isoquinoline-4-carboxylic acid ((S)-1-phenyl-propyl)-amide | 1.04 | 595.7 | 596.7 |
| **2dh** | 3-(6,9-Diaza-spiro[4.5]dec-9-ylmethyl)-1-oxo-2-phenyl-1,2-dihydro-isoquinoline-4-carboxylic acid ((S)-1-phenyl-propyl)-amide | 0.97 | 534.7 | 535.3 |
| **2di** | 3-(1,4-Diaza-spiro[5.5]undec-4-ylmethyl)-1-oxo-2-phenyl-1,2-dihydro-isoquinoline4-carboxylic acid ((S)-1-phenyl-propyl)-amide | 1.01 | 548.7 | 549.7 |
| **2dj** | 3-(3-Isopropyl-piperazin-1-ylmethyl)-1-oxo-2-phenyl-1,2-dihydro-isoquinoline-4-carboxylic acid ((S)-1-phenyl-propyl)-amide | 0.96 | 522.7 | 523.5 |
| **2dk** | 3-(3,3-Dimethyl-piperazin-1-ylmethyl)-1-oxo-2-phenyl-1,2-dihydro-isoquinoline-4-carboxylic acid ((S)-1-phenyl-propyl)-amide | 0.9 | 508.7 | 509.2 |
| **2dl** | 3-[3-(4-Fluoro-phenyl)-piperazin-1-ylmethyl]-1-oxo-2-phenyl-1,2-dihydro-isoquinoline-4-carboxylic acid ((S)-1-phenyl-propyl)-amide | 1.05 | 574.7 | 575.4 |
| **2dm** | 1-Oxo-2-phenyl-3-(3-p-tolyl-piperazin-1-ylmethyl)-1,2-dihydro-isoquinoline-4-carboxylic acid ((S)-1-phenyl-propyl)-amide | 1.08 | 570.7 | 571.7 |
| **2dn** | 4-(1-Oxo-2-phenyl-4-(1-phenyl-propylcarbamoyl)-1,2-dihydro-isoquinolin-3-ylmethyl]-piperazine-1-carboxylic acid tert-butyl ester | 1.49 | 580.7 | 581.9 |
| **2do** | 3-(4-Methylcarbamoylmethyl-piperazin-1-ylmethyl)-1-oxo-2-phenyl-1,2-dihydro-isoquinoline-4-carboxylic acid ((S)-1-phenyl-propyl)-amide | 0.86 | 551.7 | 552.9 |
| **2dp** | 8-Chloro-3-dimethylaminomethyl-1-oxo-2-phenyl-1,2-dihydro-isoquinoline-4-carboxylic acid ((S)-1-phenyl-propyl)-amide | 0.95 | 474.0 | 474.6 |
| **2dr** | 3-Cyclopentylaminomethyl-1-oxo-2-phenyl-1,2-dihydro-isoquinolinc-4-carboxylic acid ((S)-1-phenyl-propyl)-amide | 1.03 | 479.6 | 480.4 |
| **2ds** | 3-Cyclohexylaminomethyl-1-oxo-2-phenyl-1,2-dihydro-isoquinolino-4-carboxylic acid ((S)-1-phenyl-propyl)-amide | 1.1 | 493.6 | 494.4 |
| **2dt** | 3-{[(2-Hydroxy-ethyl)-methyl-amino]-methyl}-1-oxo-2-phenyl-1,2-dihydro-isoquinoline-4-carboxylic acid ((S)-1-phenyl-propyl)-amide | 0.84 | 469.6 | 470.6 |
| **2du** | 3-Imidazol-1-ylmethyl-1-oxo-2-phenyl-1,2-dihydro-isoquinoline-4-carboxylic acid ((S)-1-phenyl-propyl)-amide | 0.86 | 462.6 | 463.4 |
| **2dv** | 3-(2-Methyl-imidazol-1-ylmethyl)-1-oxo-2-phenyl-1,2-dihydro-isoquinoline-4-carboxylic acid ((S)-1-phenyl-propyl)-amide | 0.88 | 476.6 | 477.3 |
| **2dw** | 3-(4-Methyl-imidazol-1-ylmethyl)-1-oxo-2-phenyl-1,2-dihydro-isoquinoline-4-carboxylic acid ((S)-1-phenyl-propyl)-amide | 0.89 | 476.6 | 477.3 |
| **2dx** | 3-(2,5-Dihydro-pyrrol-1-ylmethyl)-1-oxo-2-phenyl-1,2-dihydro-isoquinoline-4-carboxylic acid ((S)-1-phenyl-propyl)-amide | 0.92 | 463.6 | 464.5 |
| **2dy** | 3-(2,5-Dimethyl-2,5-dihydro-pyrrol-1-ylmethyl)-1-oxo-2-phenyl-1,2-dihydro-isoquinoline-4-carboxylic acid ((S)-1-phenyl-propyl)-amide | 1.06 | 491.6 | 492.4 |
| **2dz** | 1-Oxo-2-phenyl-3-pyrrolidin-1-ylmethyl-1,2-dihydro-isoquinoline-4-carboxylic acid ((S)-1-phenyl-propyl)-amide | 0.92 | 465.6 | 466.3 |
| **2ea** | 1-Oxo-2-phenyl-3-(thiazol-2-ylaminomethyl)-1,2-dihydro-isoquinoline-4-carboxylic acid ((S)-1-phenyl-propyl)-amide | 0.88 | 494.6 | 495.6 |
| **2eb** | 1-Oxo-2-phenyl-3-(pyrirnidin-4-ylaminomethyl)-1,2-dihydro-isoquinoline-4-carboxylic acid ((S)-1-phenyl-propyl)-amide | 0.85 | 489.6 | 490.4 |
| **2ec** | 3-(tert-Butylamino-methyl)-1-oxo-2-phenyl-1,2-dihydro-isoquinoline-4-carboxylic acid ((S)-1-phenyl-propyl)-amide | 1 | 467.6 | 468.7 |
| **2ed** | 3-[(2-Hydroxy-1,1-dimethyl-ethylamino)-methyl]-1-oxo-2-phenyl-1,2-dihydro-isoquinoline-4-carboxylic acid ((S)-1-phenyl-propyl)-amide | 0.91 | 483.6 | 484.3 |
| **2ee** | 3-(Isopropylamino-methyl)-1-oxo-2-phenyl-1,2-dihydro-isoquinoline-4-carboxylic acid ((S)-1-phenyl-propyl)-amide | 0.93 | 453.6 | 454.3 |
| **2ef** | 3-[(2-Hydroxy-1-methyl-ethylamino)-methyl]-1-oxo-2-phenyl-1,2-dihydro-isoquinoline-4-carboxylic acid ((S)-1-phenyl-propyl)-amide | 0.86 | 469.6 | 470.5 |
| **2eg** | 3-[(1-Hydroxymethyl-propylamino)-methyl]-1-oxo-2-phenyl-1,2-dihydro-isoquinoline-4-carboxylic acid ((S)-1-phenyl-propyl)-amide | 0.92 | 483.6 | 484.4 |
| **2eh** | 3-[(2,2-Dimethyl-propylamino)-methyl]-1-oxo-2-phenyl-1,2-dihydro-isoquinoline-4-carboxylic acid ((S)-1-phenyl-propyl)-amide | 1.13 | 481.6 | 482.2 |
| **2ei** | 1-Oxo-2-phenyl-3-prop-2-ynylaminomethyl-1,2-dihydro-isoquinoline-4-carboxylic acid ((S)-1-phenyl-propyl)-amide | 0.95 | 449.6 | 450.2 |
| **2ej** | 3-Allylaminomethyl-1-oxo-2-phenyl-1,2-dihydro-isoquinoline-4-carboxylic acid ((S)-1-phenyl-propyl)-amide | 0.94 | 451.6 | 452.4 |
| **2ek** | 3-[(Methyl-prop-2-ynyl-amino)-methyl]-1-oxo-2-phenyl-1,2-dihydro-isoquinoline-4-carboxylic acid ((S)-1-phenyl-propyl)-amide | 1.22 | 463.6 | 464.5 |
| **2el** | 3-Diallylaminomethyl-1-oxo-2-phenyl-1,2-dihydro-isoquinoline-4-carboxylic acid ((S)-1-phenyl-propyl)-amide | 1.19 | 491.6 | 492.4 |
| **2em** | 3-Diethylaminomethyl-1-oxo-2-phenyl-1,2-dihydro-isoquinoline-4-carboxylic acid ((S)-1-phenyl-propyl)-amide | 0.98 | 467.6 | 468.5 |
| **2en** | 3-[(Isopropyl-methyl-amino)-methyl]-1-oxo-2-phenyl-1,2-dihydro-isoquinoline-4-carboxylic acid ((S)-1-phenyl-propyl)-amide | 0.96 | 467.6 | 468.7 |
| **2eo** | 3-[((S)-2-Hydroxy-)-methyl-ethylamino)-methyl-1-oxo-2-phenyl-1,2-dihydro-isoquinoline-4-carboxylic acid ((S)-1-phenyl-propyl)-amide | 0.86 | 469.6 | 470.5 |
| **2ep** | 3-[((R)-2-Hydroxy-1-methyl-ethylamino)-methyl)-1-oxo-2-phenyl-1,2-dihydro-isoquinoline-4-carboxylic acid ((S)-1-phenyl-propyl)-amide | 0.87 | 469.6 | 470.5 |
| **2eq** | 3-{[(2-Methoxy-ethyl)-methyl-amino]-methyl}-1-oxo-2-phenyl-1,2-dihydro-isoquinolinc-4-carboxylic acid ((S)-1-phenyl-propyl)-amide | 0.96 | 483.6 | 484.4 |
| **2er** | 3-((R)-3-Hydroxy-pyrrolidin-1-ylmethyl)-1-oxo-2-phenyl-1,2-dihydro-isoquinoline-4-carboxylic acid ((S)-1-phenyl-propyl)-amide | 0.85 | 481.6 | 482.3 |
| **2es** | 3-((S)-3-Hydroxy-pyrrolidin-1-ylmethyl)-1-oxo-2-phenyl-1,2-dihydro-isoquinoline-4-carboxylic acid ((S)-1-phenyl-propyl)-amide | 0.84 | 481.6 | 482.3 |
| **2et** | 3-[(Cyclopentyl-methyl-amino)-methyl]-1-oxo-2-phenyl-1,2-dihydro-isoquinoline-4-carboxylic acid ((S)-1-phenyl-propyl)-amide | 1.09 | 493.6 | 494.5 |
| **2eu** | 3-{[(2-Hydroxy-1-methyl-ethyl)-methyl-amino]-methyl}-1-oxo-2-phenyl-1,2-dihydro-isoquinoline-4-carboxylic acid ((S)-1-phenyl-propyl)-amide | 0.89 | 483.6 | 484.4 |
| **2ev** | 3-{[Ethyl-(2-hydroxy-ethyl)-amino]-methyl}-1-oxo-2-phenyl-1,2-dihydro-isoquinoline-4-carboxylic acid ((S)-1-phcnyl-propyl)-amide | 0.89 | 483.6 | 484.4 |
| **2ew** | 3-[(Ethyl-methyl-amino)-methyl]-1-oxo-2-phenyl-1,2-dihydro-isoquinoline-4-carboxylic acid ((S)-1-phenyl-propyl)-amide | 0.92 | 453.6 | 454.4 |
| **2ex** | 3-Cyclobutylaminomethyl-1-oxo-2-phenyl-1,2-dihydro-isoquinoline-4-carboxylic acid ((S)-1-phenyl-propyl)-amide | 0.98 | 465.6 | 466.4 |
| **2ey** | 3-Azetidin-1-ylmethyl-1-oxo-2-phenyl-1,2-dihydro-isoquinoline-4-carboxylic acid ((S)-1-phenyl-propyl)-amide | 0.86 | 451.6 | 452.3 |
| **2ez** | 3-(4-tert-Butyl-piperazin-1-ylmethyl)-1-oxo-2-phenyl-1,2-dihydro-isoquinoline-4-carboxylic acid ((S)-1-phenyl-propyl)-amide | 0.91 | 536.7 | 537.3 |
| **2fa** | 3-[4-(2-Hydroxy-ethyl)-piperazin-1-ylmethyl]-1-oxo-2-phenyl-1,2-dihydro-isoquinoline-4-carboxylic acid ((S)-1-phenyl-propyl)-amide | 0.86 | 524.7 | 525.6 |
| **2fb** | 3-{4-[2-(2-Hydroxy-ethoxy)-ethyl]-piperazin-1-ylmethyl}- l-oxo-2-phenyl-1 ,2-dihydro-isoquinoline-4-carboxylic acid ((S)-1-phenyl-propyl)-amide | 0.86 | 568.7 | 569.6 |
| **2fc** | 3-[4-(3-Chloro-5-trifluoromethyl-pyridin-2-yl)-piperazin-1-ylmethyl]-1-oxo-2-phenyl-1,2-dihydro-isoquinoline-4-carboxylic acid ((S)-1-phenyl-propyl)-amide | 1.77 | 660.1 | 660.8 |
| **2fd** | 3-[4-(3,5-Dichloro-pyridin-4-yl)-piperazin-1-ylmethyl]-1-oxo-2-phenyl-1,2-dihydro-isoquinoline-4-carboxylic acid ((S)-1-phenyl-propyl)-amide | 1.45 | 626.6 | 626.5 |
| **2fe** | 4-[1-Oxo-2-phenyl-4-((S)-1-phenyl-propylcarbamoyl)-1,2-dihydro-isoquinolin-3-ylmethyl]-piperazine-1-carboxylic acid benzyl ester | 1.57 | 614.7 | 615.4 |
| **2ff** | 3-[4-(3-Morpholin-4-yl-propyl)-piperazin-1-ylmethyl]-1-oxo-2-phenyl-1,2-dihydro-isoquinoline-4-carboxylic acid ((S)-1-phenyl-propyl)-amide | 0.74 | 607.8 | 609.0 |
| **2fg** | 1-Oxo-2-phenyl-3-[4-(3-piperidin-1-yl-propyl)-piperazin-1-ylmethyl]-1,2-dihydro-isoquinotine-4-carboxylic acid ((S)-1-phenyl-propyl)-amide | 0.76 | 605.8 | 607.1 |
| **2fh** | 3-[4-(4,6-Dimethoxy-pyrimidin-2-ylmethyl)-piperazin-1-ylmethyl]-1-oxo-2-phenyl-1,2-dihydro-isoquinoline-4-carboxylic acid ((S)-1-phenyl-propyl)-amide | 1.05 | 632.8 | 633.9 |
| **2fi** | 3-[4-(3-Hydroxy-propyl)-piperazin-1-ylmethyl]-1-oxo-2-phenyl-1,3-dihydro-isoquinoline-1-carboxylic acid ((S)-1-phenyl-propyl)-amide | 0.86 | 538.7 | 539.4 |
| **2fj** | 3-[4-(2,3-Dihydroxy-propyl)-piperazin-1-ylmethyl]-1-oxo-2-phenyl-1,2-dihydro-isoquinoline-4-carboxylic acid ((S)-1-phenyl-propyl)-amide | 0.84 | 554.7 | 555.7 |
| **2fk** | (2-Oxo-2-{4-[1-oxo-2-phenyl-4-(1-phenyl-propylcarbamoyl)-1,2-dihydro-isoquinolin-3-ylmethyl]-piperazin-1-yl}-ethyl)-carbamic acid tert-butyl ester | 1.39 | 637.8 | 638.5 |
| **2fl** | 3-[4-(1H-Indazol-5-yl)-piperazin-1-ylmethyl]-1-oxo-2-phenyl-1,2-dihydro-isoquinoline-4-carboxylic acid ((S)-1-phenyl-propyl)-amide | 0.99 | 596.7 | 597.7 |
| **2fm** | 1-Oxo-2-phenyl-3-(4-quinolin-6-yl-piperazin-1-ylmethyl)-1,2-dihydro-isoquinoline-4-carboxylic acid ((S)-1-phenyl-propyl)-amide | 0.95 | 607.8 | 608.7 |
| **2fn** | 3-[4-(6,7-Dimethoxy-quinazolin-4-yl)-piperazin-1-ylmethyl]-1-oxo-2-phenyl-1,2-dihydro-isoquinoline-4-carboxylic acid ((S)-1-phenyl-propyl)-amide | 1.06 | 668.8 | 669.3 |
| **2fo** | 4-{4-[1-Oxo-2-phenyl-4-(1-phenyl-propylcarbamoyl)-1,2-dihydro-isoquinolin-3-ylmethyl]-piperazin-1-yl}-piperidine-1-carboxylic acid tert-butyl ester | 1.09 | 663.9 | 664.8 |
| **2fp** | 3-{4-[2-(4-Chloro-phenoxy)-ethyl]-piperazin-1-ylmethyl}-1-oxo-2-phenyl-1,2-dihydro-isoquinoline-4-carboxylic acid ((S)-1-phenyl-propyl)-amide | 1.19 | 635.2 | 635.9 |
| **2fq** | {4-[1-Oxo-2-phenyl-4-(1-phenyl-propylcarbamoyl)-1,2-dihydro-isoquino lin-3-ylmethyl]-piperazin-1-yl}-acetic acid tert-butyl ester | 1.08 | 594.8 | 595.9 |
| **2fr** | 1-Oxo-2-phenyl-3-[4-(3,3,3-trifluoro-2-hydroxy-propyl)-piperazin-1-ylmethyl]-1,2-dihydro-isoquinoline-4-carboxylic acid ((S)-1-phenyl-propyl)-amide | 0.96 | 592.7 | 593.6 |
| **2fs** | 3-[4-(2-Hydroxy-propyl)-piperazin-1-ylmethyl]-1-oxo-2-phenyl-1,2-dihydro-isoquinoline-4-carboxylic acid ((S)-1-phenyl-propyl)-amide | 0.86 | 538.7 | 539.5 |
| **2fu** | 3-[4-(4-Amino-6,7-dimethoxy-quinazolin-2-yl)-piperazin-1-ylmethyl]-1-oxo-2-phenyl-1,2-dihydro-isoquinoline-4-carboxylic acid ((S)-1-phenyl-propyl)-amide | 1.03 | 683.8 | 684.7 |
| **2fv** | (2-{4-[1-Oxo-2-phenyl-4-(1-phenyl-propylcarbamoyl)-1,2-dihydro-isoquinolin-3-ylmethyl]-piperazin-1-yl}-ethyl)-carbamic acid tert-butyl ester | 1.06 | 623.8 | 624.5 |
| **2fw** | 1-Oxo-3-{4-[2-(2-oxo-imidazolidin-1-yl)-ethyl]-piperazin-1-ylmethyl}-2-phenyl-1,2-dihydro-isoquinoline-4-carboxylic acid ((S)-1-phenyl-propyl)-amide | 0.85 | 592.7 | 593.7 |
| **2fx** | 3-{4-[(4,6-Dimethoxy-pyrimidin-2-yl)-phenyl-methyl]-piperazin-1-ylmethyl}-1-oxo-2-phenyl-1,2-dihydro-isoquinoline-4-carboxylic acid ((S)-1-phenyl-propyl)-amide | 1.21 | 708.9 | 709.4 |
| **2fy** | 3-(4-Benzo[1,2,5]thiadiazol-4-yl-piperazin-1-ylmethyl)-1-oxo-2-phenyl-1,2-dihydro-isoquinoline-4-carboxylic acid ((S)-1-phenyl-propyl)-amide | 1.48 | 614.8 | 615.4 |
| **2fz** | 3-[4-(2,3-Dihydro-benzo[1,4]dioxin-5-yl)-piperazin-1-ylmethyl]-1-oxo-2-phenyl-1,2-dihydro-isoquinoline-4-carboxylic acid ((S)-1-phenyl-propyl)-amide | 1.2 | 614.7 | 615.3 |
| **2ga** | 3-[4-(4-Methyl-quinolin-2-yl)-piperazin-1-ylmethyl]-1-oxo-2-phenyl-1,2-dihydro-isoquinoline-4-carboxylic acid ((S)-1-phenyl-propyl)-amide | 1.15 | 621.8 | 622.7 |
| **2gb** | 1-Oxo-2-phenyl-3-[4-(pyridin-2-ylcarbamoylmethyl)-pipcrazin-1-ylmethyl]-1,2-dihydro-isoquinoline-4-carboxylic acid ((S)-1-phenyl-propyl)-amide | 0.93 | 614.7 | 615.4 |
| **2gc** | 3-[4-(6-Chloro-3-oxo-3,4-dihydro-2H-benzo[1,4]oxazin-8-yl)-pipcrazin-1-ylmethyl]-1-oxo-2-phenyl-1,2-dihydro-isoquinoline-4-carboxylic acid ((S)-1-phenyl-propyl)-amide | 1.21 | 662.2 | 662.8 |
| **2gd** | 3-(4-Carbamoylmethyl-piperazin-1-ylmethyl)-1-oxo-2-phenyl-1,2-dihydro-isoquinoline-4-carboxylic acid ((S)-1-phenyl-propyl)-amide | 0.85 | 537.7 | 538.5 |
| **2ge** | 3-(4-Hydroxy-4-phenyl-piperidin-1-ylmethyl)-1-oxo-2-phenyl-1,2-dihydro-isoquinoline-4-carboxylic acid ((S)-1-phenyl-propyl)-amide | 1.12 | 571.7 | 572.6 |
| **2gf** | 3-[4-(4-Chloro-phenyl)-4-hydroxy-piperidin-1-ylmethyl]-1-oxo-2-phenyl-1,2-dihydro-isoquinoline-4-carboxylic acid ((S)-1-phenyl-propyl)-amide | 1.23 | 606.2 | 606.8 |
| **2gg** | 1-[1-Oxo-2-phenyl-4-(1-phenyl-propylcarbamoyl)-1,2-dihydro-isoquinolin-3-ylmethyl]-piperidine-4-carboxylic acid | 0.9 | 523.6 | 524.5 |
| **2gh** | 3-(4-Cyano-4-phenyl-piperidin-1-ylmethyl)-1-oxo-2-phenyl-1,2-dihydro-isoquinoline-4-carboxylic acid ((S)-1-phenyl-propyl)-amide | 1.59 | 580.7 | 581.9 |
| **2gi** | 3-(4-Benzyl-4-hydroxy-piperidin-1-ylmethyl)-1-oxo-2-phenyl-1,2-dihydro-isoquinoline-4-carboxylic acid ((S)-1-phenyl-propyl)-amide | 1.16 | 585.7 | 586.0 |
| **2gj** | 1-[1-Oxo-2-phenyl-4-(1-phenyl-propylcarbamoyl)-1,2-dihydro-isoquinolin-3-ylmethyl]-4-phenyl-piperidine-4-carboxylic acid ethyl ester | 1.35 | 627.8 | 628.5 |
| **2gk** | 1-Oxo-2-phenyl-3-[4-(phenyl-propionyl-amino)-piperidin-1-ylmethyl]-1,2-dihydro-isoquinoline-4-carboxylic acid ((S)-1-phenyl-propyl}-amide | 1.17 | 626.8 | 627.6 |
| **2gl** | Methyl-{1-[1-oxo-2-phenyl-4-(1-phenyl-propylcarbamoyl)-1,2-dihydro-isoquinolin-3-ylmethyl]-pipendin-4-yl}-carbamic acid tert-butyl ester | 1.25 | 608.8 | 609.8 |
| **2gm** | 1-Oxo-2-phenyl-3-[4-(2-pyrrolidin-1-yl-ethyl)-piperidin-1-ylmethyl]-1,2-dihydro-isoquinoline-4-carboxylic acid ((S)-1-phenyl-propyl)-amide | 0.72 | 576.8 | 577.5 |
| **2gn** | 3-{4-[5-(4-Fluoro-phenyl)-[1,3,4]oxadiazol-2-yl]-piperidin-1-ylmethyl}-1-oxo-2-phenyl-1,2-dihydro-isoquinoline-4-carboxylic acid ((S)-1-phenyl-propyl)-amide | 1.22 | 641.7 | 642.3 |
| **2go** | 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide | 0.96 | 624.7 | 625.5 |
| **2gp** | 1-Oxo-2-phenyl-3-[4-(3-pyrazin-2-yl-[1,2,4]oxadiazol-5-yl)-piperidin-1-ylmethyl]-1,2-dihydro-isoquinoline-4-carboxylic acid ((S)-1-phenyl-propyl)-amide | 1.04 | 625.7 | 626.5 |
| **2gq** | 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide | 1.04 | 624.7 | 625.5 |
| **2gr** | 3-(4'-Hydroxy-3',4',5',6'-tetrahydro-2'H-[2,4']bipyridinyl-1'-ylmethyl)-1-oxo-2-phenyl-1,2-dihydro-isoquinoline-4-carboxylic acid ((S)-1-phenyl-propyl)-amide | 0.83 | 572.7 | 573.5 |
| **2gs** | 3-(spiro[isochroman-1,4'-piperidin]-1'-ylmethyl)-1-oxo-2-phenyl-1,2-dihydro-isoquinoline-4-carboxylic acid ((S)-1-phenyl-propyl)-amide | 1.24 | 597.8 | 598.6 |
| **2gt** | 3-[4-Hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide | 1.13 | 601.7 | 602.4 |
| **2gu** | 3-[4-(3-Chloro-phenyl)-4-hydroxy-piperidin-1-ylmethyl]- 1-oxo-2-phenyl- 1,2-dihydro-isoquinoline-4-carboxylic acid ((S)-1-phenyl-propyl)-amide | 1.23 | 606.2 | 606.8 |
| **2gv** | 3-(6-chloro-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide | 1.36 | 618.2 | 618.4 |
| **2gw** | 3-(4-{[4-Chloro-3-(4-fluoro-phenyl)-indan-1-yl]-methyl-amino}-piperidin-1-ylmethyl)-1-oxo-2-phenyl-1,2-dihydro-isoquinoline-4-carboxylic acid ((S)-1-phenyl-propyl)-amide | 1.36 | 753.4 | 753.4 |
| **2gx** | 3-(1-acetyl-spiro[indoline-3,4'-piperidin]-1'-ylmethyl)-1-oxo-2-phenyl-1,2-dihydro-isoquinoline-4-carboxylic acid ((S)-1-phenyl-propyl)-amide | 1.15 | 624.8 | 625.6 |
| **2gy** | 3-(1-acetyl-5-fluoro-spiro[indoline-3,4'-piperidin]-1'-ylmethyl)-1-oxo-2-phenyl-1,2-dihydro-isoquinoline-4-carboxylic acid ((S)-1-phenyl-propyl)-amide | 1.2 | 642.8 | 643.4 |
| **2gz** | 1-Oxo-3-(4-oxo-1-phenyl-1,3,8-triaza-spiro[4.5]dec-8-ylmethyl)-2-phenyl-1,2-dihydro-isoquinoline-4-carboxylic acid ((S)-1-phenyl-propyl)-amide | 1.15 | 625.8 | 626.6 |
| **2ha** | 3-(4-Acetylamino-piperidin-1-ylmethyl)-1-oxo-2-phenyl-1,2-dihydro-isoquinoline-4-carboxylic acid ((S)-1-phenyl-propyl)-amide | 0.83 | 536.7 | 537.4 |
| **2hb** | 1-Oxo-3-(1-oxo-2,8-diaza-spiro[4.5]dec-8-ylmethyl)-2-phenyl-1,2-dihydro-isoquinoline-4-carboxylic acid ((S)-1-phenyl-propyl)-amide | 0.85 | 548.7 | 549.7 |
| **2hc** | 3-[4-Hydroxy-4-(3-trifluoromethyl-phenyl)-piperidin-1-ylmethyl]-1-oxo-2-phenyl-1,2-dihydro-isoquinoline-4-carboxylic acid ((S)-1-phenyl-propyl)-amide | 1.29 | 639.7 | 640.3 |
| **2hd** | 1-Oxo-2-phenyl-3-(4-trifluoromethyl-piperidin-1-ylmethyl)-1,2-dihydro-isoquinoline-4-carboxylic acid ((S)-1-phenyl-propyl)-amide | 1.3 | 547.6 | 548.4 |
| **2he** | 3-[4-(4-Methyl-piperazine-1-carbonyl)-piperidin-1-ylmethyl]-1-oxo-2-phenyl-1,2-dihydro-isoquinoline-4-carboxylic acid ((S)-1-phenyl-propyl)-amide | 0.7 | 605.8 | 607.0 |
| **2hf** | 3-(5-isopropyl-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide | 1.48 | 625.8 | 626.6 |
| **2hg** | 3-[4-(Acetyl-methyl-amino)-4-phenyl-piperidin-1-ylmethyl]-1-oxo-2-phenyl-1,2-dihydro-isoquinoline-4-carboxylic acid ((S)-1-phenyl-propyl)-amide | 1.13 | 626.8 | 627.7 |
| **2hh** | 4-{1-[1-Oxo-2-phenyl-4-(1-phenyl-propylcarbamoyl)-1,2-dihydro-isoquinolin-3-ylmethyl]-piperidine-4-yl}-piperazine-1-carboxylic acid tert-butyl ester | 1.03 | 663.9 | 664.7 |
| **2hi** | (2-{1-[1-Oxo-2-phenyl-4-(1-phenyl-propylcarbamoyl)-1,2-dihydro-isoquinolin-3-ylmethyl]-piperidin-4-yl}-ethyl)-carbamic acid tert-butyl ester | 1.19 | 622.8 | 623.5 |
| **2hj** | 3-(4-Methylamino-4-phenyl-piperidin-1-ylmethyl)-1-oxo-2-phenyl-1,2-dihydro-isoquinoline-4-carboxylic acid ((S)-1-phenyl-propyl)-amide | 1.01 | 584.8 | 585.5 |
| **2hk** | 3-(4-Acetylamino-4-phenyl-piperidin-1-ylmethyl)-1-oxo-2-phenyl-1,2-dihydro-isoquinoline-4-carboxylic acid ((S)-1-phenyl-propyl)-amide | 1.06 | 612.8 | 613.3 |
| **2hl** | 3-[4-Acetylamino-4-(3-fluoro-phenyl)-piperidin-1-ylmethyl]-1-oxo-2-phenyl-1,2-dihydro-isoquinoline-4-carboxylic acid ((S)-1-phenyl-propyl)-amide | 1.06 | 630.8 | 631.5 |
| **2hm** | 1-Oxo-3-[4-(4-oxo-piperidine-1-carbonyl)-piperidin-1-ylmethyl]-2-phenyl-1,2-dihydro-isoquinoline-4-carboxylic acid ((S)-1-phenyl-propyl)-amide | 0.89 | 604.7 | 605.3 |
| **2hn** | 3-[4,4']Bipiperidinyl-1-ylmethyl-1-oxo-2-phenyl-1,2-dihydro-isoquinoline-4-carboxylic acid ((S)-1-phenyl-propyl)-amide | 0.7 | 562.8 | 563.2 |
| **2ho** | {1-[1-Oxo-2-phenyl-4-(1-phenyl-propylcarbamoyl)-1,2-dihydro-isoquinolin-3-ylmethyl]-piperidin-4-yl}-carbamic acid tert-butyl ester | 1.15 | 594.8 | 595.8 |
| **2hp** | 3-[1,4']Bipiperidinyl-1'-ylmethyl-1-oxo-2-phenyl-1,2-dihydro-isoquinoline-4-carboxylic acid [cyclopropyl-(3-fluoro-phenyl)-methyl]-amide | 0.95 | 592.8 | 593.5 |
| **2hq** | 1-Oxo-3-(4-phenethyl-piperazin-1-ylmethyl)-2-phenyl-1,2-dihydro-isoquinoline-4-carboxylic acid [cyclopropyl-(3-fluoro-phenyl)-methyl]-amide | 1.15 | 614.8 | 615.5 |
| **2hr** | 3-(4-Isopropyl-piperazin-1-ylmethyl)-1-oxo-2-phenyl-1,2-dihydro-isoquinoline-4-carboxylic acid [cyclopropyl-(3-fluoro-phenyl)-methyl]-amide | 0.97 | 552.7 | 553.8 |
| **2hs** | 3-[4-(2-Morpholin-4-yl-ethyl)-piperazin-1-ylmethyl]-1-oxo-3-phenyl-1,2-dihydro-isoquinoline-4-carboxylic acid [cyclopropyl-(3-fluoro-phenyl)-methyl]-amide | 0.81 | 623.8 | 624.6 |
| **2ht** | 3-[4-(1-Methyl-piperidin-4-yl)-piperazin-1-ylmethyl]-1-oxo-2-phenyl-1,2-dihydro-isoquinoline-4-carboxylic acid [cyclopropyl-(3-fluoro-phenyl)-methyl]-amide | 0.78 | 607.8 | 608.9 |
| **2hu** | 1-Oxo-2-phenyl-3-[4-(2-piperidin-1-yl-ethyl)-piperazin-1-ylmethyl]-1,2-dihydro-isoquinoline-4-carboxylic acid [cyclopropyl-(3-fluoro-phenyl)-methyl]-amide | 0.82 | 621.8 | 622.6 |
| **2hv** | 1-Oxo-2-phenyl-3-[4-(2-pyrrolidin-1-yl-ethyl)-piperazin-1-ylmethyl]-1,2-dihydro-isoquinolinc-4-carboxylic acid [cyclopropyl-(3-fluoro-phenyl)-methyl]-amide | 0.8 | 607.8 | 608.8 |
| **2hw** | 1-Oxo-2-phenyl-3-(4-pyridin4-methyl-piperazin-1-ylmethyl)-1,2-dihydro-isoquinoline-4-carboxylic acid [cyclopropyl-(3-fluoro-phenyl)-methyl]-amide | 0.84 | 601.7 | 602.6 |
| **2hx** | 3-(4-Hydroxy-3,4,5,6-tetrahydro-2H-[4,4']bipyridinyl-1-ylmethyl)-1-oxo-2-phenyl-1,2-dihydro-isoquinoline-4-carboxylic acid [cyclopropyl-(3-fluoro-phenyl)-methyl]-amide | 0.77 | 602.7 | 603.7 |
| **2hy** | 3-[4-(2-Morpholin-4-yl-ethyl)-piperidin-1-ylmethyl]-1-oxo-2-phenyl-1,2-dihydro-isoquinoline-4-carboxylic acid [cyclopropyl-(3-fluoro-phenyl)-methyl]-amide | 0.75 | 622.8 | 623.7 |
| **2hz** | 3-[4-Hydroxy-4-(3-methoxy-phenyl)-piperidin-1-ylmethyl]-1-oxo-2-phenyl-1,2-dihydro-isoquinoline-4-carboxylic acid [cyclopropyl-(3-fluoro-phenyl)-methyl]-amide | 1.17 | 631.7 | 632.6 |
| **2ia** | 3-[4-(Acetyl-methyl-amino)-4-phenyl-piperidin-1-ylmethyl]-1-oxo-2-phenyl-1,2-dihydro-isoquinoline-4-carboxylic acid [cyclopropyl-(3-fluoro-phenyl)-methyl]-amide | 1.18 | 656.8 | 657.8 |
| **2ib** | 2-(2-Fluoro-phenyl)-1-oxo-3-(4-phenethyl-piperazin-1-ylmethyl)-1,2-dihydro-isoquinoline-4-carboxylic acid ((S)-1-phenyl-propyl)-amide | 1.12 | 602.8 | 603.8 |
| **2ic** | 2-(2-Fluoro-phenyl)-3-(4-isopropyl-piperazin-1-ylmethyl)-1-oxo-1,2-dihydro-isoquinoline-4-carboxylic acid ((S)-1-phenyl-propyl)-amide | 0.96 | 540.7 | 541.7 |
| **2id** | 3-[1,4']Bipiperidinyl-1'-ylmethyl-2-(2-fluoro-phenyl)-1-oxo-1,2-dihydro-isoquinoline-4-carboxylic acid ((S)-1-phenyl-propyl)-amide | 1 | 580.7 | 581.7 |
| **2ie** | 2-(2-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide | 0.82 | 611.8 | 612.5 |
| **2if** | 2-(2-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide | 0.76 | 595.8 | 596.8 |
| **2ig** | 2-(2-Fluoro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide | 0.81 | 609.8 | 610.5 |
| **2ih** | 2-(2-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide | 0.8 | 595.8 | 596.7 |
| **2ii** | 2-(2-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide | 0.84 | 589.7 | 590.4 |
| **2ij** | 2-(2-Fluoro-phenyl)-3-(4-hydroxy-3,4,5,6-tetrahydro-2H-[4,4']bipyridinyl-1-ylmethyl)-1-oxo-1,2-dihydro-isoquinoline-4-carboxylic acid ((S)-1-phenyl-propyl)-amide | 0.85 | 590.7 | 591.3 |
| **2ik** | 2-(2-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide | 0.76 | 610.8 | 611.7 |
| **2il** | 2-(2-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide | 1.21 | 619.7 | 620.3 |
| **2im** | 3-[4-(2-Morpholin-4-yl-ethyl)-piperazin-1-ylmethyl]-1-oxo-2-o-tolyl-1,2-dihydro-isoquinoline-4-carboxylic acid ((S)-1-phenyl-propyl)-amide | 0.82 | 607.8 | 608.8 |
| **2in** | 1-Oxo-3-[4-(2-piperidin-1-yl-ethyl)-piperazin-1-ylmethyl]-2-o-tolyl-1,2-dihydro-isoquinoline-4-carboxylic acid ((S)-1-phenyl-propyl)-amide | 0.81 | 605.8 | 606.9 |
| **2io** | 1-Oxo-3-(4-pyridin-4-ylmethyl-piperazin-l-ylmethyl)-2-o-tolyl-1,2-dihydro-isoquinoline-4-carboxylic acid ((S)-1-phenyl-propyl)-amide | 0.84 | 585.7 | 586.4 |
| **2ip** | 2-(3-Fluoro-phenyl)-1-oxo-3-(4-phenethyl-piperazin-1-ylmethyl)-1,2-dihydro-isoquinoline-4-carboxylic acid ((S)-1-phenyl-propyl)-amide | 1.13 | 602.8 | 603.8 |
| **2iq** | 2-(3-Fluoro-phenyl)-3-(4-isopropyl-piperazin-1-ylmethyl)-1-oxo-1,2-dihydro-isoquinoline-4-carboxylic acid ((S)-1-phenyl-propyl)-amide | 0.96 | 540.7 | 541.3 |
| **2ir** | 3-[1,4']Bipiperidinyl-1'-ylmethyl-2-(3-fluoro-phenyl)-1-oxo-1,2-dihydro-isoquinoline-4-carboxylic acid ((S)-1-phenyl-propyl)-amide | 0.98 | 580.7 | 581.8 |
| **2is** | 2-(3-Fluoro-phenyl)-3-[4-(2-morpholin-4-yl-ethyl)-piperazin-1-ylmethyl]-l-oxo-1,2-dihydro-isoquinoline-4-carboxylic acid ((S)-1-phenyl-propyl)-amide | 0.81 | 611.8 | 612.6 |
| **2it** | 2-(3-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide | 0.75 | 595.8 | 596.8 |
| **2iu** | 2-(3-Fluoro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide | 0.8 | 609.8 | 610.7 |
| **2iv** | 2-(3-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide | 0.79 | 595.8 | 596.8 |
| **2iw** | 2-(3-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide | 0.83 | 589.7 | 590.2 |
| **2ix** | 2-(3-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide | 0.73 | 610.8 | 611.6 |
| **2iy** | 2-(3-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide | 1.18 | 619.7 | 620.7 |
| **2iz** | 3-[4-(Acetyl-methyl-amino)-4-phenyl-piperidin-1-ylmethyl]-2-(3-fluoro-phenyl)-1-oxo-1,2-dihydro-isoquinoline-4**-**carboxylic acid ((S)-1-phenyl-propyl)-amide | 1.2 | 644.8 | 645.7 |
| **2ja** | 2-(3-Chloro-phenyl)-1-oxo-3-(4-phenethyl-piperazin-1-ylmethyl)-1,2-dihydro-isoquinoline-4-carboxylic acid ((S)-1-phenyl-propyl)-amide | 1.17 | 619.2 | 619.4 |
| **2jb** | 2-(3-Chloro-phenyl)-3-(4-isopropyl-piperazin-1-ylmethyl)-1-oxo-1,2-dihydro-isoquinoline-4-carboxylic acid ((S)-1-phenyl-propyl)-amide | 1 | 557.1 | 557.5 |
| **2jc** | 3-[1,4']Bipiperidinyl-1'-ylmethyl-2-(3-chloro-phenyl)-1-oxo-1,2-dihydro-isoquinoline-4-carboxylic acid ((S)-1-phcnyl-propyl)-amide | 1.04 | 597.2 | 597.8 |
| **2jd** | 2-(3-Chloro-phenyl)-3-[4-(2-morpholin-4-yl-ethyl)-piperazin-1-ylmethyl]-l-oxo-1,2-dihydro-isoquinoline-4-carboxylic acid ((S)-1-phenyl-propyl)-amide | 0.86 | 628.2 | 628.6 |
| **2je** | 2-(3-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide | 0.79 | 612.2 | 612.2 |
| **2jf** | 2-(3-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide | 0.84 | 626.2 | 626.7 |
| **2jg** | 2-'3-Chloro-phenyl)-1-oxo-3-[4-(2-pyrrolidin-1-yl-ethyl)-pirerazin-1-ylmethyl]-1,2-dihydro-isoquinoline-4-carboxylic acid ((S)-1-phenyl-propyl)-amide | 0.83 | 612.2 | 612.4 |
| **2jh** | 2-(3-Chloro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide | 0.88 | 606.2 | 606.1 |
| **2ji** | 2-(3-Chloro-phenyl)-3-[4-(2-morpholin4-yl-ethyl)-piperidin-1-ylmethyl]-1-oxo-1,2-dihydro-isoquinoline-4-carboxylic acid ((S)-1-phenyl-propyl)-amide | 0.78 | 627.2 | 627.4 |
| **2jj** | 2-(3-Chloro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide | 1.23 | 636.2 | 636.7 |
| **2jk** | 3-[4-(Acetyl-methyl-amino)-4-phenyl-pipefidin-1-ylmethyl]-2-(3-chloro-phenyl)-1-oxo-1,2-dihydro-isoquinoline-4-carboxylic acid ((S)-1-phenyl-propyl)-amide | 1.26 | 661.2 | 662.0 |
| **2jl** | 1-Oxo-3-(4-phenethyl-piperazin-1-ylmethyl)-2-m-tolyl-1,2-dihydro-isoquinoline-4-carboxylic acid ((S)-1-phenyl-propyl)-amide | 1.15 | 598.8 | 599.6 |
| **2jm** | 3-[1,4']Bipiperidinyl-1'-ylmethyl-1-oxo-2-m-tolyl-1,2-dihydro-isoquinoline-4-carboxylic acid ((S)-1-phenyl-propyl)-amide | 0.96 | 576.8 | 577.5 |
| **2jn** | 3-[4-(2-Morpholin-4-yl-ethyl)-piperazin-1-ylmethyl]-1-oxo-2-m-tolyl-1,2-dihydro-isoquinoline-4-carboxylic acid ((S)- 1-phenyl-propyl)-amide | 0.83 | 607.8 | 609.0 |
| **2jo** | 3-[4-(1-Methyl-piperidin-4-yl)-piperazin-1-ylmethyl]-1-oxo-2-m-tolyl-1,2-dihydro-isoquinoline-4-carboxylic acid ((S)-1-phenyl-propyl-amide | 0.78 | 591.8 | 592.6 |
| **2jp** | 1-Oxo-3-[4-(2-piperidin-1-yl-ethyl)-piperazin-1-ylmethyl]-2-m-tolyl-1,2-dihydro-isoquinoline-4-carboxylic acid ((S)-1-phenyl-propyl)-amide | 0.83 | 605.8 | 607.1 |
| **2jq** | 1-Oxo-3-[4-(2-pyrrolidin-1-yl-ethyl)-piperazin-1-ylmethyl]-2-m-tolyl-1,2-dihydro-isoquinoline-4-carboxylic acid ((S)-1-phenyl-propyl)-amide | 0.81 | 591.8 | 592.6 |
| **2jr** | 1-Oxo-3-(4-pyridin-4-ylmethyl-piperazin-1-ylmethyl)-2-m-tolyl-1,2-dihydro-isoquinoline-4-carboxylic acid ((S)-1-phenyl-propyl)-amide | 0.85 | 585.7 | 586.0 |
| **2js** | 3-[4-(2-Morpholin-4-yl-ethyl)-piperidin-1-ylmethyl]-1-oxo-2-m-tolyl-1,2-dihydro-isoquinoline-4-carboxylic acid((S)-1-phenyl-propyl)-amide | 0.73 | 606.8 | 608.1 |
| **2jt** | 3-[4-Hydroxy-4-(3-methoxy-phenyl)-piperidin-1-ylmethyl]-1-oxo-2-m-tolyl-1,2-dihydro-isoquino line-4-carboxylic acid ((S)-1-phenyl-propyl)-amide | 1.18 | 615.8 | 616.3 |
| **2ju** | 3-[4-(Acetyl-methyl-amino)-4-phenyl-pipcridin-1-ylmethyl]-1-oxo-2-m-tolyl-1,2-dihydro-isoquinoline-4-carboxylic acid ((S)-1-phenyl-propyl)-amide | 1.19 | 640.8 | 641.7 |

### List of compounds from example 2

| | **Chemical name** | **t_{R} (min)** | **MW** | **m/z (MH+)** |
|---|---|---|---|---|
| **2ka** | 3-(4-tert-Butyl-piperazin-1-ylmethyl)-8-chloro-1-oxo-2-phenyl-1,2-dihydro-isoquinoline-4-carboxylic acid [(S)-cyclopropyl-(3-fluoro-phenyl)-methyl]-amide | 1.06 | c601.2 | 601.5 |
| **2kb** | 8-Chloro-3-(4-isopropyl-piperazin-1-ylmethyl)-1-oxo-2-phenyl-1,2-dihydro-isoquinoline-4-carboxylic acid ((S)-1-phenyl-propyl)-amide | 1.01 | 557.1 | 557.5 |
| **2kc** | 8-Chloro-3-(4-isobutyl-piperazin-1-ylmethyl)-1-oxo-2-phenyl-1,2-dihydro-isoquinoline-4-carboxylic acid ((S)-1-phenyl-propyl )-amidc | 1.07 | 571.2 | 571.7 |
| **2kd** | 8-Chloro-3-(octahydro-pyrido(1,2-a]pyrazin-2-ylmethyl)-1-oxo-2-phenyl-1,2-dihydro-isoquinoline-4-carboxylic acid ((S)-1-phenyl-propyl)-amide | 1.03 | 569.1 | 569.6 |
| **2ke** | 8-Chloro-3-(4-hydroxy-3,4,5,6-tetrahydro-2H-[4,4']bipyridinyl-1-ylmethyl)-1-oxo-2-phenyl-1,2-dihydro-isoquinoline-4-carboxylic acid ((S)-1-phenyl-propyl)-amide | 0.8 | 607.2 | 607.9 |
| **2kf** | 8-Chloro-3-(4-cyclopropylmethyl-piperazin-1-ylmethyl)-1-oxo-2-phenyl-1,2-dihydro-isoquinoline-4-carboxylic acid ((S)-1-phenyl-propyl)-amide | 1.04 | 569.1 | 569.8 |
| **2kg** | 8-Chloro-3-[4-(2-morpholin-4-yl-ethyl)-piperidin-1-ylmethyl]-1-oxo-2-phenyl-1,2-dihydro-isoquinoline-4-carboxylic acid ((S)-1-phenyl-propyl)-amide | 0.75 | 627.2 | 627.4 |
| **2kh** | 8-Chloro-3-[1,4]diazepan-1-ylmethyl-1-oxo-2-phenyl-1,2-dihydro-isoquinoline-4-carboxylic acid ((S)-1-phenyl-propyl)-amide | 0.98 | 529.1 | 529.3 |
| **2ki** | 8-Chloro-3-((S)-3-methyl-piperazin-1-ylmethyl)-1-oxo-2-phenyl-1,2-dihydro-isoquinoline-4-carboxylic acid ((S)-1-phenyl-propyl)-amide | 0.98 | 529.1 | 529.2 |
| **2kj** | 8-Chloro-3-imidazol-1-ylmethyl-1-oxo-2-phenyl-1,2-dihydro-isoquinoline-4-carboxylic acid ((S)-1-phenyl-propyl)-amide | 0.92 | 497.0 | 497.4 |
| **2kk** | 8-Chloro-3-(4-methyl-imidazol-1-ylmethyl)-1-oxo-2-phenyl-1,2-dihydro-isoquinoline-4-carboxylic acid ((S)-1-phenyl-propyl)-amide | 0.97 | 511.0 | 511.3 |
| **2kl** | 3-(tert-Butylamino-methyl)-8-chloro-1-oxo-2-phenyl-1,2-dihydro-isoquinoline-4-carboxylic acid ((S)-1-phenyl-propyl)-amide | 1.06 | 502.1 | 502.5 |
| **2km** | 8-Chloro-3-(isopropylamino-methyl)-1-oxo-2-phenyl-1,2-dihydro-isoquinoline-4-carboxylic acid ((S)-1-phenyl-propyl)-amide | 1 | 488.0 | 488.6 |
| **2kn** | 8-Chloro-3-[4-hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide | 1.2 | 636.2 | 636.8 |
| **2ko** | 3-(4-tert-Butyl-piperazin-1-ylmethyl)-8-chloro-1-oxo-2-phenyl-1,2-dihydro-isoquinoline-4-carboxylic acid ((S)-1-phenyl-propyl)-amide | 1.03 | 571.2 | 571.4 |
| **2kp** | 3-Benzoimidazol-1-ylmethyl-1-oxo-2-phenyl-1,2-dihydro-isoquinoline-4-carboxylic acid ((S)-1-phenyl-propyl)-amide | 1.02 | 512.6 | 513.3 |
| **2kq** | 1-Oxo-2-phenyl-3-pyrazol-1-ylmethyl-1,2-dihydro-isoquinoline-4-carboxylic acid ((S)-1-phenyl-propyl)-amide | 1.54 | 462.6 | 463.4 |
| **2kr** | 3-(4-Methyl-pyrazol-1-ylmethyl)-1-oxo-2-phenyl-1,2-dihydro-isoquinoline-4-carboxylic acid ((S)-1-phenyl-propyl)-amide | 1.65 | 476.6 | 477.3 |
| **2ks** | 1-Oxo-2-phenyl-3-[1,2,3]triazol-1-ylmethyl-1,2-dihydro-isoquinoline-4-carboxylic acid ((S)-1-phenyl-propyl)-amide | 1.23 | 463.5 | 464.4 |
| **2kt** | 2-(3-Methoxy-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide | 0.82 | 601.7 | 602.7 |
| **2ku** | 2-(4-Methoxy-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide | 0.8 | 623.8 | 624.5 |
| **2kv** | 2-(4-Fluoro-phenyl)-1-oxo-3-(4-phenethyl-piperazin-1-ylmethyl)-1,2-dihydro-isoquinoline-4-carboxylic acid ((S)-1-phenyl-propyl)-amide | 1.13 | 602.8 | 603.8 |
| **2kw** | 2-(4-Fluoro-phenyl)-3-(4-isoproryl-piperazin-1-ylmethyl)-1-oxo-1,2-dihydro-isoquinoline-1-carboxylic acid ((S)-1-phenyl-propyl)-amide | 0.95 | 540.7 | 541.7 |
| **2kx** | 3-[1,4']Bipiperidinyl-1'-ylmethyl-2-(4-fluoro-phenyl)-1-oxo-1,2-dihydro-isoquinoline-4-carboxylic acid ((S)-1-phenyl-propyl)-amide | 0.98 | 580.7 | 581.9 |
| **2ky** | 2-(4-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide | 0.79 | 611.8 | 612.6 |
| **2kz** | 2-(4-Fluoro-phenyl)-3-[4-(1-methyl-piperidin-4-yl)-piperazin-1-ylmethyl]-1-oxo- 1,2-dihydro-isoquinoline-4-carboxylic acid ((S)-1-phenyl-propyl)-amide | 0.74 | 595.8 | 596.8 |
| **2la** | 2-(4-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide | 0.78 | 595.8 | 596.7 |
| **21b** | 2-(4-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide | 1.18 | 619.7 | 620.4 |
| **2lc** | 2-(4-Chloro-phenyl)-1-oxo-3-(4-phenethyl-piperazin-1-ylmethyl)-1,2-dihydro-isoquinoline-4-carboxylic acid ((S)-1-phenyl-propyl)-amide | 1.17 | 619.2 | 619.3 |
| **2ld** | 2-(4-Chloro-phenyl)-3-(4-isopropyl-piperazin-1-ylmethyl)-1-oxo-1,2-dihydro-isoquinoline-4-carboxylic acid ((S)-1-phenyl-propyl)-amide | 0.99 | 557.1 | 557.4 |
| **2le** | 3-[1,4']Bipiperidinyl-1'-ylmethyl-2-(4-chloro-phenyl)-1-oxo-1,2-dihydro-isoquinoline-4-carboxylic acid ((S)-1-phenyl-propyl)-amide | 1.04 | 597.2 | 597.8 |
| **2lf** | 2-(4-Chloro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide | 0.85 | 628.2 | 628.6 |
| **2lg** | 2-(4-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide | 0.77 | 612.2 | 612.3 |
| **2lh** | 2-(4-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide | 0.84 | 626.2 | 626.8 |
| **2li** | 2-(4-Chloro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide | 0.81 | 612.2 | 612.6 |
| **2lj** | 3-(4-tert-Butyl-piperazin-1-ylmethyl)-8-fluoro-1-oxo-2-phenyl-1,2-dihydro-isoquinoline-4-carboxylic acid ((S)-1-phenyl-propyl)-amide | 0.96 | 554.7 | 555.7 |
| **2lk** | 8-Chloro-3-cyclopropylaminomethyl-1-oxo-2-phenyl-1,2-dihydro-isoquinoline-4-carboxylic acid ((S)-1-phenyl- propyl)-amide | 0.61 (method B) | 486.0 | 486.4 |
| **2ll** | 3-(4-tert-Butyl-piperazin-1-ylmethyl)-8-fluoro-1-oxo-2-phenyl-1,2-dihydro-isoquinoline-4-carboxylic acid [(S)-cyclopropyl-(3-fluoro-phenyl)-methyl]-amide | 0.98 | 584.7 | 585.5 |
| **2lm** | 8-Fluoro-1-oxo-2-phenyl-3-piperazin-1-ylmethyl-1,2-dihydro-isoquinoline-4-carboxylic acid [(S)-cyclopropyl-(3-fluoro-phenyl)-methyl]-amide (*) | 0.89 | 528.6 | 529.4 |
| **2ln** | 8-Fluoro-1-oxo-3-(3-oxo-pyrazolidin-1-ylmethyl)-2-phenyl-1,2-dihydro-isoquinoline-4-carboxylic acid [(S)-cyclopropyl-(3-fluoro-phenyl)-methyl]-amide | 1.14 | 528.6 | 528.8 |
| **2lo** | 8-Fluoro-1-oxo-3-(3-oxo-piperazin-1-ylmethyl)-2-phenyl-1,2-dihydro-isoquinoline-4-carboxylic acid [(S)-cyclopropyl-(3-fluoro-phenyl)-methyl]-amide | 1.13 | 542.6 | 543.5 |

| | | | | |
|---|---|---|---|---|
| (*): 1-(tert-Butoxycarbonyl)piperazine was used as amine and the BOC-group was removed in the next step by 2M HCl in diethyl ether and methanol (1:1) at r.t. | | | | |

### Example 3

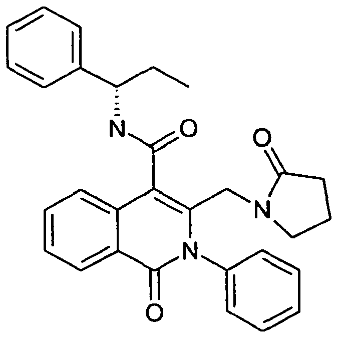

*3a 1-Oxo-3-(2-oxo-pyrrolidin-1-ylmethyl)-2-phenyl-1,2-dihydro-isoquiline-4-carboxylic acid ((S)-1-phenyl-propyl)-amide.*

To a solution of 2-pyrrolidinone (0.1 ml) in tetrahydrofurane (2 ml) sodium hydride (20 mg, 60% dispersion in mineral oil) was added. The reaction mixture was stirred at ambient temperature until the gas evolution ceased then 3-bromomethyl-1-oxo-2-phenyl-1,2-dihydro-isoquinoline-4-carboxylic acid ((S)-1-phenyl-propyl)-amide (30 mg) was added. After 30 min acetic acid (0.05 ml) was added and volatiles were removed in vacuo. The product was purified by flash chromatography on SiO₂ (5 g, 1,2-dichloroethane then gradient 50% to 100% ethyl acetate in heptane) to give 17 mg of white solid. Yield 56%. LC-MS (m/z) 480.5 (MH⁺); t_{R} = 1.21.

The following compound was obtained analogously: 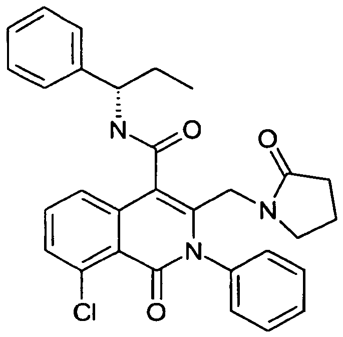 ***3b** 8-Chloro-1-oxo-3-(2-oxo-pyrrolidin-1-ylmethyl)-2-phenyl-1,2-dihydro-isoquinoline-4-carboxylic acid ((S)-1-phenyl)-propyl)-amide.*

LC-MS (m/z) 514.7 (MH⁺); t_{R} = 1.33.

Synthesis of intermediates: 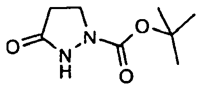 *3-Oxo-pyrazolidine-1-carboxylic acid tert-butyl ester.*

To a mixture of 3-pyrazolidinone hydrochloride (55.3 mg, 0.45 mmol), Na₂CO₃ (133 mg. 1.24 mol), water (0.5 ml) and dioxane (0.054 ml) BOC-anhydride (101 mg, 0.46 mmol) in dioxane (0.16 ml) was added and stirred at r.t. overnight. It was filtered, evaporated and used in the next step without further purification. ¹H NMR (500 MHz, DMSO-d₆): 1.4 (s, 9H), 2.32 (t, J=9 Hz, 2H), 3.6 (t, J=9 Hz, 2H). 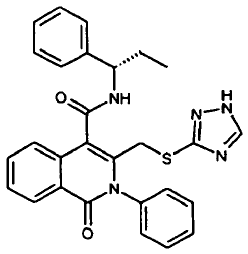 *3c 1-Oxo-2-phenyl-3-(1H-[1,2,4]triazol-3-ylsulfanylmethyl)-1,2-dihydro-isoquinoline-4-carboxylic acid ((S)-1-phenyl-propyl)-amide.*

LC-MS (m/z) 496.6 (MH⁺); t_{R} = 0.64 (method B). 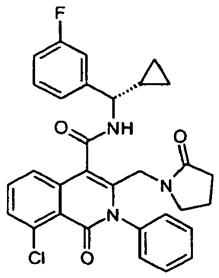 ***3d** 8-Chloro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide.*

LC-MS (m/z) 544.3 (MH⁺); t_{R} = 1.39. ¹H NMR (250 MHz, 70°C, DMSO-d₆): 0.4-0.64 (m, 4H), 1.28 (m, 1H), 1.74 (m, 2H), 1.97 (m, 2H), 2.89 (br, 1H), 3.06 (H₂O), 4.02-4.14 (br, 2H), 4.51 (t, J=8.6 Hz, 1H), 7.08 (dt, 1H), 7.2-7.3 (m, 4H), 7.37-7.7 (m, 7H), 9.07 (d, J=8.1 Hz, 1H, NH). 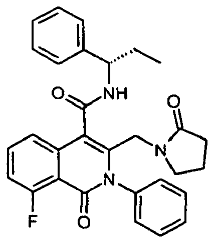 ***3e** 8-Fluoro-1-oxo-3-(2-oxo-pyrrolidin-1-ylmethyl)-2-phenyl-1,2-dihydro-isoquinolize-4-carboxylic acid ((S)-1-phenyl-propyl)-amide.*

LC-MS (m/z) 498.8 (MH⁺); t_{R} = 1.23, t_{R} = 0.68 (method B). ¹H NMR (250 MHz, 70°C, DMSO-d₆): 0.93 (t, J=7.3 Hz, 3H), 1.66-2.0 (m, 6H), 2.84 (br t, 1H), 3.01 (overlapped H₂O signal), 3.98-4.08 (br, 2H), 4.97 (t, J=7.6 Hz, 1H), 7.2-7.54 (m, 12 H), 7.69 (m, 1H), 8.77 (d, J=7.9 Hz, 1H, NH). 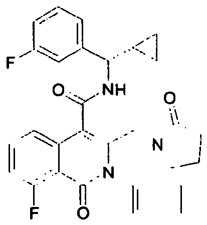 ***3f** 8-Fluoro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide.*

LC-MS (m/z) 528.7 (MH⁺); t_{R} = 1.29, t_{R} = 0.7 (method B). ¹H NMR (250 MHz, 80°C, DMSO-d₆): 0.37-0.62 (m, 4H), 1.25 (m, 1H), 1.71 (quintet, J=7.5 Hz, 2H), 1.94 (t, J=7.9 Hz, 2H), 2.85 (br, 2H), 2.94 (H₂O), 4.05 (br, 2H), 4.5 (t, J=8.6 Hz, 1H), 7.04 (m, 1H), 7.17-7.32 (m, 5H), 7.32-7.52 (m, 5H), 7.7 (m, 1H), 8.97 (d, J=8.1 Hz, 1H, NH). 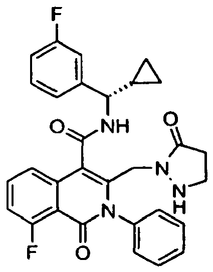 ***3g** 8-Fluoro-1-oxo-3-(5-oxo-pyrazolidin-1-ylmethyl)-2-phenyl-1,2-dihydro-isoquinoline-4-carboxylic acid [(S)-cyclopropyl-(3-fluoro-phenyl)-methy*/*)-amide.* 3-Oxo-pyrazolidine-1-carboxylic acid tert-butyl ester was used in the coupling reaction promoted by NaH. The BOC-group in the obtained intermediate 2-(4-{[(S)-cyclopropyl-(3-fluoro-phenyl)-methyl]-carbamoyl ; -8-fluoro-1-oxo-2-phenyl-1,2-dihydro-isoquinolin-3-ylmethyl)-3-oxo-yrazolidine-1-carboxylic acid tert-butyl ester was removed with 2M HCl in ethyl ether - methanol (1:1) at r.t., 30 min and the title compound was purified by preparative LC-MS. LC-MS (m/z) 529.3 (MH⁺); t_{R} = 1.15. 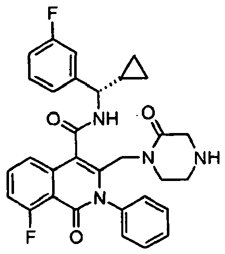 ***3h** 8-Fluoro-1-oxo-3-(2-oxo-piperazin-1-ylmethyl)-2-phenyl-1,2-dihydro-isoquinoline-4-carboxylic acid[(S)-cyclopropyl-(3-fluoro-phenyl)-methyl]-amide.*

The title compound was prepared analogously from 2-oxo-4-(tert-butoxycarbonyl)piperazine followed by BOC group removal. LC-MS (m/z) 543.6 (MH⁺); t_{R} = 0.84. 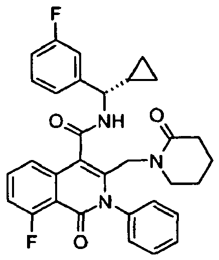 ***3i** 8-Fluoro-1-oxo-3-(2-oxo-piperidin-1-ylmethyl)-2-phenyl)-2-dihydro-isoquinoline-4-carboxylic acid [(S)-cyclopropyl-(3-fluoro-phenyl)-methyl]-amide.*

The title compound was prepared analogously from 2-piperidone. LC-MS (m/z) 542.5 (MH⁺): t_{R} = 1.35.

### Example 4

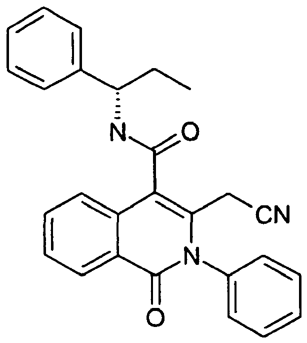

***4a** 3-Cyanomethyl-1-oxo-2-phenyl-1,2-dihydro-isoquinoline-4-carboxylic acid ((S)-1-phenyl-propyl)-amide.*

To a solution of 3-bromomethyl-1-oxo-2-phenyl-1,2-dihydro-isoquinoline-4-carboxylic acid ((S)-1-phenyl-propyl)-amide (83 mg, 0.174 mmol) in THF (1.5 ml, anhydrous) and DMF (1.5 ml, anhydrous) sodium cyanide (83 mg, excess) was added. After 5 min the obtained suspension was poured into water (50 ml) followed by addition of heptane (20 ml). The crude product was separated by filtration and purified by flash chromatography on SiO₂ (5g, 1:1 EA-heptane) to give 50 mg of colourless solid, yield 68%. LC-MS (m/z) 422.4 (MH⁺); t_{R} = 1.39. ¹H NMR (250 MHz, DMSO-d₆, T = 343 K): 0.94 (t, 3H), 1.78 and 1.85 (two overlapping qui of ABX₄, 2H, CH₂ of Et), 3.45 and 3.54 (two d of AB, 2H, CH₂CN), 4.98 (q, 1H), 7.22-7.44 (m, 8H), 7.53-7.64 (m, 4H), 7.72 (dt, 1H), 8.25 (dd, 1H), 9.05 (d, 1H). 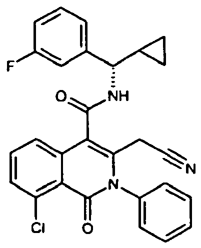 ***4b** 8-Chloro-3-cyanomethyl-1-oxo-2-phenyl-1,2-dihydro-isoquinoline-4-carboxylic acid [(S)-cyclopropyl-(3-fluoro-phenyl)-methyl]-amide.*

LC-MS (m/z) 486.5 (MH⁺); t_{R} = 1.81.

### Example 5

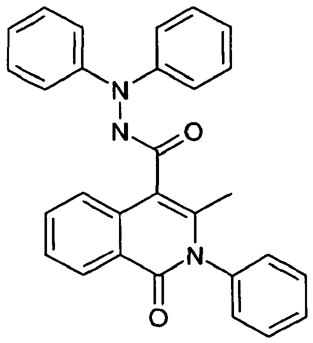

***5a** 3-Methyl-1-oxo-2 phenyl-1,2-dihydro-isoquinoline-4-carboxylic acid N',N'-diphenyl-hydrazide.*

A mixture of 3-methyl-1-oxo-2-phenyl-1,2-dihydro-isoquinoline-4-carbonyl chloride (the synthesis is described above in example **1a**) (25 mg, 0.08 mmol) and 1,1-diphenylhydrazine hydrochloride (56 mg, 0.25 mmol) in 1,2-dichloroethane (1 ml) was heated under microwave irradiation for 20 min at 140°C. Volatiles were removed in vacuo and the product was purified by preparative LC-MS to give 14 mg of colorless solid. LC-MS (m/z) 446.7 (MH⁺); t_{R} = 1.58. 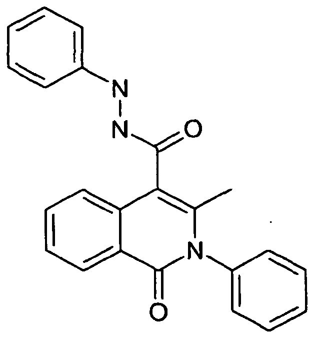 *3-Methyl-1-oxo-2-phenyl-1,2-dihydro-isoquinoline-4-carboxylic acid N'-phenyl-hydrazide.* [740-170]

The title compound was prepared analogously. LC-MS (m/z) 370.2 (MH⁺); t_{R} = 1.15. ¹H NMR (500 MHz, DMSO-d₆): 2.01 (s, 3H), 6.76 (t, 1H), 6.83 (d, 2H), 7.2 (t, 2H), 7.33 (br, 2H), 7.5-7.62 (m, 5H), 7.83 (t, 1H), 8.11 (br, 1H), 8.24 (d, 1H), 10.33 (s, 1H). 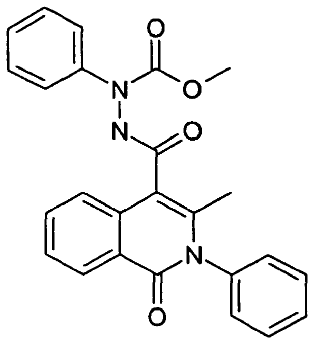 *5b N'-(3-Methyl-1-oxo-2-phenyl-1,2-dihydro-isoquinoline-4-carbonyl)-N-phenyl-hydrazinecarboxylic acid methyl ester.*

A mixture of 3-methyl-1-oxo-2-phenyl-1,2-dihydro-isoquinoline-4-carboxylic acid N'-phenyl-hydrazide (5a, 490 mg, 1.3 mmol) and methyl chloroformate (0.6 ml, 8 mmol) in 1,2-dichloroethane (10 ml) was heated under microwave irradiation for 10 min at 100°C. Volatiles were removed in vacuo and the product was purified by flash chromatography (SiO₂, gradient heptane - 1:1 ethyl acetate/heptane) to give 190 mg, of solid. LC-MS (m/z) 428.8 (MH⁺); t_{R} = 1.22.

### Example 6

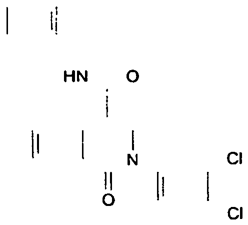

***6a** 2-(3,4-Dichloro-phenyl)-3-methyl-1-oxo-1,2-dihydro-isoquinoline-4-carboxylic acid ((S)-1-phenyl-propyl)-amide.*

A mixture of 4-acetyl-3-((S)-1-phenyl-propylamino)-isochromen-1-one (60 mg) and 3,4-dichloroaniline (250 mg) in acetonitrile (0.5 ml) were heated under microwave irradiation at 160°C for 15 min. The reaction mixture was partitioned between 2M HCl (3 ml) and ethyl acetate (3 ml). The organic solution was absorbed on SiO₂ (600 mg) and flash chromatographed on SiO₂ (20g) with gradient heptane - ethyl acetate to give 22 mg of the title compound as a white solid. LC-MS (m/z) 465.4 & 467.4 (MH⁺); t_{R} = 1.63.

The following compounds were obtained analogously from corresponding compounds of the general formula XXIII and appropriate anilines of the general formula VI: 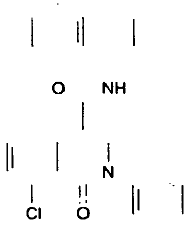 *8-Chloro-3-methyl-1-oxo-2-phenyl-1,2-dihydro-isoquinoline-4-carboxylic acid ((S)-1-phenyl-propy*/*)-amide (1i).*

The title compound and its alternative method of preparation was described in the example **1i**. 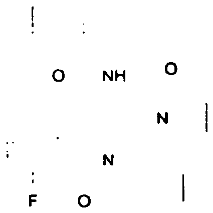 ***6b** 8-Fluoro-1-oxo-3-(2-oxo-pyrrolidin-1-ylmethyl)-2-phenyl-1,2-dihydro-isoquinoline-4-carboxylic acid ((S)-cyclopropyl-phenyl-methyl)-amide.*

LC-MS (m/z) 510.3 (MH⁺); t_{R} = 1.25. 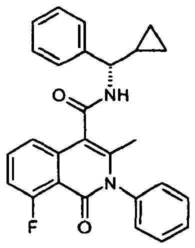 *6c 8-Fluoro-3-methyl-1-oxo-2-phenyl-1,2-dihydro-isoquinoline-4-carboxylic acid ((S)-cyclopropyl-phenyl-methyl)-amide.*

LC-MS (m/z) 427.3 (MH⁺); t_{R} = 1.39.

### Example 7

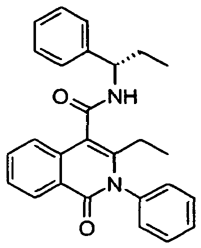

***7a** 3-Ethyl-1-oxo-2-phenyl-1,2-dihydro-isoquinoline-4-carboxylic acid ((S)-1-phenyl-propyl)-amide.*

A sealed mixture of 2-[2-oxo-1-((S)-)1-phenyl-propylcarbamoyl)-butyl]-benzoic acid (82 mg, 0.2 mmol) and aniline (0.3 ml, 3.3 mmol) in acetonitrile (0.3 ml) was heated under microwave irradiation at +160°C for 10 min. The mixture was partitioned between ethyl acetate (20 ml) and 3M aq. HCl (2 x 8 ml), sat. aq. NaHCO₃ (2 x 10 ml and brine, dried (MSO₄) and evaporated. The residue was purified by flash chromatography (SiO₂, gradient heptane - ethyl acetate) to give 32 mg of the title compound. LC-MS (m/z) 411.5 (MH⁺); t_{R} = 1.48. ¹H NMR (500 MHz, DMSO-d₆): a mixture of two atropisomers.

The following compounds were obtained analogously by condensation of compounds of the general formula XXV with appropriate aniline of the general formula VI: 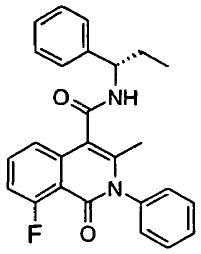 *7b 8-Fluoro-3-methyl-1-oxo-2-phenyl-1,2-dihydro-isoquinoline-4-carboxylic acid ((S)-1-phenyl-propyl)-amide.*

LC-MS (m/z) 415.5 (MH⁺); t_{R} = 1.39. 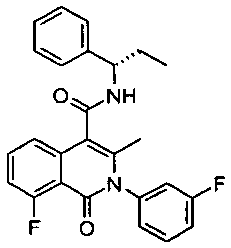 ***7c** 8-Fluoro-2-(3-fluoro-phenyl)-3-methyl-1-oxo-1,2-dihydro-isoquinoline-4-carboxylic acid ((S)-1-phenyl-propyl)-amide.*

LC-MS (m/z) 433.5 (MH⁻): t_{R} = 1.41. 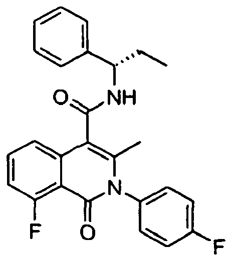 ***7d** 8-Fluoro-2-(4-fluoro-phenyl)-3-methyl-1-oxo-1,2-dihydro-isoquinoline-4-carboxylic acid ((S)-1-phenyl-propyl)-amide.*

LC-MS (m/z) 433.6 (MH⁺); t_{R} = 1.4. 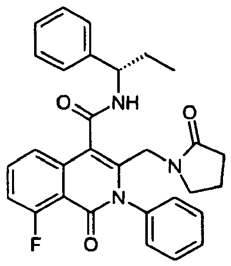 *8-Fluoro-1-oxo-3-(2-oxo-pyrrolidin-1-ylmethyl)-2-phenyl-1,2-dihydro-isoquinoline-4-carboxylic acid ((S)-1-phenyl-propyl)-amide (3e).*

The title compound and its alternative method of preparation was described above in the example 3. 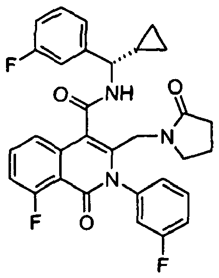 ***7f** 8-Fluoro-2-(3-fluoro-phenyl)-1-oxo-3-(2-oxo-pyrrolidin-1-ylmethyl)1,2-dihydro-isoquinoline-4-carboxylic acid [(S)-cyclopropyl-(3-fluoro-phenyl)-methyl]-amide.*

The title product was isolated by preparative TLC on silica gel. LC-MS (m/z) 546.4 (MH⁺): t_{R} = 0.71 (method B). 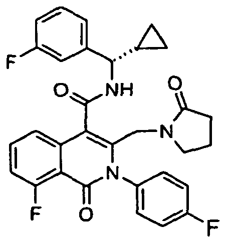 ***7g** 8-Fluoro-2-(4-fluoro-phenyl)-1-oxo-3-(2-oxo-pyrrolidin-1-ylmethyl)-1,2-dihydro-isoquinoline-4-carboxylic acid [(S)-cyclopropyl-(3-fluoro-phenyl)-methyl]-amide.*

The title product was isolated by preparative TLC on silica gel. LC-MS (m/z) 546.3 (MH⁻): t_{R} = 0.71 (method B). 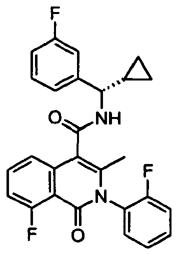 ***7i** 8-Fluoro-2-(2-fluoro-phenyl-3-methyl-1-oxo-1,2-dihydro-isoquinoline-4-carboxylic acid [(S)-cyclopropyl-(3-fluoro-phenyl)-methyl]-amide.*

LC-MS (m/z) 463.3 (MH⁺); t_{R} = 1.46. 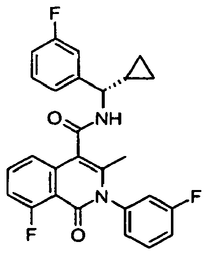 ***7j** 8-Fluoro-2-(3-fluoro-phenyl)-3-methyl-1-oxo-1,2-dihydro-isoquinoline-4-carboxylic acid [(S)-cyclopropyl-(3-fluoro-phenyl)-methyl]-amide.*

LC-MS (m/z) 463.3 (MH⁺); t_{R} = 1.46. 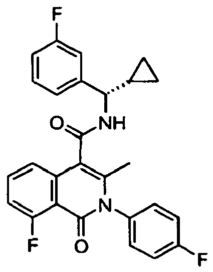 ***7k** 8-Fluoro-2-(4-fluoro-phenyl)-3-methyl-1-oxo-1,2-dihydro-isoquinoline-4-carboxylic acid (S)-cyclopropyl-(3-fluoro-phenyl)-methyl]-amide.*

LC-MS (m/z) 463.4 (MH); t_{R} = 1.45. 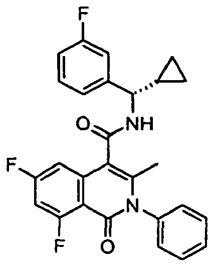 *7l 6,8-Difluoro-3-methyl-1-oxo-2-phenyl-1,2-dihydro-isoquinoline-4-carboxylic acid [(S)-cyclopropyl-(3-fluoro-phenyl)-methyl]-amide.*

LC-MS (m/z) 463.2 (MH⁺); t_{R} = 0.82 (method B). 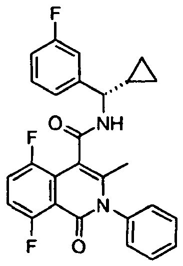 ***7m** 5,8-Difluoro-3-methyl-1-oxo-2-phenyl-1,2-dihydro-isoquinoline-4-carboxylic acid [(S)-cyclopropyl-(3-fluoro-phenyl)-methyl]-amide.*

LC-MS (m/z) 463.4 (MH⁺); t_{R} = 1.39. 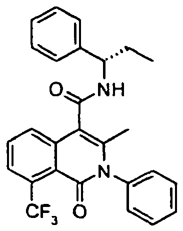 7n 3-Methyl-1-oxo-2-phenyl-8-trifluoromethyl-1,2-dihydro-isoquinoline-4-carboxylic acid ((S)-1-phenyl-propyl)-amide.

LC-MS (m/z) 465.3 (MH⁺); t_{R} = 1.57.

### Example 8

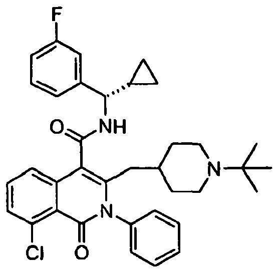

***8a** 3-(1-tert-Butyl-piperidin-4-ylmethyl)-8-chloro-1-oxo-2 phenyl-1,2-dihydro-isoquinoline-4-carboxylic acid [(S)-cyclopropyl-(3-fluoro-phenyl)-methyl]-amide.*

To a cold (ice bath) mixture of 2-(1-tert-butyl-piperidin-4-yl)-N-phenyl-acetamide (250 mg, 0.91 mmol) and 2,6-dimethylpyridine (0.139 ml, 1.2 mmol) in 1,2-dichloroethane (8 ml) oxalyl chloride (0.07 ml, 0.8 mmol) was added and stirred at 0°C for 15 min. 2-Chloro-6-({[(S)-cyclopropyl-(3-fluoro-phenyl)-methyl]-carbamoyl}methyl)-benzoic acid (70 mg, 0.2 mmol) was added and the reaction mixture was stirred in a sealed vessel at 90°C for 15 hours. It was partitioned between 0.5 M aq. NaOH (40 ml) and ethyl acetate (200 ml), the organic phase washed with water, dried and evaporated. The residue was purified by flash chromatography (SiO₂, ethyl acetate - methanol) to give 19 mg of the title compound. LC-MS (m/z) 600.6 (MH⁻): t_{R} = 1.1. ¹H NMR (500 MHz, r.t., DMSO-d₆): broad signals, a mixture of two atropisomers.

Reagents used for the preparation of the compounds.

| **Name** | **Supplier** | **CAS no.** | **Cat.no.** |
|---|---|---|---|
| Ethyl acetoacetate | Aldrich | 141-97-9 | E 964-1 |
| Methyl acetoacetate | Aldrich | 105-45-3 | 53,736-5 |
| Sodium hydride, 60% dispersion in mineral oil | Aldrich | 7646-69-7 | 45,291-2 |
| Copper (I) bromide | Aldrich | 7787-70-4 | 21,286-5 |
| 2-Bromobenzoic acid | Janssen Chimica | 88-65-3 | 41066/1 |
| 2-Bromo-4-methylbenzoic acid | Matrix Scientific | 7697-27-0 | 001816 |
| 2-Bromo-6-methylbenzoic acid | Matrix Scientific | 90259-31-7 | 001819 |
| 2,5-Dibromobenzoic acid | ABCR | 610-71-9 | AV15077 |
| 2-Bromo-4-fluorobenzoic acid | Fluorochem | 1006-41-3 | 005470 |
| 2-Bromo-5-chlorobenzoic acid | ABCR | 21739-93-5 | AV10103 |
| 2-Bromo-5-fluorobenzoic acid | Aldrich | 394-28-5 | 56,349-8 |
| 2-Bromo-3-methylbenzoic acid | Fluorochem | 53663-39-1 | 112800 |
| 2-Bromo-5-methylbenzoic acid | Fluorochem | 6967-82-4 | B2947-E |
| 2-Bromo-6-chlorobenzoic acid | Fluorochem | 93224-85-2 | 19332 |
| Aniline | Aldrich | 62-53-3 | 13,293-4 |
| 2-Fluoroaniline | Lancaster | 348-54-9 | 1236 |
| 2,6-Difluoroaniline | Aldrich | 5509-65-9 | 196614 |
| 2-Chloroaniline | Aldrich | 95-51-2 | 23300 |
| o-Toluidine | Aldrich | 95-53-4 | 18,542-6 |
| 3-Fluoroaniline | Flrochem | 372-19-0 | 1438 |
| 3-Chloroaniline | Fluka | 108-42-9 | 23330 |
| m-Toluidine | Aldrich | 108-44-1 | 511218 |
| (S)-(-)-1-Phenylpropylamine | Lancaster | 3789-59-1 | X16320G0025 |
| C-((S)-C-Cyclopropyl-C-phenyl)- methylamine | The compound was prepared according to a described procedure in WO 2005/014575 | | |
| C-[(S)-C-Cyclopropyl-C-(3-fluoro- phenyl)]-methylamine | The compound was prepared according to a described procedure in WO 2005/014575 | | |
| Tetraethoxysilane | Aldrich | 78-10-4 | 236209 |
| 1-Ethyl-3-(3-dimethylaminopropyl)carbodiimide hydrochloride (EDC) | Aldrich | 25952-53-8 | 16,146-2 |
| 1-Hydroxybenzotriazole (HOBT) | ABCR | 2592-95-2 | AV21700 |
| Dimethylamine, 33% solution in abs. Fluka Ethanol | | 124-40-3 | 38950 |
| Methylamine, 2M solution in THF | Aldrich | 74-89-5 | 42,646-6 |
| 2-Pyrrolidone | Aldrich | 616-45-5 | 240338 |
| 1,1-Diphenylhydrazine hydrochloride | Aldrich | 530-47-2 | 11,459-6 |
| Phenylhydrazine | Aldrich | 100-63-0 | P2,625-2 |
| Methyl chloroformate | Aldrich | 79-22-1 | M35304 |
| Sodium cyanide | Aldrich | 143-33-9 | 380970 |

Reagents used for the preparation of the compounds, continued.

| **Name** | **Supplier** | **CAS no.** | **Cat.no.** |
|---|---|---|---|
| Homophthalic anhydride | ABCR | 703-59-3 | AV 15538 |
| Cyclopropanecarboxaldehyde | Aldrich | 1489-69-6 | 27,221-3 |
| 1-Bromo-3-fluorobenzene | Aldrich | 1073-06-9 | B67007 |
| (S)-3-Amino-3-phenylpropanenitrile | Netchem | - | 331516 |
| 3,4-Dichloroaniline | Aldrich | 95-76-1 | 56042 |
| 2-Bromo-6-nitrobenzoic acid | Ref. 1 | 38876-67-4 | - |
| 2-Bromo-6-methoxybenzoic acid | Ref. 2 | 31786-45-5 | - |
| tert-Butyl 2-bromoacetate | Aldrich | 5292-43-3 | 12,426-0 |
| Ethyl (triphenylphosphorylidene)acetate | Aldrich | 1099-45-2 | C510-6 |
| 1-(tert-Butoxycarbonyl)piperazine | Lancaster | 57260-71-6 | 13363 |
| 3-Pyrazolidinone hydrochloride | Acros | 1752-88-1 | 335490050 |
| 2-Oxo-4-(tert-butoxycarbonyl)piperazine | Aldrich | 76003-29-7 | 64,105-7 |
| 2-Piperidone | Aldrich | 675-20-7 | V20-9 |

| | | | |
|---|---|---|---|
| Ref. 1. The compound was prepared according to a described procedure: S.C. Glossop Synthesis 2007, 7. 981. Ref. 2. The compound was prepared according to a described procedure: T. Sugaya, Y. Mimura, N. Kato, M. Ikuta, T. Mimura et al. Synthesis 1994, 73. | | | |

### Example 9 NK3 receptor binding assay

**Membranepreparation:** BHK cells stably expressing the human NK3 receptor were seeded in harvesting plates in Dulbeccos MEM containing GlutaMax (862 mg/l), 1 mM sodium pyruvate, 10% fetal calf serum, 1% Pen/Strep, I mg/ml G418 and were grown at 34 °C in a humidified atmosphere containing 10% CO₂. To increase receptor expression, 10 µM trichotatin A was added to the media 24 hours before harvest of the cells at a confluency of app 90%. Prior to the harvesting, the cells were washed twice with PBS without Mg²⁺ og Ca²⁺ and subsequently scrapped of in 10 ml PBS pr harvesting plate. The cells suspension were centrifuged at 1500xG in three minutes before resuspension in 15 mM Tris-HCl pH 7,5 buffer containing 2 mM MgCl₂; 0,3 mM EDTA and 1 mM EGTA (buffer A). The cell suspention was homogenised and subsequently centrifuged at 40000xG in 30 minutes. The membrane-pellet was resuspended in buffer A containing 250 mM sucrose, alliquoted and stored at - 80°C.

**Affinity assay description:** The assay was performed as a SPA-based competition-binding assay in a 50 mM Tris pH 7.4 assay buffer containing 120 mM NaCl, 3 mM MnCl₂, 40 µg/ml bacitracin, 2 µg/ml Chymostatin, 1 µg/ml Phosphoramidon and 4 µg/ml Leupeptin. App 0.02 nM ¹²⁵I-NKB was mixed with test compounds before addition of 4 µg of the homogenised NK3 membrane preparation and 0,025 mg SPA beads in a total volume of 60 µl. The assay plate was subsequently under agitation incubated for 90 min at RT. The plate was centrifugated 10 minutes at 500xG and counted in a topcounter 5 minuttes pr well.

The total binding, which comprised less than 5 % of added radioligand, was defined using assay buffer whereas the non-specific binding was defined in the presence of 1 µM osanetant. The non-specific binding constituted app 5 % of the total binding.

Data points were expressed in percent of the specific binding of ¹²⁵I-NKB and the IC₅₀ values (concentration causing 50 percent inhibition of ¹²⁵I-NKB specific binding) were determined by non-linear regression analysis using a sigmoidal variable slope curve fitting. The dissociation constant (Kᵢ) were calculated from the Cheng Prusoff equation (Kᵢ = IG₅₀/(1+(L/K_{D}))), where the concentration of free radioligand L is approximated to the concentration of added ¹²⁵I-NKB in the assay (app 0,02 nM).

The K_{D} of ¹²⁵I-NKB was determined to be 0,7 nM from three independent saturation assays each performed with duplicate determinations. Bmax was app 2 pmol/mg.

The compounds of the present invention generally have Kᵢ values of 500 nM or less. Many compounds, in fact, have Kᵢ values below 100 nM and down to single digit values.

The table below shows the Kᵢ for the NK3 receptor

| Compound | Kᵢ nM |
|---|---|
| Ex 2a | 180 |
| Ex 2bm | 8.2 |
| Ex 2hs | 5.3 |
| Ex 2hz | 3 |
| Ex 3a | 72 |
| Ex 5a | 55 |

See also the table in Example 10 for more affinity data for the compounds of the present invention.

### Example 10 NK3 receptor efficacy and potency assay

BHK cells stably expressing the human NK3 receptor were seeded in 100 µl media in black walled clear-base 96-wells plates (Costar) aiming at a confluency of 95-100% at the day of assay. The assay was performed according to the FLIPR Calcium 4 Assay kit (Molecular Devices). At the day of the assay, the media was removed and the cells were washed once with the HBSS buffer (Hanks BSS buffer, pH 7,4 containing 20 mM Hepes) before 100 µl of a solution of the calcium assay reagent dissolved in the HBSS buffer containing 2,5 mM probinicid was added to the cells. The plates were incubated for 60 min at 34°C, 10% CO₂ before use in the FLIPR for examination of fluorescence.

One representative plate was examined with a dose-response curve with NKB in a setup in which the wells initially were added HBSS buffer and 15 min later the various concentrations of NKB were added in order to determine the EC₅₀ and EC₈₅ of NKB. All compound plates used for NKB were precoated with a 1% BSA solution and subsequently washed three times with H₂O. NKB was diluted in HBSS buffer containing 0,1% BSA.

For efficacy and potency evaluation of compounds, these were diluted in HBSS buffer prior to test. For test of agonist activity, 25 µl of the diluted compound solution was added and the plate was analyzed for 5 minutes in the FLIPR. For test of antagonist activity, the plate was incubated for another 45 minutes before addition of 25 µl of the EC₈₅ concentration of NKB (app. 4 nM) as described above. The plates were subsequently analyzed for 5 minutes before the assay was terminated. The maximal increase in fluorescence over background following each ligand addition was determined. The IC₅₀ value was calculated using sigmoidal variable slope curve fitting, and the clC₅₀ value was determined using the equation (cIC₅₀ = IC₅₀/(1+(EC₈₅/EC₅₀))), where EC₈₅ and EC₅₀ for NKB were determined as described above.

All isoquinolinones of the present invention characterized in the NK3 receptor efficacy and potency assay have been antagonists without any observed significant agonist activity at relevant doses. The table shows affinity data (obtained as described in Example 9) and potency data obtained with the compounds of the invention. Minor diffrences between the tabulated values in Example 9 and 10 only reflects that more than one batch have been tested and/or that one batch has been tested more than once.

| | **Affinity** | | **Potency** |
|---|---|---|---|
| **Example No** | **Kᵢ/nM** | | **cIC₅₀/nM** |
| 2e | 330 | | 470 |
| 1i | 41 | | 130 |
| 3a | 88 | | 270 |
| 2r | 320 | | 420 |
| 2s | 200 | | 340 |
| 2u | 90 | | 96 |
| 2ah | 290 | | 360 |
| 2bq | 160 | | 220 |
| 2bm | 8.8 | | 14 |
| 2bn | 160 | | 300 |
| 2cw | 8,4 | | 45 |
| 2cz | 76 | | 49 |
| 2df | 340 | | 450 |
| 2ec | 210 | | 520 |
| 2eg | 240 | | 610 |
| 2es | 140 | | 240 |
| 2ey | 240 | | 480 |
| 2fm | 33 | | 140 |
| 2gt | 9,2 | | 22 |
| 2hq | 10 | | 44 |
| 2hs | 7,5 | | 21 |
| 2hu | 6,2 | | 29 |
| 2hv | 14 | | 35 |
| 2hw | 8,2 | | 63 |
| 2hx | 9,3 | | 68 |
| 2hy | 8,8 | | 63 |
| 2hz | 4,5 | | 35 |
| 2ia | 12 | | 100 |
| 2iy | 17 | | 92 |
| 1t | 27 | | 44 |
| 2kh | 82 | | 120 |
| 2kn | 8,7 | | 41 |
| 2ko | 11 | | 5,6 |
| 3d | 16 | | 46 |
| 2lj | 18 | | 16 |
| 3b | 32 | | 47 |
| 1bh | 21 | | 81 |
| 2ll | 7,9 | | 6,3 |
| 8a | 7 | | 9,9 |
| 1bn | 23 | | 23 |
| 6c | 23 | | 24 |
| 1bl | 470 | | |

## Claims

1. A compound according to formula I 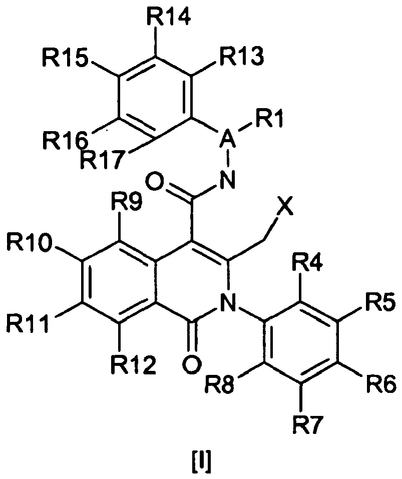 wherein A represents N, CH or CR¹;
wherein each R¹ independently represents hydrogen, C₁₋₆alkyl, C₂₋₆alkenyl, C₂₋₆ alkynyl, -C(O)-C₁₋₆alkyl, -C(O)-C₂₋₆alkenyl, -C(O)-C₂₋₆alkynyl, -C(O)-O-C₁₋₆alkyl, - C(O)-O-C₂₋₆alkenyl, -C(O)-O-C₂₋₆alkynyl or phenyl, wherein said phenyl, C₁₋₆alkyl, C₂₋₆alkenyl or C₂₋₆alkynyl is optionally substituted with one or more substituents selected from halogen, hydroxyl, haloC₁₋₆alkyl, nitro, C₁₋₆alkoxy, and NR²R³; wherein X represents hydrogen, C₁₋₆alkyl, C₂₋₆alkenyl, C₂₋₆alkynyl, cyano, -OR², -O-C(O)R², -OC(O)NR²R³, -C(O)-NR²R³, -N(R²)C(O)R³, -N(R²)-C(O)NR²R³ or NR²R³, or X represents a mono-cyclic, bi-cyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₂₋₆alkenyl, C₂₋₆alkynyl, C₁₋₆alkoxy, -C(O)-C₁₋₆alkyl, -C(O)-C₂₋₆alkenyl, -C(O)-C₂₋₆alkynyl, - C(O)-O-C₁₋₆alkyl, -C(O)-O-C₂₋₆alkenyl, -C(O)-O-C₂₋₆alkynyl, -O-C(O)-C₁₋₆alkyl, -O-C(O)-C₂₋₆alkenyl, -O-C(O)-C₂₋₆alkynyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein each of R⁴-R⁸, R⁹-R¹², and R¹³-R¹⁷ independently represent hydrogen, C₁₋₆ alkyl, C₂₋₆alkenyl, C₂₋₆alkynyl, halogen, NR²R³, hydroxy, cyano, nitro, C₁₋₆alkoxy, haloC₁₋₆alkyl or hydroxyC₁₋₆alkyl;
wherein R² and R³ independently represent hydrogen, C₁₋₆alkyl, C₂₋₆alkenyl, C₂₋₆ alkynyl, hydroxyC₁₋₆alkyl, haloC₁₋₆alkyl or phenyl;
and pharmaceutically acceptable salts thereof.

2. A compound according to claim 1, wherein R⁴-R⁸ represent hydrogen.

3. A compound according to claim 1, wherein R⁹-R¹² represent hydrogen.

4. A compound according to claim 1, wherein R¹³-R¹⁷ represent hydrogen.

5. A compound according to claim 1 of
- formula Iₐ 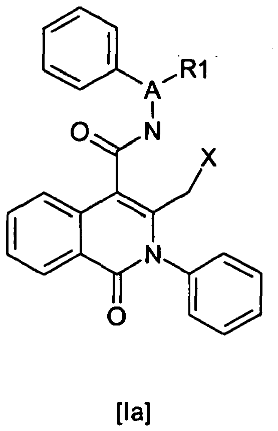 wherein A represents N, CH or CR¹;
wherein each R¹ independently represents hydrogen, C₁₋₆alkyl, -C(O)-C₁₋₆alkyl, -C(O)-O-C₁₋₆alkyl, or phenyl, wherein said phenyl or C₁₋₆alkyl, is optionally substituted with one or more substituents selected from halogen, hydroxyl, halo C₁₋₆ alkyl, nitro, C₁₋₆alkoxy, and NR²R³;
wherein X represents hydrogen, C₁₋₆alkyl, cyano, -OR², -O-C(O)R², -OC(O)NR²R³, - C(O)-NR²R³, -N(R²)C(O)R³, -N(R²)-C(O)NR²R³ or NR²R³, or X represents a mono-cyclic, bicyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ- Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein each of R⁴-R⁸, R⁹-R¹², and R¹³-R¹⁷ independently represent hydrogen, C₁₋₆ alkyl, C₂₋₆alkenyl, C₂₋₆alkynyl, halogen, NR²R³, hydroxy, cyano, nitro, C₁₋₆alkoxy, haloC₁₋₆alkyl or hydroxyC₁₋₆alkyl;
wherein R² and R³ independently represent hydrogen, C₁₋₆alkyl, C₂₋₆alkenyl, C₂₋₆ alkynyl, hydroxyC₁₋₆alkyl, haloC₁₋₆alkyl or phenyl;
and pharmaceutically acceptable salts thereof; or
- formula I_{b} 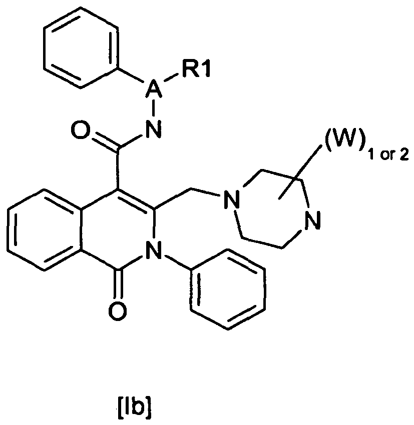 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, hatoC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula Ic 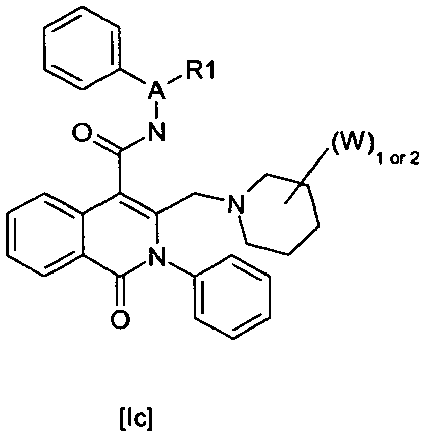 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(cH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- the formula I_{d} 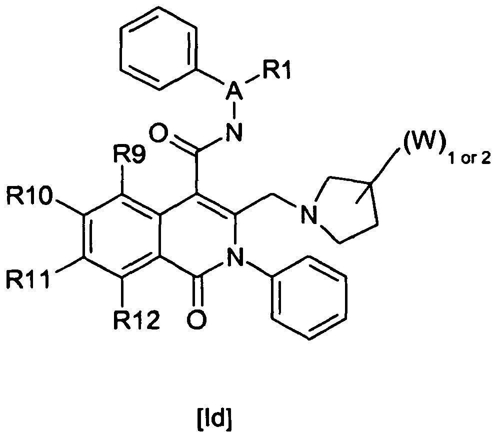 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O-C₁₋₆alkyl, -C(O)H, COOH;
wherein R² and R³ independently represent hydrogen, C₁₋₆alkyl, hydroxyC₁₋₆alkyl, haloC₁₋₆alkyl or phenyl;
wherein R⁹-R¹² independently represent hydrogen or halogen;
and pharmaceutically acceptable salts thereof; or
- formula Iₑ 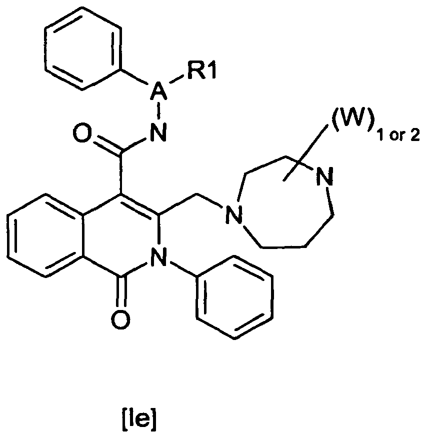 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula If 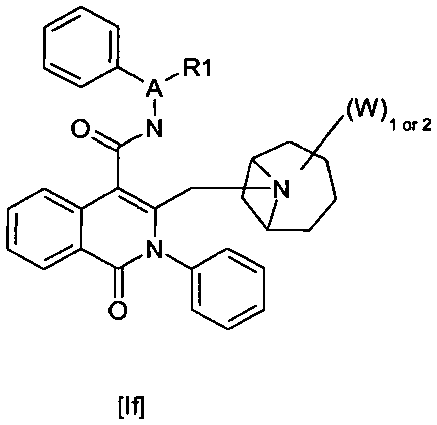 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(OP)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula I_{g} 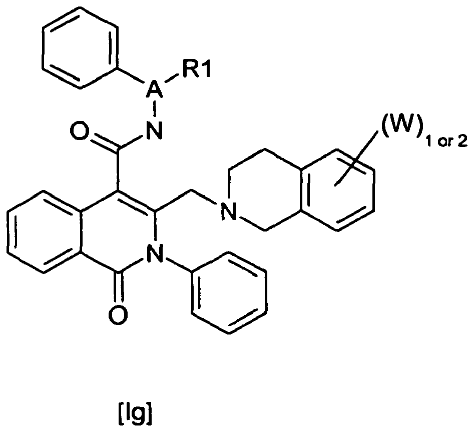 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, hatoC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -N(R²)-C(O)-R³, -C(O)H, COOH; or wherein W represents a moiety of the formula -(CH₂)ₐ-Y-(CH₂)_{b}-Z; wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula Iₕ 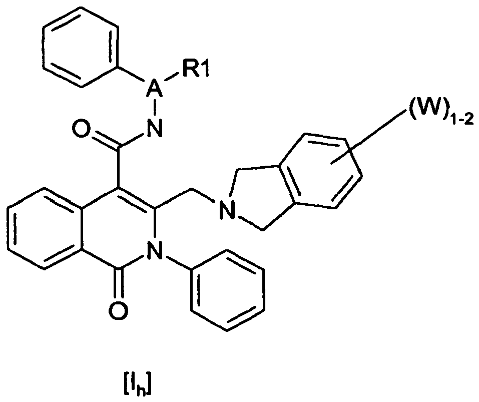 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH2)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆ₐlkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula Iᵢ 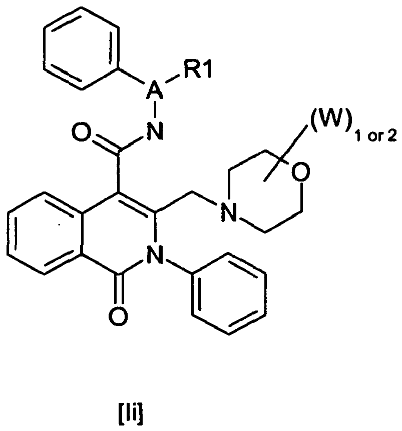 wherein A represents N, CH or CR¹;
wherein each R¹ independently represents hydrogen, C₁₋₆alkyl, -C(O)-C₁₋₆alkyl, C(O)-O-C₁₋₆alkyl, or phenyl, wherein said phenyl, or C₁₋₆alkyl is optionally substituted with one or more substituents selected from halogen, hydroxyl, haloC₁₋₆alkyl, nitro, C₁₋₆ alkoxy, and NR²R³;
wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, - C(O)-C₁₋₆alkyl, -C(O)-O-C₁₋₆alkyl, -O-C(O)-C₁₋₆alkyl, -C(O)H, COOH; or wherein X represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, NR², -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, pyrazinyl, phenyl, pyridyl, and halogen substituted phenyl;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula Ij 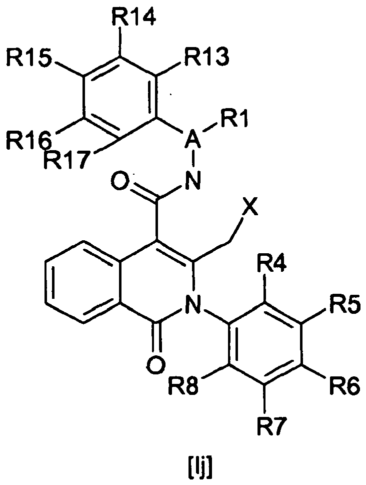 wherein A represents N, CH or CR¹;
wherein each R¹ independently represents hydrogen, C₁₋₆alkyl, -C(O)-C₁₋₆alkyl, -C(O)-O-C₁₋₆alkyl, or phenyl, wherein said phenyl or C₁₋₆alkyl is optionally substituted with one or more substituents selected from halogen, hydroxyl, haloC₁₋₆ alkyl, nitro, C₁₋₆alkoxy, and NR²R³;
wherein X represents hydrogen, C₁₋₆alkyl, cyano, -OR², -O-C(O)R², -OC(O)NR²R³, - C(O)-NR²R³, -N(R²)C(O)R³, -N(R²)-C(O)NR²R³ or NR²R³, or X represents a mono-cyclic, bicyclic or tri-cyclic moiety having 4-16 ring atoms one of which is nitrogen, and wherein one, two or three additional ring atoms may be a hetero atom selected from N, O and S, and wherein said mono-cyclic, bi-cyclic or tri-cyclic moiety may optionally be substituted with one or more substituents W, wherein W is selected from hydrogen, hydroxy, halogen, cyano, (=O), -O-(CH₂)_{c}-O-, wherein c is 2 or 3 (spiro), (CH₂)_{d}, wherein d is 5 or 6 (spiro), C₁₋₆alkyl, C₁₋₆alkoxy, -C(O)-C₁₋₆alkyl, -C(O)-O-C₁₋₆alkyl, -O-C(O)-C₁₋₆alkyl, -C(O)H, COOH; or wherein W represents a moiety of the formula -(CH₂)ₐ-Y-(CH₂)_{b}-Z;
wherein a and b independently represent an integer selected from 0, 1, 2 and 3; wherein Y represents a bond, C(O), O, S, -O-C(O), -C(O)-O-, -NR²-, -NR²-C(O)-, - C(O)-NH-, or S(O)₂;
wherein Z represents hydrogen, C₁₋₆alkyl, NR²R³, cyano or a mono-cyclic or fused bicyclic moiety comprising 4 to 12 ring atoms of which optionally one, two or three are hetero atoms selected from amongst N, O, and S, and wherein said mono-cyclic or fused bi-cyclic moiety is optionally substituted with one or more substituents selected from the group consisting of halogen, cyano, C₁₋₆alkyl, hydroxyl, and C₁₋₆alkoxy, phenyl, pyridyl, and halogen substituted phenyl;
wherein one of R⁴-R⁸ represents halogen and the other represent hydrogen; wherein one of R¹³-R¹⁷ represents halogen and the other represent hydrogen;
wherein R² and R³ independently represent hydrogen, C₁₋₆alkyl, hydroxyC₁₋₆alkyl, haloC₁₋₆alkyl or phenyl;
and pharmaceutically acceptable salts thereof; or
- formula Ik 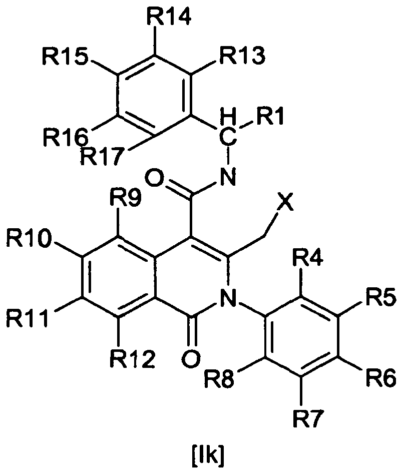 wherein R¹ represents hydrogen, C₁₋₆alkyl, C₂₋₆alkenyl or C₂₋₆alkynyl;
wherein X represents hydrogen, C₁₋₆alkyl, C₂₋₆alkenyl, C₂₋₆alkynyl or NR²R³, or **X** represents a mono-cyclic or bi-cyclic moiety having 5-9 ring atoms one of which is nitrogen, and wherein one, two or three additional ring atoms may be a hetero atom selected from N, O and S, and wherein said mono-cyclic or bi-cyclic moiety may optionally be substituted with one or more substituents W, wherein W is selected from hydrogen, halogen, hydroxy, (=O), C₁₋₆alkyl, C₂₋₆alkenyl, C₂₋₆alkynyl, C₁₋₆alkoxy, or wherein W represents a moiety of the formula -(CH₂)ₐ-Y-(CH₂)_{b}-Z;
wherein a and b independently represent an integer selected from 0, 1, 2 and 3; wherein Y represents a bond, and wherein Z represents a mono-cyclic moiety comprising 5 to 6 ring atoms of which optionally one, two or three are hetero atoms selected from amongst N, O, and S, and wherein said mono-cyclic moiety is optionally substituted with one or more substituents selected from the group consisting of halogen, C₁₋₆alkyl, hydroxyl, and C₁₋₆alkoxy;
wherein each of R⁴-R⁸ independently represents hydrogen or halogen;
wherein each of R⁹-R¹² independently represents hydrogen or halogen provided that at least one of R⁹-R¹² represents halogen;
wherein each of R¹³-R¹⁷ independently represent hydrogen or halogen;
wherein R² and R³ independently represent hydrogen, C₁₋₆alkyl, C₂₋₆alkenyl, C₂₋₆ alkynyl; and pharmaceutically acceptable salts thereof; or
- formula Ik' 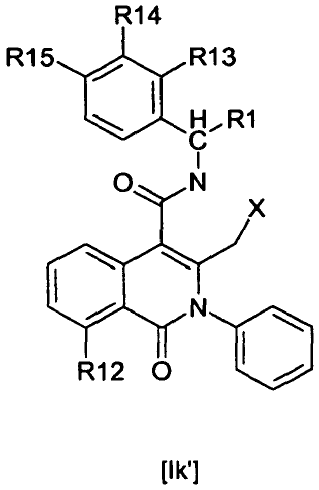 wherein R¹ is selected from C₁₋₆alkyl;
wherein X is selected from hydrogen, C₁₋₆alkyl or NR²R³, or X represents a mono-cyclic or bi-cyclic moiety having 5-9 ring atoms one of which is nitrogen, and wherein one additional ring atoms may be a hetero atom selected from N, and wherein said mono-cyclic or bi-cyclic moiety may optionally be substituted with one or more substituents W, wherein W is selected from hydrogen, hydroxy, (=O), C₁₋₆alkyl or wherein W represents a moiety of the formula -(CH₂)ₐ-Y-(CH₂)_{b}-Z;
wherein a and b independently represent an integer selected from 0, 1, 2 and 3; wherein Y represents a bond, and wherein Z represents a mono-cyclic moiety comprising 5 to 6 ring atoms of which optionally one is a hetero atom selected from amongst N, O, and S, and wherein said mono-cyclic moiety is optionally substituted with one or more substituents selected from the group consisting of halogen, C₁₋₆ alkyl, hydroxyl, and C₁₋₆alkoxy;
wherein R¹² represents halogen;
R¹³-R¹⁵ each independently represents hydrogen or halogen;
and pharmaceutically acceptable salts thereof; or
- formula Ik" 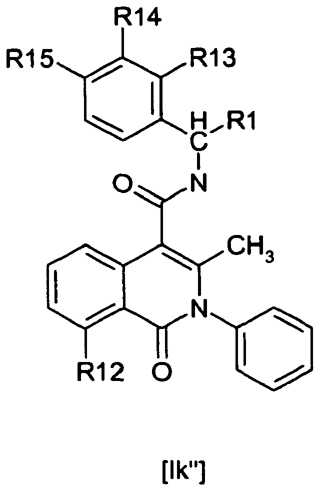 wherein R¹ represents ethyl, cylopropyl or cyclobutyl;
wherein R¹² represents chloro; and
R¹³, R¹⁴ and R¹⁵ each individually represent hydrogen, fluoro or chloro, wherein two of R¹³, R¹⁴ and R¹⁵ represent hydrogen; or
- essentially the S enantiomers as depicted in formula Ik"' 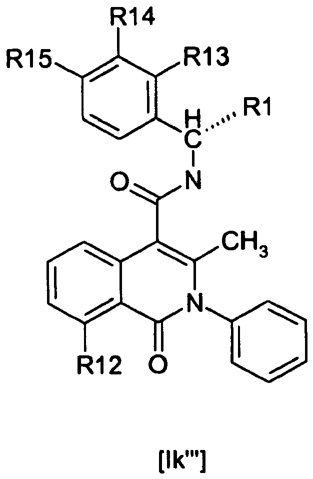 wherein R¹ represents ethyl or cylopropyl;
wherein R¹² represents chloro; and
R¹³, R¹⁴ and R¹⁵ each individually represent hydrogen, fluoro or chloro, wherein two of R¹³, R¹⁴ and R¹⁵ represent hydrogen; or
- formula Id' 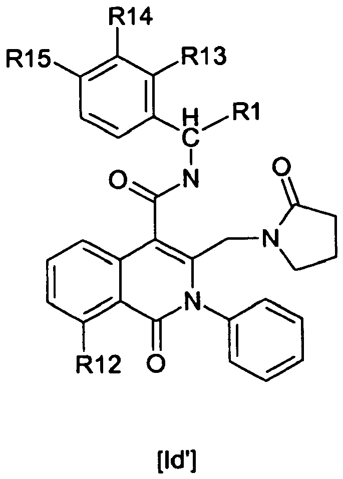 wherein R¹ represents ethyl or cyclopropyl;
R¹² represent halogen;
R¹³, R¹⁴ and R¹⁵ each independently represents hydrogen, or halogen; and pharmaceutically acceptable salts thereof; or
- formula Id" 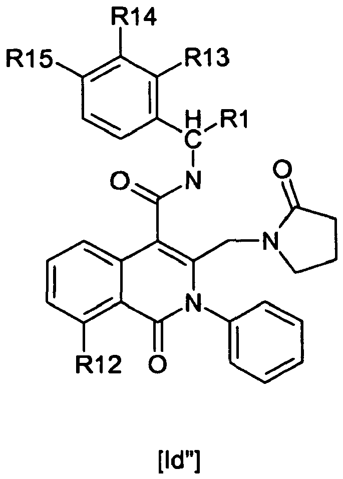 wherein R¹ represents ethyl or cyclopropyl;
R¹² represent chloro or fluoro;
R¹², R¹⁴ and R¹⁵ each independently represent hydrogen, chloro or fluoro, wherein two of R¹², R¹⁴ and R¹⁵ represent hydrogen;
and pharmaceutically acceptable salts thereof; or
- essentially the S enantiomer as depicted in formula Id"'
, wherein "essentially" defines an enantiomeric excess of at least 60%, 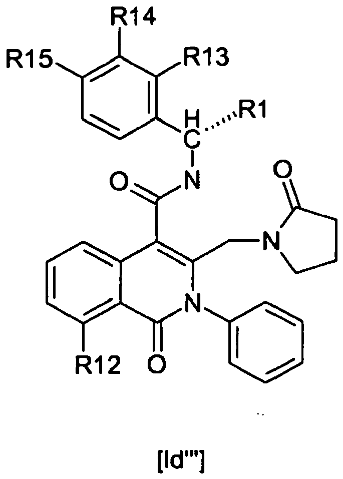 wherein R¹ represents ethyl or cyclopropyl;
R¹² represent chloro or fluoro;
R¹², R¹⁴ and R¹⁵ each independently represent hydrogen, chloro or fluoro, wherein two of R¹², R¹⁴ and R¹⁵ represent hydrogen;
and pharmaceutically acceptable salts thereof.

6. A compound according to claim 1 selected from
**1a** 3-Methyl-1-oxo-2-phenyl-1,2-dihydro-isoquinoline-4-carboxylic acid ((S)-1-phenyl-propyl)-amide
**1b** 3,6-Dimethyl-1-oxo-2-phenyl-1,2-dihydro-isoquinoline-4-carboxylic acid ((S)-1-phenyl-propyl)-amide
**1e** 7-Chloro-3-methyl-1-oxo-2-phenyl-1,2-dihydro-isoquinoline-4-carboxylic acid ((S)-1-phenyl-propyl)-amide
**1d** 7-Bromo-3-methyl-1-oxo-2-phenyl-1,2-dihydro-isoquinoline-4-carboxylic acid ((S)-1-phenyl-propyl)-amide
**1e** 6-Fluoro-3-methyl-1-oxo-2-phenyl-1,2-dihydro-isoquinoline-4-carboxylic acid ((S)-1-phenyl-propyl)-amide
**1f** 3,5-Dimethyl-1-oxo-2-phenyl-1,2-dihydro-isoquinoline-4-carboxylic acid ((S)-1-phenyl-propyl)-amide
**1g** 7-Fluoro-3-methyl-1-oxo-2-phenyl-1,2-dihydro-isoquinoline-4-carboxylic acid ((S)-1-phenyl-propyl)-amide
**1h** 3,7-Dimethyl-1-oxo-2-phenyl-1,2-dihydro-isoquinoline-4-carboxylic acid ((S)-1-phenyl-propyl)-amide
**1i** 8-Chloro-3-methyl-1-oxo-2-phenyl-1,2-dihydro-isoquinoline-4-carboxylic acid ((S)-1-phenyl-propyl)-amide
**1r** 3-Methyl-1-oxo-2-phenyl-1,2-dihydro-isoquinoline-4-carboxylic acid ((S)-cyclopropyl-phenyl-methyl)-amide
**1s** 3-Methyl-1-oxo-2-phenyl-1,2-dihydro-isoquinoline-4-carboxylic acid [(S)-cyclopropyl-(3-fluoro-phenyl)-methyl]-amide
**1j** 3,8-Dimethyl-1-oxo-2-phenyl-1,2-dihydro-isoquinoline-4-carboxylic acid ((S)-1-phenyl-propyl)-amide
**1k** 2-(2-Fluoro-phenyl)-3-methyl-1-oxo-1,2-dihydro-isoquinoline-4-carboxylic acid ((S)-1-phenyl-propyl)-amide **1l** 3-Methyl-1-oxo-2-o-tolyl-1,2-dihydro-isoquinoline-4-carboxylic acid ((S)-1-phenyl-propyl)-amide
**1m** 2-(2-Chloro-phenyl)-3-methyl-1-oxo-1,2-dihydro-isoquinoline-4-carboxylic acid ((S)-1-phenyl-propyl)-amide
**In** 2-(2,6-Difluoro-phenyl)-3-methyl-1-oxo-1,2-dihydro-isoquinoline-4-carboxylic acid ((S)-1-phenyl-propyl)-amide
**1o** 2-(3-Fluoro-phenyl)-3-methyl-1-oxo-1,2-dihydro-isoquinoline-4-carboxylic acid ((S)-1-phenyl-propyl)-amide
**1p** 3-Methyl-1-oxo-2-m-tolyl-1,2-dihydro-isoquinoline-4-carboxylic acid ((S)-1-phenyl-propyl)-amide
**1q** 2-(3-Chloro-phenyl)-3-methyl-1-oxo-1,2-dihydro-isoquinoline-4-carboxylic acid ((S)-1-phenyl-propyl)-amide
**1t** 8-Chloro-3-methyl-1-oxo-2-phenyl-1,2-dihydro-isoquinoline-4-carboxylic acid [(S)-cyclopropyl-(3-fluoro-phenyl)-methyl]-amide
**2a** 3-Dimethylaminomethyl-1-oxo-2-phenyl-1,2-dihydro-isoquinoline-4-carboxylic acid ((S)-1-phenyl-propyl)-amide
**2b** 3-Methylaminomethyl-1-oxo-2-phenyl-1,2-dihydro-isoquinoline-4-carboxylic acid ((S)-1-phenyl-propyl)-amide
**2c** 3-Ethylaminomethyl-1-oxo-2-phenyl-1,2-dihydro-isoquinoline-4-carboxylic acid ((S)-1-phenyl-propyl)-amide
**2d** 3-Cyclopropylaminomethyl-1-oxo-2-phenyl-1,2-dihydro-isoquinoline-4-carboxylic acid ((S)-1-phenyl-propyl)-amide
**2e** 3-[(Cyclopropylmethyl-amino)-methyl]-1-oxo-2-phenyl-1,2-dihydro-isoquinoline-4-carboxylic acid ((S)-1-phenyl-propyl)-amide
**2f** 3-(3,6-Dihydro-2H-pyridin-1-ylmethyl)-1-oxo-2-phenyl-1,2-dihydro-isoquino line-4-carboxylic acid ((S)-1-phenyl-propyl)-amide
**2g** 3-[4-(2-Methoxy-phenyl)-piperazin-1-ylmethyl]-1-oxo-2-phenyl-1,2-dihydro-isoquinoline-4-carboxylic acid ((S)-1-phenyl-propyl)-amide
**2h** 3-[4-(4-Fluoro-phenyl)-piperazin-1-ylmethyl]-1-oxo-2-phenyl-1,2-dihydro-isoquinoline-4-carboxylic acid ((S)-1-phenyl-propyl)-amide
**2i** 3-(4-Formyl-piperazin-1-ylmethyl)-1-oxo-2-phenyl-1,2-dihydro-isoquinoline-4-carboxylic acid ((S)-1-phenyl-propyl)-amide
**2j** 4-[1-Oxo-2-phenyl-4-(1-phenyl-propylcarbamoyl)-1,2-dihydro-isoquinolin-3-ylmethyl]-piperazine-1-carboxylic acid ethyl ester
**2k** 3-(4-Methyl-piperazin-1-ylmethyl)-1-oxo-2-phenyl-1,2-dihydro-isoquinoline-4-carboxylic acid ((S)-1-phenyl-propyl)-amide
**2l** 1-Oxo-2-phenyl-3-(1,3,4,9-tetrahydro-beta-carbolin-2-ylmethyl)-1,2-dihydro-isoquinoline-4-carboxylic acid ((S)-1-phenyl-propyl)-amide
**2m** 1-Oxo-2-phenyl-3-piperazin-1-ylmethyl-1,2-dihydro-isoquinoline-4-carboxylic acid ((S)-1-phenyl-propyl)-amide
**2n** 3-(3-Methyl-piperazin-1-ylmethyl)-1-oxo-2-phenyl-1,2-dihydro-isoquinoline-4-carboxylic acid ((S)-1-phenyl-propyl)-amide
**2o** 3-(3,5-Dimethyl-piperazin-1-ylmethyl)-1-oxo-2-phenyl-1,2-dihydro-isoquinoline-4-carboxylic acid ((S)-1-phenyl-propyl)-amide
**2p** 3-(4-Benzyl-piperazin-1-ylmethyl)-1-oxo-2-phenyl-1,2-dihydro-isoquinoline-4-carboxylic acid ((S)-1-phenyl-propyl)-amide
**2q** 1-Oxo-3-[4-(2-oxo-2-pyrrolidin-1-yl-ethyl)-piperazin-1-ylmethyl]-2-phenyl-1,2-dihydro-isoquinoline-4-carboxylic acid ((S)-1-phenyl-propyl)-amide **2r** 3-Morpholin-4-ylmethyl-1-oxo-2-phenyl-1,2-dihydro-isoquinoline-4-carboxylic acid ((S)-1-phenyl-propyl)-amide
**2s** 3-(2,6-Dimethyl-morpholin-4-ylmethyl)-1-oxo-2-phenyl-1,2-dihydro-isoquinoline-4-carboxylic acid ((S)-1-phenyl-propyl)-amide
**2t** 1-Oxo-2-phenyl-3-thiomorpholin-4-ylmethyl-1,2-dihydro-isoquinoline-4-carboxylic acid ((S)-1-phenyl-propyl)-amide
**2u** 3-(1,4-Dioxa-8-aza-spiro[4.5]dec-8-ylmethyl)-1-oxo-2-phenyl-1,2-dihydro-isoquinoline-4-carboxylic acid ((S)-1-phenyl-propyl)-amide
**2v** 1-Oxo-2-phenyl-3-piperidin-1-ylmethyl-1,2-dihydro-isoquinoline-4-carboxylic acid ((S)-1-phenyl-propyl)-amide
**2w** 3-(2-Methyl-piperidin-1-ylmethyl)-1-oxo-2-phenyl-1,2-dihydro-isoquinoline-4-carboxylic acid ((S)-1-phenyl-propyl)-amide
**2x** 3-(2,6-Dimethyl-piperidin-1-ylmethyl)-1-oxo-2-phenyl-1,2-dihydro-isoquinoline-4-carboxylic acid ((S)-1-phenyl-propyl)-amide
**2y** 3-(2-Hydroxymethyl-piperidin-1-ylmethyl)-1-oxo-2-phenyl-1,2-dihydro-isoquinoline-4-carboxylic acid ((S)-1-phenyl-propyl)-amide
**2z** 1-[1-Oxo-2-phenyl-4-(1-phenyl-propylcarbamoyl)-1,2-dihydro-isoquinolin-3-ylmethyl]-piperidine-3-carboxylic acid ethyl ester
**2aa** 3-(3-Methyl-piperidin-1-ylmethyl)-1-oxo-2-phenyl-1,2-dihydro-isoquinoline-4-carboxylic acid ((S)-1-phenyl-propyl)-amide
**2ab** 3-(4-Hydroxy-piperidin-1-ylmethyl)-1-oxo-2-phenyl-1,2-dihydro-isoquinoline-4-carboxylic acid ((S)-1-phenyl-propyl)-amide
**2ac** 1-Oxo-2-phenyl-3-(4-phenyl-piperidin-1-ylmethyl)-1,2-dihydro-isoquinoline-4-carboxylic acid ((S)-1-phenyl-propyl)-amide
**2ad** 1-[1-Oxo-2-phenyl-4-(1-phenyl-propylcarbamoyl)-1,2-dihydro-isoquinolin-3-ylmethyl]-piperidine-4-carboxylic acid ethyl ester
**2ae** 3-(4-Methyl-piperidin-1-ylmethyl)-1-oxo-2-phenyl-1,2-dihydro-isoquinoline-4-carboxylic acid ((S)-1-phenyl-propyl)-amide
**2af** 1-Oxo-2-phenyl-3-(4-pyridin-2-yl-piperazin-1-ylmethyl)-1,2-dihydro-isoquinoline-4-carboxylic acid ((S)-1-phenyl-propyl)-amide **2ag** 3-(Octahydro-quinolin-1-ylmethyl)-1-oxo-2-phenyl-1,2-dihydro-isoquinoline-4-carboxylic acid ((S)-1-phenyl-propyl)-amide
**2ah** 3-Azepan-1-ylmethyl-1-oxo-2-phenyl-1,2-dihydro-isoquinoline-4-carboxylic acid ((S)-1-phenyl-propyl)-amide
**2ai** 3-(3-Hydroxy-piperidin-1-ylmethyl)-1-oxo-2-phenyl-1,2-dihydro-isoquinoline-4-carboxylic acid ((S)-1-phenyl-propyl)-amide
**2aj** 3-[4-(2,4-Dimethyl-phenyl)-piperazin-1-ylmethyl]-1-oxo-2-phenyl-1,2-dihydro-isoquinoline-4-carboxylic acid ((S)-1-phenyl-propyl)-amide
**2ak** 3-[4-(3,4-Dimethyl-phenyl)-piperazin-1-ylmethyl]-1-oxo-2-phenyl-1,2-dihydro-isoquinoline-4-carboxylic acid ((S)-1-phenyl-propyl)-amide
**2al** 3-(4-Dimethylamino-piperidin-1-ylmethyl)-1-oxo-2-phenyl-1,2-dihydro-isoquinoline-4-carboxylic acid ((S)-1-phenyl-propyl)-amide
**2am** 3-[4-(2,5-Dimethyl-phenyl)-piperazin-1-ylmethyl]-1-oxo-2-phenyl-1,2-dihydro-isoquinoline-4-carboxylic acid ((S)-1-phenyl-propyl)-amide
**2an** 3-[4-(2-Fluoro-phenyl)-piperazin-1-ylmethyl]-1-oxo-2-phenyl-1,2-dihydro-isoquinoline-4-carboxylic acid ((S)-1-phenyl-propyl)-amide
**2ao** 3-[4-(3-Methoxy-phenyl)-piperazin-1-ylmethyl]-1-oxo-2-phenyl-1,2-dihydro-isoquinoline-4-carboxylic acid ((S)-1-phenyl-propyl)-amide
**2ap** 1-Oxo-2-phenyl-3-(4-m-tolyl-piperazin-1-ylmethyl)-1,2-dihydro-isoquinoline-4-carboxylic acid ((S)-1-phenyl-propyl)-amide
**2aq** 3-[4-(4-Methoxy-phenyl)-piperazin-1-ylmethyl]-1-oxo-2-phenyl-1,2-dihydro-isoquinoline-4-carboxylic acid ((S)-1-phenyl-propyl)-amide
**2ar** 1-Oxo-3-(4-phenethyl-piperazin-1-ylmethyl)-2-phenyl-1,2-dihydro-isoquinoline-4-carboxylic acid ((S)-1-phenyl-propyl)-amide
**2as** 1-Oxo-2-phenyl-3-(4-pyrimidin-2-yl-piperazin-1-ylmethyl)-1,2-dihydro-isoquinoline-4-carboxylic acid ((S)-1-phenyl-propyl)-amide
**2at** 3-[4-(2-Cyano-phenyl)-piperazin-1-ylmethyl]-1-oxo-2-phenyl-1,2-dihydro-isoquinoline-4-carboxylic acid ((S)-1-phenyl-propyl)-amide
**2au** 3-[4-(4-Chloro-phenyl)-piperazin-1-ylmethyl]-1-oxo-2-phenyl-1,2-dihydro-isoquinoline-4-carboxylic acid ((S)-1-phenyl-propyl)-amide
**2av** 3-((1S,3R,SR)-3-Hydroxy-8-aza-bicyclo[3.2.1]oct-8-ylmethyl)-1-oxo-2-phenyl-1,2-dihydro-isoquinoline-4-carboxylic acid ((S)-1-phenyl-propyl)-amide **2aw** 3-(4-Acetyl-piperazin-1-ylmethyl)-1-oxo-2-phenyl-1,2-dihydro-isoquinoline-4-carboxylic acid ((S)-1-phenyl-propyl)-amide
**2ax** 3-(4-ethyl-[1,4]diazepan-1-ylmethyl)-1-oxo-2-phenyl-1,2-dihydro-isoquinoline-4-carboxylic acid ((S)-1-phenyl-propyl)-amide
**2ay** 3-(4-Ethyl-piperazin-1-ylmethyl)-1-oxo-2-phenyl-1,2-dihydro-isoquinoline-4-carboxylic acid ((S)-1-phenyl-propyl)-amide
**2az** 3-((2S,6R)-2,6-Dimethyl-morpholin-4-ylmethyl)-1-oxo-2-phenyl-1,2-dihydro-isoquinoline-4-carboxylic acid ((S)-1-phenyl-propyl)-amide
**2ba** 3-[4-(2,4-Difluoro-phenyl)-piperazin-1-ylmethyl]-1-oxo-2-phenyl-1,2-dihydro-isoquino line-4-carboxylic acid ((S)-1-phenyl-propyl)-amide
**2bb** 3-[4-(3-Dimethylamino-propyl)-piperazin-1-ylmethyl]-1-oxo-2-phenyl-1,2-dihydro-isoquinoline-4-carboxylic acid ((S)-1-phenyl-propyl)-amide
**2bc** 3-(4-Isopropyl-piperazin-1-ylmethyl)-1-oxo-2-phenyl-1,2-dihydro-isoquino line-4-carboxylic acid ((S)-1-phenyl-propyl)-amide
**2bd** 3-(3-Aza-bicyclo[3.2.2]non-3-ylmethyl)-1-oxo-2-phenyl-1,2-dihydro-isoquinoline-4-carboxylic acid ((S)-1-phenyl-propyl)-amide
**2be** 3-(4-Cyclopentyl-piperazin-1-ylmethyl)-1-oxo-2-phenyl-1,2-dihydro-isoquinoline-4-carboxylic acid ((S)-1-phenyl-propyl)-amide
**2bf** 3-[1,4']Bipiperidinyl-1'-ylmethyl-1-oxo-2-phenyl-1,2-dihydro-isoquinoline-4-carboxylic acid ((S)-1-phenyl-propyl)-amide
**2bg** 3-(3,4-Dihydro-1H-isoquinolin-2-ylmethyl)-1-oxo-2-phenyl-1,2-dihydro-isoquinoline-4-carboxylic acid ((S)-1-phenyl-propyl)-amide
**2bh** 3-[4-(2-Dimethylamino-ethyl)-piperazin-1-ylmethyl]-1-oxo-2-phenyl-1,2-dihydro-isoquinoline-4-carboxylic acid ((S)-1-phenyl-propyl)-amide
**2bi** 3-(4-Hydroxymethyl-piperidin-1-ylmethyl)-1-oxo-2-phenyl-1,2-dihydro-isoquinoline-4-carboxylic acid ((S)-1-phenyl-propyl)-amide
**2bj** 1-Oxo-2-phenyl-3-[4-(tetrahydro-furan-2-carbonyl)-piperazin-1-ylmethyl]-1,2-dihydro-isoquino line-4-carboxylic acid ((S)-1-phenyl-propyl)-amide
**2bk** 3-(4-Isobutyl-piperazin-1-ylmethyl)-1-oxo-2-phenyl-1,2-dihydro-isoquinoline-4-carboxylic acid ((S)-1-phenyl-propyl)-amide
**2bl** 3-[4-(2-Methoxy-ethyl)-piperazin-1-ylmethyl]-1-oxo-2-phenyl-1,2-dihydro-isoquinoline-4-carboxylic acid ((S)-1-phenyl-propyl)-amide
**2bm** 3-[4-(2-Morpholin-4-yl-ethyl)-piperazin-1-ylmethyl]-1-oxo-2-phenyl-1,2-dihydro-isoquinoline-4-carboxylic acid ((S)-1-phenyl-propyl)-amide
**2bn** 3-(1,3-Dihydro-isoindol-2-ylmethyl)-1-oxo-2-phenyl-1,2-dihydro-isoquinoline-4-carboxylic acid ((S)-1-phenyl-propyl)-amide
**2bo** 3-[4-(1-Methyl-piperidin-4-yl)-piperazin-1-ylmethyl]-1-oxo-2-phenyl-1,2-dihydro-isoquinoline-4-carboxylic acid ((S)-1-phenyl-propyl)-amide
**2bp** 1-Oxo-2-phenyl-3-[4-(2-piperidin-1-yl-ethyl)-piperazin-1-ylmethyl]-1,2-dihydro-isoquinoline-4-carboxylic acid ((S)-1-phenyl-propyl)-amide
**2bq** 1-Oxo-2-phenyl-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2br** 3-(4-Dimethylcarbamoylmethyl-piperazin-1-ylmethyl)-1-oxo-2-phenyl-1,2-dihydro-isoquinoline-4-carboxylic acid ((S)-1-phenyl-propyl)-amide
**2bs** 3-(Octahydro-pyrido[1,2-a]pyrazin-2-ylmethyl)-1-oxo-2-phenyl-1,2-dihydro-isoquino line-4-carboxylic acid ((S)-1-phenyl-propyl)-amide
**2bt** 3-(4-Formyl-[1,4]diazepan-1-ylmethyl)-1-oxo-2-phenyl-1,2-dihydro-isoquinoline-4-carboxylic acid ((S)-1-phenyl-propyl)-amide
**2bu** 3-[4-(4-Cyano-phenyl)-piperazin-1-ylmethyl]-1-oxo-2-phenyl-1,2-dihydro-isoquinoline-4-carboxylic acid ((S)-1-phenyl-propyl)-amide
**2bv** 1-Oxo-2-phenyl-3-(4-pyridin-4-ylmethyl-piperazin-1-ylmethyl)-1,2-dihydro-isoquinoline-4-carboxylic acid ((S)-1-phenyl-propyl)-amide
**2bw** 1-Oxo-2-phenyl-3-[4-(3-pyrrolidin-1-yl-propyl)-piperazin-1-ylmethyl]-1,2-dihydro-isoquino line-4-carboxylic acid ((S)-1-phenyl-propyl)-amide
**2bx** 1-Oxo-2-phenyl-3-(4-pyridin-2-ylmethyl-piperazin-1-ylmethyl)-1,2-dihydro-isoquinoline-4-carboxylic acid ((S)-1-phenyl-propyl)-amide
**2by** 3-(4-Ethanesulfonyl-piperazin-1-ylmethyl)-1-oxo-2-phenyl-1,2-dihydro-isoquino line-4-carboxylic acid ((S)-1-phenyl-propyl)-amide
**2bz** 3-(4-sec-Butyl-piperazin-1-ylmethyl)-1-oxo-2-phenyl-1,2-dihydro-isoquinoline-4-carboxylic acid ((S)-1-phenyl-propyl)-amide
**2ca** 3-[4-(1-Ethyl-propyl)-piperazin-1-ylmethyl]-1-oxo-2-phenyl-1,2-dihydro-isoquinoline-4-carboxylic acid ((S)-1-phenyl-propyl)-amide
**2cb** 3-[4-(2-Cyano-ethyl)-piperazin-1-ylmethyl]-1-oxo-2-phenyl-1,2-dihydro-isoquinoline-4-carboxylic acid ((S)-1-phenyl-propyl)-amide
**2cc** 3-(4-Methanesulfonyl-piperazin-1-ylmethyl)-1-oxo-2-phenyl-1,2-dihydro-isoquinoline-4-carboxylic acid ((S)-1-phenyl-propyl)-amide
**2cd** {1-[1-Oxo-2-phenyl-4-(1-phenyl-propylcarbamoyl)-1,2-dihydro-isoquinolin-3-ylmethyl]-piperidin-4-yl}-acetic acid ethyl ester
**2ce** 3-[4-(3-Fluoro-phenyl)-piperazin-1-ylmethyl]-1-oxo-2-phenyl-1,2-dihydro-isoquinoline-4-carboxylic acid ((S)-1-phenyl-propyl)-amide
**2cf** 1-Oxo-2-phenyl-3-((S)-3-phenyl-piperidin-1-ylmethyl)-1,2-dihydro-isoquinoline-4-carboxylic acid ((S)-1-phenyl-propyl)-amide
**2cg** 1-Oxo-2-phenyl-3-[4-(pyrrolidine-1-carbonyl)-piperazin-1-ylmethyl]-1,2-dihydro-isoquinoline-4-carboxylic acid ((S)-1-phenyl-propyl)-amide
**2ch** 3-[4-(Morpholine-4-carbonyl)-piperazin-1-ylmethyl]-1-oxo-2-phenyl-1,2-dihydro-isoquinoline-4-carboxylic acid ((S)-1-phenyl-propyl)-amide
**2ci** 3-(4-Methoxy-piperidin-1-ylmethyl)-1-oxo-2-phenyl-1,2-dihydro-isoquinoline-4-carboxylic acid ((S)-1-phenyl-propyl)-amide
**2cj** 3-(4'-Hydroxy-3',4',5',6'-tetrahydro-2'H-[3,4']bipyridinyl-1'-ylmethyl)-1-oxo-2-phenyl-1,2-dihydro-isoquinoline-4-carboxylic acid ((S)-1-phenyl-propyl)-amide
**2ck** 3-(4-Hydroxy-3,4,5,6-tetrahydro-2H-[4,4']bipyridinyl-1-ylmethyl)-1-oxo-2-phenyl-1,2-dihydro-isoquinoline-4-carboxylic acid ((S)-1-phenyl-propyl)-amide
**2cl** 3-(3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide
**2cm** 3-(4-Hydroxy-4-methyl-piperidin-1-ylmethyl)-1-oxo-2-phenyl-1,2-dihydro-isoquinoline-4-carboxylic acid ((S)-1-phenyl-propyl)-amide
**2cn** 3-(hexahydro-spiro[benzo[1,3]dioxole-2,4'-piperidin]-1'-ylmethyl)-1-oxo-2-phenyl-1,2-dihydro-isoquinoline-4-carboxylic acid ((S)-1-phenyl-propyl)-amide
**2co** 1-Oxo-2-phenyl-3-(3,4,5,6-tetrahydro-2H-[4,4']bipyridinyl-1-ylmethyl)-1,2-dihydro-isoquinoline-4-carboxylic acid ((S)-1-phenyl-propyl)-amide
**2cp** 3-[4-(2-Dimethylamino-ethyl)-piperidin-1-ylmethyl]-1-oxo-2-phenyl-1,2-dihydro-isoquinoline-4-carboxylic acid ((S-1-phenyl-propyl)-amide
**2cq** 3-(4-Dimethylsulfamoyl-piperazin-1-ylmethyl)-1-oxo-2-phenyl-1,2-dihydro-isoquinoline-4-carboxylic acid ((S)-1-phenyl-propyl)-amide
**2cr** 3-(1,1-Dioxo-thiomorpholin-4-ylmethyl)-1-oxo-2-phenyl-1,2-dihydro-isoquinoline-4-carboxylic acid ((S)-1-phenyl-propyl)-amide
**2cs** 1-Oxo-2-phenyl-3-(2-pyridin-2-ylmethyl-piperidin-1-ylmethyl)-1,2-dihydro-isoquinoline-4-carboxylic acid ((S)-1-phenyl-propyl)-amide
**2ct** 3-(2-Morpholin-4-ylmethyl-piperidin-1-ylmethyl)-1-oxo-2-phenyl-1,2-dihydro-isoquinoline-4-carboxylic acid ((S)-1-phenyl-propyl)-amide
**2cu** 3-(4-Furo[3,2-c]pyridin-4-yl-piperazin-1-ylmethyl)-1-oxo-2-phenyl-1,2-dihydro-isoquino line-4-carboxylic acid ((S)-1-phenyl-propyl)-amide
**2cv** 3-(4-Cyclopropylmethyl-piperazin-1-ylmethyl)-1-oxo-2-phenyl-1,2-dihydro-isoquinoline-4-carboxylic acid ((S)-1-phenyl-propyl)-amide
**2cw** 3-[4-(2-Morpholin-4-yl-ethyl)-piperidin-1-ylmethyl]-1-oxo-2-phenyl-1,2-dihydro-isoquinoline-4-carboxylic acid ((S)-1-phenyl-propyl)-amide
**2cx** 1-Oxo-2-phenyl-3-(4-pyrimidin-2-yl-[1,4]diazepan-1-ylmethyl)-1,2-dihydro-isoquinoline-4-carboxylic acid ((S)-1-phenyl-propyl)-amide
**2cy** 3-(4-Methyl-6,7-dihydro-4H-thieno[3,2-c]pyridin-5-ylmethyl)-1-oxo-2-phenyl-1,2-dihydro-isoquinoline-4-carboxylic acid ((S)-1-phenyl-propyl)-amide
**2cz** 3-[1,4]Diazepan-1-ylmethyl-1-oxo-2-phenyl-1,2-dihydro-isoquinoline-4-carboxylic acid ((S)-1-phenyl-propyl)-amide
**2da** 3-((2S,5R)-2,5-Dimethyl-piperazin-1-ylmethyl)-1-oxo-2-phenyl-1,2-dihydro-isoquinoline-4-carboxylic acid ((S)-1-phenyl-propyl)-amide
**2db** 3-((S)-3-Methyl-piperazin-1-ylmethyl)-1-oxo-2-phenyl-1,2-dihydro-isoquinoline-4-carboxylic acid ((S)-1-phenyl-propyl)-amide
**2dc** 3-((R)-3-Methyl-piperazin-1-ylmethyl)-1-oxo-2-phenyl-1,2-dihydro-isoquinoline-4-carboxylic acid ((S)-1-phenyl-propyl)-amide
**2dd** 3-[3-(3-Chloro-phenyl)-piperazin-1-ylmethyl]-1-oxo-2-phenyl-1,2-dihydro-isoquinoline-4-carboxylic acid ((S)-1-phenyl-propyl)-amide
**2de** 3-[4-(1H-Indol-4-yl)-piperazin-1-ylmethyl]-1-oxo-2-phenyl-1,2-dihydro-isoquinoline-4-carboxylic acid ((S)-1-phenyl-propyl)-amide
**2df** 1-Oxo-3-(3-oxo-piperazin-1-ylmethyl)-2-phenyl-1,2-dihydro-isoquinoline-4-carboxylic acid ((S)-1-phenyl-propyl)-amide
**2dg** 3-[4-(1H-Indol-5-yl)-piperazin-1-ylmethyl]-1-oxo-2-phenyl-1,2-dihydro-isoquinoline-4-carboxylic acid ((S)-1-phenyl-propyl)-amide
**2dh** 3-(6,9-Diaza-spiro[4.5]dec-9-ylmethyl)-1-oxo-2-phenyl-1,2-dihydro-isoquinoline-4-carboxylic acid ((S)-1-phenyl-propyl)-amide
**2di** 3-(1,4-Diaza-spiro[5.5]undec-4-ylmethyl)-1-oxo-2-phenyl-1,2-dihydro-isoquino line-4-carboxylic acid ((S)-1-phenyl-propyl)-amide
**2dj** 3-(3-Isopropyl-piperazin-1-ylmethyl)-1-oxo-2-phenyl-1,2-dihydro-isoquinoline-4-carboxylic acid ((S)-1-phenyl-propyl)-amide
**2dk** 3-(3,3-Dimethyl-piperazin-1-ylmethyl)-1-oxo-2-phenyl-1,2-dihydro-isoquinoline-4-carboxylic acid ((S)-1-phenyl-propyl)-amide
**2dl** 3-[3-(4-Fluoro-phenyl)-piperazin-1-ylmethyl]-1-oxo-2-phenyl-1,2-dihydro-isoquinoline-4-carboxylic acid ((S)-1-phenyl-propyl)-amide
**2dm** 1-Oxo-2-phenyl-3-(3-p-tolyl-piperazin-1-ylmethyl)-1,2-dihydro-isoquinoline-4-carboxylic acid ((S)-1-phenyl-propyl)-amide
**2dn** 4-[1-Oxo-2-phenyl-4-(1-phenyl-propylcarbamoyl)-1,2-dihydro-isoquinolin-3-ylmethyl]-piperazine-1-carboxylic acid tert-butyl ester
**2do** 3-(4-Methylcarbamoylmethyl-piperazin-1-ylmethyl)-1-oxo-2-phenyl-1,2-dihydro-isoquinoline-4-carboxylic acid ((S)-1-phenyl-propyl)-amide
**2dp** 8-Chloro-3-dimethylaminomethyl-1oxo-2-phenyl-1,2-dihydro-isoquinoline-4-carboxylic acid ((S)-1-phenyl-propyl)-amide
**2dr** 3-Cyclopentylaminomethyl-1-oxo-2-phenyl-1,2-dihydro-isoquinoline-4-carboxylic acid ((S)-1-phenyl-propyl)-amide
**2ds** 3-Cyclohexylaminomethyl-1-oxo-2-phenyl-1,2-dihydro-isoquinoline-4-carboxylic acid ((S)-1-phenyl-propyl)-amide
**2dt** 3-{[(2-Hydroxy-ethyl)-methyl-amino]-methyl}-1-oxo-2-phenyl-1,2-dihydro-isoquino line-4-carboxylic acid ((S)-1-phenyl-propyl)-amide
**2du** 3-Imidazol-1-ylmethyl-1-oxo-2-phenyl-1,2-dihydro-isoquinoline-4-carboxylic acid ((S)-1-phenyl-propyl)-amide
**2dv** 3-(2-Methyl-imidazol-1-ylmethyl)-1-oxo-2-phenyl-1,2-dihydro-isoquinoline-4-carboxylic acid ((S)-1-phenyl-propyl)-amide
**2dw** 3-(4-Methyl-imidazol-1-ylmethyl)-1-oxo-2-phenyl-1,2-dihydro-isoquinoline-4-carboxylic acid ((S)-1-phenyl-propyl)-amide
**2dx** 3-(2,5-Dihydro-pyrrol-1-ylmethyl)-1-oxo-2-phenyl-1,2-dihydro-isoquinoline-4-carboxylic acid ((S)-1-phenyl-propyl)-amide
**2dy** 3-(2,5-Dimethyl-2,5-dihydro-pyrrol-1-ylmethyl)-1-oxo-2-phenyl-1,2-dihydro-isoquinoline-4-carboxylic acid ((S)-1-phenyl-propyl)-amide
**2dz** 1-Oxo-2-phenyl-3-pyrrolidin-1-ylmethyl-1,2-dihydro-isoquinoline-4-carboxylic acid ((S)-1-phenyl-propyl)-amide
**2ea** 1-Oxo-2-phenyl-3-(thiazol-2-ylaminomethyl)-1,2-dihydro-isoquinoline-4-carboxylic acid ((S)-1-phenyl-propyl)-amide
**2eb** 1-Oxo-2-phenyl-3-(pyrimidin-4-ylaminomethyl)-1,2-dihydro-isoquinoline-4-carboxylic acid ((S)-1-phenyl-propyl)-amide
**2ec** 3-(tert-Butylamino-methyl)-1-oxo-2-phenyl-1,2-dihydro-isoquinoline-4-carboxylic acid ((S)-1-phenyl-propyl)-amide
**2ed** 3-[(2-Hydroxy-1,1-dimethyl-ethylamino)-methyl]-1-oxo-2-phenyl-1,2-dihydro-isoquinoline-4-carboxylic acid ((S)-1-phenyl-propyl)-amide
**2ee** 3-(Isopropylamino-methyl)-1-oxo-2-phenyl-1,2-dihydro-isoquinoline-4-carboxylic acid ((S)-1-phenyl-propyl)-amide
**2ef** 3-[(2-Hydroxy-1-methyl-ethylamino)-methyl]-1-oxo-2-phenyl-1,2-dihydro-isoquinoline-4-carboxylic acid ((S)-1-phenyl-propyl)-amide
**2eg** 3-[(1-Hydroxymethyl-propylamino)-methyl]-1-oxo-2-phenyl-1,2-dihydro-isoquinoline-4-carboxylic acid ((S)-1-phenyl-propyl)-amide
**2eh** 3-[(2,2-Dimethyl-propylamino)-methyl]-1-oxo-2-phenyl-1,2-dihydro-isoquinoline-4-carboxylic acid ((S)-1-phenyl-propyl)-amide
**2ei** 1-Oxo-2-phenyl-3-prop-2-ynylaminomethyl-1,2-dihydro-isoquinoline-4-carboxylic acid ((S)-1-phenyl-propyl)-amide
**2ej** 3-Allylaminomethyl-1-oxo-2-phenyl-1,2-dihydro-isoquinoline-4-carboxylic acid ((S)-1-phenyl-propyl)-amide
**2ek** 3-[(Methyl-prop-2-ynyl-amino)-methyl]-1-oxo-2-phenyl-1,2-dihydro-isoquino line-4-carboxylic acid ((S)-1-phenyl-propyl)-amide
**2el** 3-Diallylaminomethyl-1-oxo-2-phenyl-1,2-dihydro-isoquinoline-4-carboxylic acid ((S)-1-phenyl-propyl)-amide
**2em** 3-Diethylaminomethyl-1-oxo-2-phenyl-1,2-dihydro-isoquinoline-4-carboxylic acid ((S)-1-phenyl-propyl)-amide
**2en** 3-[(Isopropyl-methyl-amino)-methyl]-1-oxo-2-phenyl-1,2-dihydro-isoquinoline-4-carboxylic acid ((S)-1-phenyl-propyl)-amide
**2eo** 3-[((S)-2-Hydroxy-1-methyl-ethylamino)-methyl]-1-oxo-2-phenyl-1,2-dihydro-isoquinoline-4-carboxylic acid ((S)-1-phenyl-propyl)-amide
**2ep** 3-[((R)-2-Hydroxy-1-methyl-ethylamino)-methyl]-1-oxo-2-phenyl-1,2-dihydro-isoquinoline-4-carboxylic acid ((S)-1-phenyl-propyl)-amide
**2eq** 3-{[(2-Methoxy-ethyl)-methyl-amino]-methyl}-1-oxo-2-phenyl-1,2-dihydro-isoquinoline-4-carboxylic acid ((S)-1-phenyl-propyl)-amide
**2er** 3-((R)-3-Hydroxy-pyrrolidin-1-ylmethyl)-1-oxo-2-phenyl-1,2-dihydro-isoquinoline-4-carboxylic acid ((S)-1-phenyl-propyl)-amide
**2es** 3-((S)-3-Hydroxy-pyrrolidin-1-ylmethyl)-1-oxo-2-phenyl-1,2-dihydro-isoquinoline-4-carboxylic acid ((S)-1-phenyl-propyl)-amide
**2et** 3-[(Cyclopentyl-methyl-amino)-methyl]-1-oxo-2-phenyl-1,2-dihydro-isoquino line-4-carboxylic acid ((S)-1-phenyl-propyl)-amide
**2eu** 3-{[(2-Hydroxy-1-methyl-ethyl)-methyl-amino]-methyl}-1-oxo-2-phenyl-1,2-dihydro-isoquinoline-4-carboxylic acid ((S)-1-phenyl-propyl)-amide
**2ev** 3- {[Ethyl-(2-hydroxy-ethyl)-amino]-methyl}-1-oxo-2-phenyl-1,2-dihydro-isoquinoline-4-carboxylic acid ((S)-1-phenyl-propyl)-amide
**2ew** 3-[(Ethyl-methyl-amino)-methyl]-1-oxo-2-phenyl-1,2-dihydro-isoquinoline-4-carboxylic acid ((S)-1-phenyl-propyl)-amide
**2ex** 3-Cyclobutylaminomethyl-1-oxo-2-phenyl-1,2-dihydro-isoquinoline-4-carboxylic acid ((S)-1-phenyl-propyl)-amide
**2ey** 3-Azetidin-1-ylmethyl-1-oxo-2-phenyl-1,2-dihydro-isoquinoline-4-carboxylic acid ((S)-1-phenyl-propyl)-amide
**2ez** 3-(4-tert-Butyl-piperazin-1-ylmethyl)-1-oxo-2-phenyl-1,2-dihydro-isoquinoline-4-carboxylic acid ((S)-1-phenyl-propyl)-amide
**2fa** 3-[4-(2-Hydroxy-ethyl)-piperazin-1-ylmethyl]-1-oxo-2-phenyl-1,2-dihydro-isoquino line-4-carboxylic acid ((S)-1-phenyl-propyl)-amide
**2fb** 3-{4-[2-(2-Hydroxy-ethoxy)-ethyl]-piperazin-1-ylmethyl}-1-oxo-2-phenyl-1,2-dihydro-isoquinoline-4-carboxylic acid ((S)-1-phenyl-propyl)-amide
**2fc** 3-[4-(3-Chloro-5-trifluoromethyl-pyridin-2-yl)-piperazin-1-ylmethyl]-1-oxo-2-phenyl-1,2-dihydro-isoquinoline-4-carboxylic acid ((S)-1-phenyl-propyl)-amide
**2fd** 3-[4-(3,5-Dichloro-pyridin-4-yl)-piperazin-1-ylmethyl]-1-oxo-2-phenyl-1,2-dihydro-isoquinoline-4-carboxylic acid ((S)-1-phenyl-propyl)-amide
**2fe** 4-[1-Oxo-2-phenyl-4-((S)-1-phenyl-propylcarbamoyl)-1,2-dihydro-isoquinolin-3-ylmethyl]-piperazine-1-carboxylic acid benzyl ester
**2ff** 3-[4-(3-Morpholin-4-yl-propyl)-piperazin-1-ylmethyl]-1-oxo-2-phenyl-1,2-dihydro-isoquinoline-4-carboxylic acid ((S)-1-phenyl-propyl)-amide **2fg** 1-Oxo-2-phenyl-3-[4-(3-piperidin-1-yl-propyl)-piperazin-1-ylmethyl]-1,2-dihydro-isoquinoline-4-carboxylic acid ((S)-1-phenyl-propyl)-amide
**2fh** 3-[4-(4,6-Dimethoxy-pyrimidin-2-ylmethyl)-piperazin-1-ylmethyl]-1-oxo-2-phenyl-1,2-dihydro-isoquinoline-4-carboxylic acid ((S)-1-phenyl-propyl)-amide
**2fi** 3-[4-(3-Hydroxy-propyl)-piperazin-1-ylmethyl]-1-oxo-2-phenyl-1,2-dihydro-isoquinoline-4-carboxylic acid ((S)-1-phenyl-propyl)-amide
**2fj** 3-[4-(2,3-Dihydroxy-propyl)-piperazin-1-ylmethyl]-1-oxo-2-phenyl-1,2-dihydro-isoquinoline-4-carboxylic acid ((S)-1-phenyl-propyl)-amide
**2fk** (2-Oxo-2-{4-[1-oxo-2-phenyl-4-(1-phenyl-propylcarbamoyl)-1,2-dihydro-isoquinolin-3-ylmethyl]-piperazin-1-yl}-ethyl)-carbamic acid tert-butyl ester
**2fl** 3-[4-(1H-Indazol-5-yl)-piperazin-1-ylmethyl]-1-oxo-2-phenyl-1,2-dihydro-isoquinoline-4-carboxylic acid ((S)-1-phenyl-propyl)-amide
**2fm** 1-Oxo-2-phenyl-3-(4-quinolin-6-yl-piperazin-1-ylmethyl)-1,2-dihydro-isoquinoline-4-carboxylic acid ((S)-1-phenyl-propyl)-amide
**2fn** 3-[4-(6,7-Dimethoxy-quinazolin-4-yl)-piperazin-1-ylmethyl]-1-oxo-2-phenyl-1,2-dihydro-isoquinoline-4-carboxylic acid ((S)-1-phenyl-propyl)-amide
**2fo** 4-{4-[1-Oxo-2-phenyl-4-(1-phenyl-propylcarbamoyl)-1,2-dihydro-isoquinolin-3-ylmethyl]-piperazin-1-yl}-piperidine-1-carboxylic acid tert-butyl ester
**2fp** 3-{4-[2-(4-Chloro-phenoxy)-ethyl]-piperazin-1-ylmethyl}-1-oxo-2-phenyl-1,2-dihydro-isoquinoline-4-carboxylic acid ((S)-1-phenyl-propyl)-amide
**2fq** {4-[1-Oxo-2-phenyl-4-(1-phenyl-propylcarbamoyl)-1,2-dihydro-isoquinolin-3-ylmethyl]-piperazin-1-yl}-acetic acid tert-butyl ester
**2fr** 1-Oxo-2-phenyl-3-[4-(3,3,3-trifluoro-2-hydroxy-propyl)-piperazin-1-ylmethyl]-1,2-dihydro-isoquinoline-4-carboxylic acid ((S)-1-phenyl-propyl)-amide
**2fs** 3-[4-(2-Hydroxy-propyl)-piperazin-1-ylmethyl]-1-oxo-2-phenyl-1,2-dihydro-isoquinoline-4-carboxylic acid ((S)-1-phenyl-propyl)-amide
**2fu** 3-[4-(4-Amino-6,7-dimethoxy-quinazolin-2-yl)-piperazin-1-ylmethyl]-1-oxo-2-phenyl-1,2-dihydro-isoquinoline-4-carboxylic acid ((S)-1-phenyl-propyl)-amide
**2fv** (2-{4-[1-Oxo-2-phenyl-4-(1-phenyl-propylcarbamoyl)-1,2-dihydro-isoquinolin-3-ylmethyl]-piperazin-1-yl}-ethyl)-carbamic acid tert-butyl ester
**2fw** 1-Oxo-3-{4-[2-(2-oxo-imidazolidin-1-yl)-ethyl]-piperazin-1-ylmethyl}-2-phenyl-1,2-dihydro-isoquinoline-4-carboxylic acid ((S)-1-phenyl-propyl)-amide
**2fx** 3-{4-[(4,6-Dimethoxy-pyrimidin-2-yl)-phenyl-methyl]-piperazin-1-ylmethyl}-1-oxo-2-phenyl-1,2-dihydro-isoquinoline-4-carboxylic acid ((S)-1-phenyl-propyl)-amide
**2fy** 3-(4-Benzo[1,2,5]thiadiazol-4-yl-piperazin-1-ylmethyl)-1-oxo-2-phenyl-1,2-dihydro-isoquino line-4-carboxylic acid ((S)-1-phenyl-propyl)-amide
2fz 3-[4-(2,3-Dihydro-benzo[1,4]dioxin-5-yl)-piperazin-1-ylmethyl]-1-oxo-2-phenyl-1,2-dihydro-isoquinoline-4-carboxylic acid ((S)-1-phenyl-propyl)-amide
**2ga** 3-[4-(4-Methyl-quinolin-2-yl)-piperazin-1-ylmethyl]-1-oxo-2-phenyl-1,2-dihydro-isoquinoline-4-carboxylic acid ((S)-1-phenyl-propyl)-amide
**2gb** 1-Oxo-2-phenyl-3-[4-(pyridin-2-ylcarbamoylmethyl)-piperazin-1-ylmethyl]-1,2-dihydro-isoquinoline-4-carboxylic acid ((S)-1-phenyl-propyl)-amide
**2gc** 3-[4-(6-Chloro-3-oxo-3,4-dihydro-2H-benzo[1,4]oxazin-8-yl)-piperazin-1-ylmethyl]-1-oxo-2-phenyl-1,2-dihydro-isoquinoline-4-carboxylic acid ((S)-1-phenyl-propyl)-amide
**2gd** 3-(4-Carbamoylmethyl-piperazin-1-ylmethyl)-1-oxo-2-phenyl-1,2-dihydro-isoquinoline-4-carboxylic acid ((S)-1-phenyl-propyl)-amide
**2ge** 3-(4-Hydroxy-4-phenyl-piperidin-1-ylmethyl)-1-oxo-2-phenyl-1,2-dihydro-isoquino line-4-carboxylic acid ((S)-1-phenyl-propyl)-amide
**2gf** 3-[4-(4-Chloro-phenyl)-4-hydroxy-piperidin-1-ylmethyl]-1-oxo-2-phenyl-1,2-dihydro-isoquino line-4-carboxylic acid ((S)-1-phenyl-propyl)-amide
**2gg** 1-[1-Oxo-2-phenyl-4-(1-phenyl-propylcarbamoyl)-1,2-dihydro-isoquinolin-3-ylmethyl]-piperidine-4-carboxylic acid
**2gh** 3-(4-Cyano-4-phenyl-piperidin-1-ylmethyl)-1-oxo-2-phenyl-1,2-dihydro-isoquino line-4-carboxylic acid ((S)-1-phenyl-propyl)-amide
**2gi** 3-(4-Benzyl-4-hydroxy-piperidin-1-ylmethyl)-1-oxo-2-phenyl-1,2-dihydro-isoquino line-4-carboxylic acid ((S)-1-phenyl-propyl)-amide
**2gj** 1-[1-Oxo-2-phenyl-4-(1-phenyl-propylcarbamoyl)-1,2-dihydro-isoquinolin-3-ylmethyl]-4-phenyl-piperidine-4-carboxylic acid ethyl ester
**2gk** 1-Oxo-2-phenyl-3-[4-(phenyl-propionyl-amino)-piperidin-1-ylmethyl]-1,2-dihydro-isoquinoline-4-carboxylic acid ((S)-1-phenyl-propyl)-amide
**2gl** Methyl-{1-[1-oxo-2-phenyl-4-(1-phenyl-propylcarbamoyl)-1,2-dihydro-isoquinolin-3-ylmethyl]-piperidin-4-yl}-carbamic acid tert-butyl ester
**2gm** 1-Oxo-2-phenyl-3-[4-(2-pyrrolidin-1-yl-ethyl)-piperidin-1-ylmethyl]-1,2-dihydro-isoquinoline-4-carboxylic acid ((S)-1-phenyl-propyl)-amide
**2gn** 3-{4-[5-(4-Fluoro-phenyl)-[1,3,4]oxadiazol-2-yl]-piperidin-1-ylmethyl}-1-oxo-2-phenyl-1,2-dihydro-isoquinoline-4-carboxylic acid ((S)-1-phenyl-propyl)-amide
**2go** 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide
**2gp** 1-Oxo-2-phenyl-3-[4-(3-pyrazin-2-yl-[1,2,4]oxadiazol-5-yl)-piperidin-1-ylmethyl]-1,2-dihydro-isoquinoline-4-carboxylic acid ((S)-1-phenyl-propyl)-amide
**2gq** 1-Oxo-2-phenyl-3-[4-(3-pyridin-4-yl-[1,2,4]oxadiazol-5-yl)-piperidin-1-ylmethyl]-1,2-dihydro-isoquinoline-4-carboxylic acid ((S)-1-phenyl-propyl)-amide
**2gr** 3-(4'-Hydroxy-3',4',5',6'-tetrahydro-2'H-[2,4']bipyridinyl-1'-ylmethyl)-1-oxo-2-phenyl-1,2-dihydro-isoquinoline-4-carboxylic acid ((S)-1-phenyl-propyl)-amide
**2gs** 3-(spiro[isochroman-1,4'-piperidin]-1'-ylmethyl)-1-oxo-2-phenyl-1,2-dihydro-isoquinoline-4-carboxylic acid ((S)-1-phenyl-propyl)-amide
**2gt** 3-[4-Hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide
**2gu** 3-[4-(3-Chloro-phenyl)-4-hydroxy-piperidin-1-ylmethyl]-1-oxo-2-phenyl-1,2-dihydro-isoquinoline-4-carboxylic acid ((S)-1-phenyl-propyl)-amide
**2gv** 3-(6-chloro-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide
**2gw** 3-(4-{[4-Chloro-3-(4-fluoro-phenyl)-indan-1-yl]-methyl-amino}-piperidin-1-ylmethyl)-1-oxo-2-phenyl-1,2-dihydro-isoquinoline-4-carboxylic acid ((S)-1-phenyl-propyl)-amide
**2gx** 3-(1-acetyl-spiro[indoline-3,4'-piperidin]-1'-ylmethyl)-1-oxo-2-phenyl-1,2-dihydro-isoquinoline-4-carboxylic acid ((S)-1-phenyl-propyl)-amide
**2gy** 3-(1-acetyl-5-fluoro-spiro[indoline-3,4'-piperidin]-1'-ylmethyl)-1-oxo-2-phenyl-1,2-dihydro-isoquinoline-4-carboxylic acid ((S)-1-phenyl-propyl)-amide
**2gz** 1-Oxo-3-(4-oxo-1-phenyl-1,3,8-triaza-spiro[4.5]dec-8-ylmethyl)-2-phenyl-1,2-dihydro-isoquinoline-4-carboxylic acid ((S)-1-phenyl-propyl)-amide
**2ha** 3-(4-Acetylamino-piperidin-1-ylmethyl)-1-oxo-2-phenyl-1,2-dihydro-isoquino line-4-carboxylic acid ((S)-1-phenyl-propyl)-amide
**2hb** 1-Oxo-3-(1-oxo-2,8-diaza-spiro[4.5]dec-8-ylmethyl)-2-phenyl-1,2-dihydro-isoquinoline-4-carboxylic acid ((S)-1-phenyl-propyl)-amide
**2hc** 3-[4-Hydroxy-4-(3-trifluoromethyl-phenyl)-piperidin-1-ylmethyl]-1-oxo-2-phenyl- 1,2-dihydro-isoquinoline-4-carboxylic acid ((S)-1-phenyl-propyl)-amide
**2hd** 1-Oxo-2-phenyl-3-(4-trifluoromethyl-piperidin-1-ylmethyl)-1,2-dihydro-isoquinoline-4-carboxylic acid ((S)-1-phenyl-propyl)-amide
**2he** 3-[4-(4-Methyl-piperazine-1-carbonyl)-piperidin-1-ylmethyl]-1-oxo-2-phenyl-1,2-dihydro-isoquinoline-4-carboxylic acid ((S)-1-phenyl-propyl)-amide
**2hf** 3-(5-isopropyl-3H-spiro[isobenzofuran-1,4'-piperidin]-1'-ylmethyl)-1-oxo-2-phenyl-1,2-dihydro-isoquinoline-4-carboxylic acid ((S)-1-phenyl-propyl)-amide
**2hg** 3-[4-(Acetyl-methyl-amino)-4-phenyl-piperidin-1-ylmethyl]-1-oxo-2-phenyl-1,2-dihydro-isoquinoline-4-carboxylic acid ((S)-1-phenyl-propyl)-amide
**2hh** 4-{1-[1-Oxo-2-phenyl-4-(1-phenyl-propylcarbamoyl)-1,2-dihydro-isoquinolin-3-ylmethyl]-piperidin-4-yl}-piperazine-1-carboxylic acid tert-butyl ester
**2hi** (2-{1-[1-Oxo-2-phenyl-4-(1-phenyl-propylcarbamoyl)-1,2-dihydro-isoquinolin-3-ylmethyl]-piperidin-4-yl}-ethyl)-carbamic acid tert-butyl ester
**2hj** 3-(4-Methylamino-4-phenyl-piperidin-1-ylmethyl)-1-oxo-2-phenyl-1,2-dihydro-isoquinoline-4-carboxylic acid ((S)-1-phenyl-propyl)-amide
**2hk** 3-(4-Acetylamino-4-phenyl-piperidin-1-ylmethyl)-1-oxo-2-phenyl-1,2-dihydro-isoquinoline-4-carboxylic acid ((S)-1-phenyl-propyl)-amide
**2hl** 3-[4-Acetylamino-4-(3-fluoro-phenyl)-piperidin-1-ylmethyl]-1-oxo-2-phenyl-1,2-dihydro-isoquinoline-4-carboxylic acid ((S)-1-phenyl-propyl)-amide
**2hm** 1-Oxo-3-[4-(4-oxo-piperidine-1-carbonyl)-piperidin-1-ylmethyl]-2-phenyl-1,2-dihydro-isoquinoline-4-carboxylic acid ((S)-1-phenyl-propyl)-amide
**2hn** 3-[4,4']Bipiperidinyl-1-ylmethyl-1-oxo-2-phenyl-1,2-dihydro-isoquinoline-4-carboxylic acid ((S)-1-phenyl-propyl)-amide
**2ho** {1-[1-Oxo-2-phenyl-4-(1-phenyl-propylcarbamoyl)-1,2-dihydro-isoquinolin-3-ylmethyl]-piperidin-4-yl}-carbamic acid tert-butyl ester
**2hp** 3-[1,4']Bipiperidinyl-1'-ylmethyl-1-oxo-2-phenyl-1,2-dihydro-isoquinoline-4-carboxylic acid [cyclopropyl-(3-fluoro-phenyl)-methyl]-amide
**2hq** 1-Oxo-3-(4-phenethyl-piperazin-1-ylmethyl)-2-phenyl-1,2-dihydro-isoquinoline-4-carboxylic acid [cyclopropyl-(3-fluoro-phenyl)-methyl]-amide
**2hr** 3-(4-Isopropyl-piperazin-1-ylmethyl)-1-oxo-2-phenyl-1,2-dihydro-isoquinoline-4-carboxylic acid [cyclopropyl-(3-fluoro-phenyl)-methyl]-amide
**2hs** 3-[4-(2-Morpholin-4-yl-ethyl)-piperazin-1-ylmethyl]-1-oxo-2-phenyl-1,2-dihydro-isoquinoline-4-carboxylic acid [cyclopropyl-(3-fluoro-phenyl)-methyl]-amide
**2ht** 3-[4-(1-Methyl-piperidin-4-yl)-piperazin-1-ylmethyl]-1-oxo-2-phenyl-1,2-dihydro-isoquinoline-4-carboxylic acid [cyclopropyl-(3-fluoro-phenyl)-methyl]-amide
**2hu** 1-Oxo-2-phenyl-3-[4-(2-piperidin-1-yl-ethyl)-piperazin-1-ylmethyl]-1,2-dihydro-isoquinoline-4-carboxylic acid [cyclopropyl-(3-fluoro-phenyl)-methyl]-amide
**2hv** 1-Oxo-2-phenyl-3-[4-(2-pyrrolidin-1-yl-ethyl)-piperazin-1-ylmethyl]-1,2-dihydro-isoquinoline-4-carboxylic acid [cyclopropyl-(3-fluoro-phenyl)-methyl]-amide
**2hw** 1-Oxo-2-phenyl-3-(4-pyridin-4-ylmethyl-piperazin-1-ylmethyl)-1,2-dihydro-isoquinoline-4-carboxylic acid [cyclopropyl-(3-fluoro-phenyl)-methyl]-amide
**2hx** 3-(4-Hydroxy-3,4,5,6-tetrahydro-2H-[4,4']bipyridinyl-1-ylmethyl)-1-oxo-2-phenyl-1,2-dihydro-isoquinoline-4-carboxylic acid [cyclopropyl-(3-fluoro-phenyl)-methyl]-amide
**2hy** 3-[4-(2-Morpholin-4-yl-ethyl)-piperidin-1-ylmethyl]-1-oxo-2-phenyl-1,2-dihydro-isoquinoline-4-carboxylic acid [cyclopropyl-(3-fluoro-phenyl)-methyl]-amide
**2hz** 3-[4-Hydroxy-4-(3-methoxy-phenyl)-piperidin-1-ylmethyl]-1-oxo-2-phenyl-1,2-dihydro-isoquinoline-4-carboxylic acid [cyclopropyl-(3-fluoro-phenyl)-methyl]-amide
**2ia** 3-[4-(Acetyl-methyl-amino)-4-phenyl-piperidin-1-ylmethyl]-1-oxo-2-phenyl-1,2-dihydro-isoquinoline-4-carboxylic acid [cyclopropyl-(3-fluoro-phenyl)-methyl]-amide
**2ib** 2-(2-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2ic** 2-(2-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2id** 3-[1,4']Bipiperidinyl-1'-ylmethyl-2-(2-fluoro-phenyl)-1-oxo-1,2-dihydro-isoquinoline-4-carboxylic acid ((S)-1-phenyl-propyl)-amide
**2ie** 2-(2-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2if** 2-(2-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2ig** 2-(2-Fluoro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2ih** 2-(2-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2ii** 2-(2-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ij** 2-(2-Fluoro-phenyl)-3-(4-hydroxy-3,4,5,6-tetrahydro-2H-[4,4']bipyridinyl-1-ylmethyl)-1-oxo-1,2-dihydro-isoquinoline-4-carboxylic acid ((S)-1-phenyl-propyl)-amide
**2ik** 2-(2-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide
**2il** 2-(2-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2im** 3-[4-(2-Morpholin-4-yl-ethyl)-piperazin-1-ylmethyl]-1-oxo-2-o-tolyl-1,2-dihydro-isoquinoline-4-carboxylic acid ((S)-1-phenyl-propyl)-amide
**2in** 1-Oxo-3-[4-(2-piperidin-1-yl-ethyl)-piperazin-1-ylmethyl]-2-o-tolyl-1,2-dihydro-isoquinoline-4-carboxylic acid ((S)-1-phenyl-propyl)-amide
**2io** 1-Oxo-3-(4-pyridin-4-ylmethyl-piperazin-1-ylmethyl)-2-o-tolyl-1,2-dihydro-isoquinoline-4-carboxylic acid ((S)-1-phenyl-propyl)-amide
**2ip** 2-(3-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2iq** 2-(3-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2ir** 3-[1,4']Bipiperidinyl-1'-ylmethyl-2-(3-fluoro-phenyl)-1-oxo-1,2-dihydro-isoquinoline-4-carboxylic acid ((S)-1-phenyl-propyl)-amide
**2is** 2-(3-Fluoro-phenyl)-3-[4-(2-morpholin-4-yl-ethyl)-piperazin-1-ylmethyl]-1-oxo-1,2-dihydro-isoquinotine-4-carboxylic acid ((S)-1-phenyl-propyl)-amide
**2it** 2-(3-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2iu** 2-(3-Fluoro-phenyl)-1-oxo-3-[4(2-piperidin-1-yl-ethyl)-piperazin-1-ylmethyl]-1,2-dihydro-isoquinoline-4-carboxylic acid ((S)-1-phenyl-propyl)-amide
**2iv** 2-(3-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2iw** 2-(3-Fluoro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ix** 2-(3-Fluoro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide **2iy** 2-(3-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2iz** 3-[4-(Acetyl-methyl-amino)-4-phenyl-piperidin-1-ylmethyl]-2-(3-fluoro-phenyl)-1-oxo-1,2-dihydro-isoquinoline-4-carboxylic acid ((S)-1-phenyl-propyl)-amide
**2ja** 2-(3-Chloro-phenyl)-1-oxo-3-(4-phenethyl-piperazin-1-ylmethyl)-1,2-dihydro-isoquinoline-4-carboxylic acid ((S)-1-phenyl-propyl)-amide
**2jb** 2-(3-Chloro-phenyl)-3-(4-isopropyl-piperazin-1-ylmethyl)-1-oxo-1,2-dihydro-isoquinoline-4-carboxylic acid ((S)-1-phenyl-propyl)-amide
**2jc** 3-[1,4']Bipiperidinyl-1'-ylmethyl-2-(3-chloro-phenyl)-1-oxo-1,2-dihydro-isoquinoline-4-carboxylic acid ((S)-1-phenyl-propyl)-amide
**2jd** 2-(3-Chloro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2je** 2-(3-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2jf** 2-(3-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2jg** 2-(3-Chloro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2jh** 2-(3-Chloro-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ji** 2-(3-Chloro-phenyl)-3-[4-(2-morpholin-4-yl-ethyl)-piperidin-1-ylmethyl]-1-oxo-1,2-dihydro-isoquinoline-4-carboxylic acid ((S)-1-phenyl-propyl)-amide
**2jj** 2-(3-Chloro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2jk** 3-[4-(Acetyl-methyl-amino)-4-phenyl-piperidin-1-ylmethyl]-2-(3-chloro-phenyl)-1-oxo-1,2-dihydro-isoquinoline-4-carboxylic acid ((S)-1-phenyl-propyl)-amide
**2jl** 1-Oxo-3-(4-phenethyl-piperazin-1-ylmethyl)-2-m-tolyl-1,2-dihydro-isoquinoline-4-carboxylic acid ((S)-1-phenyl-propyl)-amide
**2jm** 3-[1,4']Bipiperidinyl-1'-ylmethyl-1-oxo-2-m-tolyl-1,2-dihydro-isoquinoline-4-carboxylic acid ((S)-1-phenyl-propyl)-amide
**2jn** 3-[4-(2-Morpholin-4-yl-ethyl)-piperazin-1-ylmethyl]-1-oxo-2-m-tolyl-1,2-dihydro-isoquinoline-4-carboxylic acid ((S)-1-phenyl-propyl)-amide
**2jo** 3-[4-(1-Methyl-piperidin-4-yl)-piperazin-1-ylmethyl]-1-oxo-2-m-tolyl-1,2-dihydro-isoquinoline-4-carboxylic acid ((S)-1-phenyl-propyl)-amide
**2jp** 1-Oxo-3-[4-(2-piperidin-1-yl-ethyl)-piperazin-1-ylmethyl]-2-m-tolyl-1,2-dihydro-isoquinoline-4-carboxylic acid ((S)-1-phenyl-propyl)-amide
**2jq** 1-Oxo-3-[4-(2-pyrrolidin-1-yl-ethyl)-piperazin-1-ylmethyl]-2-m-tolyl-1,2-dihydro-isoquinoline-4-carboxylic acid ((S)-1-phenyl-propyl)-amide
**2jr** 1-Oxo-3-(4-pyridin-4-ylmethyl-piperazin-1-ylmethyl)-2-m-tolyl-1,2-dihydro-isoquinoline-4-carboxylic acid ((S)-1-phenyl-propyl)-amide
**2js** 3-[4-(2-Morpholin-4-yl-ethyl)-piperidin-1-ylmethyl]-1-oxo-2-m-tolyl-1,2-dihydro-isoquinoline-4-carboxylic acid ((S)-1-phenyl-propyl)-amide
**2jt** 3-[4-Hydroxy-4-(3-methoxy-phenyl)-piperidin-1-ylmethyl]-1-oxo-2-m-tolyl-1,2-dihydro-isoquino line-4-carboxylic acid ((S)-1-phenyl-propyl)-amide
**2ju** 3-[4-(Acetyl-methyl-amino)-4-phenyl-piperidin-1-ylmethyl]-1-oxo-2-m-tolyl-1,2-dihydro-isoquino line-4-carboxylic acid ((S)-1-phenyl-propyl)-amide
**3a** 1-Oxo-3-(2-oxo-pyrrolidin-1-ylmethyl)-2-phenyl-1,2-dihydro-isoquinoline-4-carboxylic acid ((S)-1-phenyl-propyl)-amide
**3b** 8-Chloro-1-oxo-3-(2-oxo-pyrrolidin-1-ylmethyl)-2-phenyl-1,2-dihydro-isoquinoline-4-carboxylic acid ((S)-1-phenyl-propyl)-amide
**4a** 3-Cyanomethyl-1-oxo-2-phenyl-1,2-dihydro-isoquinoline-4-carboxylic acid ((S)-1-phenyl-propyl)-amide
**5a** 3-Methyl-1-oxo-2-phenyl-1,2-dihydro-isoquinoline-4-carboxylic acid N,N-diphenyl-hydrazide
**5b** N'-(3-Methyl-1-oxo-2-phenyl-1,2-dihydro-isoquinoline-4-carbonyl)-N-phenyl-hydrazinecarboxylic acid methyl ester
**1aa** 2-(3-Methoxy-phenyl)-3-methyl-1-oxo-1,2-dihydro-isoquinoline-4-carboxylic acid ((S)-1-phenyl-propyl)-amide
**1ab** 2-(4-Methoxy-phenyl)-3-methyl-1-oxo-1,2-dihydro-isoquinoline-4-carboxylic acid ((S)-1-phenyl-propyl)-amide
**1ac** 2-(4-Fluoro-phenyl)-3-methyl-1-oxo-1,2-dihydro-isoquinoline-4-carboxylic acid ((S)-1-phenyl-propyl)-amide
**1ad** 2-(4-Chloro-phenyl)-3-methyl-1-oxo-1,2-dihydro-isoquinoline-4-carboxylic acid ((S)-1-phenyl-propyl)-amide
**1ae** 3-Methyl-1-oxo-2-p-tolyl-1,2-dihydro-isoquinoline-4-carboxylic acid ((S)-1-phenyl-propyl)-amide
**1af** 2-(3-Methoxy-phenyl)-3-methyl-1-oxo-1,2-dihydro-isoquinoline-4-carboxylic acid [(S)-cyclopropyl-(3-fluoro-phenyl)-methyl]-amide
**1ag** 2-(4-Methoxy-phenyl)-3-methyl-1-oxo-1,2-dihydro-isoquinoline-4-carboxylic acid [(S)-cyclopropyl-(3-fluoro-phenyl)-methyl]-amide
**1ah** 2-(4-Fluoro-phenyl)-3-methyl-1-oxo-1,2-dihydro-isoquinoline-4-carboxylic acid [(S)-cyclopropyl-(3-fluoro-phenyl)-methyl]-amide
**1ai** 2-(4-Chloro-phenyl)-3-methyl-1-oxo-1,2-dihydro-isoquinoline-4-carboxylic acid [(S)-cyclopropyl-(3-fluoro-phenyl)-methyl]-amide
**1aj** 3-Methyl-1-oxo-2-p-tolyl-1,2-dihydro-isoquinoline-4-carboxylic acid [(S)-cyclopropyl-(3-fluoro-phenyl)-methyl]-amide
**1ak** 2-(2-Methoxy-phenyl)-3-methyl-1-oxo-1,2-dihydro-isoquinoline-4-carboxylic acid ((S)-1-phenyl-propyl)-amide
**1al** 2-(2-Methoxy-phenyl)-3-methyl-1-oxo-1,2-dihydro-isoquinoline-4-carboxylic acid [(S)-cyclopropyl-(3-fluoro-phenyl)-methyl]-amide
**1am** 3-Methyl-8-nitro-1-oxo-2-phenyl-1,2-dihydro-isoquinoline-4-carboxylic acid ((S)-1-phenyl-propyl)-amide
**1an** 3-Methyl-8-nitro-1-oxo-2-phenyl-1,2-dihydro-isoquinoline-4-carboxylic acid [(S)-cyclopropyl-(3-fluoro-phenyl)-methyl]-amide
**1ao** 8-Methoxy-3-methyl-1-oxo-2-phenyl-1,2-dihydro-isoquinoline-4-carboxylic acid ((S)-1-phenyl-propyl)-amide
**1ap** 8-Methoxy-3-methyl-1-oxo-2-phenyl-1,2-dihydro-isoquinoline-4-carboxylic acid [(S)-cyclopropyl-(3-fluoro-phenyl)-methyl]-amide
**1aq** 8-Amino-3-methyl-1-oxo-2-phenyl-1,2-dihydro-isoquinoline-4-carboxylic acid ((S)-1-phenyl-propyl)-amide
**1ar** 8-Cyano-3-methyl-1-oxo-2-phenyl-1,2-dihydro-isoquinoline-4-carboxylic acid ((S)-1-phenyl-propyl)-amide
**1as** 8-Chloro-3-methyl-1-oxo-2-phenyl-1,2-dihydro-isoquinoline-4-carboxylic acid [(S)-1-(4-chloro-phenyl)-propyl]-amide
**1at** 8-Chloro-3-methyl-1-oxo-2-phenyl-1,2-dihydro-isoquinoline-4-carboxylic acid [(S)-1-(4-fluoro-phenyl)-propyl]-amide
**1au** 8-Chloro-3-methyl-1-oxo-2-phenyl-1,2-dihydro-isoquinoline-4-carboxylic acid [(S)-1-(2-fluoro-phenyl)-propyl]-amide
**1av** 8-Chloro-3-methyl-1-oxo-2-phenyl-1,2-dihydro-isoquinoline-4-carboxylic acid [(S)-1-(3-chloro-phenyl)-propyl]-amide
**1aw** 8-Chloro-3-methyl-1-oxo-2-phenyl-1,2-dihydro-isoquinoline-4-carboxylic acid [(S)-(3-chloro-phenyl)-cyclopropyl-methyl]-amide
**1ax** 8-Chloro-3-methyl-1-oxo-2-phenyl-1,2-dihydro-isoquinoline-4-carboxylic acid [(S)-(4-chloro-phenyl)-cyclopropyl-methyl]-amide
**1ay** 8-Chloro-3-methyl-1-oxo-2-phenyl-1,2-dihydro-isoquinoline-4-carboxylic acid [(S)-1-(3-fluoro-phenyl)-propyl]-amide
**1az** 8-Chloro-3-methyl-1-oxo-2-phenyl-1,2-dihydro-isoquinoline-4-carboxylic acid ((S)-2-methyl-1-phenyl-propyl)-amide
**1ba** 8-Chloro-3-methyl-1-oxo-2-phenyl-1,2-dihydro-isoquinoline-4-carboxylic acid [(S)-cyclopropyl-(4-fluoro-phenyl)-methyl]-amide
**1bb** 8-Chloro-3-methyl-1-oxo-2-phenyl-1,2-dihydro-isoquinoline-4-carboxylic acid [(S)-1-(2-chloro-phenyl)-propyl]-amide
**1bc** 8-Chloro-3-methyl-1-oxo-2-phenyl-1,2-dihydro-isoquino line-4-carboxylic acid ((S)-cyclopentyl-phenyl-methyl)-amide
**1bd** 8-Chloro-3-methyl-1-oxo-2-phenyl-1,2-dihydro-isoquinoline-4-carboxylic acid [(S)-(2-chloro-phenyl)-cyclopropyl-methyl]-amide
**1be** 8-Chloro-3-methyl-1-oxo-2-phenyl-1,2-dihydro-isoquinoline-4-carboxylic acid [(S)-cyclopropyl-(2-fluoro-phenyl)-methyl]-amide
**1bf** 8-Chloro-3-methyl-1-oxo-2-phenyl-1,2-dihydro-isoquinoline-4-carboxylic acid ((S)-cyclohexyl-phenyl-methyl)-amide
**1bg** 8-Chloro-3-methyl-1-oxo-2-phenyl-1,2-dihydro-isoquinoline-4-carboxylic acid ((S)-cyclopropyl-phenyl-methyl)-amide
**1bh** 8-Chloro-3-methyl-1-oxo-2-phenyl-1,2-dihydro-isoquinoline-4-carboxylic acid [(S)-cyclobutyl-(3-fluoro-phenyl)-methyl]-amide
**1bi** 8-Chloro-3-methyl-1-oxo-2-phenyl-1,2-dihydro-isoquinoline-4-carboxylic acid [(S)-cyclobutyl-(4-fluoro-phenyl)-methyl]-amide
**1bl** 8-Chloro-3-methyl-1-oxo-2-phenyl-1,2-dihydro-isoquinoline-4-carboxylic acid [(R)-cyclopropyl-(3-fluoro-phenyl)-methyl]-amide
**1bn** 8-Chloro-3-methyl-1-oxo-2-phenyl-1,2-dihydro-isoquinoline-4-carboxylic acid ((S)cyclobutyl-phenyl-methyl)-amide
**1bo** 8-Chloro-3-methyl-1-oxo-2-phenyl-1,2-dihydro-isoquinoline-4-carboxylic acid [cyclobutyl-(2-fluoro-phenyl)-methyl]-amide
**2ka** 3-(4-tert-Butyl-piperazin-1-ylmethyl)-8-chloro-1-oxo-2-phenyl-1,2-dihydro-isoquinoline-4-carboxylic acid [(S)-cyclopropyl-(3-fluoro-phenyl)-methyl]-amide
**2kb** 8-Chloro-3-(4-isopropyl-piperazin-1-ylmethyl)-1-oxo-2-phenyl-1,2-dihydro-isoquinoline-4-carboxylic acid ((S)-1-phenyl-propyl)-amide
**2kc** 8-Chloro-3-(4-isobutyl-piperazin-1-ylmethyl)-1-oxo-2-phenyl-1,2-dihydro-isoquinoline-4-carboxylic acid ((S)-1-phenyl-propyl)-amide
**2kd** 8-Chloro-3-(octahydro-pyrido[1,2-a]pyrazin-2-ylmethyl)-1-oxo-2-phenyl-1,2-dihydro-isoquinoline-4-carboxylic acid ((S)-1-phenyl-propyl)-amide **2ke** 8-Chloro-3-(4-hydroxy-3,4,5,6-tetrahydro-2H-[4,4']bipyridinyl-1-ylmethyl)-1-oxo-2-phenyl-1,2-dihydro-isoquinoline-4-carboxylic acid ((S)-1-phenyl-propyl)-amide
**2kf** 8-Chloro-3-(4-cyclopropylmethyl-piperazin-1-ylmethyl)-1-oxo-2-phenyl-1,2-dihydro-isoquinoline-4-carboxylic acid ((S)-1-phenyl-propyl)-amide
**2kg** 8-Chloro-3-[4-(2-morpholin-4-yl-ethyl)-piperidin-1-ylmethyl]-1-oxo-2-phenyl-1,2-dihydro-isoquinoline-4-carboxylic acid ((S)-1-phenyl-propyl)-amide
**2kh** 8-Chloro-3-[1,4]diazepan-1-ylmethyl-1-oxo-2-phenyl-1,2-dihydro-isoquinoline-4-carboxylic acid ((S)-1-phenyl-propyl)-amide
**2ki** 8-Chloro-3-((S)-3-methyl-piperazin-1-ylmethyl)-1-oxo-2-phenyl-1,2-dihydro-isoquinoline-4-carboxylic acid ((S)-1-phenyl-propyl)-amide
**2kj** 8-Chloro-3-imidazol-1-ylmethyl-1-oxo-2-phenyl-1,2-dihydro-isoquinoline-4-carboxylic acid ((S)-1-phenyl-propyl)-amide
**2kk** 8-Chloro-3-(4-methyl-imidazol-1-ylmethyl)-1-oxo-2-phenyl-1,2-dihydro-isoquinoline-4-carboxylic acid ((S)-1-phenyl-propyl)-amide
**2kl** 3-(tert-Butylamino-methyl)-8-chloro-1-oxo-2-phenyl-1,2-dihydro-isoquino line-4-carboxylic acid ((S)-1-phenyl-propyl)-amide
**2km** 8-Chloro-3-(isopropylamino-methyl)-1-oxo-2-phenyl-1,2-dihydro-isoquinoline-4-carboxylic acid ((S)-1-phenyl-propyl)-amide
**2kn** 8-Chloro-3-[4-hydroxy-4-(3-methoxy-phenyl)-piperidin-1-ylmethyl]-1-oxo-2-phenyl-1,2-dihydro-isoquinoline-4-carboxylic acid ((S)-1-phenyl-propyl)-amide
**2ko** 3-(4-tert-Butyl-piperazin-1-ylmethyl)-8-chloro-1-oxo-2-phenyl-1,2-dihydro-isoquinoline-4-carboxylic acid ((S)-1-phenyl-propyl)-amide
**2kp** 3-Benzoimidazol-1-ylmethyl-1-oxo-2-phenyl-1,2-dihydro-isoquinoline-4-carboxylic acid ((S)-1-phenyl-propyl)-amide
**2kq** 1-Oxo-2-phenyl-3-pyrazol-1-ylmethyl-1,2-dihydro-isoquinoline-4-carboxylic acid ((S)-1-phenyl-propyl)-amide
**2kr** 3-(4-Methyl-pyrazol-1-ylmethyl)-1-oxo-2-phenyl-1,2-dihydro-isoquinoline-4-carboxylic acid ((S)-1-phenyl-propyl)-amide
**2ks** 1-Oxo-2-phenyl-3-[1,2,3]triazol-1-ylmethyl-1,2-dihydro-isoquinoline-4-carboxylic acid ((S)-1-phenyl-propyl)-amide
**2kt** 2-(3-Methoxy-phenyl)-1-oxo-3-(4-pyridin-4-ylmethyl-piperazin-1-ylmethyl)-1,2-dihydro-isoquinoline-4-carboxylic acid ((S)-1-phenyl-propyl)-amide
**2ku** 2-(4-Methoxy-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2kv** 2-(4-Fluoro-phenyl)-1-oxo-3-(4-phenethyl-piperazin-1-ylmethyl)-1,2-dihydro-isoquinoline-4-carboxylic acid ((S)-1-phenyl-propyl)-amide
**2kw** 2-(4-Fluoro-phenyl)-3-(4-isopropyl-piperazin-1-ylmethyl)-1-oxo-1,2-dihydro-isoquinoline-4-carboxylic acid ((S)-1-phenyl-propyl)-amide
**2kx** 3-[1,4']Bipiperidinyl-1'-ylmethyl-2-(4-fluoro-phenyl)-1-oxo-1,2-dihydro-isoquinoline-4-carboxylic acid ((S)-1-phenyl-propyl)-amide
**2ky** 2-(4-Fluoro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2kz** 2-(4-Fluoro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2la** 2-(4-Fluoro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2lb** 2-(4-Fluoro-phenyl)-3-[4-hydroxy-4-(3-methoxy-phenyl)-piperidin-1-ylmethyl]-1-oxo-1,2-dihydro-isoquinoline-4-carboxylic acid ((S)-1-phenyl-propyl)-amide
**2lc** 2-(4-Chloro-phenyl)-1-oxo-3-(4-phenethyl-piperazin-1-ylmethyl)-1,2-dihydro-isoquinoline-4-carboxylic acid ((S)-1-phenyl-propyl)-amide
**2ld** 2-(4-Chloro-phenyl)-3-(4-isopropyl-piperazin-1-ylmethyl)-1-oxo-1,2-dihydro-isoquino line-4-carboxylic acid ((S)-1-phenyl-propyl)-amide
**2le** 3-[1,4']Bipiperidinyl-1'-ylmethyl-2-(4-chloro-phenyl)-1-oxo-1,2-dihydro-isoquinoline-4-carboxylic acid ((S)-1-phenyl-propyl)-amide
**2lf** 2-(4-Chloro-phenyl)-3-[4-(2-morpholin-4-yl-ethyl)-piperazin-1-ylmethyl]-1-oxo-1,2-dihydro-isoquinoline-4-carboxylic acid ((S)-1-phenyl-propyl)-amide
**2lg** 2-(4-Chloro-phenyl)-3-[4-(1-methyl-piperidin-4-yl)-piperazin-1-ylmethyl]-1-oxo-1,2-dihydro-isoquinoline-4-carboxylic acid ((S)-1-phenyl-propyl)-amide
**2lh** 2-(4-Chloro-phenyl)-1-oxo-3-[4-(2-piperidin-1-yl-ethyl)-piperazin-1-ylmethyl]-1,2-dihydro-isoquinoline-4-carboxylic acid ((S)-1-phenyl-propyl)-amide
**2li** 2-(4-Chloro-phenyl)-1-oxo-3-[4-(2-pyrrolidin-1-yl-ethyl)-piperazin-1-ylmethyl]-1,2-dihydro-isoquinoline-4-carboxylic acid ((S)-1-phenyl-propyl)-amide
**2lj** 3-(4-tert-Butyl-piperazin-1-ylmethyl)-8-fluoro-1-oxo-2-phenyl-1,2-dihydro-isoquinoline-4-carboxylic acid ((S)-1-phenyl-propyl)-amide
**2lk** 8-Chloro-3-cyclopropylaminomethyl-1-oxo-2-phenyl-1,2-dihydro-isoquinoline-4-carboxylic acid ((S)-1-phenyl-propyl)-amide
**2ll** 3-(4-tert-Butyl-piperazin-1-ylmethyl)-8-fluoro-1-oxo-2-phenyl-1,2-dihydro-isoquinoline-4-carboxylic acid [(S)-cyclopropyl-(3-fluoro-phenyl)-methyl]-amide
**2lm** 8-Fluoro-1-oxo-2-phenyl-3-piperazin-1-ylmethyl-1,2-dihydro-isoquinoline-4-carboxylic acid [(S)-cyclopropyl-(3-fluoro-phenyl)-methyl]-amide
**2ln** 8-Fluoro-1-oxo-3-(3-oxo-pyrazolidin-1-ylmethyl)-2-phenyl-1,2-dihydro-isoquinoline-4-carboxylic acid [(S)-cyclopropyl-(3-fluoro-phenyl)-methyl]-amide
**2lo** 8-Fluoro-1-oxo-3-(3-oxo-piperazin-1-ylmethyl)-2-phenyl-1,2-dihydro-isoquinoline-4-carboxylic acid [(S)-cyclopropyl-(3-fluoro-phenyl)-methyl]-amide
**3c** 1-Oxo-2-phenyl-3-(1H-[1,2,4]triazol-3-ylsulfanylmethyl)-1,2-dihydro-isoquinoline-4-carboxylic acid ((S)-1-phenyl-propyl)-amide
**3d** 8-Chloro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide
**3e** 8-Fluoro-1-oxo-3-(2-oxo-pyrrolidin-1-ylmethyl)-2-phenyl-1,2-dihydro-isoquinoline-4-carboxylic acid ((S)-1-phenyl-propyl)-amide
**3f** 8-Fluoro-1-oxo-3-(2-oxo-pyrrolidin-1-ylmethyl)-2-phenyl-1,2-dihydro-isoquinoline-4-carboxylic acid [(S)-cyclopropyl-(3-fluoro-phenyl)-methyl]-amide
**3g** 8-Fluoro-1-oxo-3-(5-oxo-pyrazolidin-1-ylmethyl)-2-phenyl-1,2-dihydro-isoquinoline-4-carboxylic acid [(S)-cyclopropyl-(3-fluoro-phenyl)-methyl]-amide
**3h** 8-Fluoro-1-oxo-3-(2-oxo-piperazin-1-ylmethyl)-2-phenyl-1,2-dihydro-isoquinoline-4-carboxylic acid [(S)-cyclopropyl-(3-fluoro-phenyl)-methyl]-amide
**3i** 8-Fluoro-1-oxo-3-(2-oxo-piperidin-1-ylmethyl)-2-phenyl-1,2-dihydro-isoquinoline-4-carboxylic acid [(S)-cyclopropyl-(3-fluoro-phenyl)-methyl]-amide
**4b** 8-Chloro-3-cyanomethyl-1-oxo-2-phenyl-1,2-dihydro-isoquinoline-4-carboxylic acid [(S)-cyclopropyl-(3-fluoro-phenyl)-methyl]-amide
**6a** 2-(3,4-Dichloro-phenyl)-3-methyl-1-oxo-1,2-dihydro-isoquinoline-4-carboxylic acid ((S)-1-phenyl-propyl)-amide
**6b** 8-Fluoro-1-oxo-3-(2-oxo-pyrrolidin-1-ylmethyl)-2-phenyl-1,2-dihydro-isoquinoline-4-carboxylic acid ((S)-cyclopropyl-phenyl-methyl)-amide
**6c** 8-Fluoro-3-methyl-1-oxo-2-phenyl-1,2-dihydro-isoquinoline-4-carboxylic acid ((S)-cyclopropyl-phenyl-methyl)-amide
**7a** 3-Ethyl-1-oxo-2-phenyl-1,2-dihydro-isoquinoline-4-carboxylic acid ((S)-1-phenyl-propyl)-amide
**7b** 8-Fluoro-3-methyl-1-oxo-2-phenyl-1,2-dihydro-isoquinoline-4-carboxylic acid ((S)-1-phenyl-propyl)-amide
**7c** 8-Fluoro-2-(3-fluoro-phenyl)-3-methyl-1-oxo-1,2-dihydro-isoquinoline-4-carboxylic acid ((S)-1-phenyl-propyl)-amide
**7d** 8-Fluoro-2-(4-fluoro-phenyl)-3-methyl-1-oxo-1,2-dihydro-isoquinoline-4-carboxylic acid ((S)-1-phenyl-propyl)-amide
**7f** 8-Fluoro-2-(3-fluoro-phenyl)-1-oxo-3-(2-oxo-pyrrolidin-1-ylmethyl)-1,2-dihydro-isoquino line-4-carboxylic acid [(S)-cyclopropyl-(3-fluoro-phenyl)-methyl]-amide
**7g** 8-Fluoro-2-(4-fluoro-phenyl)-1-oxo-3-(2-oxo-pyrrolidin-1-ylmethyl)-1,2-dihydro-isoquinoline-4-carboxylic acid [(S)-cyclopropyl-(3-fluoro-phenyl)-methyl]-amide
**7i** 8-Fluoro-2-(2-fluoro-phenyl)-3-methyl-1-oxo-1,2-dihydro-isoquinoline-4-carboxylic acid [(S)-cyclopropyl-(3-fluoro-phenyl)-methyl]-amide
**7j** 8-Fluoro-2-(3-fluoro-phenyl)-3-methyl-1-oxo-1,2-dihydro-isoquinoline-4-carboxylic acid [(S)-cyclopropyl-(3-fluoro-phenyl)-methyl]-amide
**7k** 8-Fluoro-2-(4-fluoro-phenyl)-3-methyl-1-oxo-1,2-dihydro-isoquinoline-4-carboxylic acid [(S)-cyclopropyl-(3-fluoro-phenyl)-methyl)-amide
**71**6,8-Difluoro-3-methyl-1-oxo-2-phenyl-1,2-dihydro-isoquinoline-4-carboxylic acid [(S)-cyclopropyl-(3-fluoro-phenyl)-methyl]-amide
**7m** 5,8-Difluoro-3-methyl-1-oxo-2-phenyl-1,2-dihydro-isoquinoline-4-carboxytic acid [(S)-cyclopropyl-(3-fluoro-phenyl)-methyl]-amide
**7n** 3-Methyl-1-oxo-2-phenyl-8-trifluoromethyl-1,2-dihydro-isoquinoline-4-carboxylic acid ((S)-1-phenyl-propyl)-amide
**8a** 3-(1-tert-Butyl-piperidin-4-ylmethyl)-8-chloro-1-oxo-2-phenyl-1,2-dihydro-isoquinoline-4-carboxylic acid [(S)-cyclopropyl-(3-fluoro-phenyl)-methyl]-amide; and pharmaceutically acceptable salts of any of these compounds.

7. A compound according to any of claims 1-6 for use in therapy.

8. A compound according to any of claims 1-6 for use in the treatment of a disease selected from psychosis; schizophrenia; schizophrenoform disorder; schizoaffective disorder; delusional disorder; brief psychotic disorder; shared psychotic disorder; psychotic disorder due to a general medical condition; substance or drug induced psychotic disorder (cocaine, alcohol, amphetamine etc); schizoid personality disorder; schizoptypal personality disorder; psychosis or schizophrenia associated with major depression, bipolar disorder, Alzheimer's disease or Parkinson's disease; major depression; general anxiety disorder; bipolar disorder (maintenance treatment, recurrence prevention and stabilization); mania; hypomania; cognitive impairment; ADHD; obesity; appetite reduction; Alzheimer's disease; Parkinson's disease; pain; convulsions; cough; asthma; airway hyperresponsiveness; microvascular hypersensitivity; bronchoconstriction; chronic obstructive pulmonary disease; urinary incontinence; gut inflammation; and inflammatory bowel syndrome.

9. The use of a compound according to any of claims 1-6 for the manufacture of a medicament for the treatment of a disease selected from psychosis; schizophrenia; schizophrenoform disorder; schizoaffective disorder; delusional disorder; brief psychotic disorder; shared psychotic disorder; psychotic disorder due to a general medical condition; substance or drug induced psychotic disorder (cocaine, alcohol, amphetamine etc); schizoid personality disorder; schizoptypal personality disorder; psychosis or schizophrenia associated with major depression, bipolar disorder, Alzheimer's disease or Parkinson's disease; major depression; general anxiety disorder; bipolar disorder (maintenance treatment, recurrence prevention and stabilization); mania; hypomania; cognitive impairment; ADHD; obesity; appetite reduction; Alzheimer's disease; Parkinson's disease; pain; convulsions; cough; asthma; airway hyperresponsiveness; microvascular hypersensitivity; bronchoconstriction; chronic obstructive pulmonary disease; urinary incontinence; gut inflammation; and inflammatory bowel syndrome.

10. The use according to claim 9 wherein said manufacture further comprises the use of a compound selected from the list consisting of D2 antagonists, D2 partial agonists, PDE10 antagonists, 5-HT_{2A} antagonists, 5-HT₆ antagonists and KCNQ4 antagonists

11. A pharmaceutical composition comprising a compound according to any of claims 1-10 and one or more pharmaceutically acceptable carrier or excipient.

12. A composition according to claim 11 which composition additionally comprises a compound selected from the list consisting of D2 antagonists, D2 partial agonists, PDE10 antagonists, 5 -HT_{2A} antagonists, 5-HT₆ antagonists and KCNQ4 antagonists.

13. A kit comprising a compound according to any of claims 1-12 together with a compound selected from the list consisting of D2 antagonists, D2 partial agonists, PDE10 antagonists, 5-HT_{2A} antagonists, 5-HT₆ antagonists and KCNQ4 antagonists.

## Patentansprüche

1. Verbindung nach Formel I 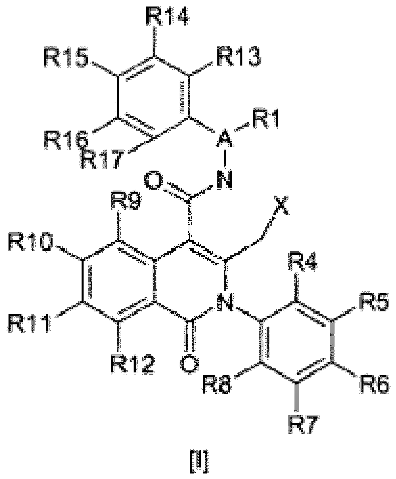 wobei A N, CH oder CR¹ darstellt;
wobei R¹ unabhängig Wasserstoff, C₁₋₆-Alkyl, C₂₋₆-Alkenyl, C₂₋₆ -Alkynyl, -C(O)-C₁₋₆-Alkyl, -C(O)-C₂₋₆-Alkenyl, -C(O)-C₂₋₆-Alkynyl, -C(O)-O-C₁₋₆-Alkyl, -C(O)-O-C₂₋₆-Alkenyl, -C(O)-O-C₂₋₆-Alkynyl oder Phenyl darstellt, wobei das Phenyl, C₁₋₆-Alkyl, C₂₋₆-Alkenyl oder C₂₋₆-Alkynyl gegebenenfalls mit einem oder mehr Substituenten substituiert ist, die aus Halogen, Hydroxyl, Halo-C₁₋₆-alkyl, Nitro, C₁₋₆-Alkoxy und NR²R³ ausgewählt sind; wobei X Wasserstoff, C₁₋₆-Alkyl, C₂₋₆-Alkenyl, C₂₋₆-Alkynyl, Cyan, -OR², -O-C(O)R², -OC(O)NR²R³, -C(O)-NR²R³, -N(R²)C(O)R³, -N(R²)-C(O)NR²R³ oder NR²R³ darstellt, oder X einen monocyclischen, bicyclischen oder tricyclischen Rest mit 4 bis 16 Ringatomen darstellt, von welchen eines Stickstoff ist, und wobei ein, zwei oder drei zusätzliche Ringatome ein Heteroatom sein können, das aus N, O und S ausgewählt ist, und wobei der monocyclische, bicyclische oder tricyclische Rest gegebenenfalls mit einem oder mehreren Substituenten W substituiert sein kann, wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₂₋₆ -Alkenyl, C₂₋₆-Alkynyl, C₁₋₆-Alkoxy, -C(O)-C₁₋₆-Alkyl, -C(O)-C₂₋₆-Alkenyl, - C(O)-C₂₋₆-Alkynyl, -C(O)-O-C₁₋₆-Alkyl, -C(O)-O-C₂₋₆-Alkenyl, -C(O)-O-C₂₋₆-Alkynyl, -O-C(O)-C₁₋₆-Alkyl, -O-C(O)-C₂₋₆-Alkenyl, -O-C(O)-C₂₋₆-Alkynyl, - C(O)H, COOH ausgewählt ist; oder wobei W einen Rest der Formel - (CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, -NR²-, - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertes Phenyl ausgewählt sind;
wobei R⁴ bis R⁸, R⁹ bis R¹² und R¹³ bis R¹⁷ unabhängig Wasserstoff, C₁₋₆-Alkyl, C₂₋₆-Alkenyl, C₂₋₆-Alkynyl, Halogen, NR²R³, Hydroxy, Cyan, Nitro, C₁₋₆-Alkoxy, Halo-C₁₋₆-alkyl oder Hydroxy-C₁₋₆-alkyl darstellen;
wobei R² und R³ unabhängig Wasserstoff, C₁₋₆-Alkyl, C₂₋₆-Alkenyl, C₂₋₆-Alkynyl, Hydroxy-C₁₋₆-alkyl, HalO-C₁₋₆-alkyl oder Phenyl darstellen;
und pharmazeutisch unbedenkliche Salze davon.

2. Verbindung nach Anspruch 1, wobei R⁴ bis R⁸ Wasserstoff darstellen.

3. Verbindung nach Anspruch 1, wobei R⁹ bis R¹² Wasserstoff darstellen.

4. Verbindung nach Anspruch 1, wobei R¹³ bis R¹⁷ Wasserstoff darstellen.

5. Verbindung nach Anspruch 1 der
- Formel Iₐ 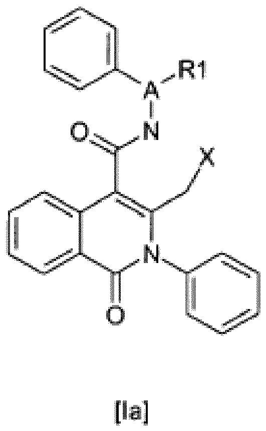 wobei A N, CH oder CR¹ darstellt;
wobei R¹ jeweils unabhängig Wasserstoff, C₁₋₆-Alkyl, -C(O)-C₁₋₆ -Alkyl, -C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei X Wasserstoff, C₁₋₆-Alkyl, Cyan, -OR², -O-C(O)R², - OC(O)NR²R³, C(O)-NR²R³, -N(R²)C(O)R³, -N(R²)-C(O)NR²R³ oder NR²R³ darstellt, oder X einen monocyclischen, bicyclischen oder tricyclischen Rest mit 4 bis 16 Ringatomen darstellt, von welchen eines Stickstoff ist,
und wobei ein, zwei oder drei zusätzliche Ringatome ein Heteroatom sein können, das aus N, O und S ausgewählt ist, und wobei der monocyclische, bicyclische oder tricyclische Rest gegebenenfalls mit einem oder mehreren Substituenten W substituiert sein kann, wobei W aus Wasserstoff, Hydroxy, Halogen, Cyan, (=O), -OP-(CH²)_{c}-O-, wobei c für 2 oder 3 (Spiro) steht, (CH₂)_{d}, wobei d für 5 oder 6 (Spiro) steht, C₁₋₆-Alkyl, C₁₋₆ -Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, -NR²-, - NR²-C(O)-, - C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R⁴ bis R⁸, R⁹ bis R¹² und R¹³ bis R¹⁷ jeweils unabhängig Wasserstoff, C₁₋₆-Alkyl, C₂₋₆-Alkenyl, C₂₋₆-Alkynyl, Halogen, NR²R³, Hydroxy, Cyan, Nitro, C₁₋₆-Alkoxy, Halo-C₁₋₆-alkyl oder Hydroxy-C₁₋₆-alkyl darstellen;
wobei R² und R³ unabhängig Wasserstoff, C₁₋₆-Alkyl, C₂₋₆-Alkenyl, C₂₋₆-Alkynyl, Hydroxy-C₁₋₆-alkyl, Halo-C₁₋₆-alkyl oder Phenyl darstellen;
und pharmazeutisch unbedenkliche Salze davon; oder der
- Formel I_{b} 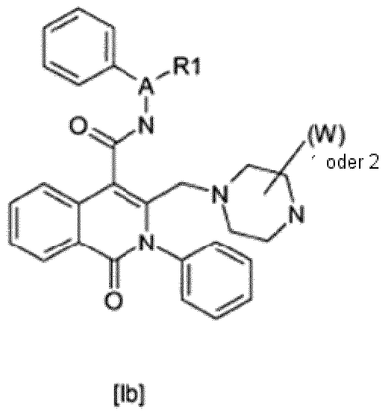 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, -NR²-, - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel I_{c} 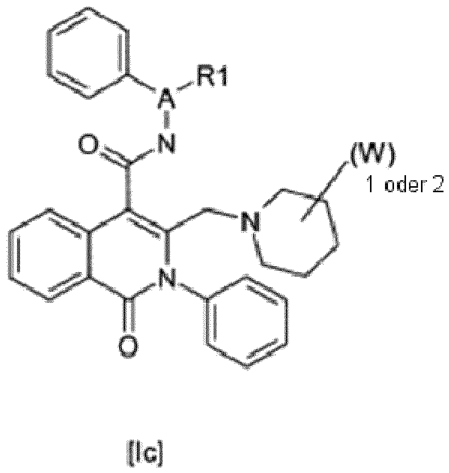 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, -NR²-, - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel I_{d} 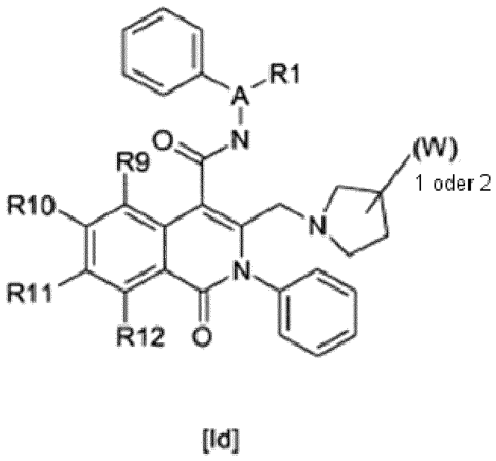 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁-₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist;
wobei R² und R³ unabhängig Wasserstoff, C₁₋₆-Alkyl, Hydroxy-C₁₋₆-alkyl, Halo-C₁₋₆-alkyl oder Phenyl darstellen;
wobei R⁹ bis R¹² unabhängig Wasserstoff oder Halogen darstellen;
und pharmazeutisch unbedenkliche Salze davon; oder der
- Formel Iₑ 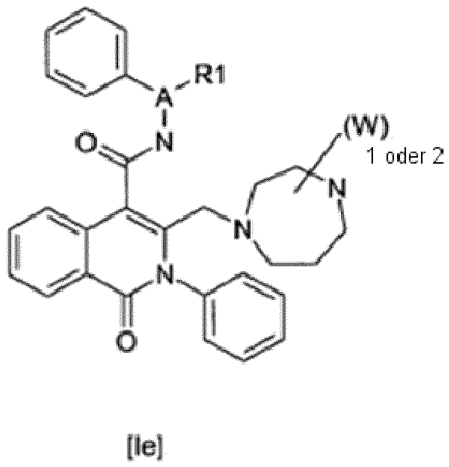 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy, und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, NR², - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel I_{f} 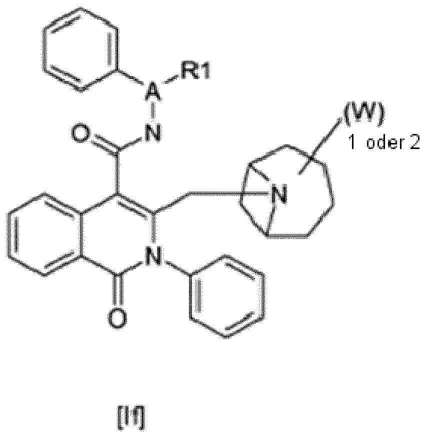 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, NR², -NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel I_{g} 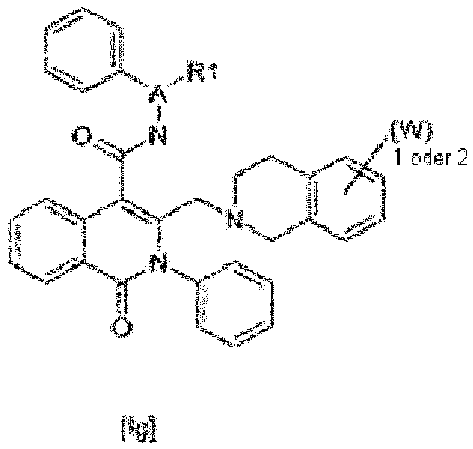 wobei A N, CH oder CR¹;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -N(R²)-C(O)-R³, -C(O)H, COOH ausgewählt ist; oder wobei W einen Rest der Formel -(CH₂)ₐ-Y-(CH2)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, NR², -NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierten bicyclischen Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel Iₕ 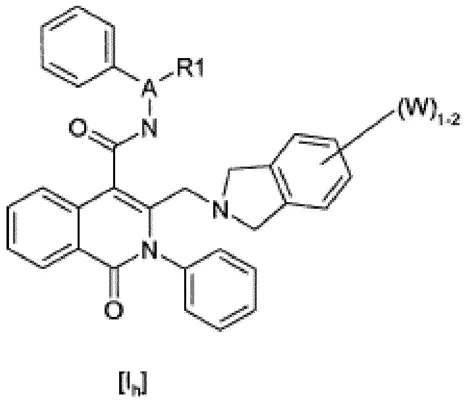 wobei A N, CH oder CR¹ darstellt;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, NR², -NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel Iᵢ 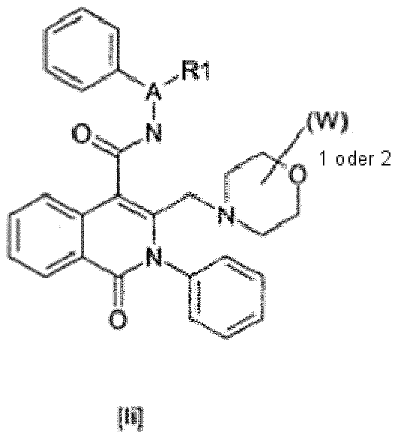 wobei A N, CH oder CR¹;
wobei R¹ jeweils unabhängig Wasserstoff, C₁₋₆-Alkyl, -C(O)-C₁₋₆ -Alkyl, C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy, und NR²R³ ausgewählt sind;
wobei W aus Wasserstoff, Hydroxy, Halogen, Cyan, (=O), -O-(CH₂)_{c}-O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei X einen Rest der Formel -(CH₂)ₐ-Y-(CH2)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, NR², - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Pyrazinyl, Phenyl, Pyridyl und halogensubstituiertem Phenyl ausgewählt sind;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel Ij 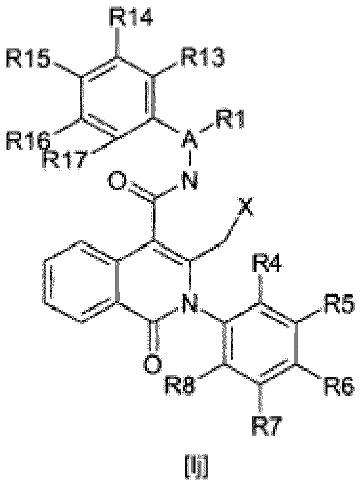 wobei A N, CH oder CR¹ darstellt;
wobei R¹ jeweils unabhängig Wasserstoff, C₁₋₆-Alkyl, -C(O)-C₁₋₆ -Alkyl, -C(O)-O-C₁₋₆-Alkyl oder Phenyl darstellt, wobei das Phenyl oder C₁₋₆-Alkyl gegebenenfalls mit einem oder mehreren Substituenten substituiert ist, die aus Halogen, Hydroxyl, Halo-C₁₋₆-alkyl, Nitro, C₁₋₆-Alkoxy und NR²R³ ausgewählt sind;
wobei X Wasserstoff, C₁₋₆-Alkyl, Cyan, -OR², -O-C(O)R², -OC(O)NR²R³, -C(O)-NR²R³, -N(R²)C(O)R³, -N(R²)-C(O)NR²R³ oder NR²R³ darstellt, oder X einen monocyclischen, bicyclischen oder tricyclischen Rest mit 4 bis 16 Ringatomen darstellt, von welchen eines Stickstoff ist, und wobei ein, zwei oder drei zusätzliche Ringatome ein Heteroatom sein können, das aus N, O und S ausgewählt ist, und wobei der monocyclische, bicyclische oder tricyclische Rest gegebenenfalls mit einem oder mehreren Substituenten W substituiert sein kann, wobei W aus Wasserstoff, Hydroxy, Halogen, Cyan, (=O), -O-(CH₂)_{c} O-, wobei c für 2 oder 3 (Spiro) steht, (CH₂)_{d}, wobei d für 5 oder 6 (Spiro) steht, C₁₋₆-Alkyl, C₁₋₆-Alkoxy, -C(O)-C₁₋₆-Alkyl, -C(O)-O-C₁₋₆-Alkyl, -O-C(O)-C₁₋₆-Alkyl, -C(O)H, COOH ausgewählt ist; oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung, C(O), O, S, -O-C(O), -C(O)-O-, -NR²-, - NR²-C(O)-, -C(O)-NH- oder S(O)₂ darstellt;
wobei Z Wasserstoff, C₁₋₆-Alkyl, NR²R³, Cyan oder einen monocyclischen oder fusionierten bicyclischen Rest mit 4 bis 12 Ringatomen darstellt, von welchen eines, zwei oder drei gegebenenfalls Heteroatome sind, die aus N, O und S ausgewählt sind, und wobei der monocyclische oder fusionierte bicyclische Rest gegebenenfalls mit einem oder mehreren Substituenten substituiert ist, die aus der Gruppe bestehend aus Halogen, Cyan, C₁₋₆-Alkyl, Hydroxyl und C₁₋₆-Alkoxy, Phenyl, Pyridyl und halogensubstituiertem Phenyl ausgewählt sind;
wobei eines von R⁴ bis R⁸ Halogen darstellt und die anderen Wasserstoff darstellen; wobei eines von R¹³ bis R¹⁷ Halogen darstellt und die anderen Wasserstoff darstellen;
wobei R² und R³ unabhängig Wasserstoff, C₁₋₆-Alkyl, Hydroxy-C₁₋₆-alkyl, Halo-C₁₋₆-alkyl oder Phenyl darstellen;
und pharmazeutisch unbedenkliche Salze davon; oder der
- Formel Ik 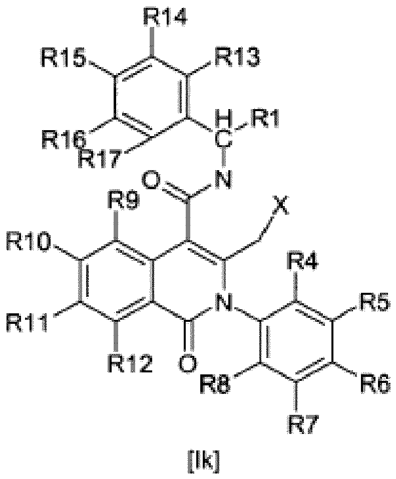 wobei R¹ Wasserstoff, C₁₋₆-Alkyl, C₂₋₆-Alkenyl oder C₂₋₆-Alkynyl darstellt;
wobei X Wasserstoff, Cₗ-₆-Alkyl, C₂-₆-Alkenyl, C₂-₆-Alkynyl oder NR²R³ darstellt, oder X einen monocyclischen oder bicyclischen Rest mit 5 bis 9 Ringatomen darstellt, von welchen eines Stickstoff ist, und wobei ein, zwei oder drei zusätzliche Ringatome ein Heteroatom sein können, die aus N, O und S ausgewählt sind, und wobei der monocyclische oder bicyclische Rest gegebenenfalls mit einem oder mehreren Substituenten W substituiert sein kann, wobei W aus Wasserstoff, Halogen, Hydroxy, (=O), C₁₋₆-Alkyl, C₂₋₆ -Alkenyl, C₂₋₆-Alkynyl, C₁₋₆-Alkoxy ausgewählt ist, oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung darstellt, und wobei Z einen monocyclischen Rest mit 5 bis 6 Ringatomen darstellt, von welchen eines, zwei oder drei gegebenenfalls Heteroatome sind, die aus N, O und S ausgewählt sind, und wobei der monocyclische Rest gegebenenfalls mit einem oder mehreren Substituenten substituiert ist, die aus der Gruppe bestehend aus Halogen, C₁₋₆-Alkyl, Hydroxyl und C₁₋₆-Alkoxy ausgewählt sind;
wobei R⁴ bis R⁸ jeweils unabhängig Wasserstoff oder Halogen darstellen;
wobei R⁹ bis R¹² jeweils unabhängig Wasserstoff oder Halogen darstellen, vorausgesetzt, dass mindestens eines von R⁹ bis R¹² Halogen darstellt;
wobei R¹³ bis R¹⁷ jeweils unabhängig Wasserstoff oder Halogen darstellen;
wobei R² und R³ unabhängig Wasserstoff, C₁₋₆-Alkyl, C₂₋₆-Alkenyl, C₂₋₆-Alkynyl darstellen; und pharmazeutisch unbedenkliche Salze davon; oder der
- Formel Ik' 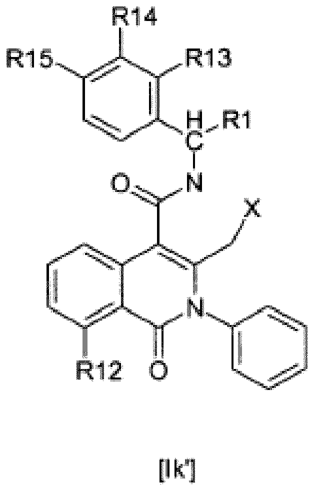 wobei R¹ aus C₁₋₆-Alkyl ausgewählt ist;
wobei X aus Wasserstoff, C₁₋₆-Alkyl oder NR²R³ ausgewählt ist, oder X einen monocyclischen oder bicyclischen Rest mit 5 bis 9 Ringatomen darstellt, von welchen eines Stickstoff ist, und wobei ein zusätzliches Ringatom ein Heteroatom sein kann, das aus N ausgewählt ist, und wobei der monocyclische oder bicyclische Rest gegebenenfalls mit einem oder mehreren Substituenten W substituiert sein kann, wobei W aus Wasserstoff, Hydroxy, (=O), C₁₋₆-Alkyl ausgewählt ist, oder wobei W einen Rest der Formel -(CH₂)ₐ-Y-(CH₂)_{b}-Z darstellt;
wobei a und b unabhängig eine ganze Zahl darstellen, die aus 0, 1, 2 und 3 ausgewählt ist;
wobei Y eine Bindung darstellt, und wobei Z einen monocyclischen Rest mit 5 bis 6 Ringatomen darstellt, von welchen eines gegebenenfalls ein Heteroatom ist, das aus N, O und S ausgewählt ist, und wobei der monocyclische Rest gegebenenfalls mit einem oder mehreren Substituenten substituiert ist, die aus der Gruppe bestehend aus Halogen, C₁₋₆-Alkyl, Hydroxyl und C₁₋₆-Alkoxy ausgewählt sind;
wobei R¹² Halogen darstellt;
R¹³ bis R¹⁵ jeweils unabhängig Wasserstoff oder Halogen darstellen;
und pharmazeutisch unbedenkliche Salze davon; oder der
- Formel 1 k" 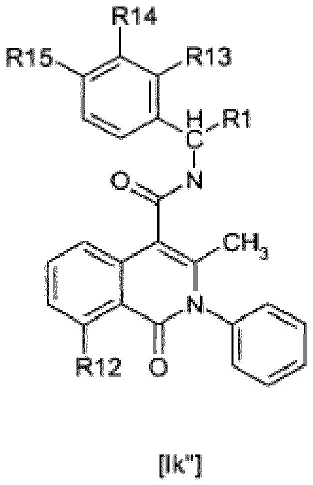 wobei R¹ Ethyl, Cylopropyl oder Cyclobutyl darstellt; wobei R¹² Chlor darstellt; und
R¹³, R¹⁴ und R¹⁵ jeweils individuell Wasserstoff, Fluor oder Chlor darstellen, wobei zwei von R¹³, R¹⁴ und R¹⁵ Wasserstoff darstellen; oder
- im Wesentlichen die S-Enantiomere wie dargestellt in Formel Ik"' 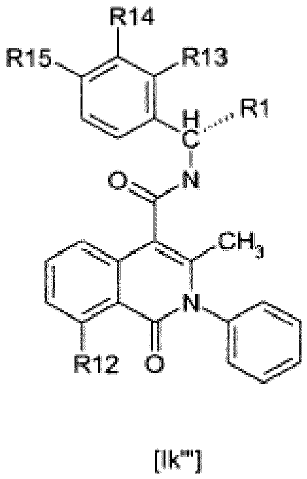 wobei R¹ Ethyl oder Cylopropyl darstellt;
wobei R¹² Chlor darstellt; und
R¹³, R¹⁴ und R¹⁵ jeweils individuell Wasserstoff, Fluor oder Chlor darstellen, wobei zwei von R¹³, R¹⁴ und R¹⁵ Wasserstoff darstellen; oder der
- Formel Id' 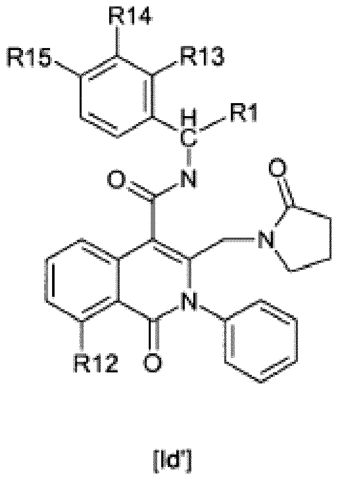 wobei R¹ Ethyl oder Cyclopropyl darstellt;
R¹² Halogen darstellt;
R¹³, R¹⁴ und R¹⁵ jeweils unabhängig Wasserstoff oder Halogen darstellen;
und pharmazeutisch unbedenkliche Salze davon; oder der
- Formel Id" 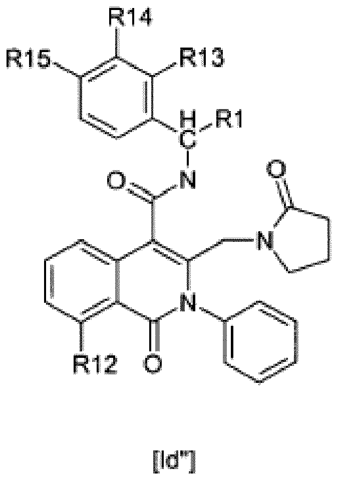 wobei R¹ Ethyl oder Cyclopropyl darstellt;
R¹² Chlor oder Fluor darstellt;
R¹² , R¹⁴ und R¹⁵ jeweils unabhängig Wasserstoff, Chlor oder Fluor darstellen, wobei zwei von R¹² , R¹⁴ und R¹⁵ Wasserstoff darstellen;
und pharmazeutisch unbedenkliche Salze davon; oder
- im Wesentlichen das S-Enantiomer wie in Formel Id"' dargestellt, wobei "im Wesentlichen" einen Enantiomerenüberschuss von mindestens 60 % definiert, 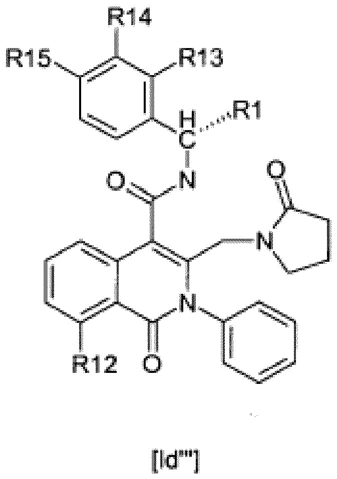 wobei R¹ Ethyl oder Cyclopropyl darstellt;
R¹² Chlor oder Fluor darstellt;
R¹² , R¹⁴ und R¹⁵ jeweils unabhängig Wasserstoff, Chlor oder Fluor darstellen, wobei zwei von R¹² , R¹⁴ und R¹⁵ Wasserstoff darstellen;
und pharmazeutisch unbedenkliche Salze davon.

6. Verbindung nach Anspruch 1, ausgewählt aus:
1a 3-Methyl-1-oxo-2-phenyl-1,2-dihydroisochinolin-4-carbonsäure ((S)-1-phenylpropyl)-amid
1 b 3,6-Dimethyl-1-oxo-2-phenyl-1,2-dihydroisochinolin-4-carbonsäure ((S)-1-phenylpropyl)-amid
1c 7-Chlor-3-methyl-1-oxo-2-phenyl-1,2-dihydroisochinolin-4-carbonsäure ((S)-1-phenylpropyl)-amid
1d 7-Brom-3-methyl-1-oxo-2-phenyl-1,2-dihydroisochinolin-4-carbonsäure ((S)-1-phenylpropyl)-amid
1e 6-Fluor-3-methyl-1-oxo-2-phenyl-1,2-dihydroisochinolin-4-carbonsäure ((S)-1-phenylpropyl)-amid
1f f 3,5-Dimethyl-1-oxo-2-phenyl-1,2-dihydroisochinolin-4-carbonsäure ((S)-1-phenylpropyl)-amid
1g 7-Fluor-3-methyl-1-oxo-2-phenyl-1,2-dihydroisochinolin-4-carbonsäure ((S)-1-phenylpropyl)-amid
1 h 3,7-Dimethyl-1 -oxo-2-phenyl-1,2-dihydroisochinolin-4-carbonsäure ((S)-1 -phenylpropyl)-amid
1i 8-Chlor-3-methyl-1-oxo-2-Phenyl-1,2-dihydroisochinolin-4-carbonsäure ((S)-1-phenylpropyl)-amid
1 r 3-Methyl-1-oxo-2-phenyl-1,2-dihydroisochinolin-4-carbonsäure ((S)-cyclopropylphenylmethyl)-amid
1s 3-Methyl-1-oxo-2-phenyl-1,2-dihydroisochinolin-4-carbonsäure [(S)-cyclopropyl-(3-fluorphenyl)-methyl]-amid
1j 3,8-Dimethyl-1 -oxo-2-phenyl-1,2-dihydroisochinolin-4-carbonsäure ((S)-1-phenylpropyl)-amid
1 k 2-(2-Fluorphenyl)-3-methyl-1-oxo-1,2-dihydroisochinolin-4-carbonsäure ((S)-1-phenylpropyl)-amid
1l 3-Methyl-1-oxo-2-o-tolyl-1,2-dihydroisochinolin-4-carbonsäure ((S)-1-phenylpropyl)-amid
1 m 2-(2-Chlorphenyl)-3-methyl-1-oxo-1,2-dihydroisochinolin-4-carbonsäure ((S)-1-phenylpropyl)-amid
1 n 2-(2,6-Difluorphenyl)-3-methyl-1-oxo-1,2-dihydroisochinolin-4-carbonsäure ((S)-1-phenylpropyl)-amid
1o 2-(3-Fluorphenyl)-3-methyl-1-oxo-1,2-dihydroisochinolin-4-carbonsäure ((S)-1-phenylpropyl)-amid
1 p 3-Methyl-1-oxo-2-m-tolyl-1,2-dihydroisochinolin-4-carbonsäure ((S)-1-phenylpropyl)-amid
1q 2-(3-Chlorphenyl)-3-methyl-1-oxo-1,2-dihydroisochinolin-4-carbonsäure ((S)-1-phenylpropyl)-amid
1t 8-Chlor-3-methyl-1-oxo-2-phenyl-1,2-dihydroisochinolin-4-carbonsäure [(S)-cyclopropyl-(3-fluorphenyl)-methyl]-amid
2a 3-Dimethylaminomethyl-1-oxo-2-Phenyl-1,2-dihydroisochinolin-4-carbonsäure ((S)-1-phenylpropyl)-amid
2b 3-Methylaminomethyl-1-oxo-2-phenyl-1,2-dihydroisochinolin-4-carbonsäure ((S)-1-phenylpropyl)-amid
2c 3-Ethylaminomethyl-1-oxo-2-phenyl-1,2-dihydroisochinolin-4-carbonsäure ((S)-1-phenylpropyl)-amid
2d 3-Cyclopropylaminomethyl-1-oxo-2-phenyl-1,2-dihydroisochinolin-4-carbonsäure ((S)-1-phenylpropyl)-amid
2e 3-[(Cyclopropylmethylamino)-methyl]-1-oxo-2-phenyl-1,2-dihydroisochinolin-4-carbonsäure ((S)-1-phenylpropyl)-amid
2f 3-(3,6-Dihydro-2H-pyridin-1-ylmethyl)-1-oxo-2-phenyl-1,2-dihydroisochinolin-4-carbonsäure ((S)-1-phenylpropyl)-amid
2g 3-[4-(2-Methoxyphenyl)-piperazin-1-ylmethyl]-1-oxo-2-phenyl-1,2-dihydroisochinolin-4-carbonsäure ((S)-1-phenylpropyl)-amid
2h 3-[4-(4-Fluorphenyl)-piperazin-1-ylmethyl]-1-oxo-2-phenyl-1,2-dihydroisochinolin-4-carbonsäure ((S)-1-phenylpropyl)-amid
2i 3-(4-Formylpiperazin-1-ylmethyl)-1-oxo-2-phenyl-1,2-dihydroisochinolin-4-carbonsäure ((S)-1-phenylpropyl)-amid
2j 4-[1-Oxo-2-phenyl-4-(1-phenylpropylcarbamoyl)-1,2-dihydroisochinolin-3-ylmethyl]-piperazine-1-carbonsäureethylester
2k 3-(4-Methylpiperazin-1-ylmethyl)-1-oxo-2-phenyl-1,2-dihydroisochinolin-4-carbonsäure ((S)-1-phenylpropyl)-amid
2l 1-Oxo-2-phenyl-3-(1,3,4,9-tetrahydro-beta-carbolin-2-ylmethyl)-1,2-dihydroisochinolin-4-carbonsäure ((S)-1-phenylpropyl)-amid
2m 1-Oxo-2-phenyl-3-piperazin-1-ylmethyl-1,2-dihydroisochinolin-4-carbonsäure ((S)-1-phenylpropyl)-amid
2n 3-(3-Methylpiperazin-1-ylmethyl)-1-oxo-2-phenyl-1,2-dihydroisochinolin-4-carbonsäure ((S)-1-phenylpropyl)-amid
2o 3-(3,5-Dimethylpiperazin-1-ylmethyl)-1-oxo-2-phenyl-1,2-dihydroisochinolin-4-carbonsäure ((S)-1-phenylpropyl)-amid
2p 3-(4-Benzylpiperazin-1-ylmethyl)-1-oxo-2-phenyl-1,2-dihydroisochinolin-4-carbonsäure ((S)-1-phenylpropyl)-amid
2q 1-Oxo-3-[4-(2-oxo-2-pyrrolidin-1-yl-ethyl)-piperazin-1-ylmethyl]-2-phenyl-1,2-dihydroisochinolin-4-carbonsäure ((S)-1-phenylpropyl)-amid
2r 3-Morpholin-4-ylmethyl-1-oxo-2-phenyl-1,2-dihydroisochinolin-4-carbonsäure ((S)-1-phenylpropyl)-amid
2s 3-(2,6-Dimethylmorpholin-4-ylmethyl)-1-oxo-2-phenyl-1,2-dihydroisochinolin-4-carbonsäure ((S)-1-phenylpropyl)-amid
2t 1-Oxo-2-phenyl-3-thiomorpholin-4-ylmethyl-1,2-dihydroisochinolin-4-carbonsäure ((S)-1-phenylpropyl)-amid
2u 3-(1,4-Dioxa-8-azaspiro[4.5]dec-8-ylmethyl)-1-oxo-2-phenyl-1,2-dihydroisochinolin-4-carbonsäure ((S)-1-phenylpropyl)-amid
2v 1-Oxo-2-phenyl-3-piperidin-1-ylmethyl-1,2-dihydroisochinolin-4-carbonsäure ((S)-1-phenylpropyl)-amid
2w 3-(2-Methylpiperidin-1-ylmethyl)-1-oxo-2-phenyl-1,2-dihydroisochinolin-4-carbonsäure ((S)-1-phenylpropyl)-amid
2x 3-(2,6-Dimethylpiperidin-1-ylmethyl)-1-oxo-2-phenyl-1,2-dihydroisochinolin-4-carbonsäure ((S)-1-phenylpropyl)-amid
2y 3-(2-Hydroxymethylpiperidin-1-ylmethyl)-1-oxo-2-phenyl-1,2-dihydroisochinolin-4-carbonsäure ((S)-1-phenylpropyl)-amid
2z 1-[1-Oxo-2-phenyl-4-(1-phenylpropylcarbamoyl)-1,2-dihydroisochinolin-3-ylmethyl]-piperidin-3-carbonsäureethylester
2aa 3-(3-Methylpiperidin-1-ylmethyl)-1-oxo-2-phenyl-1,2-dihydroisochinolin-4-carbonsäure ((S)-1-phenylpropyl)-amid
2ab 3-(4-Hydroxypiperidin-1-ylmethyl)-1-oxo-2-Phenyl-1,2-dihydroisochinolin-4-carbonsäure ((S)-1-phenylpropyl)-amid
2ac 1-Oxo-2-phenyl-3-(4-phenylpiperidin-1-ylmethyl)-1,2-dihydroisochinolin-4-carbonsäure ((S)-1-phenylpropyl)-amid
2ad 1-[1-Oxo-2-phenyl-4-(1-phenylpropylcarbamoyl)-1,2-dihydroisochinolin-3-ylmethyl]-piperidin-4-carbonsäureethylester
2ae 3-(4-Methylpiperidin-1-ylmethyl)-1-oxo-2-phenyl-1,2-dihydroisochinolin-4-carbonsäure ((S)-1-phenylpropyl)-amid
2af 1-Oxo-2-phenyl-3-(4-pyridin-2-yl-piperazin-1-ylmethyl)-1,2-dihydroisochinolin-4-carbonsäure ((S)-1-phenylpropyl)-amid
2ag 3-(Octahydrochinolin-1-ylmethyl)-1-oxo-2-phenyl-1,2-dihydroisochinolin-4-carbonsäure ((S)-1-phenylpropyl)-amid
2ah 3-Azepan-1-ylmethyl-1-oxo-2-Phenyl-1,2-dihydroisochinolin-4-carbonsäure ((S)-1-phenylpropyl)-amid
2ai 3-(3-Hydroxypiperidin-1-ylmethyl)-1-oxo-2-phenyl-1,2-dihydroisochinolin-4-carbonsäure ((S)-1-phenylpropyl)-amid
2aj 3-[4-(2,4-Dimethylphenyl)-piperazin-1-ylmethyl]-1-oxo-2-phenyl-1,2-dihydroisochinolin-4-carbonsäure ((S)-1-phenylpropyl)-amid
2ak 3-[4-(3,4-Dimethylphenyl)-piperazin-1-ylmethyl]-1-oxo-2-phenyl-1,2-dihydroisochinolin-4-carbonsäure ((S)-1-phenylpropyl)-amid
2al 3-(4-Dimethylam inopiperid in-1-ylmethyl)-1-oxo-2-phenyl-1,2-dihydroisochinolin-4-carbonsäure ((S)-1-phenylpropyl)-amid
2am 3-[4-(2,5-Dimethylphenyl)-piperazin-1-ylmethyl]-1-oxo-2-phenyl-1,2-dihydroisochinolin-4-carbonsäure ((S)-1-phenylpropyl)-amid
2an 3-[4-(2-Fluorphenyl)-piperazin-1-ylmethyl]-1-oxo-2-phenyl-1,2-dihydroisochinolin-4-carbonsäure ((S)-1-phenylpropyl)-amid
2ao 3-[4-(3-Methoxyphenyl)-piperazin-1-ylmethyl]-1-oxo-2-phenyl-1,2-dihydroisochinolin-4-carbonsäure ((S)-1-phenylpropyl)-amid
2ap 1-Oxo-2-phenyl-3-(4-m-tolyl-piperazin-1-ylmethyl)-1,2-dihydroisochinolin-4-carbonsäure ((S)-1-phenylpropyl)-amid
2aq 3-[4-(4-Methoxyphenyl)-piperazin-1-ylmethyl]-1-oxo-2-phenyl-1,2-dihydroisochinolin-4-carbonsäure ((S)-1-phenylpropyl)-amid
2ar 1 -Oxo-3-(4-phenethylpiperazin-1-ylmethyl)-2-phenyl-1,2-dihydroisochinolin-4-carbonsäure ((S)-1-phenylpropyl)-amid
2as 1-Oxo-2-phenyl-3-(4-pyrimidin-2-yl-piperazin-1-ylmethyl)-1,2-dihydroisochinolin-4-carbonsäure ((S)-1-phenylpropyl)-amid
2at 3-[4-(2-Cyanphenyl)-piperazin-1-ylmethyl]-1-oxo-2-phenyl-1,2-dihydroisochinolin-4-carbonsäure ((S)-1-phenylpropyl)-amid
2au 3-[4-(4-Chlorphenyl)-piperazin-1-ylmethyll-1-oxo-2-phenyl-1,2-dihydroisochinolin-4-carbonsäure ((S)-1-phenylpropyl)-amid
2av 3-((1S,3R,5R)-3-Hydroxy-8-azabicyclo[3.2.1]oct-8-ylmethyl)-1-oxo-2-phenyl-1,2-dihydroisochinolin-4-carbonsäure ((S)-1-phenylpropyl)-amid
2aw 3-(4-Acetylpiperazin-1-ylmethyl)-1-oxo-2-phenyl-1,2-dihydroisochinolin-4-carbonsäure ((S)-1-phenylpropyl)-amid
2ax 3-(4-Methyl-[1,4]diazepan-1-ylmethyl)-1-oxo-2-phenyl-1,2-dihydroisochinolin-4-carbonsäure ((S)-1-phenylpropyl)-amid
2ay 3-(4-Ethylpiperazin-1-ylmethyl)-1-oxo-2-phenyl-1,2-dihydroisochinolin-4-carbonsäure ((S)-1-phenylpropyl)-amid
2az 3-((2S,6R)-2,6-Dimethylmorpholin-4-ylmethyl)-1-oxo-2-phenyl-1,2-dihydroisochinolin-4-carbonsäure ((S)-1-phenylpropyl)-amid
2ba 3-[4-(2,4-Difluorphenyl)-piperazin-1-ylmethyl]-1-oxo-2-phenyl-1,2-dihydroisochinolin-4-carbonsäure ((S)-1-phenylpropyl)-amid
2bb 3-[4-(3-Dimethylaminopropyl)-piperazin-1-ylmethyl]-1-oxo-2-phenyl-1,2-dihydroisochinolin-4-carbonsäure ((S)-1-phenylpropyl)-amid
2bc 3-(4-Isopropylpiperazin-1-ylmethyl)-1-oxo-2-phenyl-1,2-dihydroisochinolin-4-carbonsäure ((S)-1-Phenyl-propyl)-amid
2bd 3-(3-Azabicyclo[3.2.2]non-3-ylmethyl)-1-oxo-2-phenyl-1,2-dihydroisochinolin-4-carbonsäure ((S)-1-phenylpropyl)-amid
2be 3-(4-Cyclopentylpiperazin-1-ylmethyl)-1-oxo-2-phenyl-1,2-dihydroisochinolin-4-carbonsäure ((S)-1-phenylpropyl)-amid
2bf 3-[1,4']Bipiperidinyl-1'-ylmethyl-1-oxo-2-phenyl-1,2-dihydroisochinolin-4-carbonsäure ((S)-1-phenylpropyl)-amid
2bg 3-(3,4-Dihydro-1H-isochinolin-2-ylmethyl)-1-oxo-2-phenyl-1,2-dihydroisochinolin-4-carbonsäure ((S)-1-phenylpropyl)-amid
2bh 3-[4-(2-Dimethylaminoethyl)-piperazin-1-ylmethyl]-1-oxo-2-phenyl-1,2-dihydroisochinolin-4-carbonsäure ((S)-1-phenylpropyl)-amid
2bi 3-(4-Hydroxymethylpiperidin-1-ylmethyl)-1-oxo-2-phenyl-1,2-dihydroisochinolin-4-carbonsäure ((S)-1-phenylpropyl)-amid
2bj 1-Oxo-2-phenyl-3-[4-(tetrahydrofuran-2-carbonyl)-piperazin-1-ylmethyl]-1,2-dihydroisochinolin-4-carbonsäure ((S)-1-phenylpropyl)-amid
2bk 3-(4-Isobutylpiperazin-1-ylmethyl)-1-oxo-2-phenyl-1,2-dihydroisochinolin-4-carbonsäure ((S)-1-phenylpropyl)-amid
2bl 3-[4-(2-Methoxyethyl)-piperazin-1-ylmethyl]-1-oxo-2-phenyl-1,2-dihydroisochinolin-4-carbonsäure ((S)-1-phenylpropyl)-amid
2bm 3-[4-(2-Morpholin-4-yl-ethyl)-piperazin-1-ylmethyl]-1-oxo-2-phenyl-1,2-dihydroisochinolin-4-carbonsäure ((S)-1-phenylpropyl)-amid
2bn 3-(1,3-Dihydroisoindol-2-ylmethyl)-1-oxo-2-phenyl-1,2-dihydroisochinolin-4-carbonsäure ((S)-1-phenylpropyl)-amid
2bo 3-[4-(1-Methylpiperidin-4-yl)-piperazin-1-ylmethyl]-1-oxo-2-phenyl-1,2-dihydroisochinolin-4-carbonsäure ((S)-1-phenylpropyl)-amid
2bp 1-Oxo-2-phenyl-3-[4-(2-piperidin-1-yl-ethyl)-piperazin-1-ylmethyl]-1,2-dihydroisochinolin-4-carbonsäure ((S)-1-phenylpropyl)-amid
2bq 1-Oxo-2-phenyl-3-[4-(2-pyrrolidin-1-yl-ethyl)-piperazin-1-ylmethyl]-1,2-dihydroisochinolin-4-carbonsäure ((S)-1-phenylpropyl)-amid
2br 3-(4-Dimethylcarbamoylmethylpiperazin-1-ylmethyl)-1-oxo-2-phenyl-1,2-dihydroisochinolin-4-carbonsäure ((S)-1-phenylpropyl)-amid
2bs 3-(Octahydropyrido[1,2-a]pyrazin-2-ylmethyl)-1-oxo-2-phenyl-1,2-dihydroisochinolin-4-carbonsäure ((S)-1-phenylpropyl)-amid
2bt 3-(4-Formyl-[1,4]diazepan-1-ylmethyl)-1-oxo-2-phenyl-1,2-dihydroisochinolin-4-carbonsäure ((S)-1-phenylpropyl)-amid
2bu 3-[4-(4-Cyanphenyl)-piperazin-1-ylmethyl]-1-oxo-2-phenyl-1,2-dihydroisochinolin-4-carbonsäure ((S)-1-phenylpropyl)-amid
2bv 1-Oxo-2-phenyl-3-(4-pyridin-4-ylmethyl-piperazin-1-ylmethyl)-1,2-dihydroisochinolin-4-carbonsäure ((S)-1-phenylpropyl)-amid
2bw 1-Oxo-2-phenyl-3-[4-(3-pyrrolidin-1-yl-propyl)-piperazin-1-ylmethyl]-1,2-dihydroisochinolin-4-carbonsäure ((S)-1-phenylpropyl)-amid
2bx 1-Oxo-2-phenyl-3-(4-pyridin-2-ylmethyl-piperazin-1-ylmethyl)-1,2-dihydroisochinolin-4-carbonsäure ((S)-1-phenylpropyl)-amid
2by 3-(4-Ethansulfonylpiperazin-1-ylmethyl)-1-oxo-2-phenyl-1,2-dihydroisochinolin-4-carbonsäure ((S)-1-phenylpropyl)-amid
2bz 3-(4-sec-Butylpiperazin-1-ylmethyl)-1-oxo-2-phenyl-1,2-dihydroisochinolin-4-carbonsäure ((S)-1-phenylpropyl)-amid
2ca 3-[4-(1-Ethylpropyl)-piperazin-1-ylmethyl]-1-oxo-2-Phenyl-1,2-dihydroisochinolin-4-carbonsäure ((S)-1-phenylpropyl)-amid
2cb 3-[4-(2-Cyanethyl)-piperazin-1-ylmethyl]-1-oxo-2-phenyl-1,2-dihydroisochinolin-4-carbonsäure ((S)-1-phenylpropyl)-amid
2cc 3-(4-Methansulfonylpiperazin-1-ylmethyl)-1-oxo-2-phenyl-1,2-dihydroisochinolin-4-carbonsäure ((S)-1-phenylpropyl)-amid
2cd {1-[1-Oxo-2-phenyl-4-(1-phenylpropylcarbamoyl)-1,2-dihydroisochinolin-3-ylmethyl]-piperidin-4-yl}-essigsäureethylester
2ce 3-[4-(3-Fluorphenyl)-piperazin-1-ylmethyl]-1-oxo-2-phenyl-1,2-dihydroisochinolin-4-carbonsäure ((S)-1-phenylpropyl)-amid
2cf 1-Oxo-2-phenyl-3-((S)-3-phenylpiperidin-1-ylmethyl)-1,2-dihydroisochinolin-4-carbonsäure ((S)-1-phenylpropyl)-amid
2cg 1-Oxo-2-phenyl-3-[4-(pyrrolidin-1-carbonyl)-piperazin-1-ylmethyl]-1,2-dihydroisochinolin-4-carbonsäure ((S)-1-phenylpropyl)-amid
2ch 3-[4-(Morpholin-4-carbonyl)-piperazin-1-ylmethyl]-1-oxo-2-phenyl-1,2-dihydroisochinolin-4-carbonsäure ((S)-1-phenylpropyl)-amid
2ci 3-(4-Methoxypiperidin-1-ylmethyl)-1-oxo-2-phenyl-1,2-dihydroisochinolin-4-carbonsäure ((S)-1-phenylpropyl)-amid
2cj 3-(4'-Hydroxy-3',4',5',6'-tetrahydro-2'H-[3,4']bipyridinyl-1'-ylmethyl)-1-oxo-2-phenyl-1,2-dihydroisochinolin-4-carbonsäure ((S)-1-phenylpropyl)-amid
2ck 3-(4-Hydroxy-3,4,5,6-tetrahydro-2H-[4,4']bipyridinyl-1-ylmethyl)-1-oxo-2-phenyl-1,2-dihydroisochinolin-4-carbonsäure ((S)-1-phenylpropyl)-amid
2cl 3-(3H-Spiro[isobenzofuran-1,4'-piperidin]-1'-ylmethyl)-1-oxo-2-phenyl-1,2-dihydroisochinolin-4-carbonsäure ((S)-1-phenylpropyl)-amid
2cm 3-(4-Hydroxy-4-methylpiperidin-1-ylmethyl)-1-oxo-2-phenyl-1,2-dihydroisochinolin-4-carbonsäure ((S)-1-phenylpropyl)-amid
2cn 3-(Hexahydrospiro[benzo[1,3]dioxol-2,4'-piperidin]-1'-ylmethyl)-1-oxo-2-phenyl-1,2-dihydroisochinolin-4-carbonsäure ((S)-1-phenylpropyl)-amid
2co 1-Oxo-2-phenyl-3-(3,4,5,6-tetrahydro-2H-[4,4']bipyridinyl-1-ylmethyl)-1,2-dihydroisochinolin-4-carbonsäure ((S)-1-phenylpropyl)-amid
2cp 3-[4-(2-Dimethylaminoethyl)-piperidin-1-ylmethyl]-1-oxo-2-phenyl-1,2-dihydroisochinolin-4-carbonsäure ((S)-1-phenylpropyl)-amid
2cq 3-(4-Dimethylsulfamoylpiperazin-1-ylmethyl)-1-oxo-2-phenyl-1,2-dihydroisochinolin-4-carbonsäure ((S)-1-phenylpropyl)-amid
2cr 3-(1,1-Dioxothiomorpholin-4-ylmethyl)-1-oxo-2-phenyl-1,2-dihydroisochinolin-4-carbonsäure ((S)-1-phenylpropyl)-amid
2cs 1-Oxo-2-phenyl-3-(2-pyridin-2-ylmethyl-piperidin-1-ylmethyl)-1,2-dihydroisochinolin-4-carbonsäure ((S)-1-phenylpropyl)-amid
2ct 3-(2-Morpholin-4-ylmethyl-piperidin-1-ylmethyl)-1-oxo-2-phenyl-1,2-dihydroisochinolin-4-carbonsäure ((S)-1-phenylpropyl)-amid
2cu 3-(4-Furo[3,2-c]pyridin-4-yl-piperazin-1-ylmethyl)-1-oxo-2-phenyl-1,2-dihydroisochinolin-4-carbonsäure ((S)-1-phenylpropyl)-amid
2cv 3-(4-Cyclopropylmethyl-piperazin-1-ylmethyl)-1-oxo-2-phenyl-1,2-dihydroisochinolin-4-carbonsäure ((S)-1-phenylpropyl)-amid
2cw 3-[4-(2-Morpholin-4-yl-ethyl)-piperidin-1-ylmethyl]-1-oxo-2-phenyl-1,2-dihydroisochinolin-4-carbonsäure ((S)-1-phenylpropyl)-amid
2cx 1-Oxo-2-phenyl-3-(4-pyrimidin-2-yl[1,4]diazepan-1-ylmethyl)-1,2-dihydroisochinolin-4-carbonsäure ((S)-1-phenylpropyl)-amid
2cy 3-(4-Methyl-6,7-dihydro-4H-thieno[3,2-c]pyridin-5-ylmethyl)-1-oxo-2-phenyl-1,2-dihydroisochinolin-4-carbonsäure ((S)-1-phenylpropyl)-amid
2cz 3-[1,4]Diazepan-1-ylmethyl-1-oxo-2-phenyl-1,2-dihydroisochinolin-4-carbonsäure ((S)-1-phenylpropyl)-amid
2da 3-((2S,5R)-2,5-Dimethylpiperazin-1-ylmethyl)-1-oxo-2-phenyl-1,2-dihydroisochinolin-4-carbonsäure ((S)-1-phenylpropyl)-amid
2db 3-((S)-3-Methylpiperazin-1-ylmethyl)-1-oxo-2-phenyl-1,2-dihydroisochinolin-4-carbonsäure ((S)-1-phenylpropyl)-amid
2dc 3-((R)-3-Methylpiperazin-1-ylmethyl)-1-oxo-2-phenyl-1,2-dihydroisochinolin-4-carbonsäure ((S)-1-phenylpropyl)-amid
2dd 3-[3-(3-Chlorphenyl)-piperazin-1-ylmethyl]-1-oxo-2-phenyl-1,2-dihydroisochinolin-4-carbonsäure ((S)-1-phenylpropyl)-amid
2de 3-[4-(1H-lndol-4-yl)-piperazin-1-ylmethyl]-1-oxo-2-phenyl-1,2-dihydroisochinolin-4-carbonsäure ((S)-1-phenylpropyl)-amid
2df 1-Oxo-3-(3-oxo-piperazin-1-ylmethyl)-2-phenyl-1,2-dihydroisochinolin-4-carbonsäure ((S)-1-phenylpropyl)-amid
2dg 3-[4-(1H-lndol-5-yl)-piperazin-1-ylmethyl]-1-oxo-2-phenyl-1,2-dihydroisochinolin-4-carbonsäure ((S)-1-phenylpropyl)-amid
2dh 3-(6,9-Diazaspiro[4.5]dec-9-ylmethyl)-1-oxo-2-phenyl-1,2-dihydroisochinolin-4-carbonsäure ((S)-1-phenylpropyl)-amid
2di 3-(1,4-Diazaspiro[5.5]undec-4-ylmethyl)-1-oxo-2-phenyl-1,2-dihydroisochinolin-4-carbonsäure ((S)-1-phenylpropyl)-amid
2dj 3-(3-Isopropylpiperazin-1-ylmethyl)-1-oxo-2-phenyl-1,2-dihydroisochinolin-4-carbonsäure ((S)-1-phenylpropyl)-amid
2dk 3-(3,3-Dimethylpiperazin-1-ylmethyl)-1-oxo-2-phenyl-1,2-dihydroisochinolin-4-carbonsäure ((S)-1-phenylpropyl)-amid
2dl 3-[3-(4-Fluorphenyl)-piperazin-1-ylmethyl]-1-oxo-2-phenyl-1,2-dihydroisochinolin-4-carbonsäure ((S)-1-phenylpropyl)-amid
2dm 1-Oxo-2-phenyl-3-(3-p-tolylpiperazin-1-ylmethyl)-1,2-dihydroisochinolin-4-carbonsäure ((S)-1-phenylpropyl)-amid
2dn 4-[1-Oxo-2-phenyl-4-(1-phenylpropylcarbamoyl)-1,2-dihydroisochinolin-3-ylmethyl]-piperazin-1-carbonsäure-tert-butylester
2do 3-(4-Methylcarbamoylmethylpiperazin-1-ylmethyl)-1-oxo-2-phenyl-1,2-dihydroisochinolin-4-carbonsäure ((S)-1-phenylpropyl)-amid
2dp 8-Chlor-3-dimethylaminomethyl-1-oxo-2-phenyl-1,2-dihydroisochinolin-4-carbonsäure ((S)-1-phenylpropyl)-amid
2dr 3-Cyclopentylaminomethyl-1-oxo-2-phenyl-1,2-dihydroisochinolin-4-carbonsäure ((S)-1-phenylpropyl)-amid
2ds 3-Cyclohexylaminomethyl-1-oxo-2-phenyl-1,2-dihydroisochinolin-4-carbonsäure ((S)-1-phenylpropyl)-amid
2dt 3-{[(2-Hydroxyethyl)-methylamino]-methyl}-1-oxo-2-phenyl-1,2-dihydroisochinolin-4-carbonsäure ((S)-1-phenylpropyl)-amid
2du 3-Imidazol-1-ylmethyl-1-oxo-2-phenyl-1,2-dihydroisochinolin-4-carbonsäure ((S)-1-phenylpropyl)-amid
2dv 3-(2-Methylimidazol-1-ylmethyl)-1-oxo-2-phenyl-1,2-dihydroisochinolin-4-carbonsäure ((S)-1-phenylpropyl)-amid
2dw 3-(4-Methylimidazol-1-ylmethyl)-1-oxo-2-phenyl-1,2-dihydroisochinolin-4-carbonsäure ((S)-1-phenylpropyl)-amid
2dx 3-(2,5-Dihydropyrrol-1-ylmethyl)-1-oxo-2-phenyl-1,2-dihydroisochinolin-4-carbonsäure ((S)-1-phenylpropyl)-amid
2dy 3-(2,5-Dimethyl-2,5-dihydropyrrol-1-ylmethyl)-1-oxo-2-phenyl-1,2-dihydroisochinolin-4-carbonsäure ((S)-1-phenylpropyl)-amid
2dz 1-Oxo-2-phenyl-3-pyrrolidin-1-ylmethyl-1,2-dihydroisochinolin-4-carbonsäure ((S)-1-phenylpropyl)-amid
2ea 1-Oxo-2-phenyl-3-(thiazol-2-ylaminomethyl)-1,2-dihydroisochinolin-4-carbonsäure ((S)-1-phenylpropyl)-amid
2eb 1-Oxo-2-phenyl-3-(pyrimidin-4-ylaminomethyl)-1,2-dihydroisochinolin-4-carbonsäure ((S)-1-phenylpropyl)-amid
2ec 3-(tert-Butylaminomethyl)-1-oxo-2-phenyl-1,2-dihydroisochinolin-4-carbonsäure ((S)-1-phenylpropyl)-amid
2ed 3-[(2-Hydroxy-1,1-dimethyl-ethylamino)-methyl]-1-oxo-2-phenyl-1,2-dihydroisochinolin-4-carbonsäure ((S)-1-phenylpropyl)-amid
2ee 3-(Isopropylaminomethyl)-1-oxo-2-phenyl-1,2-dihydroisochinolin-4-carbonsäure ((S)-1-phenylpropyl)-amid
2ef 3-[(2-Hydroxy-1-methyl-ethylamino)-methyl]-1-oxo-2-phenyl-1,2-dihydroisochinolin-4-carbonsäure ((S)-1-phenylpropyl)-amid
2eg 3-[(1-Hydroxymethylpropylamino)-methyl]-1-oxo-2-phenyl-1,2-dihydroisochinolin-4-carbonsäure ((S)-1-phenylpropyl)-amid
2eh 3-[(2,2-Dimethylpropylamino)-methyl]-1-oxo-2-phenyl-1,2-dihydroisochinolin-4-carbonsäure ((S)-1-phenylpropyl)-amid
2ei 1-Oxo-2-phenyl-3-prop-2-ynylaminomethyl-1,2-dihydroisochinolin-4-carbonsäure ((S)-1-phenylpropyl)-amid
2ej 3-Allylaminomethyl-1-oxo-2-phenyl-1,2-dihydroisochinolin-4-carbonsäure ((S)-1-phenylpropyl)-amid
2ek 3-[(Methylprop-2-ynyl-amino)-methyl]-1-oxo-2-phenyl-1,2-dihydroisochinolin-4-carbonsäure ((S)-1-phenylpropyl)-amid
2el 3-Diallylaminomethyl-1-oxo-2-phenyl-1,2-dihydroisochinolin-4-carbonsäure ((S)-1-phenylpropyl)-amid
2em 3-Diethylaminomethyl-1-oxo-2-phenyl-1,2-dihydroisochinolin-4-carbonsäure ((S)-1-phenylpropyl)-amid
2en 3-[(Isopropylmethylamino)-methyl]-1-oxo-2-phenyl-1,2-dihydroisochinolin-4-carbonsäure ((S)-1-phenylpropyl)-amid
2eo 3-[((S)-2-Hydroxy-1-methyl-ethylamino)-methyl]-1-oxo-2-phenyl-1,2-dihydroisochinolin-4-carbonsäure ((S)-1-phenylpropyl)-amid
2ep 3-[((R)-2-Hydroxy-1-methylethylamino)-methyl]-1-oxo-2-Phenyl-1,2-dihydroisochinolin-4-carbonsäure ((S)-1-phenylpropyl)-amid
2eq 3-{[(2-Methoxyethyl)-methylamino]-methyl}-1-oxo-2-phenyt-1,2-dihydroisochinolin-4-carbonsäure ((S)-1-phenylpropyl)-amid
2er 3-((R)-3-Hydroxypyrrolidin-1-ylmethyl)-1-oxo-2-phenyl-1,2-dihydroisochinolin-4-carbonsäure ((S)-1-phenylpropyl)-amid
2es 3-((S)-3-Hydroxypyrrolidin-1-ylmethyl)-1-oxo-2-phenyl-1,2-dihydroisochinolin-4-carbonsäure ((S)-1-phenylpropyl)-amid
2et 3-[(Cyclopentylmethylamino)-methyl]-1-oxo-2-phenyl-1,2-dihydroisochinolin-4-carbonsäure ((S)-1-phenylpropyl)-amid
2eu 3-{[(2-Hydroxy-1-methylethyl)-methylamino]-methyl}-1-oxo-2-phenyl-1,2-dihydroisochinolin-4-carbonsäure ((S)-1-phenylpropyl)-amid
2ev 3-{[Ethyl-(2-Hydroxyethyl)-amino]-methyl}-1-oxo-2-phenyl-1,2-dihydroisochinolin-4-carbonsäure ((S)-1-phenylpropyl)-amid
2ew 3-[(Ethylmethylamino)-methyl]-1-oxo-2-phenyl-1,2-dihydroisochinolin-4-carbonsäure ((S)-1-phenylpropyl)-amid
2ex 3-Cyclobutylaminomethyl-1-oxo-2-phenyl-1,2-dihydroisochinolin-4-carbonsäure ((S)-1-phenylpropyl)-amid
2ey 3-Azetidin-1-ylmethyl-1-oxo-2-phenyl-1,2-dihydroisochinolin-4-carbonsäure ((S)-1-phenylpropyl)-amid
2ez 3-(4-tert-Butylpiperazin-1-ylmethyl)-1-oxo-2-phenyl-1,2-dihydroisochinolin-4-carbonsäure ((S)-1-phenylpropyl)-amid
2fa 3-[4-(2-Hydroxyethyl)-piperazin-1-ylmethylkl-oxo-2-phenyl-1,2-dihydroisochinolin-4-carbonsäure ((S)-1-phenylpropyl)-amid
2fb 3-{4-[2-(2-Hydroxyethoxy)-ethyl]-piperazin-1-ylmethyl}-1-oxo-2-phenyl-1,2-dihydroisochinolin-4-carbonsäure ((S)-1-phenylpropyl)-amid
2fc 3-[4-(3-Chlor-5-trifluormethylpyridin-2-yl)-piperazin-1-ylmethyl]-1-oxo-2-phenyl-1,2-dihydroisochinolin-4-carbonsäure ((S)-1-phenylpropyl)-amid
2fd 3-[4-(3,5-Dichlorpyridin-4-yl)-piperazin-1-ylmethyl]-1-oxo-2-phenyl-1,2-dihydroisochinolin-4-carbonsäure ((S)-1-phenylpropyl)-amid
2fe 4-[1-Oxo-2-phenyl-4-((S)-1-phenylpropylcarbamoyl)-1,2-dihydroisochinolin-3-ylmethyl]-piperazin-1-carbonsäurebenzylester
2ff 3-[4-(3-Morpholin-4-yl-propyl)-piperazin-1-ylmethyl]-1-oxo-2-Phenyl-1,2-dihydroisochinolin-4-carbonsäure ((S)-1-phenylpropyl)-amid
2fg 1-Oxo-2-phenyl-3-[4-(3-piperidin-1-yl-propyl)-piperazin-1-ylmethyl]-1,2-dihydroisochinolin-4-carbonsäure ((S)-1-phenylpropyl)-amid
2fh 3-[4-(4,6-Dimethoxypyrimidin-2-ylmethyl)-piperazin-1-ylmethyl]-1-oxo-2-phenyl-1,2-dihydroisochinolin-4-carbonsäure ((S)-1-phenylpropyl)-amid
2fi 3-[4-(3-Hydroxypropyl)-piperazin-1-ylmethyl]-1-oxo-2-phenyl-1,2-dihydroisochinolin-4-carbonsäure ((S)-1-phenylpropyl)-amid
2fj 3-[4-(2,3-Dihydroxypropyl)-piperazin-1-ylmethyl]-1-oxo-2-phenyl-1,2-dihydroisochinolin-4-carbonsäure ((S)-1-phenylpropyl)-amid
2fk (2-Oxo-2-{4-[1-oxo-2-phenyl-4-(1-phenylpropylcarbamoyl)-1,2-dihydroisochinolin-3-ylmethyl]-piperazin-1-yl}-ethyl)-carbamidsäure-tert-butylester
2fl 3-[4-(1H-Indazol-5-yl)-piperazin-1-ylmethyl]-1-oxo-2-phenyl-1,2-dihydroisochinolin-4-carbonsäure ((S)-1-phenylpropyl)-amid
2fm 1-Oxo-2-phenyl-3-(4-chinolin-6-yl-piperazin-1-ylmethyl)-1,2-dihydroisochinolin-4-carbonsäure ((S)-1-phenylpropyl)-amid
2fn 3-[4-(6,7-Dimethoxychinazolin-4-yl)-piperazin-1-ylmethyl]-1-oxo-2-phenyl-1,2-dihydroisochinolin-4-carbonsäure ((S)-1-phenylpropyl)-amid
2fo 4-{4-[1-Oxo-2-phenyl-4-(1-phenylpropylcarbamoyl)-1,2-dihydroisochinolin-3-ylmethyl]-piperazin-1-yl}-piperidin-1-carbonsäure-tert-butylester
2fp 3-{4-[2-(4-Chlorphenoxy)-ethyl]-piperazin-1-ylmethyl}-1-oxo-2-phenyl-1,2-dihydroisochinolin-4-carbonsäure ((S)-1-phenylpropyl)-amid
2fq {4-[1-Oxo-2-phenyl-4-(1-phenylpropylcarbamoyl)-1,2-dihydroisochinolin-3-ylmethyl]-piperazin-1-yl}-essigsäure-tert-butylester
2fr 1-Oxo-2-phenyl-3-[4-(3,3,3-trifluor-2-hydroxypropyl)-piperazin-1-ylmethyl]-1,2-dihydroisochinolin-4-carbonsäure ((S)-1-phenylpropyl)-amid
2fs 3-[4-(2-Hydroxypropyl)-piperazin-1-ylmethyl]-1-oxo-2-phenyl-1,2-dihydroisochinolin-4-carbonsäure ((S)-1-phenylpropyl)-amid
2fu 3-[4-(4-Amino-6,7-dimethoxychinazolin-2-yl)-piperazin-1-ylmethyl]-1-oxo-2-phenyl-1,2-dihydroisochinolin-4-carbonsäure ((S)-1-phenylpropyl)-amid
2fv (2-{4-[1-Oxo-2-phenyl-4-(1-phenylpropylcarbamoyl)-1,2-dihydroisochinolin-3-ylmethyl]-piperazin-1-yl}-ethyl)-carbamidsäure-tert-butylester
2fw 1-Oxo-3-{4-[2-(2-oxo-imidazolidin-1-yl)-ethyl]-piperazin-1-ylmethyl}-2-phenyl-1,2-dihydroisochinolin-4-carbonsäure ((S)-1-phenylpropyl)-amid
2fx 3-(4-[(4,6-Dimethoxypyrimidin-2-yl)-phenylmethyl]-piperazin-1-ylmethyl}-1-oxo-2-phenyl-1,2-dihydroisochinolin-4-carbonsäure ((S)-1-phenylpropyl)-amid
2fy 3-(4-Benzo[1,2,5]thiadiazol-4-yl-piperazin-1-ylmethyl)-1-oxo-2-phenyl-1,2-dihydroisochinolin-4-carbonsäure ((S)-1-phenylpropyl)-amid
2fz 3-[4-(2,3-Dihydrobenzo[1,4]dioxin-5-yl)-piperazin-1-ylmethyl]-1-oxo-2-phenyl-1,2-dihydroisochinolin-4-carbonsäure ((S)-1-phenylpropyl)-amid
2ga 3-[4-(4-Methylchinolin-2-yl)-piperazin-1-ylmethyl]-1-oxo-2-phenyl-1,2-dihydroisochinolin-4-carbonsäure ((S)-1-phenylpropyl)-amid
2gb 1-Oxo-2-phenyl-3-[4-(pyridin-2-ylcarbamoylmethyl)-piperazin-1-ylmethyl]-1,2-dihydroisochinolin-4-carbonsäure ((S)-1-phenylpropyl)-amid
2gc 3-[4-(6-Chlor-3-oxo-3,4-dihydro-2H-benzo[1,4]oxazin-8-yl)-piperazin-11-ylmethyl]-1-oxo-2-phenyl-1,2-dihydroisochinolin-4-carbonsäure ((S)-1-phenylpropyl)-amid
2gd 3-(4-Carbamoylmethylpiperazin-1-ylmethyl)-1-oxo-2-phenyl-1,2-dihydroisochinolin-4-carbonsäure ((S)-1-phenylpropyl)-amid
2ge 3-(4-Hydroxy-4-phenylpiperidin-1-ylmethyl)-1-oxo-2-phenyl-1,2-dihydroisochinolin-4-carbonsäure ((S)-1-phenylpropyl)-amid
2gf 3-[4-(4-Chlorphenyl)-4-hydroxypiperidin-1-ylmethyl]-1-oxo-2-phenyl-1,2-dihydroisochinolin-4-carbonsäure ((S)-1-phenylpropyl)-amid
2gg 1-[1-Oxo-2-phenyl-4-(1-phenylpropylcarbamoyl)-1,2-dihydroisochinolin-3-ylmethyl]-piperidin-4-carbonsäure
2gh 3-(4-Cyan-4-phenylpiperidin-1-ylmethyl)-1-oxo-2-phenyl-1,2-dihydroisochinolin-4-carbonsäure ((S)-1-phenylpropyl)-amid
2gi 3-(4-Benzyl-4-Hydroxy-piperidin-1-ylmethyl)-1-oxo-2-phenyl-1,2-dihydroisochinolin-4-carbonsäure ((S)-1-phenylpropyl)-amid
2gj 1-[1-Oxo-2-phenyl-4-(1-phenylpropylcarbamoyl)-1,2-dihydroisochinolin-3-ylmethyl]-4-phenylpiperidin-4-carbonsäureethylester
2gk 1-Oxo-2-phenyl-3-[4-(phenylpropionylamino)-piperidin-1-ylmethyl]-1,2-dihydroisochinolin-4-carbonsäure ((S)-1-phenylpropyl)-amid
2gl Methyl-{1-[1-oxo-2-phenyl-4-(1-phenylpropylcarbarnoyl)-1,2-dihydroisochinolin-3-ylmethyl]-piperidin-4-yl}-carbamidsäure-tert-butylester
2gm 1-Oxo-2-phenyl-3-[4-(2-pyrrolidin-1-yl-ethyl)-piperidin-1-ylmethyl]-1,2-dihydroisochinolin-4-carbonsäure ((S)-1-phenylpropyl)-amid
2gn 3-{4-[5-(4-Fluorphenyl)-[1,3,4]oxadiazol-2-yl]-piperidin-1-ylmethyl}-1-oxo-2-phenyl-1,2-dihydroisochinolin-4-carbonsäure ((S)-1-phenylpropyl)-amid
2go 1-Oxo-2-phenyl-3-[4-(3-pyridin-4-yl[1,2,4]oxadiazol-5-yl)-piperidin-1-ylmethyl]-1,2-dihydroisochinolin-4-carbonsäure ((S)-1-phenylpropyl)-amid
2gp 1-Oxo-2-phenyl-3-[4-(3-pyrazin-2-yl-[1,2,4]oxadiazo1-5-yl)-piperidin-1-ylmethyl]-1,2-dihydroisochinolin-4-carbonsäure ((S)-1-phenylpropyl)-amid
2gq 1-Oxo-2-phenyl-3-[4-(3-pyridin-4-yl-[1,2,4]oxadiazol-5-yl)-piperidin-1-ylmethyl]-1,2-dihydroisochinolin-4-carbonsäure ((S)-1-phenylpropyl)-amid
2gr 3-(4'-Hydroxy-3',4',5',6'-tetrahydro-2'H-[2,4']bipyridinyl-1'-ylmethyl)-1-oxo-2-phenyl-1,2-dihydroisochinolin-4-carbonsäure ((S)-1-phenylpropyl)-amid
2gs 3-(Spiro[isochroman-1,4'-piperidin]-1'-ylmethyl)-1-oxo-2-phenyl-1,2-dihydroisochinolin-4-carbonsäure ((S)-1-phenylpropyl)-amid
2gt 3-[4-Hydroxy-4-(3-methoxy-phenyl)-piperidin-1-ylmethyl]-1-oxo-2-phenyl-1,2-dihydroisochinolin-4-carbonsäure ((S)-1-phenylpropyl)-amid
2gu 3-[4-(3-Chlorphenyl)-4-hydroxypiperidin-1-ylmethyl]-1-oxo-2-phenyl-1,2-dihydroisochinolin-4-carbonsäure ((S)-1-phenylpropyl)-amid
2gv 3-(6-Chlor-3H-spiro[isobenzofuran-1,4'-piperidin]-1'-ylmethyl)-1-oxo-2-phenyl-1,2-dihydroisochinolin-4-carbonsäure ((S)-1-phenylpropyl)-amid
2gw 3-(4-{[4-Chlor-3-(4-fluorphenyl)-indan-1-yl]-methyl-amino}-piperidin-1-ylmethyl)-1-oxo-2-phenyl-1,2-dihydroisochinolin-4-carbonsäure ((S)-1-Phenylpropyl)-amid
2gx 3-(1-Acetylspiro[indolin-3,4'-piperidin]-1'-ylmethyl)-1-oxo-2-phenyl-1,2-dihydroisochinolin-4-carbonsäure ((S)-1-phenylpropyl)-amid
2gy 3-(1-Acetyl-5-fluorspiro[indolin-3,4'-piperidin]-1'-ylmethyl)-1-oxo-2-phenyl-1,2-dihydroisochinolin-4-carbonsäure ((S)-1-phenylpropyl)-amid
2gz 1-Oxo-3-(4-oxo-1-phenyl-1,3,8-triazaspiro[4.5]dec-8-ylmethyl)-2-phenyl-1,2-dihydroisochinolin-4-carbonsäure ((S)-1-phenylpropyl)-amid
2ha 3-(4-Acetylaminopiperidin-1-ylmethyl)-1-oxo-2-phenyl-1,2-dihydroisochinolin-4-carbonsäure ((S)-1-phenylpropyl)-amid
2hb 1-Oxo-3-(1-oxo-2,8-diazaspiro[4.5]dec-8-ylmethyl)-2-phenyl-1,2-dihydroisochinolin-4-carbonsäure ((S)-1-phenylpropyl)-amid
2hc 3-[4-Hydroxy-4-(3-trifluormethylphenyl)-piperidin-1-ylmethyl]-1-oxo-2-phenyl-1,2-dihydroisochinolin-4-carbonsäure ((S)-1-phenylpropyl)-amid
2hd 1-Oxo-2-phenyl-3-(4-trifluormethylpiperidin-1-ylmethyl)-1,2-dihydroisochinolin-4-carbonsäure ((S)-1-phenylpropyl)-amid
2he 3-[4-(4-Methylpiperazin-1-carbonyl)-piperidin-1-ylmethyl]-1-oxo-2-henyl-1,2-dihydroisochinolin-4-carbonsäure ((S)-1-phenylpropyl)-amid
2hf 3-(5-Isopropyl-3H-spiro[isobenzofuran-1,4'-piperidin]-1'-ylmethyl)-1-oxo-2-phenyl-1,2-dihydroisochinolin-4-carbonsäure ((S)-1-phenylpropyl)-amid
2hg 3-[4-(Acetylmethylamino)-4-phenyl-piperidin-1-ylmethyl]-1-oxo-2-phenyl-1,2-dihydroisochinolin-4-carbonsäure ((S)-1-phenylpropyl)-amid
2hh 4-{1-[1-Oxo-2-phenyl-4-(1-phenylpropylcarbamoyl)-1,2-dihydroisochinolin-3-ylmethyl]-piperidin-4-yl}-piperazin-1-carbonsäure-tert-butylester
2hi (2-{1-[1-Oxo-2-phenyl-4-(1-phenylpropylcarbamoyl)-1,2-dihydroisochinolin-3-ylmethyl]-piperidin-4-yl}-ethyl)-carbamidsäure-tert-butylester
2hj 3-(4-Methylamino-4-Phenyl-piperidin-1-ylmethyl)-1-oxo-2-phenyl-1,2-dihydroisochinolin-4-carbonsäure ((S)-1-phenylpropyl)-amid
2hk 3-(4-Acetylamino-4-phenylpiperidin-1-ylmethyl)-1-oxo-2-phenyl-1,2-dihydroisochinolin-4-carbonsäure ((S)-1-phenylpropyl)-amid
2hl 3-[4-Acetylamino-4-(3-fluorphenyl)-piperidin-1-ylmethyl]-1-oxo-2-phenyl-1,2-dihydroisochinolin-4-carbonsäure ((S)-1-phenylpropyl)-amid
2hm 1-Oxo-3-[4-(4-oxo-piperidin-1-carbonyl)-piperidin-1-ylmethyl]-2-phenyl-1,2-dihydroisochinolin-4-carbonsäure ((S)-1-phenylpropyl)-amid
2hn 3-[4,4']Bipiperidinyl-1-ylmethyl-1-oxo-2-phenyl-1,2-dihydroisochinolin-4-carbonsäure ((S)-1-phenylpropyl)-amid
2ho {1-[1-Oxo-2-phenyl-4-(1-phenylpropylcarbamoyl)-1,2-dihydroisochuinolin-3-ylmethyl]-piperidin-4-yl}-carbamidsäure-tert-butylester
2hp 3-[1,4']Bipiperidinyl-1'-ylmethyl-1-oxo-2-phenyl-1,2-dihydroisochinolin-4-carbonsäure [cyclopropyl-(3-fluorphenyl)-methyl]-amid
2hq 1-Oxo-3-(4-phenethylpiperazin-1-ylmethyl)-2-phenyl-1,2-dihydroisochinolin-carbonsäure [cyclopropyl-(3-fluorphenyl)-methyl]-amid
2hr 3-(4-Isopropylpiperazin-1-ylmethyl)-1-oxo-2-Phenyl-1,2-dihydroisochinolin-4-carbonsäure [cyclopropyl-(3-fluorphenyl)-methyl]-amid
2hs 3-[4-(2-Morpholin-4-yl-ethyl)-piperazin-1-ylmethyl]-1-oxo-2-phenyl-1,2-dihydroisochinolin-4-carbonsäure [cyclopropyl-(3-fluorphenyl)-methyl]-amid
2ht 3-[4-(1-Methylpiperidin-4-yl)-piperazin-1-ylmethyl]-1-oxo-2-phenyl-1,2-dihydroisochinolin-4-carbonsäure [cyclopropyl-(3-fluorphenyl)-methyl]-amid
2hu 1-Oxo-2-phenyl-3-[4-(2-piperidin-1-yl-ethyl)-piperazin-1-ylmethyl]-1,2-dihydroisochinolin-4-carbonsäure [cyclopropyl-(3-fluorphenyl)-methyl]-amid
2hv 1-Oxo-2-phenyl-3-[4-(2-pyrrolidin-1-yl-ethyl)-piperazin-1-ylmethyl]-1,2-dihydroisochinolin-4-carbonsäure [cyclopropyl-(3-fluorphenyl)-methyl]-amid
2hw 1-Oxo-2-phenyl-3-(4-pyridin-4-ylmethyl-piperazin-1-ylmethyl)-1,2-dihydroisochinolin-4-carbonsäure [cyclopropyl-(3-fluorphenyl)-methyl]-amid
2hx 3-(4-Hydroxy-3,4,5,6-tetrahydro-2H-[4,4']bipyridinyl-1-ylmethyl)-1-oxo-2-phenyl-1,2-dihydroisochinolin-4-carbonsäure [cyclopropyl-(3-fluorphenyl)-methyl]-amid
2hy 3-[4-(2-Morpholin-4-yl-ethyl)-piperidin-1-ylmethyl]-1-oxo-2-phenyl-1,2-dihydroisochinolin-4-carbonsäure [cyclopropyl-(3-fluorphenyl)-methyl]-amid
2hz 3-[4-Hydroxy-4-(3-methoxy-phenyl)-piperidin-1-ylmethyl]-1-oxo-2-phenyl-1,2-dihydroisochinolin-4-carbonsäure [cyclopropyl-(3-fluorphenyl)-methyl]-amid
2ia 3-[4-(Acetylmethylamino)-4-phenylpiperidin-1-ylmethyl]-1-oxo-2-phenyl-1,2-dihydroisochinolin-4-carbonsäure [cyclopropyl-(3-fluorphenyl)-methyl]-amid
2ib 2-(2-Fluorphenyl)-1-oxo-3-(4-phenethylpiperazin-1-ylmethyl)-1,2-dihydroisochinolin-4-carbonsäure ((S)-1-phenylpropyl)-amid
2ic 2-(2-Fluorphenyl)-3-(4-isopropylpiperazin-1-ylmethyl)-1-oxo-1,2-dihydroisochinolin-4-carbonsäure ((S)-1-phenylpropyl)-amid
2id 3-[1,4']Bipiperidinyl-1'-ylmethyl-2-(2-fluorphenyl)-1-oxo-1,2-dihydroisochinolin-4-carbonsäure ((S)-1-phenylpropyl)-amid
2ie 2-(2-Fluorphenyl)-3-[4-(2-morpholin-4-yl-ethyl)-piperazin-1-ylmethyl]-1-oxo-1,2-dihydroisochinolin-4-carbonsäure ((S)-1-phenylpropyl)-amid
2if 2-(2-Fluorphenyl)-3-[4-(1-methyl-piperidin-4-yl)-piperazin-1-ylmethyl]-1-oxo-1,2-dihydroisochinolin-4-carbonsäure ((S)-1-phenylpropyl)-amid
2ig 2-(2-Fluorphenyl)-1-oxo-3-[4-(2-piperidin-1-yl-ethyl)-piperazin-1-ylmethyl]-1,2-dihydroisochinolin-4-carbonsäure ((S)-1-phenylpropyl)-amid
2ih 2-(2-Fluorphenyl)-1-oxo-3-[4-(2-pyrrolidin-1-yl-ethyl)-piperazin-1-ylmethyl]-1,2-dihydroisochinolin-4-carbonsäure ((S)-1-phenylpropyl)-amid
2ii 2-(2-Fluorphenyl)-1-oxo-3-(4-pyridin-4-ylmethylpiperazin-1-ylmethyl)-1,2-dihydroisochinolin-4-carbonsäure ((S)-1-phenylpropyl)-amid
2ij 2-(2-Fluorphenyl)-3-(4-hydroxy-3,4,5,6-tetrahydro-2H-[4,4']bipyridinyl-1-ylmethyl)-1-oxo-1,2-dihydroisochinolin-4-carbonsäure ((S)-1-phenylpropyl)-amid
2ik 2-(2-Fluorphenyl)-3-[4-(2-morpholin-4-yl-ethyl)-piperidin-1-ylmethyl]-1-oxo-1,2-dihydroisochinolin-4-carbonsäure ((S)-1-phenylpropyl)-amid
2il 2-(2-Fluorphenyl)-3-[4-Hydroxy-4-(3-methoxy-phenyl)-piperidin-1-ylmethyl]-1-oxo-1,2-dihydroisochinolin-4-carbonsäure ((S)-1-phenylpropyl)-amid
2im 3-[4-(2-Morpholin-4-yl-ethyl)-piperazin-1-ylmethyl]-1-oxo-2-o-tolyl-1,2-dihydroisochinolin-4-carbonsäure ((S)-1-phenylpropyl)-amid
2in 1-Oxo-3-[4-(2-piperidin-1-yl-ethyl)-piperazin-1-ylmethyl]-2-o-tolyl-1,2-dihydroisochinolin-4-carbonsäure ((S)-1-phenylpropyl)-amid
2io 1-Oxo-3-(4-pyridin-4-ylmethylpiperazin-1-ylmethyl)-2-o-tolyl-1,2-dihydroisochinolin-4-carbonsäure ((S)-1-phenylpropyl)-amid
2ip 2-(3-Fluorphenyl)-1-oxo-3-(4-phenethylpiperazin-1-ylmethyl)-1,2-dihydroisochinolin-4-carbonsäure ((S)-1-phenylpropyl)-amid
2iq 2-(3-Fluorphenyl)-3-(4-isopropylpiperazin-1-ylmethyl)-1-oxo-1,2-dihydroisochinolin-4-carbonsäure ((S)-1-phenylpropyl)-amid
2ir 3-[1,4']Bipiperidinyl-1'-ylmethyl-2-(3-fluorphenyl)-1-oxo-1,2-dihydroisochinolin-4-carbonsäure ((S)-1-phenylpropyl)-amid
2is 2-(3-Fluorphenyl)-3-[4-(2-morpholin-4-yl-ethyl)-piperazin-1-ylmethyll-1-oxo-1,2-dihydroisochinolin-4-carbonsäure ((S)-1-phenylpropyl)-amid
2it 2-(3-Fluorphenyl)-3-[4-(1-methylpiperidin-4-yl)-piperazin-1-ylmethyl]-1-oxo-1,2-dihydroisochinolin-4-carbonsäure ((S)-1-phenylpropyl)-amid
2iu 2-(3-Fluorphenyl)-1-oxo-3-[4-(2-piperidin-1-yl-ethyl)-piperazin-1-ylmethyl]-1,2-dihydroisochinolin-4-carbonsäure ((S)-1-phenylpropyl)-amid
2iv 2-(3-Fluorphenyl)-1-oxo-3-[4-(2-pyrrolidin-1-yl-ethyl)-piperazin-1-ylmethyl]-1,2-dihydroisochinolin-4-carbonsäure ((S)-1-phenylpropyl)-amid
2iw 2-(3-Fluorphenyl)-1-oxo-3-(4-pyridin-4-ylmethylpiperazin-1-ylmethyl)-1,2-dihydroisochinolin-4-carbonsäure ((S)-1-Phenyl-propyl)-amid
2ix 2-(3-Fluorphenyl)-3-[4-(2-morpholin-4-yl-ethyl)-piperidin-1-ylmethyll-1-oxo-1,2-dihydroisochinolin-4-carbonsäure ((S)-1-phenylpropyl)-amid
2iy 2-(3-Fluorphenyl)-3-[4-hydroxy-4-(3-methoxyphenyl)-piperidin-1-ylmethyl]-1-oxo-1,2-dihydroisochinolin-4-carbonsäure ((S)-1-phenylpropyl)-amid
2iz 3-[4-(Acetylmethylamino)-4-phenyl-piperidin-1-ylmethyl]-2-(3-fluorphenyl)-1-oxo-1,2-dihydroisochinolin-4-carbonsäure ((S)-1-phenylpropyl)-amid
2ja 2-(3-Chlorphenyl)-1-oxo-3-(4-phenethylpiperazin-1-ylmethyl)-1,2-dihydroisochinolin-4-carbonsäure ((S)-1-phenylpropyl)-amid
2jb 2-(3-Chlorphenyl)-3-(4-isopropyl-piperazin-1-ylmethyl)-1-oxo-1,2-dihydroisochinolin-4-carbonsäure ((S)-1-phenylpropyl)-amid
2jc 3-[1,4']Bipiperidinyl-1'-ylmethyl-2-(3-chlorphenyl)-1-oxo-1,2-dihydroisochinolin-4-carbonsäure ((S)-1-phenylpropyl)-amid
2jd 2-(3-Chlorphenyl)-3-[4-(2-morpholin-4-yl-ethyl)-piperazin-1-ylmethyl]-1-oxo-1,2-dihydroisochinolin-4-carbonsäure ((S)-1-phenylpropyl)-amid
2je 2-(3-Chlorphenyl)-3-[4-(1-methylpiperidin-4-yl)-piperazin-1-ylmethyl]-1-oxo-1,2-dihydroisochinolin-4-carbonsäure ((S)-1-phenylpropyl)-amid
2jf 2-(3-Chlorphenyl)-1-oxo-3-[4-(2-piperidin-1-yl-ethyl)-piperazin-1-ylmethyl]-1,2-dihydroisochinolin-4-carbonsäure ((S)-1-phenylpropyl)-amid
2jg 2-(3-Chlorphenyl)-1-oxo-3-[4-(2-pyrrolidin-1-yl-ethyl)-piperazin-1-ylmethyl]-1,2-dihydroisochinolin-4-carbonsäure ((S)-1-phenylpropyl)-amid
2jh 2-(3-Chlorphenyl)-1-oxo-3-(4-pyridin-4-ylmethylpiperazin-1-ylmethyl)-1,2-dihydroisochinolin-4-carbonsäure ((S)-1-phenylpropyl)-amid
2ji 2-(3-Chlorphenyl)-344-(2-morpholin-4-yl-ethyl)-piperidin-1-ylmethyl]-1-oxo-1,2-dihydroisochinolin-4-carbonsäure ((S)-1-phenylpropyl)-amid
2jj 2-(3-Chlorphenyl)-3-[4-hydroxy-4-(3-methoxyphenyl)-piperidin-1-ylmethyl]-1-oxo-1,2-dihydroisochinolin-4-carbonsäure ((S)-1-phenylpropyl)-amid
2jk 3-[4-(Acetylmethylamino)-4-phenyl-piperidin-1-ylmethyl]-2-(3-chlorphenyl)-1-oxo-1,2-dihydroisochinolin-4-carbonsäure ((S)-1-phenylpropyl)-amid
2jl 1-Oxo-3-(4-phenethylpiperazin-1-ylmethyl)-2-m-tolyl-1,2-dihydroisochinolin-4-carbonsäure ((S)-1-phenylpropyl)-amid
2jm 3-[1,4']Bipiperidinyl-1'-ylmethyl-1-oxo-2-m-tolyl-1,2-dihydroisochinolin-4-carbonsäure ((S)-1-phenylpropyl)-amid
2jn 3-[4-(2-Morpholin-4-yl-ethyl)-piperazin-1-ylmethyl]-1-oxo-2-m-tolyl-1,2-dihydroisochinolin-4-carbonsäure ((S)-1-phenylpropyl)-amid
2jo 3-[4-(1-Methylpiperidin-4-yl)-piperazin-1-ylmethyl]-1-oxo-2-m-tolyl-1,2-dihydroisochinolin-4-carbonsäure ((S)-1-phenylpropyl)-amid
2jp 1-Oxo-3-[4-(2-piperidin-1-yl-ethyl)-piperazin-1-ylmethyl]-2-m-tolyl-1,2-dihydroisochinolin-4-carbonsäure ((S)-1-phenylpropyl)-amid
2jq 1-Oxo-3-[4-(2-pyrrolidin-1-yl-ethyl)-piperazin-1-ylmethyl]-2-m-tolyl-1,2-dihydroisochinolin-4-carbonsäure ((S)-1-phenylpropyl)-amid
2jr 1-Oxo-3-(4-pyridin-4-ylmethyl-piperazin-1-ylmethyl)-2-m-tolyl-1,2-dihydroisochinolin-4-carbonsäure ((S)-1-phenylpropyl)-amid
2js 3-[4-(2-Morpholin-4-yl-ethyl)-piperidin-1-ylmethyl]-1-oxo-2-m-tolyl-1,2-dihydroisochinolin-4-carbonsäure ((S)-1-phenylpropyl)-amid
2jt 3-[4-Hydroxy-4-(3-methoxyphenyl)-piperidin-1-ylmethyl]-1-oxo-2-m-tolyl-1,2-dihydroisochinolin-4-carbonsäure ((S)-1-phenylpropyl)-amid
2ju 3-[4-(Acetylmethylamino)-4-phenylpiperidin-1-ylmethyl]-1-oxo-2-m-tolyl-1,2-dihydroisochinolin-4-carbonsäure ((S)-1-phenylpropyl)-amid
3a 1-Oxo-3-(2-oxo-pyrrolidin-1-ylmethyl)-2-Phenyl-1,2-dihydroisochinolin-4-carbonsäure ((S)-1-phenylpropyl)-amid
3b 8-Chlor-1-oxo-3-(2-oxo-pyrrolidin-1-ylmethyl)-2-phenyl-1,2-dihydroisochinolin-4-carbonsäure ((S)-1-phenylpropyl)-amid
4a 3-Cyanmethyl-1-oxo-2-phenyl-1,2-dihydroisochinolin-4-carbonsäure ((S)-1-phenylpropyl)-amid
5a 3-Methyl-1-oxo-2-phenyl-1,2-dihydroisochinolin-4-carbonsäure-N,N-diphenylhydrazid
5b N'-(3-Methyl-1-oxo-2-phenyl-1,2-dihydroisochinolin-4-carbonyl)-N-phenyl-hydrazincarbonsäuremethylester
1aa 2-(3-Methoxyphenyl)-3-methyl-1-oxo-1,2-dihydroisochinolin-4-carbonsäure ((S)-1-phenylpropyl)-amid
1ab 2-(4-Methoxyphenyl)-3-methyl-1-oxo-1,2-dihydroisochinolin-4-carbonsäure ((S)-1-phenylpropyl)-amid
1ac 2-(4-Fluorphenyl)-3-methyl-1-oxo-1,2-dihydroisochinolin-4-carbonsäure ((S)-1-phenylpropyl)-amid
1ad 2-(4-Chlorphenyl)-3-methyl-1-oxo-1,2-dihydroisochinolin-4-carbonsäure((S)-1-phenylpropyl)-amid
1ae 3-Methyl-1-oxo-2-p-tolyl-1,2-dihydroisochinolin-4-carbonsäure ((S)-1-phenylpropyl)-amid
1 af 2-(3-Methoxyphenyl)-3-methyl-1-oxo-1,2-dihydroisochinolin-4-carbonsäure[(S)-cyclopropyl-(3-fluorphenyl)-methyl]-amid
1ag 2-(4-Methoxyphenyl)-3-methyl-1-oxo-1,2-dihydroisochinolin-4-carbonsäure[(S)-cyclopropyl-(3-fluorphenyl)-methyl]-amid
1ah 2-(4-Fluorphenyl)-3-methyl-1-oxo-1,2-dihydroisochinolin-4-carbonsäure[(S)-cyclopropyl-(3-fluorphenyl)-methyl]-amid
1ai 2-(4-Chlorphenyl)-3-methyl-1-oxo-1,2-dihydroisochinolin-4-carbonsäure [(S)-cyclopropyl-(3-fluorphenyl)-methyl]-amid
1 aj 3-Methyl-1-oxo-2-p-tolyl-1,2-dihydroisochinolin-4-carbonsäure [(S)-cyclopropyl-(3-fluorphenyl)-methyl]-amid
1ak 2-(2-Methoxyphenyl)-3-methyl-1-oxo-1,2-dihydroisochinolin-4-carbonsäure((S)-1-phenylpropyl)-amid
1al 2-(2-Methoxyphenyl)-3-methyl-1-oxo-1,2-dihydroisochinolin-4-carbonsäure[(S)-cyclopropyl-(3-fluorphenyl)-methyl]-amid
1am 3-Methyl-8-nitro-1-oxo-2-phenyl-1,2-dihydroisochinolin-4-carbonsäure ((S)-1-phenylpropyl)-amid
1an 3-Methyl-8-nitro-1-oxo-2-phenyl-1,2-dihydroisochinolin-4-carbonsäure[(S)-cyclopropyl-(3-fluorphenyl)-methyl]-amid
1ao 8-Methoxy-3-methyl-1-oxo-2-phenyl-1,2-dihydroisochinolin-4-carbonsäure((S)-1 -phenylpropyl)-amid
1ap 8-Methoxy-3-methyl-1-oxo-2-Phenyl-1,2-dihydroisochinolin-4-carbonsäure[(S)-cyclopropyl-(3-fluorhenyl)-methyl]-amid
1aq 8-Amino-3-methyl-1-oxo-2-phenyl-1,2-dihydroisochinolin-4-carbonsäure((S)-1-phenylpropyl)-amid
1ar 8-Cyan-3-methyl-1-oxo-2-phenyl-1,2-dihydroisochinolin-4-carbonsäure((S)-1-phenylpropyl)-amid
1as 8-Chlor-3-methyl-1-oxo-2-phenyl-1,2-dihydroisochinolin-4-carbonsäure [(S)-1-chlorphenyl)-propyl]-amid 1 at 8-Chlor-3-methyl-1-oxo-2-phenyl-1,2-dihydroisochinolin-4-carbonsäure [(S)-1-[4-fluorphenyl)-propyl]-amid
1au 8-Chlor-3-methyl-1-oxo-2-phenyl-1,2-dihydroisochinolin-4-carbonsäure [(S)-1-(2-fluorphenyl)-propyl]-amid 1 av 8-Chlor-3-methyl-1-oxo-2-phenyl-1,2-dihydroisochinolin-4-carbonsäure [(S)-1-(3-chlorphenyl)-propyl]-amid
1aw 8-Chlor-3-methyl-1-oxo-2-phenyl-1,2-dihydroisochinolin-4-carbonsäure[(S)-(3-chlorphenyl)-cyclopropylmethyl]-amid
1 ax 8-Chlor-3-methyl-1-oxo-2-phenyl-1,2-dihydroisochinolin-4-carbonsäure[(S)-(4-chlorphenyl)-cyclopropylmethyl]-amid
1 ay 8-Chlor-3-methyl-1-oxo-2-phenyl-1,2-dihydroisochinolin-4-carbonsäure [(S)-1-(3-fluorphenyl)-propyl]-amid
1 az 8-Chlor-3-methyl-1-oxo-2-Phenyl-1,2-dihydroisochinolin-4-carbonsäure ((S)-2-methyl-1-phenylpropyl)-amid
1 ba 8-Chlor-3-methyl-1-oxo-2-phenyl-1,2-dihydroisochinolin-4-carbonsäure[(S)-cyclopropyl-(4-fluorphenyl)-methyl]-amid
1bb 8-Chlor-3-methyl-1-oxo-2-phenyl-1,2-dihydroisochinolin-4-carbonsäure [(S)-1-(2-chlorphenyl)-propyl]-amid
1 bc 8-Chlor-3-methyl-1-oxo-2-phenyl-1,2-dihydroisochinolin-4-carbonsäure((S)-cyclopentylphenylmethyl)-amid
1 bd 8-Chlor-3-methyl-1-oxo-2-phenyl-1,2-dihydroisochinolin-4-carbonsäure[(S)-(2-chlorphenyl)-cyclopropylmethyl]-amid
1 be 8-Chlor-3-methyl-1-oxo-2-phenyl-1,2-dihydroisochinolin-4-carbonsäure[(S)-cyclopropyl-(2-fluorphenyl)-methyl]-amid
1 bf 8-Chlor-3-methyl-1-oxo-2-phenyl-1,2-dihydroisochinolin-4-carbonsäure((S)-cyclohexylphenylmethyl)-amid
1 bg 8-Chlor-3-methyl-1-oxo-2-phenyl-1,2 dihydroisochinolin-4-carbonsäure((S)-cyclopropylphenylmethyl)-amid
1bh 8-Chlor-3-methyl-1-oxo-2-phenyl-1,2-dihydroisochinolin-4-carbonsäure[(S)-cyclobutyl-(3-fluorphenyl)-methyl]-amid
1bi 8-Chlor-3-methyl-1-oxo-2-phenyl-1,2-dihydroisochinolin-4-carbonsäure [(S)-cyclobutyl-(4-fluorphenyl)-methyl]-amid
1bl 8-Chlor-3-methyl-1-oxo-2-phenyl-1,2-dihydroisochinolin-4-carbonsäure[(S)-cyclopropyl-(3-fluorphenyl)-methyl]-amid
1bn 8-Chlor-3-methyl-1-oxo-2-phenyl-1,2-dihydroisochinolin-4-carbonsäure ((S)-cyclobutylphenylmethyl)-amid 1bo 8-Chlor-3-methyl-1-oxo-2-phenyl-1,2-dihydroisochinolin-4-carbonsäure [cyclobutyl-(2-fluorphenyl)-methyl]-amid
2ka 3-(4-tert-Butylpiperazin-1-ylmethyl)-8-chlor-1-oxo-2-phenyl-1,2-dihydroisochinolin-4-carbonsäure [(S)-cyclopropyl-(3-fluorphenyl)-methyl]-amid
2kb 8-Chlor-3-(4-isopropylpiperazin-1-ylmethyl)-1-oxo-2-phenyl-1,2-dihydroisochinolin-4-carbonsäure ((S)-1-phenylpropyl)-amid
2kc 8-Chlor-3-(4-isobutylpiperazin-1-ylmethyl)-1-oxo-2-phenyl-1,2-dihydroisochinolin-4-carbonsäure ((S)-1-phenylpropyl)-amid
2kd 8-Chlor-3-(octahydropyrido[1,2-a]pyrazin-2-ylmethyl)-1-oxo-2-phenyl-1,2-dihydroisochinolin-4-carbonsäure ((S)-1-phenylpropyl)-amid
2ke 8-Chlor-3-(4-hydroxy-3,4,5,6-tetrahydro-2H-[4,4']bipyridinyl-1-ylmethyl)-1-oxo-2-phenyl-1,2-dihydroisochinolin-4-carbonsäure ((S)-1-phenylpropyl)- amid
2kf 8-Chlor-3-(4-cyclopropylmethylpiperazin-1-ylmethyl)-1-oxo-2-phenyl-1,2-dihydroisochinolin-4-carbonsäure ((S)-1-phenylpropyl)-amid
2kg 8-Chlor-3-[4-(2-morpholin-4-yl-ethyl)-piperidin-1-ylmethyl]-1-oxo-2-phenyl-1,2-dihydroisochinolin-4-carbonsäure ((S)-1-phenylpropyl)-amid
2kh 8-Chlor-3-[1,4]diazepan-1-ylmethyl-1-oxo-2-phenyl-1,2-dihydroisochinolin-4-carbonsäure ((S)-1-phenylpropyl)-amid
2ki 8-Chlor-3-((S)-3-methylpiperazin-1-ylmethyl)-1-oxo-2-phenyl-1,2-dihydroisochinolin-4-carbonsäure ((S)-1-phenylpropyl)-amid
2kj 8-Chlor-3-imidazol-1-ylmethyl-1-oxo-2-phenyl-1,2-dihydroisochinolin-4-carbonsäure ((S)-1-phenylpropyl)-amid
2kk 8-Chlor-3-(4-methylimidazol-1-ylmethyl)-1-oxo-2-phenyl-1,2-dihydroisochinolin-4-carbonsäure ((S)-1-phenylpropyl)-amid
2kl 3-(tert-Butylaminomethyl)-8-chlor-1-oxo-2-phenyl-1,2-dihydroisochinolin-4-carbonsäure((S)-1-phenylpropyl)-amid
2km 8-Chlor-3-(isopropylaminomethyl)-1-oxo-2-phenyl-1,2-dihydroisochinolin-4-carbonsäure((S)-1-phenylpropyl)-amid
2kn 8-Chlor-3-[4-hydroxy-4-(3-methoxyphenyl)-piperidin-1-ylmethyl]-1-oxo-2-phenyl-1,2-dihydroisochinolin-4-carbonsäure((S)-1-phenylpropyl)-amid
2ko 3-(4-tert-Butylpiperazin-1-ylmethyl)-8-chlor-1-oxo-2-phenyl-1,2-dihydroisochinolin-4-carbonsäure ((S)-1-phenylpropyl)-amid
2kp 3-Benzoimidazol-1-ylmethyl-1-oxo-2-phenyl-1,2-dihydroisochinolin-4-carbonsäure((S)-1-phenylpropyl)-amid
2kq 1-Oxo-2-phenyl-3-pyrazol-1-ylmethyl-1,2-dihydroisochinolin-4-carbonsäure((S)-1-phenylpropyl)-amid
2kr 3-(4-Methylpyrazol-1-ylmethyl)-1-oxo-2-phenyl-1,2-dihydroisochinolin-4-carbonsäure((S)-1-phenylpropyl)-amid
2ks 1-Oxo-2-phenyl-3-[1,2,3]triazol-1-ylmethyl-1,2-dihydroisochinolin-4-carbonsäure((S)-1-phenylpropyl)-amid
2kt 2-(3-Methoxyphenyl)-1-oxo-3-(4-pyridin-4-ylmethylpiperazin-1-ylmethyl)-1,2-dihydroisochinolin-4-carbonsäure ((S)-1-phenylpropyl)-amid
2ku 2-(4-Methoxyphenyl)-3-[4-(2-morpholin-4-yl-ethyl)-piperazin-1-ylmethyl]-1-oxo-1,2-dihydroisochinolin-4-carbonsäure ((S)-1-phenylpropyl)-amid
2kv 2-(4-Fluorphenyl)-1-oxo-3-(4-phenethylpiperazin-1-ylmethyl)-1,2-dihydroisochinolin-4-carbonsäure ((S)-1-phenylpropyl)-amid
2kw 2-(4-Fluorphenyl)-3-(4-isopropylpiperazin-1-ylmethyl)-1-oxo-1,2-dihydroisochinolin-4-carbonsäure((S)-1-phenylpropyl)-amid
2kx 3-[1,4']Bipiperidinyl-1'-ylmethyl-2-(4-fluorphenyl)-1-oxo-1,2-dihydroisochinolin-4-carbonsäure ((S)-1-phenylpropyl)-amid
2ky 2-(4-Fluorphenyl)-3-[4-(2-morpholin-4-yl-ethyl)-piperazin-1-ylmethyl]-1-oxo-1,2-dihydroisochinolin-4-carbonsäure((S)-1-phenylpropyl)-amid
2kz 2-(4-Fluorphenyl)-3-[4-(1-methylpiperidin-4-yl)-piperazin-1-ylmethyl]-1-oxo-1,2-dihydroisochinolin-4-carbonsäure((S)-1-phenylpropyl)-amid
2la 2-(4-Fluorphenyl)-1-oxo-3-[4-(2-pyrrolidin-1-yl-ethyl)-piperazin-1-ylmethyl]-1,2-dihydroisochinolin-4-carbonsäure((S)-1-phenylpropyl)-amid
2lb 2-(4-Fluorphenyl)-3-[4-hydroxy-4-(3-methoxyphenyl)-piperidin-1 -ylmethyl]-1 -oxo-1,2-dihydroisochinolin-4-carbonsäure((S)-1-phenylpropyl)-amid
2lc 2-(4-Chlorphenyl)-1-oxo-3-(4-phenethylpiperazin-1-ylmethyl)-1,2-dihydroisochinolin-4-carbonsäure((S)-1-phenylpropyl)-amid
2ld 2-(4-Chlorphenyl)-3-(4-isopropylpiperazin-1-ylmethyl)-1-oxo-1,2-dihydroisochinolin-4-carbonsäure((S)-1-phenylpropyl)-amid
2le 3-[1,4']Bipiperidinyl-1'-ylmethyl-2-(4-chlorphenyl)-1-oxo-1,2-dihydroisochinolin-4-carbonsäure((S)-1-phenylpropyl)-amid
2lf 2-(4-Chlorphenyl)-3-[4-(2-morpholin-4-yl-ethyl)-piperazin-1-ylmethyl]-1-oxo-1,2-dihydroisochinolin-4-carbonsäure((S)-1-phenylpropyl)-amid
2lg 2-(4-Chlorphenyl)-3-[4-(1-methylpiperidin-4-yl)-piperazin-1-ylmethyl]-1-oxo-1,2-dihydroisochinolin-4-carbonsäure((S)-1-phenylpropyl)-amid
2lh 2-(4-Chlorphenyl)-1-oxo-3-[4-(2-piperidin-1-yl-ethyl)-piperazin-1-ylmethyl]-1,2-dihydroisochinolin-4-carbonsäure((S)-1-phenylpropyl)-amid
2li 2-(4-Chlorphenyl)-1-oxo-3-[4-(2-pyrrolidin-1-yl-ethyl)-piperazin-1-ylmethyl]-1,2-dihydroisochinolin-4-carbonsäure((S)-1-phenylpropyl)-amid
21j 3-(4-tert-Butylpiperazin-1-ylmethyl)-8-fluor-1-oxo-2-phenyl-1,2-dihydroisochinolin-4-carbonsäure((S)-1-phenylpropyl)-amid
2lk 8-Chlor-3-cyclopropylaminomethyl-1-oxo-2-phenyl-1,2-dihydroisochinolin-4-carbonsäure((S)-1-phenylpropyl)-amid
2ll 3-(4-tert-Butylpiperazin-1-ylmethyl)-8-fluor-1-oxo-2-phenyl-1,2-dihydroisochinolin-4-carbonsäure [(S)-cyclopropyl-(3-fluorphenyl)-methyl]-amid
2lm 8-Fluor-1-oxo-2-phenyl-3-piperazin-1-ylmethyl-1,2-dihydroisochinolin-4-carbonsäure[(S)-cyclopropyl-(3-fluorphenyl)-methyl]-amid
2ln 8-Fluor-1-oxo-3-(3-oxo-pyrazolidin-1-ylmethyl)-2-phenyl-1,2-dihydroisochinolin-4-carbonsäure [(S)-cyclopropyl-(3-fluorphenyl)-methyl]-amid
2lo 8-Fluor-1-oxo-3-(3-oxo-piperazin-1-ylmethyl)-2-phenyl-1,2-dihydroisochinolin-4-carbonsäure[(S)-cyclopropyl-(3-fluorphenyl)-methyl]-amid
3c 1-Oxo-2-phenyl-3-(1H-[1,2,4]triazol-3-ylsulfanylmethyl)-1,2-dihydroisochinolin-4-carbonsäure((S)-1-phenylpropyl)-amid
3d 8-Chlor-1-oxo-3-(2-oxo-pyrrolidin-1-ylmethyl)-2-phenyl-1,2-dihydroisochinolin-4-carbonsäure [(S)-cyclopropyl-(3-fluorphenyl)-methyl]-amid
3e 8-Fluor-1-oxo-3-(2-oxo-pyrrolidin-1-ylmethyl)-2-phenyl-1,2-dihydroisochinolin-4-carbonsäure ((S)-1-phenylpropyl)-amid
3f 8-Fluor-1-oxo-3-(2-oxo-pyrrolidin-1-ylmethyl)-2-phenyl-1,2-dihydroisochinolin-4-carbonsäure [(S)-cyclopropyl-(3-fluorphenyl)-methyl]-amid
3g 8-Fluor-1-oxo-3-(5-oxo-pyrazolidin-1-ylmethyl)-2-phenyl-1,2-dihydroisochinolin-4-carbonsäure [(S)-cyclopropyl-(3-fluorphenyl)-methyl]-amid
3h 8-Fluor-1-oxo-3-(2-oxo-piperazin-1-ylmethyl)-2-phenyl-1,2-dihydroisochinolin-4-carbonsäure [(S)-cyclopropyl-(3-fluorphenyl)-methyl]-amid
3i 8-Fluor-1-oxo-3-(2-oxo-piperidin-1-ylmethyl)-2-phenyl-1,2-dihydroisochinolin-4-carbonsäure [(S)-cyclopropyl-(3-fluorphenyl)-methyl]-amid
4b 8-Chlor-3-cyanmethyl-1-oxo-2-phenyl-1,2-dihydroisochinolin-4-carbonsäure[(S)-cyclopropyl-(3-fluorphenyl)-methyl]-amid
6a 2-(3,4-Dichlorphenyl)-3-methyl-1-oxo-1,2-dihydroisochinolin-4-carbonsäure((S)-1-phenylpropyl)-amid
6b 8-Fluor-1-oxo-3-(2-oxo-pyrrolidin-1-ylmethyl)-2-Pphenyl-1,2-dihydroisochinolin-4-carbonsäure((S)-cyclopropylphenylmethyl)-amid
6c 8-Fluor-3-methyl-1-oxo-2-phenyl-1,2-dihydroisochinolin-4-carbonsäure ((S)-cyclopropylphenylmethyl)-amid
7a 3-Ethyl-1-oxo-2-phenyl-1,2-dihydroisochinolin-4-carbonsäure ((S)-1-phenylpropyl)-amid
7b 8-Fluor-3-methyl-1-oxo-2-phenyl-1,2-dihydroisochinolin-4-carbonsäure((S)-1-phenylpropyl)-amid
7c 8-Fluor-2-(3-fluorphenyl)-3-methyl-1-oxo-1,2-dihydroisochinolin-4-carbonsäure((S)-1-phenylpropyl)-amid
7d 8-Fluor-2-(4-fluorphenyl)-3-methyl-1-oxo-1,2-dihydroisochinolin-4-carbonsäure ((S)-1-phenylpropyl)-amid
7f 8-Fluor-2-(3-fluorphenyl)-1-oxo-3-(2-oxo-pyrrolidin-1-ylmethyl)-1,2-dihydroisochinolin-4-carbonsäure [(S)-cyclopropyl-(3-fluorphenyl)-methyl]-amid
7g 8-Fluor-2-(4-fluorphenyl)-1-oxo-3-(2-oxo-pyrrolidin-1-ylmethyl)-1,2-dihydroisochinolin-4-carbonsäure [(S)-cyclopropyl-(3-fluorphenyl)-methyl]-amid
7i 8-Fluor-2-(2-fluorphenyl)-3-methyl-1-oxo-1,2-dihydroisochinolin-4-carbonsäure[(S)-cyclopropyl-(3-fluorphenyl)-methyl]-amid
7j 8-Fluor-2-(3-fluorphenyl)-3-methyl-1-oxo-1,2-dihydroisochinolin-4-carbonsäure[(S)-cyclopropyl-(3-fluorphenyl)-methyl]-amid
7k 8-Fluor-2-(4-fluorphenyl)-3-methyl-1-oxo-1,2-dihydroisochinolin-4-carbonsäure[(S)-cyclopropyl-(3-fluorphenyl)-methyl]-amid
7l 6,8-Difluor-3-methyl-1-oxo-2-phenyl-1,2-dihydroisochinolin-4-carbonsäure[(S)-cyclopropyl-(3-fluorphenyl)-methyl]-amid
7m 5,8-Difluor-3-methyl-1-oxo-2-phenyl-1,2-dihydroisochinolin-4-carbonsäure[(S)-cyclopropyl-(3-fluorphenyl)-methyl]-amid
7n 3-Methyl-1-oxo-2-henyl-8-trifluormethyl-1,2-dihydroisochinolin-4-carbonsäure ((S)-1-phenylpropyl)-amid
8a 3-(1-tert-Butylpiperidin-4-ylmethyl)-8-chlor-1-oxo-2-phenyl-1,2-dihydroisochinolin-4-carbonsäure[(S)-cyclopropyl-(3-fluorphenyl)-methyl]-amid; und pharmazeutisch unbedenkliche Salze von beliebigen dieser Verbindungen.

7. Verbindung nach einem der Ansprüche 1 bis 6 zur Therapieverwendung.

8. Verbindung nach einem der Ansprüche 1 bis 6 zur Verwendung bei der Behandlung einer Krankheit, die ausgewählt ist aus: Psychose; Schizophrenie; schizophreniformer Störung; schizoaffektiver Störung; wahnhafter Störung; kurzzeitiger psychotischer Störung; geteilter psychotischer Störung; psychotischer Störung infolge eines allgemeinmedizinischen Leidens; substanz- oder drogeninduzierter psychotischer Störung (Kokain, Alkohol, Amphetamin usw.); schizoider Persönlichkeitsstörung; schizotypischer Persönlichkeitsstörung; mit typischer Depression, bipolarer Störung, Alzheimer'scher Krankheit oder Parkinson'scher Krankheit verbundener Psychose oder Schizophrenie; typischer Depression; allgemeiner Angststörung; bipolarer Störung (Erhaltungstherapie, Rezidivprävention und Stabilisierung); Manie; Hypomanie; kognitiver Störung; ADHD; Fettleibigkeit; Appetitreduzierung; Alzheimer'scher Krankheit; Parkinson'scher Krankheit; Schmerz; Konvulsionen; Huste; Asthma; Hyperreaktivität der Atemwege; mikrovaskulärer Hypersensibilität; Bronchokonstriktion; chronischobstruktiver Lungenerkrankung; Harninkontinenz; Darmentzündung; und entzündlicher Darmerkrankung.

9. Verwendung einer Verbindung nach einem der Ansprüche 1 bis 6 zur Herstellung eines Medikaments zur Behandlung einer Krankheit, die ausgewählt ist aus: Psychose; Schizophrenie; schizophreniformer Störung; schizoaffektiver Störung; wahnhafter Störung; kurzzeitiger psychotischer Störung; geteilter psychotischer Störung; psychotischer Störung infolge eines allgemeinmedizinischen Leidens; substanz- oder drogeninduzierter psychotischer Störung (Kokain, Alkohol, Amphetamin usw.); schizoider Persönlichkeitsstörung; schizotypischer Persönlichkeitsstörung; mit typischer Depression, bipolarer Störung, Alzheimer'scher Krankheit oder Parkinson'scher Krankheit verbundener Psychose oder Schizophrenie; typischer Depression; allgemeiner Angststörung; bipolarer Störung (Erhaltungstherapie, Rezidivprävention und Stabilisierung); Manie; Hypomanie; kognitiver Störung; ADHD; Fettleibigkeit; Appetitreduzierung; Alzheimer'scher Krankheit; Parkinson'scher Krankheit; Schmerz; Konvulsionen; Huste; Asthma; Hyperreaktivität der Atemwege; mikrovaskulärer Hypersensibilität; Bronchokonstriktion; chronischobstruktiver Lungenerkrankung; Harninkontinenz; Darmentzündung; und entzündlicher Darmerkrankung.

10. Verwendung nach Anspruch 9, wobei die Herstellung ferner die Verwendung einer Verbindung umfasst, die aus der Liste bestehend aus D2-Antagonisten, partiellen D2-Agonisten, PDE10-Antagonisten, 5-HT_{2A}-Antagonisten, 5-HT₆-Antagonisten und KCNQ4-Antagonisten ausgewählt ist.

11. Pharmazeutische Zusammensetzung, umfassend eine Verbindung nach einem der Ansprüche 1 bis 10 und einen oder mehrere pharmazeutisch unbedenkliche Träger oder Exzipienten.

12. Zusammensetzung nach Anspruch 11, wobei die Zusammensetzung zusätzlich eine Verbindung umfasst, die aus der Liste bestehend aus D2-Antagonisten, partiellen D2-Agonisten, PDE10-Antagonisten, 5-HT_{2A}-Antagonisten, 5-HT₆-Antagonisten und KCNQ4-Antagonisten ausgewählt ist.

13. Kit, umfassend eine Verbindung nach einem der Ansprüche 1 bis 12 zusammen mit einer Verbindung, die aus der Liste bestehend aus D2-Antagonisten, partiellen D2-Agonisten, PDE10-Antagonisten, 5-HT_{2A}-Antagonisten, 5-HT₆-Antagonisten und KCNQ4-Antagonisten ausgewählt ist.

## Revendications

1. Composé selon la formule I 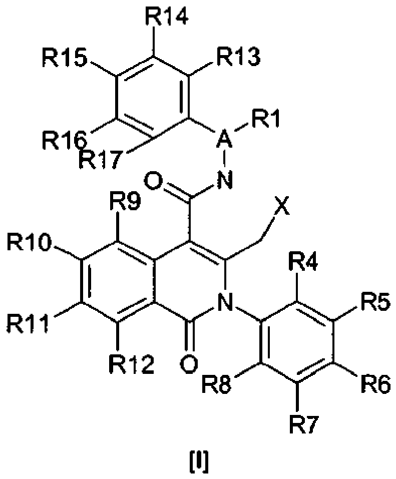 dans laquelle A représente N, CH ou CR¹ ;
dans laquelle chaque R¹ représente indépendamment un hydrogène, un alkyle en C_{1 à 6}, un alcényle en C_{2 à 6}, un alcynyle en C_{2 à 6}, -C(O)-alkyle en C_{1 à 6}, -C(O)-alcényle en C_{2 à 6}, -C(O)-alcynyle en C_{2 à 6}, -C(O)-O-alkyle en C_{1 à 6}, -C(O)-O-alcényle en C_{2 à 6}, -C(O)-O-alcynyle en C_{2 à} où phényle, où ledit phényle, alkyle en C_{1 à 6}, alcényle en C_{2 à 6} ou alcynyle en C_{2 à 6} est éventuellement substitué avec un ou plusieurs substituants choisis parmi halogène, hydroxyle, haloalkyle en C_{1 à 6}, nitro, alcoxy en C_{1 à 6}, et NR²R³ ; dans laquelle X représente un hydrogène, un alkyle en C_{1 à 6}, un alcényle en C_{2 à 6}, un alcynyle en C_{2 à 6}, cyano, -OR², -O-C(O)R², -OC(O)NR²R³, -C(O)-NR²R³, -N(R²)C(O)R³, -N(R²)-C(O)NR²R³ ou NR²R³, ou X représente un fragment monocyclique, bicyclique ou tricyclique comportant 4 à 16 atomes de cycle, dont un est l'azote, et dans laquelle un, deux ou trois atomes de cycle supplémentaires peuvent être un hétéroatome choisi parmi N, O et S, et dans laquelle ledit fragment monocyclique, bicyclique ou tricyclique peut facultativement être substitué avec un ou plusieurs substituants W, où W est choisi parmi hydrogène, hydroxy, halogène, cyano, (=O), -O-(CH₂)_{c}-O-, où c est 2 ou 3 (spiro), (CH₂)_{d}, où d est 5 ou 6 (spiro), alkyle en C_{1 à 6}, alcényle en C_{2 à 6}, alcynyle en C_{2 à 6}, alcoxy en C_{2 à 6}, -C(O)- alkyle en C_{1 à 6}, -C(O)-alcényle en C_{2 à 6}, -C(O)-alcynyle en C_{2 à 6}, -C(O)-O- alkyle en C_{1 à 6}, -C(O)-O-alcényle en C_{2 à 6}, -C(O)-O-alcynyle ex C_{2 à 6}, -O-C(O)-alkyle en C_{1 à 6}, -O-C(O)-alcényle en C_{2 à 6}, -O-C(O)-alcynyle en C_{2 à 6}, -C(O)H, COOH ; ou dans laquelle W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle chacun de R⁴ à R⁸, R⁹ à R¹², et R¹³ à R¹⁷ représente indépendamment un hydrogène, un alkyle en C_{1 à 6}, un alcényle en C_{2 à 6}, un alcynyle en C_{2 à 6}, un halogène, NR²R³, un hydroxy, un cyano, un nitro, un alcoxy en C_{1 à 6}, un haloalkyle en C_{1 à 6} ou un hydroxyalkyle en C_{1 à 6} ;
dans laquelle R² et R³ représentent indépendamment un hydrogène, un alkyle en C_{1 à 6}, un alcényle en C_{2 à 6}, un alcynyle en C_{2 à 6}, un hydroxyalkyle en C_{1 à 6}, un haloalkyle en C_{1 à 6} ou un phényle ;
et leurs sels acceptables sur le plan pharmaceutique.

2. Composé selon la revendication 1, dans lequel R⁴ à R⁸ représentent l'hydrogène.

3. Composé selon la revendication 1, dans lequel R⁹ à R¹² représentent l'hydrogène.

4. Composé selon la revendication 1, dans lequel R¹³ à R¹⁷ représentent l'hydrogène.

5. Composé selon la revendication 1 de
- formule Iₐ 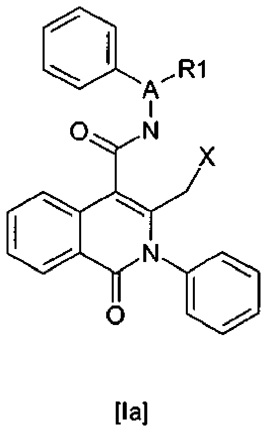 dans laquelle A représente N, CH ou CR¹ ;
dans laquelle chaque R¹ représente indépendamment un hydrogène, un alkyle en C_{1 à 6}, -C(O)-alkyle en C_{1 à 6}, -C(O)-O-alkyle en C_{1 à 6}, ou un phényle, dans laquelle ledit phényle ou alkyle en C_{1 à 6} est éventuellement substitué avec un ou plusieurs substituants choisis parmi halogène, hydroxyle, haloalkyle en C_{1à 6}, nitro, alcoxy en C_{1 à 6}, et NR²R³;
dans laquelle X représente un hydrogène, un alkyle en C_{1 à 6}, un cyano, -OR², -O-C(O)R², -OC(O)NR²R³, -C(O)-NR²R³, -N(R²)C(O)R³, -N(R²)-C(O)NR²R³ ou NR²R³, ou X représente un fragment monocyclique, bicyclique ou tricyclique comportant 4 à 16 atomes de cycle dont un est l'azote, et dans laquelle un, deux ou trois atomes de cycle supplémentaires peuvent être un hétéroatome choisi parmi N, O et S, et dans laquelle ledit fragment monocyclique, bicyclique ou tricyclique peut facultativement être substitué avec un ou plusieurs substituants W, où W est choisi parmi hydrogène, hydroxy, halogène, cyano, (=O), -O-(CH₂)_{c}-O-, où c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle chacun de R⁴ à R⁸, R⁹ à R¹², et R¹³ à R¹⁷ représente indépendamment un hydrogène, un alkyle en C_{1 à 6}, un alcényle en C_{2 à 6}, un alcynyle en C_{2 à 6}, un halogène, NR²R³, un hydroxy, un cyano, un nitro, un alcoxy en C_{1 à 6}, un haloalkyle en C_{1 à 6} ou un hydroxyalkyle en C_{1 à 6} ;
dans laquelle R² et R³ représentent indépendamment un hydrogène, un alkyle en C_{1 à 6}, un alcényle en C_{2 à 6}, un alcynyle en C_{2 à 6}, un hydroxyalkyle en C_{1 à 6}, un haloalkyle en C_{1 à 6} ou un phényle ;
et leurs sels acceptables sur le plan pharmaceutique ; ou
- formule 1b 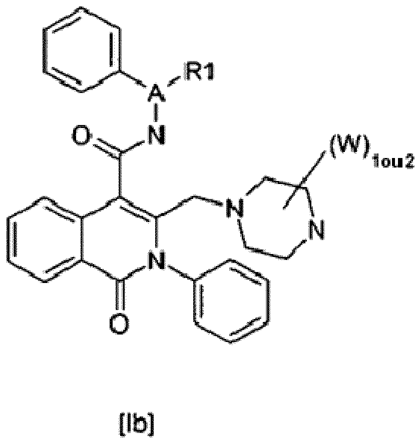 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³ ;
dans laquelle W est choisi parmi hydrogène, hydroxy, halogène, cyano, (=O), -O-(CH₂)_{c}O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle W représente un fragment de formule -(CH₂)ₐ-Y-(CH₂)b-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule Ic 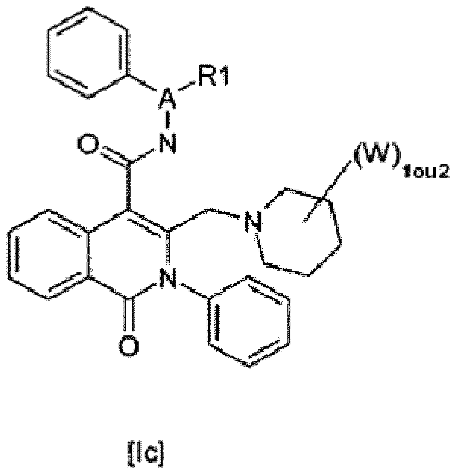 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³ ;
dans laquelle W est choisi parmi un hydrogène, un hydroxy, un halogène, cyano, (=O), -O-(CH₂)_{c}-O-, où c est 2 ou 3 (spiro), (CH2)d, où d est 5 ou 6 (spiro), alkyle en C_{1 à 6}, alcoxy en C_{1 à 6}, -C(O)-alkyle en C_{1 à 6}, -C(O)-O-alkyl en C_{1 à 6}, -O-C(O)-alkyle en C_{1 à 6}, -C(O)H, COOH ; ou dans laquelle W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- la formule Id 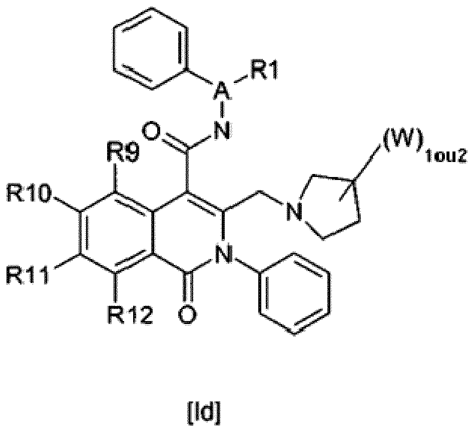 dans laquelle A représente N, CH ou CR¹ ;
dans laquelle chaque R¹ représente indépendamment un hydrogène, un alkyle en C_{1 à 6}, -C(O)-alkyle en C_{1 à} , C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C₁ à ₆, et NR²R³¹;
dans laquelle W est choisi parmi hydrogène, hydroxy, halogène, cyano, (=O), -O-(CH₂)_{c}-O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
dans laquelle R⁹-R¹² représentent indépendamment un hydrogène ou un halogène ;
et leurs sels acceptables sur le plan pharmaceutique ; ou
- formule Iₑ 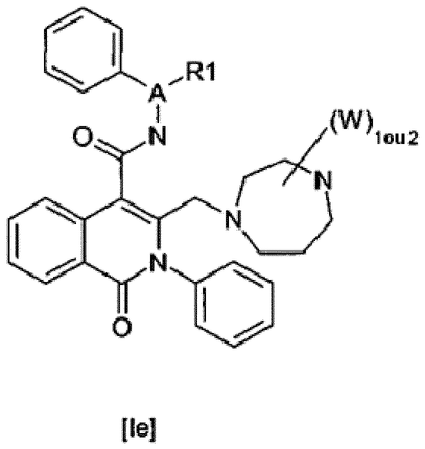 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³ ;
dans laquelle W est choisi parmi hydrogène, hydroxy, halogène, cyano, (=O), -O-(CH₂)_{c}O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule I_{f} 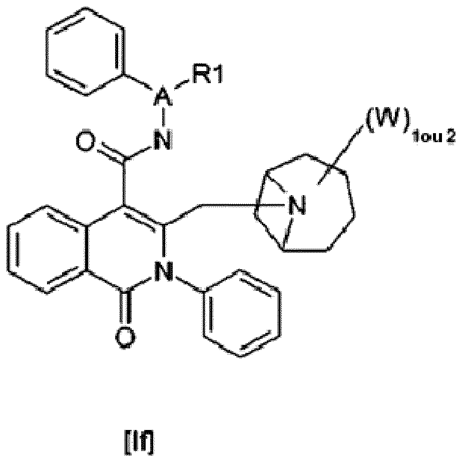 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou un phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³;
dans laquelle W est choisi parmi hydrogène, hydroxy, halogène, cyano, (=O), -O-(CH₂)_{c}-O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à} , -C(O)-O-alkyle en C_{1 à 6}, -O-C(O)-alkyle en C_{1 à 6}, -C(O)H, COOH ; ou dans laquelle W représente un fragment de formule -(CH₂)ₐ-Y-(CH2)b-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule I_{g} 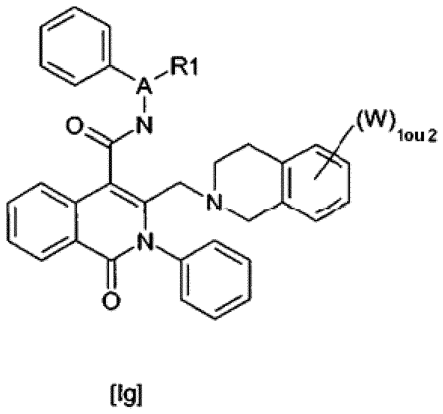 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³ ;
dans laquelle W est choisi parmi hydrogène, hydroxy, halogène, cyano, (=O), -O-(CH₂)_{c}-O-, dans laquelle c est 2 ou 3 (spiro), (CH₂)ₐ, où d est 5 ou 6 (spiro), alkyle en C_{1 à 6}, alcoxy en C_{1 à 6}, -C(O)-alkyle en C_{1 à 6}, -C(O)-O- alkyle en C_{1 à 6}, -O-C(O)- alkyle en C_{1 à 6}, -N(R²)-C(O)-R³, -C(O)H, COOH ; ou dans laquelle W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule Iₕ 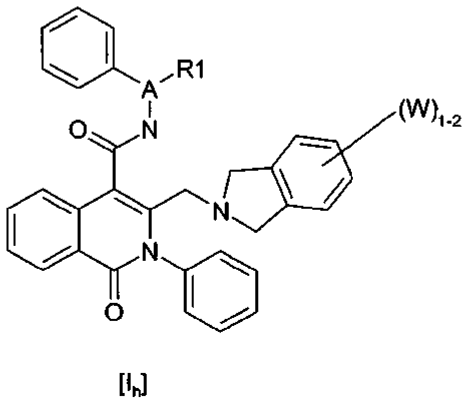 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³;
dans laquelle W est choisi parmi hydrogène, hydroxy, halogène, cyano, (=O), -O-(CH₂)_{c}-O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle W représente un fragment de formule -(CH2)ₐ-Y-(CH2)b-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule Iᵢ 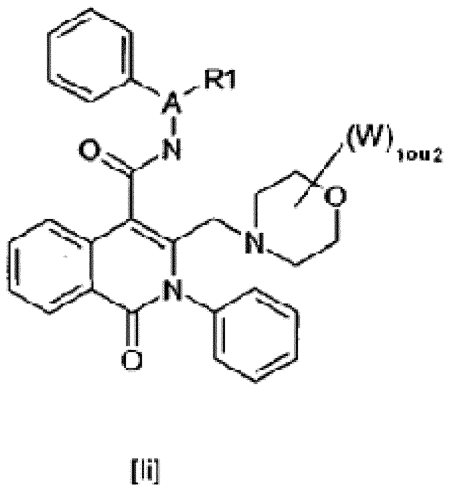 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³¹;
dans laquelle W est choisi parmi hydrogène, hydroxy, halogène, cyano, (=O), -O-(CH₂)_{c}-O-, dans laquelle c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle X représente un fragment de formule -(CH₂)ₐ-Y-(CH2)b-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, pyrazinyle, phényle, pyridyle, et phényle à substitution halogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule Iⱼ 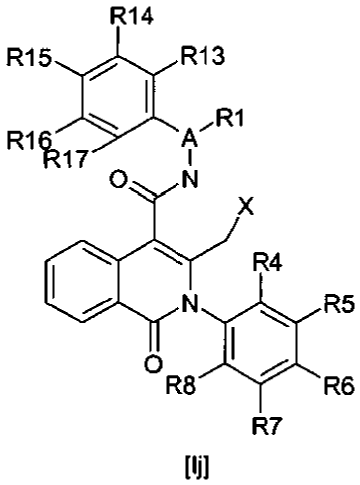 dans laquelle A représente N, CH ou CR¹ ;
dans laquelle chaque R¹ représente indépendamment un hydrogène, un alkyle en C_{1 à 6}, -C(O)-alkyle en C_{1 à 6}, C(O)-O-alkyle en C_{1 à 6}, ou phényle, dans laquelle ledit phényle, ou alkyle en C_{1 à 6} est éventuellement substitué avec un ou plusieurs substituants choisis parmi un halogène, un hydroxyl, un haloalkyle en C_{1 à 6}, un nitro, un alcoxy en C_{1 à 6}, et NR²R³¹;
dans laquelle X représente un hydrogène, un alkyle en C_{1 à 6}, un cyano, -OR², -O-C(O)R², -OC(O)NR²R³, -C(O)-NR²R³, -N(R²)C(O)R³, -N(R²)-C(O)NR²R³ ou NR²R³, ou X représente un fragment monocyclique, bicyclique ou tricyclique comportant 4 à 16 atomes de cycle dont un est l'azote, et dans laquelle un, deux ou trois atomes de cycle supplémentaires peuvent être un hétéroatome choisi parmi N, O et S, et dans laquelle ledit fragment monocyclique, bicyclique ou tricyclique peut facultativement être substitué avec un ou plusieurs substituants W, où W est choisi parmi hydrogène, hydroxy, halogène, cyano, (=O), -O-(CH₂)_{c}-O-, où c est 2 ou 3 (spiro), (CH₂)_{d}, où d est 5 ou 6 (spiro), alkyle en C_{1 à 6}, alcoxy en C_{1 à 6}, -C(O)-alkyle en C_{1 à 6}, -C(O)-O-alkyle en C_{1 à 6}, -O-C(O)-alkyle en C_{1 à 6}, -C(O)H, COOH ; ou dans laquelle W représente un fragment de formule -(CH₂)a-Y-(CH₂)_{b}-Z ;
dans laquelle a et b représentent indépendamment un nombre entier choisi parmi 0, 1, 2 et 3 ;
dans laquelle Y représente une liaison, C(O), O, S, -OC(O), -C(O)-O-, -NR²-, -NR²-C(O)-, -C(O)-NH-, ou S(O)₂ ;
dans laquelle Z représente un hydrogène, un alkyle en C_{1 à 6}, NR²R³, cyano ou un fragment monocyclique ou bicyclique fusionné comprenant 4 à 12 atomes de cycle dont éventuellement un, deux ou trois sont des hétéroatomes choisis parmi N, O, et S, et dans laquelle ledit fragment monocyclique ou bicyclique fusionné est éventuellement substitué avec un ou plusieurs substituants choisis parmi le groupe constitué d'halogène, cyano, alkyle en C_{1 à 6}, hydroxyle, et alcoxy en C_{1 à 6}, phényle, pyridyle, et phényle à substitution halogène ;
dans laquelle un de R⁴ à R⁸ représente un halogène et les autres représentent un hydrogène ;
dans laquelle un de R¹³ à R¹⁷ représente un halogène et les autres représentent un hydrogène ;
dans laquelle R² et R³ représentent indépendamment un hydrogène, un alkyle en C_{1 à 6}, un hydroxyalkyle en C_{1 à 6}, un haloalkyle en C_{1 à 6} ou un phényle ;
et leurs sels acceptables sur le plan pharmaceutique ; ou
- formule Ik 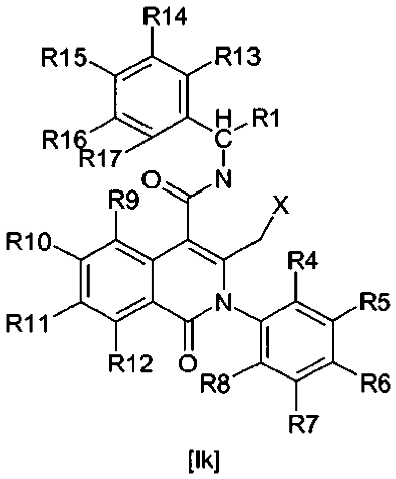 dans laquelle R¹ représente un hydrogène, un alkyle en C_{1 à 6}, un alcényle en C_{2 à 6} ou un alcynyle en C_{2 à 6} ;
dans laquelle X représente un hydrogène, un alkyle en C_{1 à 6}, un alcényle en C_{2 à 6}, un alcynyle en C_{2 à 6} ou NR²R³, ou X représente un fragment monocyclique ou bicyclique comportant 5 à 9 atomes de cycle, dont un est l'azote, et où un, deux ou trois atomes de cycle supplémentaires peuvent être un hétéroatome choisi parmi N, O et S, et où ledit fragment monocyclique ou bicyclique peut facultativement être substitué avec un ou plusieurs substituants W, où W est choisi parmi hydrogène, halogène, hydroxy, (=O), alkyle en C_{1 à 6}, alcényle en C_{2 à 6}, alcynyle en C_{2 à 6}, alcoxy en C_{1 à 6}, ou dans laquelle formule W représente un fragment de formule -(CH₂)ₐ-Y-(CH₂)_{b}-Z ;
dans laquelle a et b représentent indépendamment un nombre entier choisi parmi 0, 1, 2 et 3 ;
dans laquelle Y représente une liaison, et dans laquelle Z représente un fragment monocyclique comprenant 5 à 6 atomes de cycle dont éventuellement un, deux ou trois sont des hétéroatomes choisis parmi N, O, et S, et dans laquelle ledit fragment monocyclique est éventuellement substitué avec un ou plusieurs substituants choisis parmi le groupe constitué d'un halogène, un alkyle en C_{1 à 6}, un hydroxyle, et un alcoxy en C_{1 à 6} ;
dans laquelle chacun de R⁴ à R⁸ représente indépendamment un hydrogène ou un halogène ;
dans laquelle chacun de R⁹ à R¹² représente indépendamment un hydrogène ou un halogène pour autant qu'au moins un parmi R⁹ à R¹² représente un halogène ;
dans laquelle chacun de R¹³ à R¹⁷ représente indépendamment un hydrogène ou un halogène ;
dans laquelle R² et R³ représentent indépendamment un hydrogène, un alkyle en C_{1 à 6}, un alcényle en C_{2 à 6}, un alcynyle en C_{2 à 6} ; et leurs sels acceptables sur le plan pharmaceutique ; ou
- formule Ik' 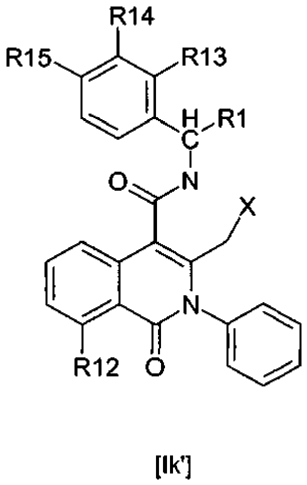 dans laquelle R¹ est choisi parmi un alkyle en C_{1 à 6} ;
dans laquelle X est choisi parmi un hydrogène, un alkyle en C₁ à ₆ ou NR²R³, ou X représente un fragment monocyclique ou bicyclique comportant 5 à 9 atomes de cycle, dont un est l'azote, et où un, deux ou trois atomes de cycle supplémentaires peuvent être un hétéroatome choisi parmi N, O et S, et où ledit fragment monocyclique ou bicyclique peut facultativement être substitué avec un ou plusieurs substituants W, où W est choisi parmi hydrogène, hydroxy, (=O), alkyle en C_{1 à 6} ou dans laquelle formule W représente un fragment de formule -(CH₂)ₐ-Y-(CH₂)_{b}-Z;
dans laquelle a et b représentent indépendamment un nombre entier choisi parmi 0, 1, 2 et 3 ;
dans laquelle Y représente une liaison, et dans laquelle Z représente un fragment monocyclique comprenant 5 à 6 atomes de cycle dont éventuellement un est un hétéroatome choisi parmi N, O, et S, et dans laquelle ledit fragment monocyclique est éventuellement substitué avec un ou plusieurs substituants choisis parmi le groupe constitué d'un halogène, un alkyle en C_{1 à 6}, un hydroxyle, et un alcoxy en C_{1 à 6} ;
dans laquelle R¹² représente un halogène ;
R¹³ à R¹⁵ représentent chacun indépendamment un hydrogène ou un halogène ;
et leurs sels acceptables sur le plan pharmaceutique ; ou
- formule Ik" 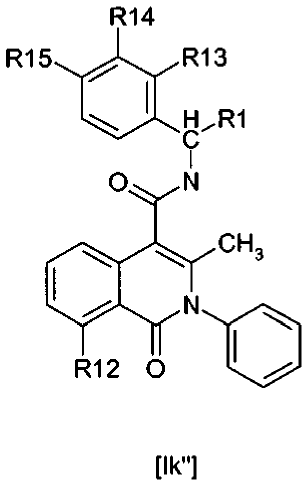 dans laquelle R¹ représente un éthyle, un cyclopropyle ou un cyclobutyle ;
dans laquelle R¹² représente un radical chloro ; et
R¹³, R¹⁴ et R¹⁵ représentent chacun individuellement un hydrogène, un radical fluoro ou chloro, dans laquelle deux parmi R¹³, R¹⁴ et R¹⁵ représentent un hydrogène ; ou
- pratiquement les énantiomères S tels que représentés dans la formule Ik"' 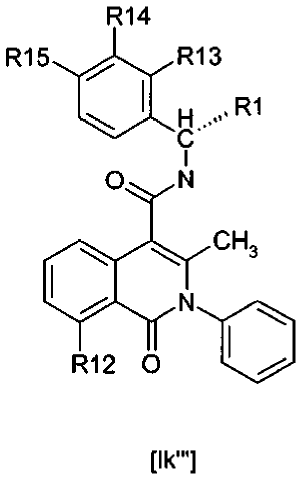 dans laquelle R¹ représente un éthyle ou un cyclopropyle ; dans laquelle R¹² représente un radical chloro ; et
R¹³, R¹⁴ et R¹⁵ représentent chacun individuellement un hydrogène, un radical fluoro ou chloro, dans laquelle deux parmi R¹³, R¹⁴ et R¹⁵ représentent un hydrogène ; ou
- formule Id' 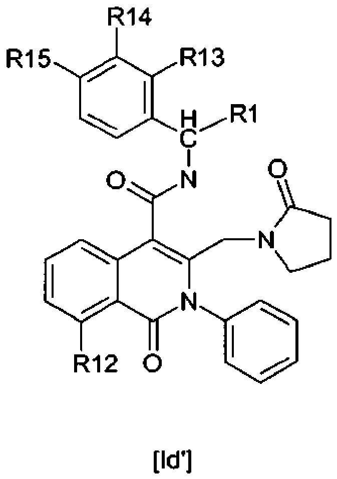 dans laquelle R¹ représente un éthyle ou un cyclopropyle ; R¹² représente un halogène ;
R¹³, R¹⁴ et R¹⁵ représentent chacun indépendamment un hydrogène ou un halogène ;
et leurs sels acceptables sur le plan pharmaceutique ; ou
- formule Id" 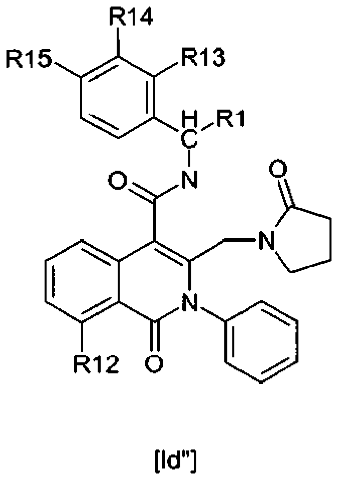 dans laquelle R¹ représente un éthyle ou un cyclopropyle ; R¹² représente un radical chloro ou fluoro ;
R¹², R¹⁴ et R¹⁵ représentent chacun indépendamment un hydrogène, un radical chloro ou fluoro, où deux parmi R¹², R¹⁴ et R¹⁵ représentent un hydrogène ;
et leurs sels acceptables sur le plan pharmaceutique ; ou
- pratiquement l'énantiomère S tel que représenté dans la formule Id"'
où « pratiquement » définit un excès d'énantiomère d'au
moins 60%, 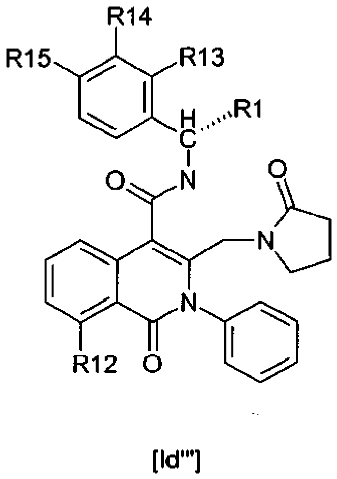 dans laquelle R¹ représente un éthyle ou un cyclopropyle ; R¹² représente un radical chloro ou fluoro ;
R¹², R¹⁴ et R¹⁵ représentent chacun indépendamment un hydrogène, un radical chloro ou fluoro, où deux parmi R¹², R¹⁴ et R¹⁵ représentent un hydrogène ;
et leurs sels acceptables sur le plan pharmaceutique.

6. Composé selon la revendication 1, choisi parmi :
1a Acide 3 -méthyl -1 -oxo -2 -phényl -1,2 -dihydro -isoquinoline -4 - carboxylique ((S) -1 -phényl -propyl) -amide
1b Acide 3,6 -diméthyl -1 -oxo -2 -phényl -1,2 -dihydro -isoquinoline -4 - carboxylique ((S) -1 -phényl -propyl) -amide
1c Acide 7 -chloro -3 -méthyl -1 -oxo -2 -phényl -1,2 -dihydro - isoquinoline -4 -carboxylique ((S) -1 -phényl -propyl) -amide
1d Acide 7 -bromo -3 -méthyl -1 -oxo -2 -phényl -1,2 -dihydro - isoquinoline -4 -carboxylique ((S) -1 -phényl -propyl) -amide
1e Acide 6 -fluoro -3 -méthyl -1 -oxo -2 -phényl -1 ,2 -dihydro - isoquinoline -4 -carboxylique ((S) -1 -phényl -propyl) -amide
1f Acide 3,5 -diméthyl -1 -oxo -2 -phényl -1,2 -dihydro -isoquinoline -4 - carboxylique ((S) -1 -phényl -propyl) -amide
1g Acide 7 -fluoro -3 -méthyl -1 -oxo -2 -phényl -1,2 -dihydro - isoquinoline -4 -carboxylique ((S) -1 -phényl -propyl) -amide
1h Acide 3,7 -diméthyl -1 -oxo -2 -phényl -1,2 -dihydro -isoquinoline -4 - carboxylique ((S) -1 -phényl -propyl) -amide
1i Acide 8 -chloro -3 -méthyl -1 -oxo -2 -phényl -1,2 -dihydro - isoquinoline -4 -carboxylique ((S) -1 -phényl -propyl) -amide
1r Acide 3 -méthyl -1 -oxo -2 -phényl -1,2 -dihydro -isoquinoline -4 - carboxylique ((S) - cyclopropyl -phényl -méthyl) -amide
1s Acide 3 -méthyl -1 -oxo -2 -phényl -1,2 -dihydro -isoquinoline -4 - carboxylique [(S) -cyclopropyl -(3 -fluoro -phényl) -méthyl] -amide
1j Acide 3,8 -diméthyl -1 -oxo -2 -phényl -1,2 -dihydro -isoquinoline -4 - carboxylique ((S) -1 -phényl -propyl) -amide
1k Acide 2 -(2 -fluoro -phényl) -3 -méthyl -1 -oxo -1,2 -dihydro - isoquinoline -4 -carboxylique ((S) -1 -phényl -propyl) -amide
1l Acide 3 -Méthyl -1 -oxo -2 -o -tolyl -1,2 -dihydro -isoquinoline -4 - carboxylique ((S) -1 -phényl -propyl) -amide
1m Acide 2 -(2 -chloro -phényl) -3 -méthyl -1 -oxo -1,2 -dihydro - isoquinoline -4 -carboxylique ((S) -1 -phényl -propyl) -amide
1n Acide 2 -(2,6 -difluoro -phényl) -3 -méthyl -1 -oxo -1,2 -dihydro - isoquinoline -4 -carboxylique ((S) -1 -phényl -propyl) -amide
1o Acide 2 -(3 -fluoro -phényl) -3 -méthyl -1 -oxo -1,2 -dihydro - isoquinoline -4 -carboxylique ((S) -1 -phényl -propyl) -amide
1p Acide 3 -méthyl -1 -oxo -2 -m -tolyl -1,2 -dihydro -isoquinoline -4 - carboxylique ((S) -1 -phényl -propyl) -amide
1q Acide 2 -(3 -chloro -phényl) -3 -méthyl -1 -oxo -1,2 -dihydro - isoquinoline -4 -carboxylique ((S) -1 -phényl -propyl) -amide
1t Acide 8 -chloro -3 -méthyl -1 -oxo -2 -phényl -1,2 -dihydro - isoquinoline -4 -carboxylique [(S) -cyclopropyl -(3 -fluoro -phényl) - méthyl] -amide
2a Acide 3 -diméthylaminométhyl -1 -oxo -2 -phényl -1,2 -dihydro - isoquinoline -4 -carboxylique ((S) -1 -phényl -propyl) -amide
2b Acide 3 -méthylaminométhyl -1 -oxo -2 -phényl -1,2 -dihydro - isoquinoline -4 -carboxylique ((S) -1 -phényl -propyl) -amide
2c Acide 3 -éthylaminométhyl -1 -oxo -2 -phényl -1 ,2 -dihydro - isoquinoline -4 -carboxylique ((S) -1 -phényl -propyl) -amide
2d Acide 3 -cyclopropylaminométhyl -1 -oxo -2 -phényl -1,2 -dihydro - isoquinoline -4 -carboxylique ((S) -1 -phényl -propyl) -amide
2e Acide 3 -[(cyclopropylméthyl -amino) -méthyl] -1 -oxo -2 -phényl -1,2 - dihydro -isoquinoline -4 -carboxylique ((S) -1 -phényl -propyl) -amide
2f Acide 3 -(3,6 -dihydro -2H -pyridin -1 -ylméthyl) -1 -oxo -2 -phényl -1,2 -dihydro -isoquinoline -4 -carboxylique ((S) -1 -phényl -propyl) -amide
2g Acide 3 -[4 -(2 -méthoxy -phényl) -pipérazin -1 -ylméthyl] -1 -oxo -2 - phényl -1,2 -dihydro -isoquinoline -4 -carboxylique ((S) -1 -phényl - propyl) -amide
2h Acide 3 -[4 -(4 -fluoro -phényl) -pipérazin -1 -ylméthyl] -1 -oxo -2 - phényl -1,2 -dihydro -isoquinoline -4 -carboxylique ((S) -1 -phényl - propyl) -amide
2i Acide 3 -(4 -formyl -pipérazin -1 -ylméthyl) -1 -oxo -2 -phényl -1,2 - dihydro -isoquinoline -4 -carboxylique ((S) -1 -phényl -propyl) -amide
2j Acide 4-[1 -oxo -2 -phényl -4 -(1 -phényl -propylcarbamoyl) -1,2 - dihydro -isoquinolin -3 -ylméthyl] -pipérazine -1 -carboxylique éthyl ester
2k Acide 3 -(4 -méthyl -pipérazin -1 -ylméthyl) -1 -oxo -2 -phényl -1,2 - dihydro -isoquinoline -4 -carboxylique ((S) -1 -phényl -propyl) -amide
2l Acide 1 -oxo -2 -phényl -3 -(1,3,4,9 -tétrahydro -bêta -carbolin -2 - ylméthyl) -1,2 -dihydro -isoquinoline -4 -carboxylique ((S) -1 -phényl - propyl) -amide
2m Acide 1 -oxo -2 -phényl -3 -pipérazin -1 -ylméthyl -1,2 -dihydro - isoquinoline -4 -carboxylique ((S) -1 -phényl -propyl) -amide
2n Acide 3 -(3 -méthyl -pipérazin -1 -ylméthyl) -1 -oxo -2 -phényl -1,2 - dihydro -isoquinoline -4 -carboxylique ((S) -1 -phényl -propyl) -amide
2o Acide 3 -(3,5 -diméthyl -pipérazin -1 -ylméthyl) -1 -oxo -2 -phényl -1,2 -dihydro -isoquinoline -4 -carboxylique ((S) -1 -phényl -propyl) -amide
2p Acide 3 -(4 -benzyl -pipérazin -1 -ylméthyl) -1 -oxo -2 -phényl -1,2 - dihydro -isoquinoline -4 -carboxylique ((S) -1 -phényl -propyl) -amide
2q Acide 1 -oxo -3 -[4 -(2 -oxo -2 -pyrrolidin -1 -yl -éthyl) -pipérazin -1 - ylméthyl] -2 -phényl -1,2 -dihydro -isoquinoline -4 -carboxylique ((S) -1 - phényl -propyl) -amide
2r Acide 3 -morpholin -4 -ylméthyl -1 -oxo -2 -phényl -1,2 -dihydro - isoquinoline -4 -carboxylique ((S) -1 -phényl -propyl) -amide
2s Acide 3 -(2,6 -diméthyl -morpholin -4 -ylméthyl) -1 -oxo -2 -phényl -1,2 -dihydro -isoquinoline -4 -carboxylique ((S) -1 -phényl -propyl) -amide
2t Acide 1 -oxo -2 -phényl -3 -thiomorpholin -4 -ylméthyl -1,2 -dihydro - isoquinoline -4 -carboxylique ((S) -1 -phényl -propyl) -amide
2u Acide 3 -(1,4 -dioxa -8 -aza -spiro[4.5]dec -8 -ylméthyl) -1 -oxo -2 - phényl -1,2 -dihydro -isoquinoline -4 -carboxylique ((S) -1 -phényl - propyl) -amide
2v Acide 1 -oxo -2 -phényl -3 -pipéridin -1 -ylméthyl -1,2 -dihydro - isoquinoline -4 -carboxylique ((S) -1 -phényl -propyl) -amide
2w Acide 3 -(2 -méthyl -pipéridin -1 -ylméthyl) -1 -oxo -2 -phényl -1,2 - dihydro -isoquinoline -4 -carboxylique ((S) -1 -phényl -propyl) -amide
2x Acide 3 -(2,6 -diméthyl -pipéridin -1 -ylméthyl) -1 -oxo -2 -phényl -1,2 - dihydro -isoquinoline -4 -carboxylique ((S) -1 -phényl -propyl) -amide
2y Acide 3 -(2 -hydroxyméthyl -pipéridin -1 -ylméthyl) -1 -oxo -2 -phényl - 1,2 -dihydro -isoquinoline -4 -carboxylique ((S) -1 -phényl -propyl) -amide
2z Acide 1 -[1 -oxo -2 -phényl -4 -(1 -phényl -propylcarbamoyl) -1,2 - dihydro -isoquinolin -3 -ylméthyl] -pipéridine -3 -carboxylique éthyl ester
2aa Acide 3 -(3 -méthyl -pipéridin -1 -ylméthyl) -1 -oxo -2 -phényl -1,2 - dihydro -isoquinoline -4 -carboxylique ((S) -1 -phényl -propyl) -amide
2ab Acide 3 -(4 -hydroxy -pipéridin -1 -ylméthyl) -1 -oxo -2 -phényl -1,2 - dihydro -isoquinoline -4 -carboxylique ((S) -1 -phényl -propyl) -amide
2ac Acide 1 -oxo -2 -phényl -3 -(4 -phényl -pipéridin -1 -ylméthyl) -1,2 - dihydro -isoquinoline -4 -carboxylique ((S) -1 -phényl -propyl) -amide
2ad Acide 1 -[1 -oxo -2 -phényl -4 -(1 -phényl -propylcarbamoyl) -1,2 - dihydro -isoquinolin -3 -ylméthyl] -pipéridine -4 -carboxylique éthyl ester
2ae Acide 3 -(4 -méthyl -pipéridin -1 -ylméthyl) -1 -oxo -2 -phényl -1,2 - dihydro -isoquinoline -4 -carboxylique ((S) -1 -phényl -propyl) -amide
2af Acide 1 -oxo -2 -phényl -3 -(4 -pyridin -2 -yl -pipérazin -1 -ylméthyl) - 1,2 -dihydro -isoquinoline -4 -carboxylique ((S) -1 -phényl -propyl) -amide
2ag Acide 3 -(octahydro -quinolin -1 -ylméthyl) -1 -oxo -2 -phényl -1,2 - dihydro -isoquinoline -4 -carboxylique ((S) -1 -phényl -propyl) -amide
2ah Acide 3 -azépan -1 -ylméthyl -1 -oxo -2 -phényl -1,2 -dihydro - isoquinoline -4 -carboxylique ((S) -1 -phényl -propyl) -amide
2ai Acide 3 -(3 -hydroxy -pipéridin -1 -ylméthyl) -1 -oxo -2 -phényl -1,2 - dihydro -isoquinoline -4 -carboxylique ((S) -1 -phényl -propyl) -amide
2aj Acide 3 -[4 -(2,4 -diméthyl -phényl) -pipérazin -1 -ylméthyl] - 1 -oxo -2 -phényl -1,2 -dihydro -isoquinoline -4 -carboxylique ((S) -1 -phényl - propyl) -amide
2ak Acide 3 -[4 -(3,4 -diméthyl -phényl) -pipérazin -1 -ylméthyl] -1 -oxo -2 -phényl -1,2 -dihydro -isoquinoline -4 -carboxylique ((S) -1 -phényl - propyl) -amide
2al Acide 3 -(4 -diméthylamino -pipéridin -1 -ylméthyl) -1 -oxo -2 -phényl -1,2 -dihydro -isoquinoline -4 -carboxylique ((S) -1 -phényl -propyl) - amide
2am Acide 3 -[4 -(2,5 -diméthyl -phényl) -pipérazin -1 -ylméthyl] -1 -oxo - 2 -phényl -1,2 -dihydro -isoquinoline -4 -carboxylique ((S) -1 -phényl - propyl) -amide
2an Acide 3 -[4 -(2 -fluoro -phényl) -pipérazin -1 -ylméthyl] -1 -oxo -2 - phényl -1,2 -dihydro -isoquinoline -4 -carboxylique ((S) -1 -phényl - propyl) -amide
2ao Acide 3 -[4 -(3 -méthoxy -phényl) -pipérazin -1 -ylméthyl] -1 -oxo -2 - phényl -1,2 -dihydro -isoquinoline -4 -carboxylique ((S) -1 -phényl - propyl) -amide
2ap Acide 1 -oxo -2 -phényl -3 -(4 -m -tolyl -pipérazin -1 -ylméthyl) -1,2 - dihydro -isoquinoline -4 -carboxylique ((S) -1 -phényl -propyl) -amide
2aq Acide 3 -[4 -(4 -méthoxy -phényl) -pipérazin -1 -ylméthyl] -1 -oxo -2 - phényl -1,2 -dihydro -isoquinoline -4 -carboxylique ((S) -1 -phényl - propyl) -amide
2ar Acide 1 -oxo -3 -(4 -phénétyl -pipérazin -1 -ylméthyl) -2 -phényl -1,2 - dihydro -isoquinoline -4 -carboxylique ((S) -1 -phényl -propyl) -amide
2as Acide 1 -oxo -2 -phényl -3 -(4 -pyrimidin -2 -yl -pipérazin -1 - ylméthyl) -1,2 -dihydro -isoquinoline -4 -carboxylique ((S) -1 -phényl - propyl) -amide
2at Acide 3 -[4 -(2 -cyano -phényl) -pipérazin -1 -ylméthyl] -1 -oxo -2 - phényl -1,2 -dihydro -isoquinoline -4 -carboxylique ((S) -1 -phényl - propyl) -amide
2au Acide 3 -[4 -(4 -chloro -phényl) -pipérazin -1 -ylméthyl] -1 -oxo -2 - phényl -1,2 -dihydro -isoquinoline -4 -carboxylique ((S) -1 -phényl - propyl) -amide
2av Acide 3 -((1 S,3R,5R) -3 -hydroxy -8 -aza -bicyclo[3.2.1 ]oct -8 - ylméthyl) -1 -oxo -2 -phényl -1,2 -dihydro -isoquinoline -4 -carboxylique ((S) -1 -phényl -propyl) -amide
2aw Acide 3 -(4 -acétyl -pipérazin -1 -ylméthyl) -1 -oxo -2 -phényl -1,2 - dihydro -isoquinoline -4 -carboxylique ((S) -1 -phényl -propyl) -amide
2ax Acide 3 -(4 -méthyl -[1,4]diazépan -1 -ylméthyl) -1 -oxo -2 -phényl - 1,2 -dihydro -isoquinoline -4 -carboxylique ((S) -1 -phényl -propyl) -amide
2ay Acide 3 -(4 -éthyl -pipérazin -1 -ylméthyl) -1 -oxo -2 -phényl -1,2 - dihydro -isoquinoline -4 -carboxylique ((S) -1 -phényl -propyl) -amide
2az Acide 3 -((2S,6R) -2,6 -diméthyl -morpholin -4 -ylméthyl) -1 -oxo -2 - phényl -1,2 -dihydro -isoquinoline -4 -carboxylique ((S) -1 -phényl - propyl) -amide
2ba Acide 3 -[4 -(2,4 -difluoro -phényl) -pipérazin -1 -ylméthyl] -1 -oxo -2 -phényl -1,2 -dihydro -isoquinoline -4 -carboxylique ((S) -1 -phényl - propyl) -amide
2bb Acide 3 -[4 -(3 -diméthylamino -propyl) -pipérazin -1 -ylméthyl] -1 - oxo -2 -phényl -1,2 -dihydro -isoquinoline -4 -carboxylique ((S) -1 -phényl -propyl) -amide
2bc Acide 3 -(4 -Isopropyl -pipérazin -1 -ylméthyl) -1 -oxo -2 -phényl -1,2 -dihydro -isoquinoline -4 -carboxylique ((S) -1 -phényl -propyl) -amide
2bd Acide 3 -(3 -aza -bicyclo[3.2.2]non -3 -ylméthyl) -1 -oxo -2 -phényl - 1,2 -dihydro -isoquinoline -4 -carboxylique ((S) -1 -phényl -propyl) -amide
2be Acide 3 -(4 -cyclopentyl -pipérazin -1 -ylméthyl) -1 -oxo -2 -phényl -1 ,2 -dihydro -isoquinoline -4 -carboxylique ((S) -1 -phényl -propyl) -amide
2bf Acide 3 -[1,4']bipipéridinyl -1' -ylméthyl -1 -oxo -2 -phényl -1,2 - dihydro -isoquinoline -4 -carboxylique ((S) -1 -phényl -propyl) -amide
2bg Acide 3 -(3,4 -dihydro -1 h -isoquinolin -2 -ylméthyl) -1 -oxo -2 - phényl -1 ,2 -dihydro -isoquinoline -4 -carboxylique ((S) -1 -phényl - propyl) -amide
2bh Acide 3 -[4 -(2 -diméthylamino -éthyl) -pipérazin -1 -ylméthyl] -1 -oxo -2 -phényl -1,2 -dihydro -isoquinoline -4 -carboxylique ((S) -1 -phényl - propyl) -amide
2bi Acide 3 -(4 -hydroxyméthyl -pipéridin -1 -ylméthyl) -1 -oxo -2 -phényl -1,2 -dihydro -isoquinoline -4 -carboxylique ((S) -1 -phényl -propyl) - amide
2bj Acide 1 -oxo -2 -phényl -3 -[4 -(tétrahydrofuran -2 -carbonyl) - pipérazin -1 -ylméthyl] -1,2 -dihydro -isoquinoline -4 -carboxylique ((S) -1 -phényl -propyl) -amide
2bk Acide 3 -(4 -isobutyl -pipérazin -1 -ylméthyl) -1 -oxo -2 -phényl -1,2 - dihydro -isoquinoline -4 -carboxylique ((S) -1 -phényl -propyl) -amide
2bl Acide 3 -[4 -(2 -méthoxy -éthyl) -pipérazin -1 -ylméthyl] -1 -oxo -2 - phényl -1,2 -dihydro -isoquinoline -4 -carboxylique ((S) -1 -phényl - propyl) -amide
2bm Acide 3 -[4 -(2 -morpholin -4 -yl -éthyl) -pipérazin -1 -ylméthyl] -1 - oxo -2 -phényl -1,2 -dihydro -isoquinoline -4 -carboxylique ((S) -1 -phényl -propyl) -amide
2bn Acide 3 -(1,3 -dihydro -iso-indol -2 -ylméthyl) -1 -oxo -2 -phényl -1,2 -dihydro -isoquinoline -4 -carboxylique ((S) -1 -phényl -propyl) -amide 2bo Acide 3 -[4 -(1 -méthyl -pipéridin -4 -yl) -pipérazin -1 -ylméthyl] -1 -
oxo -2 -phényl -1,2 - dihydro -isoquinoline -4 -carboxylique ((S) -1 -phényl -propyl) -amide
2bp Acide 1 -oxo -2 -phényl -3 -[4 -(2 -pipéridin -1 -yl -éthyl) -pipérazin -1 -ylméthyl] -1,2 -dihydro -isoquinoline -4 -carboxylique ((S) -1 -phényl - propyl) -amide
2bq Acide 1 -oxo -2 -phényl -3 -[4 -(2 -pyrrolidin -1 -yl -éthyl) -pipérazin - 1 -ylméthyl] -1,2 -dihydro -isoquinoline -4 -carboxylique ((S) -1 -phényl - propyl) -amide
2br Acide 3 -(4 -diméthylcarbamoylméthyl -pipérazin -1 -ylméthyl) -1 -oxo -2 -phényl -1,2 -dihydro -isoquinoline -4 -carboxylique ((S) -1 -phényl - propyl) -amide
2bs Acide 3 -(octahydro -pyrido[1,2 -a]pyrazin -2 -ylméthyl) -1 -oxo -2 - phényl -1,2 -dihydro -isoquinoline -4 -carboxylique ((S) -1 -phényl - propyl) -amide
2bt Acide 3 -(4 -formyl -[1,4]diazépan -1 -ylméthyl) -1 -oxo -2 -phényl -1,2 -dihydro -isoquinoline -4 -carboxylique ((S) -1 -phényl -propyl) -amide
2bu Acide 3 -[4 -(4 -cyano -phényl) -pipérazin -1 -ylméthyl] -1 -oxo -2 - phényl -1,2 -dihydro -isoquinoline -4 -carboxylique ((S) -1 -phényl - propyl) -amide
2bv Acide 1 -oxo -2 -phényl -3 -(4 -pyridin -4 -ylméthyl -pipérazin -1 - ylméthyl) -1,2 -dihydro -isoquinoline -4 -carboxylique ((S) -1 -phényl - propyl) -amide
2bw Acide 1 -oxo -2 -phényl -3 -[4 -(3 -pyrrolidin - 1 -yl -propyl) - pipérazin -1 -ylméthyl] -1,2 -dihydro -isoquinoline -4 -carboxylique ((S) -1 -phényl -propyl) -amide
2bx Acide 1 -oxo -2 -phényl -3 -(4 -pyridin -2 -ylméthyl -pipérazin -1 - ylméthyl) -1,2 -dihydro -isoquinoline -4 -carboxylique ((S) -1 -phényl - propyl) -amide
2by Acide 3 -(4 -éthanesulfonyl -pipérazin -1 -ylméthyl) -1 -oxo -2 - phényl -1,2 -dihydro -isoquinoline -4 -carboxylique ((S) -1 -phényl - propyl) -amide
2bz Acide 3 -(4 -sec -butyl -pipérazin -1 -ylméthyl) -1 -oxo -2 -phényl -1,2 -dihydro -isoquinoline -4 -carboxylique ((S) -1 -phényl -propyl) -amide
2ca Acide 3 -[4 -(1 -Ethyl -propyl) -pipérazin -1 -ylméthyl] -1 -oxo -2 - phényl -1,2 -dihydro -isoquinoline -4 -carboxylique ((S) -1 -phényl - propyl) -amide
2cb Acide 3 -[4 -(2 -cyano -éthyl) -pipérazin -1 -ylméthyl] -1 -oxo -2 - phényl -1,2 -dihydro -isoquinoline -4 -carboxylique ((S) -1 -phényl - propyl) -amide
2cc Acide 3 -(4 -méthanesulfonyl -pipérazin -1 -ylméthyl) -1 -oxo -2 - phényl -1,2 -dihydro -isoquinoline -4 -carboxylique ((S) -1 -phényl - propyl) -amide
2cd Acide {1-[1 -oxo -2 -phényl -4 -(1 -phényl -propylcarbamoyl) -1,2 - dihydro -isoquinolin -3 -ylméthyl] -pipéridin -4 -yl} -acétic éthyl ester
2ce Acide 3 -[4 -(3 -fluoro -phényl) -pipérazin -1 -ylméthyl] -1 -oxo -2 - phényl -1,2 -dihydro -isoquinoline -4 -carboxylique ((S) -1 -phényl - propyl) -amide
2cf Acide 1 -oxo -2 -phényl -3 -((S) -3 -phényl -pipéridin -1 -ylméthyl) -1,2 -dihydro -isoquinoline -4 -carboxylique ((S) -1 -phényl -propyl) -amide
2cg Acide 1 -oxo -2 -phényl -3 -[4 -(pyrrolidine -1 -carbonyl) -pipérazin -1 -ylméthyl]-1,2-dihydro-isoquinoline-4-carboxylique((S)-1-phényl-propyl) -amide
2ch Acide 3 -[4 -(morpholine -4 -carbonyl) -pipérazin -1 -ylméthyl] -1 -oxo -2-phényl-1,2-dihydro-isoquinoline-4-carboxylique((S)-1-phényl-propyl) -amide
2ciAcide3-(4-méthoxy-pipéridin-1-ylméthyl)-1-oxo-2-phényl-1,2-dihydro-isoquinoline-4-carboxylique((S)-1-phényl-propyl)-amide
2cj Acide 3 -(4' -hydroxy -3',4',5',6' -tétrahydro -2'h -[3,4']bipyridinyl -1' - ylméthyl) -1 -oxo -2 -phényl -1,2 -dihydro -isoquinoline -4 -carboxylique ((S) -1 -phényl -propyl) -amide
2ck Acide 3 -(4 -hydroxy -3,4,5,6 -tétrahydro -2h -[4,4']bipyridinyl -1 - ylméthyl) -1 -oxo -2 -phényl -1,2 -dihydro -isoquinoline -4 -carboxylique ((S) -1 -phényl -propyl) -amide
2cl Acide 3 -(3h -spiro[isobenzofuran -1,4' -pipéridin] -1 ' -ylméthyl) -1 - oxo -2 -phényl -1,2 -dihydro -isoquinoline -4 -carboxylique ((S) -1 -phényl -propyl) -amide
2cm Acide 3 -(4 -hydroxy -4 -méthyl -pipéridin -1 -ylméthyl) -1 -oxo -2 - phényl -1,2 -dihydro -isoquinoline -4 -carboxylique ((S) -1 -phényl - propyl) -amide
2cn Acide 3 -(hexahydro -spiro[benzo[1,3]dioxole -2,4' -pipéridin] -r - ylméthyl) -1 -oxo -2 -phényl -1,2 -dihydro -isoquinoline -4 -carboxylique ((S) -1 -phényl -propyl) -amide
2co Acide 1 -oxo -2 -phényl -3 -(3,4,5,6 -tétrahydro -2h -[4,4']bipyridinyl - 1 -ylméthyl) -1,2 -dihydro -isoquinoline -4 -carboxylique ((S) -1 -phényl - propyl) -amide
2cp Acide 3 -[4 -(2 -diméthylamino -éthyl) -pipéridin -1 -ylméthyl] -1 -oxo -2 -phényl -1,2 -dihydro -isoquinoline -4 -carboxylique ((S) -1 -phényl - propyl) -amide
2cq Acide 3 -(4 -diméthylsulfamoyl -pipérazin -1 -ylméthyl) -1 -oxo -2 - phényl -1,2 -dihydro -isoquinoline -4 -carboxylique ((S) -1 -phényl - propyl) -amide
2cr Acide 3 -(1,1 -dioxo -thiomorpholin -4 -ylméthyl) -1 -oxo -2 -phényl - 1,2 -dihydro -isoquinoline -4 -carboxylique ((S) -1 -phényl -propyl) -amide
2cs Acide 1 -oxo -2 -phényl -3 -(2 -pyridin -2 -ylméthyl -pipéridin -1 - ylméthyl) -1,2 -dihydro -isoquinoline -4 -carboxylique ((S) -1 -phényl - propyl) -amide
2ct Acide 3 -(2 -morpholin -4 -ylméthyl -pipéridin -1 -ylméthyl) -1 -oxo -2 - phényl -1,2 -dihydro -isoquinoline -4 -carboxylique ((S) -1 -phényl - propyl) -amide
2cu Acide 3 -(4 -furo[3,2 -c]pyridin -4 -yl -pipérazin -1 -ylméthyl) -1 -oxo - 2 -phényl -1,2 -dihydro -isoquinoline -4 -carboxylique ((S) -1 -phényl - propyl) -amide
2cv Acide 3 -(4 -cyclopropylméthyl -pipérazin -1 -ylméthyl) -1 -oxo -2 - phényl -1,2 -dihydro -isoquinoline -4 -carboxylique ((S) -1 -phényl - propyl) -amide
2cw Acide 3 -[4 -(2 -morpholin -4 -yl -éthyl) -pipéridin -1 -ylméthyl] -1 - oxo -2 -phényl -1,2 -dihydro -isoquinoline -4 -carboxylique ((S) -1 -phényl -propyl) -amide
2cx Acide 1 -oxo -2 -phényl -3 -(4 -pyrimidin -2 -yl -[1,4]diazépan -1 - ylméthyl) -1,2 -dihydro -isoquinoline -4 -carboxylique ((S) -1 -phényl - propyl) -amide
2cy Acide 3 -(4 -méthyl -6,7 -dihydro -4h -thiéno[3,2 -c]pyridin -5 - ylméthyl) -1 -oxo -2 -phényl -1,2 -dihydro -isoquinoline -4 -carboxylique ((S) -1 -phényl -propyl) -amide
2cz Acide 3 -[1,4]diazépan -1 -ylméthyl -1 -oxo -2 -phényl -1,2 -dihydro - isoquinoline -4 -carboxylique ((S) -1 -phényl -propyl) -amide
2da Acide 3 -((2S,5R) -2,5 -diméthyl -pipérazin -1 -ylméthyl) -1 -oxo -2 - phényl -1,2 -dihydro -isoquinoline -4 -carboxylique ((S) -1 -phényl - propyl) -amide
2db Acide 3 -((S) -3 -méthyl -pipérazin -1 -ylméthyl) -1 -oxo -2 -phényl - 1,2 -dihydro -isoquinoline -4 -carboxylique ((S) -1 -phényl -propyl) -amide 2dc Acide 3 -((R) -3 -méthyl -pipérazin -1 -ylméthyl) -1 -oxo -2 -phényl - 1,2 -dihydro -isoquinoline -4 -carboxylique ((S) -1 -phényl -propyl) -amide
2dd Acide 3 -[3 -(3 -chloro -phényl) -pipérazin -1 -ylméthyl] -1 -oxo -2 - phényl -1,2 -dihydro -isoquinoline -4 -carboxylique ((S) -1 -phényl - propyl) -amide
2de Acide 3 -[4 -(1 h -indol -4 -yl) -pipérazin -1 -ylméthyl] -1 -oxo -2 - phényl -1,2 -dihydro -isoquinoline -4 -carboxylique ((S) -1 -phényl - propyl) -amide
2df Acide 1 -oxo -3 -(3 -oxo -pipérazin -1 -ylméthyl) -2 -phényl -1,2 - dihydro -isoquinoline -4 -carboxylique ((S) -1 -phényl -propyl) -amide
2dg Acide 3 -[4 -(1 h -Indol -5 -yl) -pipérazin -1 -ylméthyl] -1 -oxo -2 - phényl -1,2 -dihydro -isoquinoline -4 -carboxylique ((S) -1 -phényl - propyl) -amide
2dh Acide 3 -(6,9 -diaza -spiro[4.5]déc -9 -ylméthyl) -1 -oxo -2 -phényl - 1,2 -dihydro -isoquinoline -4 -carboxylique ((S) -1 -phényl -propyl) -amide
2di Acide 3 -(1,4 -diaza -spiro[5.5]undéc -4 -ylméthyl) -1 -oxo -2 -phényl - 1,2 -dihydro -isoquinoline -4 -carboxylique ((S) -1 -phényl -propyl) -amide
2dj Acide 3 -(3 -isopropyl -pipérazin -1 -ylméthyl) -1 -oxo -2 -phényl -1,2 - dihydro -isoquinoline -4 -carboxylique ((S) -1 -phényl -propyl) -amide
2dk Acide 3 -(3,3 -diméthyl -pipérazin -1 -ylméthyl) -1 -oxo -2 -phényl - 1,2 -dihydro -isoquinoline -4 -carboxylique ((S) -1 -phényl -propyl) -amide
2dl Acide 3 -[3 -(4 -fluoro -phényl) -pipérazin -1 -ylméthyl] -1 -oxo -2 - phényl -1,2 -dihydro -isoquinoline -4 -carboxylique ((S) -1 -phényl - propyl) -amide
2dm Acide 1 -oxo -2 -phényl -3 -(3 -p -tolyl -pipérazin -1 -ylméthyl) -1,2 - dihydro -isoquinoline -4 -carboxylique ((S) -1 -phényl -propyl) -amide
2dn Acide 4 -[1 -oxo -2 -phényl -4 -(1 -phényl -propylcarbamoyl) -1,2 - dihydro -isoquinolin -3 -ylméthyl] -pipérazine -1 -carboxylique tert -butyl ester
2do Acide 3 -(4 -méthylcarbamoylméthyl -pipérazin -1 -ylméthyl) -1 -oxo - 2 -phényl -1,2 -dihydro -isoquinoline -4 -carboxylique ((S) -1 -phényl - propyl) -amide
2dp Acide 8 -chloro -3 -diméthylaminométhyl -1 -oxo -2 -phényl -1,2 - dihydro -isoquinoline -4 -carboxylique ((S) -1 -phényl -propyl) -amide
2dr Acide 3 -cyclopentylaminométhyl -1 -oxo -2 -phényl -1,2 -dihydro - isoquinoline -4 -carboxylique ((S) -1 -phényl -propyl) -amide
2ds Acide 3 -cyclohexylaminométhyl -1 -oxo -2 -phényl -1,2 -dihydro - isoquinoline -4 -carboxylique ((S) -1 -phényl -propyl) -amide
2dt Acide 3 - {[(2 -hydroxy -éthyl) -méthyl -amino] -méthyl} -1 -oxo -2 - phényl -1,2 -dihydro -isoquinoline -4 -carboxylique ((S) -1 -phényl - propyl) -amide
2du Acide 3 -imidazol -1 -ylméthyl -1 -oxo -2 -phényl -1,2 -dihydro - isoquinoline -4 -carboxylique ((S) -1 -phényl -propyl) -amide
2dv Acide 3 -(2 -méthyl -imidazo 1 -1 -ylméthyl) -1 -oxo -2 -phényl -1,2 - dihydro -isoquinoline -4 -carboxylique ((S) -1 -phényl -propyl) -amide
2dw Acide 3 -(4 -méthyl -imidazol -1 -ylméthyl) -1 -oxo -2 -phényl -1,2 - dihydro -isoquinoline -4 -carboxylique ((S) -1 -phényl -propyl) -amide
2dx Acide 3 -(2,5 -dihydro -pyrrol -1 -ylméthyl) -1 -oxo -2 -phényl -1,2 - dihydro -isoquinoline -4 -carboxylique ((S) -1 -phényl -propyl) -amide
2dy Acide 3 -(2,5 -diméthyl -2,5 -dihydro -pyrrol -1 -ylméthyl) -1 -oxo -2 - phényl -1,2 -dihydro -isoquinoline -4 -carboxylique ((S) -1 -phényl - propyl) -amide
2dz Acide 1 -oxo -2 -phényl -3 -pyrrolidin - 1 -ylméthyl -1,2 -dihydro - isoquinoline -4 -carboxylique ((S) -1 -phényl -propyl) -amide
2ea Acide 1 -oxo -2 -phényl -3 -(thiazol -2 -ylamino méthyl) -1,2 -dihydro -isoquinoline -4 -carboxylique ((S) -1 -phényl -propyl) -amide
2eb Acide 1 -oxo -2 -phényl -3 -(pyrimidin -4 -ylaminométhyl) -1,2 - dihydro -isoquinoline -4 -carboxylique ((S) -1 -phényl -propyl) -amide
2ec Acide 3 -(tert -butylamino -méthyl) -1 -oxo -2 -phényl -1,2 -dihydro - isoquinoline -4 -carboxylique ((S) -1 -phényl -propyl) -amide
2ed Acide 3 -[(2 -hydroxy -1,1 -diméthyl -éthylamino) -méthyl] -1 -oxo -2 - phényl -1,2 -dihydro -isoquinoline -4 -carboxylique ((S) -1 -phényl - propyl) -amide
2ee Acide 3 -(isopropylamino -méthyl) -1 -oxo -2 -phényl -1,2 -dihydro - isoquinoline -4 -carboxylique ((S) -1 -phényl -propyl) -amide
2ef Acide 3 -[(2 -hydroxy -1 -méthyl -éthylamino) -méthyl] -1 -oxo -2 - phényl -1,2 -dihydro -isoquinoline -4 -carboxylique ((S) -1 -phényl - propyl) -amide
2eg Acide 3 -[(1 -hydroxyméthyl -propylamino) -méthyl] -1 -oxo -2 - phényl -1,2 -dihydro -isoquinoline -4 -carboxylique ((S) -1 -phényl - propyl) -amide
2eh Acide 3 -[(2,2 -diméthyl -propylamino) -méthyl] -1 -oxo -2 -phényl - 1,2 -dihydro -isoquinoline -4 -carboxylique ((S) -1 -phényl -propyl) -amide
2ei Acide 1 -oxo -2 -phényl -3 -prop -2 -ynylaminométhyl -1,2 -dihydro - isoquinoline -4 -carboxylique ((S) -1 -phényl -propyl) -amide
2ej Acide 3 -allylaminométhyl -1 -oxo -2 -phényl -1,2 -dihydro - isoquinoline -4 -carboxylique ((S) -1 -phényl -propyl) -amide
2ek Acide 3 -[(méthyl -prop -2 -ynyl -amino) -méthyl] -1 -oxo -2 -phényl - 1,2 -dihydro -isoquinoline -4 -carboxylique ((S) -1 -phényl -propyl) -amide
2el Acide 3 -diallylaminométhyl -1 -oxo -2 -phényl -1,2 -dihydro - isoquinoline -4 -carboxylique ((S) -1 -phényl -propyl) -amide
2em Acide 3 -diéthylaminométhyl -1 -oxo -2 -phényl -1,2 -dihydro - isoquinoline -4 -carboxylique ((S) -1 -phényl -propyl) -amide
2en Acide 3 -[(Isopropyl -méthyl -amino) -méthyl] -1 -oxo -2 -phényl -1,2 - dihydro -isoquinoline -4 -carboxylique ((S) -1 -phényl -propyl) -amide
2eo Acide 3 -[((S) -2 -hydroxy -1 -méthyl -éthylamino) -méthyl] - 1 -oxo -2 -phényl -1,2 -dihydro -isoquinoline -4 -carboxylique ((S) -1 -phényl - propyl) -amide
2ep Acide 3 -[((R) -2 -hydroxy -1 -méthyl -éthylamino) -méthyl] -1 -oxo -2 -phényl -1,2 -dihydro -isoquinoline -4 -carboxylique ((S) -1 -phényl - propyl) -amide
2eq Acide 3 -{[(2 -méthoxy -éthyl) -méthyl -amino] -méthyl} -1 -oxo -2 - phényl -1,2 -dihydro -isoquinoline -4 -carboxylique ((S) -1 -phényl - propyl) -amide
2er Acide 3 -((R) -3 -hydroxy -pyrrolidin -1 -ylméthyl) -1 -oxo -2 -phényl - 1,2 -dihydro -isoquinoline -4 -carboxylique ((S) -1 -phényl -propyl) -amide
2es Acide 3 -((S) -3 -hydroxy -pyrrolidin -1 -ylméthyl) -1 -oxo -2 -phényl - 1,2 -dihydro -isoquinoline -4 -carboxylique ((S) -1 -phényl -propyl) -amide
2et Acide 3 -[(cyclopentyl -méthyl -amino) -méthyl] -1 -oxo -2 -phényl -1,2 -dihydro -isoquinoline -4 -carboxylique ((S) -1 -phényl -propyl) -amide
2eu Acide 3 - {[(2 -hydroxy -1 -méthyl -éthyl) -méthyl -amino] -méthyl} -1 - oxo -2 -phényl -1,2 -dihydro -isoquinoline -4 -carboxylique ((S) -1 -phényl -propyl) -amide
2ev Acide 3 - {[Ethyl -(2 -hydroxy -éthyl) -amino] -méthyl} -1 -oxo -2 - phényl -1,2 -dihydro -isoquinoline -4 -carboxylique ((S) -1 -phényl - propyl) -amide
2ew Acide 3 -[(Ethyl -méthyl -amino) -méthyl] -1 -oxo -2 -phényl -1,2 - dihydro -isoquinoline -4 -carboxylique ((S) -1 -phényl -propyl) -amide
2ex Acide 3 -cyclobutylaminométhyl -1 -oxo -2 -phényl -1,2 -dihydro - isoquinoline -4 -carboxylique ((S) -1 -phényl -propyl) -amide
2ey Acide 3 -azétidin -1 -ylméthyl -1 -oxo -2 -phényl -1,2 -dihydro - isoquinoline -4 -carboxylique ((S) -1 -phényl -propyl) -amide
2ez Acide 3 -(4 -tert -butyl -pipérazin -1 -ylméthyl) -1 -oxo -2 -phényl -1,2 -dihydro -isoquinoline -4 -carboxylique ((S) -1 -phényl -propyl) -amide
2fa Acide 3 -[4 -(2 -hydroxy -éthyl) -pipérazin -1 -ylméthyl] -1 -oxo -2 - phényl -1,2 -dihydro -isoquinoline -4 -carboxylique ((S) -1 -phényl - propyl) -amide
2fb Acide 3 - {4 -[2 -(2 -hydroxy -éthoxy) -éthyl] -pipérazin -1 -ylméthyl} - 1 -oxo -2 -phényl -1,2 -dihydro -isoquinoline -4 -carboxylique ((S) -1 - phényl -propyl) -amide
2fc Acide 3 -[4 -(3 -chloro -5 -trifluorométhyl -pyridin -2 -yl) -pipérazin -1 - ylméthyl] -1 -oxo -2 -phényl -1,2 -dihydro -isoquinoline -4 -carboxylique ((S) -1 -phényl -propyl) -amide
2fd Acide 3 -[4 -(3,5 -dichloro -pyridin -4 -yl) -pipérazin -1 -ylméthyl] -1 - oxo -2 -phényl -1,2 -dihydro -isoquinoline -4 -carboxylique ((S) -1 -phényl -propyl) -amide
2fe Acide 4 -[1 -oxo -2 -phényl -4 -((S) -1 -phényl -propylcarbamoyl) -1,2 -dihydro -isoquinolin -3 -ylméthyl] -pipérazine -1 -carboxylique benzyl ester
2ff Acide 3 -[4 -(3 -morpholin -4 -yl -propyl) -pipérazin -1 -ylméthyl] -1 - oxo -2 -phényl -1,2 -dihydro -isoquinoline -4 -carboxylique ((S) -1 -phényl -propyl) -amide
2fg Acide 1 -oxo -2 -phényl -3 -[4 -(3 -pipéridin -1 -yl -propyl) -pipérazin - 1 -ylméthyl] -1,2 -dihydro -isoquinoline -4 -carboxylique ((S) -1 -phényl - propyl) -amide
2fh Acide 3 -[4 -(4,6 -diméthoxy -pyrimidin -2 -ylméthyl) -pipérazin -1 - ylméthyl] -1 -oxo -2 -phényl -1,2 -dihydro -isoquinoline -4 -carboxylique ((S) -1 -phényl -propyl) -amide
2fi Acide 3 -[4 -(3 -hydroxy -propyl) -pipérazin -1 -ylméthyl] -1 -oxo -2 - phényl -1,2 -dihydro -isoquinoline -4 -carboxylique ((S) -1 -phényl - propyl) -amide
2fj Acide 3 -[4 -(2,3 -dihydroxy -propyl) -pipérazin -1 .ylméthyl] -1 -oxo -2 -phényl -1,2 -dihydro -isoquinoline -4 -carboxylique ((S) -1 -phényl - propyl) -amide
2fk Acide (2 -oxo -2 - {4 -[1 -oxo -2 -phényl -4 -(1 -phényl - propylcarbamoyl) -1,2 -dihydro -isoquinoline -3 -ylméthyl] -pipérazin -1 - yl}-éthyl) -carbamique tert -butyl ester
2fl Acide 3 -[4 -(1 h -indazol -5 -yl) -pipérazin -1 -ylméthyl] -1 -oxo -2 - phényl -1,2 -dihydro -isoquinoline -4 -carboxylique ((S) -1 -phényl - propyl) -amide
2fm Acide 1 -oxo -2 -phényl -3 -(4 -quinolin -6 -yl -pipérazin -1 -ylméthyl) -1,2 -dihydro -isoquinoline -4 -carboxylique ((S) -1 -phényl -propyl) - amide
2fn Acide 3 -[4 -(6,7 -diméthoxy -quinazolin -4 -yl) -pipérazin -1 -ylméthyl] -1 -oxo -2 -phényl -1,2 -dihydro -isoquinoline -4 -carboxylique ((S) -1 - phényl -propyl) -amide
2fo Acide 4 - {4-[1 -oxo -2 -phényl -4 -(1 -phényl -propylcarbamoyl) -1,2 - dihydro -isoquinolin -3 -ylméthyl] -pipérazin -1 -yl} -pipéridine -1 - carboxylique tert -butyl ester
2fp Acide 3 -{4 -[2 -(4 -chloro -phénoxy) -éthyl] -pipérazin -1 -ylméthyl}-1 -oxo -2 -phényl -1,2 -dihydro -isoquinoline -4 -carboxylique ((S) -1 - phényl -propyl) -amide
2fq Acide {4 -[1 -oxo -2 -phényl -4 -(1 -phényl -propylcarbamoyl) -1,2 - dihydro -isoquinolin -3 -ylméthyl] -pipérazin -1 -yl}-acétique tert -butyl ester
2fr Acide 1 -oxo -2 -phényl -3 -[4 -(3,3,3 -trifluoro -2 -hydroxy -propyl) - pipérazin -1 -ylméthyl] -1,2 -dihydro -isoquinoline -4 -carboxylique ((S) -1 -phényl -propyl) -amide
2fs Acide 3 -[4 -(2 -hydroxy -propyl) -pipérazin -1 -ylméthyl] -1 -oxo -2 - phényl -1,2 -dihydro -isoquinoline -4 -carboxylique ((S) -1 -phényl - propyl) -amide
2fu Acide 3 -[4 -(4 -Amino -6,7 -diméthoxy -quinazolin -2 -yl) -pipérazin - 1 -ylméthyl] -1 -oxo -2 -phényl -1,2 -dihydro -isoquinoline -4 - carboxylique ((S) -1 -phényl -propyl) -amide
2fv Acide (2 - {4 -[1 -oxo -2 -phényl -4 -(1 -phényl -propylcarbamoyl) -1,2 -dihydro -isoquinolin -3 -ylméthyl] -pipérazin -1 -yl}-éthyl) -carbamique tert -butyl ester
2fw Acide 1 -oxo -3 - {4-[2 -(2 -oxo -imidazolidin-1 -yl) -éthyl] -pipérazin -1 -ylméthyl}-2 -phényl -1,2 -dihydro -isoquinoline -4 -carboxylique ((S) - 1 -phényl -propyl) -amide
2fx Acide 3 - {4-[(4,6 -diméthoxy -pyrimidin -2 -yl) -phényl -méthyl] - pipérazin -1 -ylméthyl}-1 -oxo -2 -phényl -1,2 -dihydro -isoquinoline -4 - carboxylique ((S) -1 -phényl -propyl) -amide
2fy Acide 3 -(4 -benzo[1,2,5]thiadiazo 1 -4 -yl -pipérazin -1 -ylméthyl) -1 - oxo -2 -phényl -1,2 -dihydro -isoquinoline -4 -carboxylique ((S) -1 -phényl -propyl) -amide
2fz Acide 3 -[4 -(2,3 -dihydro -benzo[1,4]dioxin -5 -yl) -pipérazin -1 - ylméthyl] -1 -oxo -2 -phényl 1,2 -dihydro -isoquinoline -4 -carboxylique ((S) -1 -phényl -propyl) -amide
2ga Acide 3 -[4 -(4 -méthyl -quinolin -2 -yl) -pipérazin -1 -ylméthyl] -1 - oxo -2 -phényl -1,2 -dihydro -isoquinoline -4 -carboxylique ((S) - 1 - phényl -propyl) -amide
2gb Acide 1 -oxo -2 -phényl -3 -[4 -(pyridin -2 -ylcarbamoylméthyl) - pipérazin -1 -ylméthyl] -1,2 -dihydro -isoquinoline -4 -carboxylique ((S) -1 -phényl -propyl) -amide
2gc Acide 3 -[4 -(6 -chloro -3 -oxo -3,4 -dihydro -2h -benzo[1,4]oxazin -8 -yl)-pipérazin -1 -ylméthyl] -1 -oxo -2 -phényl -1,2 -dihydro -isoquinoline -4 -carboxylique ((S)-1 -phényl -propyl) -amide
2gd Acide 3 -(4 -carbamoylméthyl -pipérazin -1 -ylméthyl) -1 -oxo -2 - phényl -1,2 -dihydro -isoquinoline -4 -carboxylique ((S) -1 -phényl - propyl) -amide
2ge Acide 3 -(4 -hydroxy -4 -phényl -pipéridin -1 -ylméthyl) -1 -oxo -2 - phényl -1,2 -dihydro -isoquinoline -4 -carboxylique ((S) -1 -phényl - propyl) -amide
2gf Acide 3 -[4 -(4 -chloro -phényl) -4 -hydroxy -pipéridin -1 -ylméthyl] -1 -oxo -2 -phényl -1,2 -dihydro -isoquinoline -4 -carboxylique ((S) -1 - phényl -propyl) -amide
2gg Acide 1 -[1 -oxo -2 -phényl -4 -(1 -phényl -propylcarbamoyl) -1,2 - dihydro -isoquinolin -3 -ylméthyl] -pipéridine -4 -carboxylique
2gh Acide 3 -(4 -cyano -4 -phényl -pipéridin -1 -ylméthyl) -1 -oxo -2 - phényl -1,2 -dihydro -isoquinoline -4 -carboxylique ((S) -1 -phényl - propyl) -amide
2gi Acide 3 -(4 -benzyl -4 -hydroxy -pipéridin -1 -ylméthyl) -1 -oxo -2 - phényl -1,2 -dihydro -isoquinoline -4 -carboxylique ((S) -1 -phényl - propyl) -amide
2gj Acide 1 -[1 -oxo -2 -phényl -4 -(1 -phényl -propylcarbamoyl) -1,2 - dihydro -isoquinolin -3 -ylméthyl] -4 -phényl -pipéridine -4 -carboxylique éthyl ester
2gk Acide 1 -oxo -2 -phényl -3 -[4 -(phényl -propionyl -amino) -pipéridin - 1 -ylméthyl] -1,2 -dihydro -isoquinoline -4 -carboxylique ((S) -1 -phényl - propyl) -amide
2gl Acide méthyl - {1-[1 -oxo -2 -phényl -4 -(1 -phényl -propylcarbamoyl) -1,2 -dihydro -isoquinolin -3 -ylméthyl] -pipéridin -4 -yl}-carbamique tert - butyl ester
2gm Acide 1 -oxo -2 -phényl -3 -[4 -(2 -pyrrolidin -1 -yl-éthyl) -pipéridin - 1 -ylméthyl] -1,2 -dihydro -isoquinoline -4 -carboxylique ((S) -1 -phényl - propyl) -amide
2gn Acide 3 -{4 -[5 -(4 -fluoro -phényl) -[1,3,4]oxadiazol -2 -yl] -pipéridin - 1 -ylméthyl}-1 -oxo -2 -phényl -1,2 -dihydro -isoquinoline -4 - carboxylique ((S) -1 -phényl -propyl) -amide
2goAcide 1 -oxo -2 -phényl -3 -[4 -(3 -pyridin -4 -yl -[1,2,4]oxadiazol -5 - yl)-pipéridin -1 -ylméthyl] -1,2 -dihydro -isoquinoline -4 -carboxylique ((S) -1 -phényl -propyl) -amide
2gp Acide 1 -oxo -2 -phényl -3 -[4 -(3 -pyrazin -2 -yl -[1,2,4]oxadiazol -5 - yl)-pipéridin -1 -ylméthyl] -1,2 -dihydro-isoquinoline -4 -carboxylique ((S) -1 -phényl-propyl) -amide
2gq Acide 1 -oxo -2 -phényl -3 -[4 -(3 -pyridin -4 -yl -[1,2,4]oxadiazol -5 - yl)-pipéridin -1 -ylméthyl] -1,2 -dihydro -isoquinoline -4 -carboxylique ((S) -1 -phényl -propyl) -amide
2gr Acide 3 -(4' -hydroxy -3',4',5',6* -tétrahydro -2'h -[2,4']bipyridinyl-1'-ylméthyl) -1 -oxo -2 -phényl -1,2 -dihydro -isoquinoline -4 -carboxylique ((S) -1 -phényl -propyl) -amide
2gs Acide 3 -(spiro[isochroman -1,4' -pipéridin]-1 ' -ylméthyl) -1 -oxo -2 - phényl -1,2 -dihydro -isoquinoline -4 -carboxylique ((S) -1 -phényl-propyl) -amide
2gt Acide 3 -[4 -hydroxy -4 -(3 -méthoxy -phényl)-pipéridin -1 -ylméthyl] - 1 -oxo -2 -phényl -1,2 -dihydro -isoquinoline -4 -carboxylique ((S) -1 - phényl -propyl) -amide
2gu Acide 3 -[4 -(3 -chloro -phényl) -4 -hydroxy -pipéridin -1 -ylméthyl] -1 -oxo -2 -phényl -1,2 -dihydro -isoquinoline -4 -carboxylique ((S) -1 - phényl -propyl) -amide
2gv Acide 3 -(6 -chloro -3h -spiro[isobenzofliran -1,4' -pipéridin] -1' - ylméthyl) -1 -oxo -2 -phényl -1,2 -dihydro -isoquinoline -4 -carboxylique ((S) -1 -phényl -propyl) -amide
2gw Acide 3 -(4 - {[4-chloro -3 -(4 -fluoro -phényl)-indan -1 -yl]-méthyl - amino}-pipéridin -1 -ylméthyl) -1 -oxo -2 -phényl -1,2 -dihydro-isoquinoline -4 -carboxylique ((S) -1 -phényl -propyl) -amide
2gx Acide 3 -(1 -acétyl -spiro[indoline -3,4'-pipéridin] -1'-ylméthyl) -1 - oxo -2 -phényl -1,2 -dihydro -isoquinoline -4 -carboxylique ((S) -1 -phényl -propyl) -amide
2gy Acide 3 -(1 -acétyl -5 -fluoro -spiro[indoline -3,4' -pipéridin]-1'-ylméthyl) -1 -oxo -2 -phényl -1,2 -dihydro -isoquinoline -4 -carboxylique ((S) -1 -phényl -propyl) -amide
2gz Acide 1 -oxo -3 -(4 -oxo -1 -phényl -1,3,8 -triaza -spiro[4.5]dec -8 - ylméthyl) -2 -phényl -1,2 -dihydro -isoquinoline -4 -carboxylique ((S) -1 - phényl -propyl) -amide
2ha Acide 3 -(4 -acétylamino -pipéridin -1 -ylméthyl) -1 -oxo -2 -phényl - 1,2 -dihydro -isoquinoline -4 -carboxylique ((S) -1 -phényl -propyl) -amide
2hb Acide 1 -oxo -3 -(1 -oxo -2,8 -diaza -spiro[4.5]déc -8 -ylméthyl) -2 - phényl -1,2 -dihydro -isoquinoline -4 -carboxylique ((S) -1 -phényl - propyl) -amide
2hc Acide 3 -[4 -hydroxy -4 -(3 -trifluorométhyl -phényl) -pipéridin -1 - ylméthyl] -1 -oxo -2 -phényl -1,2 -dihydro -isoquinoline -4 -carboxylique ((S) -1 -phényl -propyl) -amide
2hd Acide 1 -oxo -2 -phényl -3 -(4 -trifluorométhyl -pipéridin -1 -ylméthyl) -1,2 -dihydro -isoquinoline -4 -carboxylique ((S) -1 -phényl -propyl) - amide
2he Acide 3 -[4 -(4 -méthyl -pipérazine -1 -carbonyl) -pipéridin -1 - ylméthyl] -1 -oxo -2 -phényl -1,2 -dihydro -isoquinoline -4 -carboxylique ((S) -1 -phényl -propyl) -amide
2hf Acide 3 -(5 -isopropyl -3h -spiro[isobenzofuran -1,4' -pipéridin] -1' - ylméthyl) -1 -oxo -2 -phényl -1,2 -dihydro -isoquinoline -4 -carboxylique ((S) -1 -phényl -propyl) -amide
2hg Acide 3 -[4 -(acétyl -méthyl -amino) -4 -phényl -pipéridin -1 - ylméthyl] -1 -oxo -2 -phényl -1,2 -dihydro -isoquinoline -4 -carboxylique ((S) -1 -phényl -propyl) -amide
2hh Acide 4 -{1-[1 -oxo -2 -phényl -4 -(1 -phényl-propylcarbamoyl) -1,2 - dihydro -isoquinolin -3 -ylméthyl] -pipéridin-4 -yl}-pipérazine-1 - carboxylique tert -butyl ester
2hi Acide (2 -{1-[1 -oxo -2 -phényl-4 -(1 -phényl -propylcarbamoyl) -1,2 - dihydro -isoquinolin -3 -ylméthyl] -pipéridin-4 -yl}-éthyl)-carbamique tert -butyl ester
2hj Acide 3 -(4 -méthylamino -4 -phényl -pipéridin -1 -ylméthyl) -1 -oxo -2 -phényl -1,2 -dihydro -isoquinoline -4 -carboxylique ((S) -1 -phényl-propyl) -amide
2hk Acide 3 -(4 -acétylamino -4 -phényl -pipéridin -1 -ylméthyl) -1 -oxo -2 -phényl -1,2 -dihydro -isoquinoline -4 -carboxylique ((S) -1 -phényl - propyl) -amide
2hl Acide 3 -[4 -acétylamino -4 -(3 -fluoro -phényl) -pipéridin -1 -ylméthyl] -1 -oxo -2 -phényl -1,2 -dihydro -isoquinoline -4 -carboxylique ((S) -1 - phényl -propyl) -amide
2hm Acide 1 -oxo -3 -[4 -(4 -oxo -pipéridine -1 -carbonyl) -pipéridin -1 - ylméthyl] -2 -phényl -1,2 -dihydro -isoquinoline -4 -carboxylique ((S) -1 - phényl -propyl) -amide
2hn Acide 3 -[4,4']bipipéridinyl -1 -ylméthyl -1 -oxo -2 -phényl -1,2 - dihydro -isoquinoline -4 -carboxylique ((S) -1 -phényl -propyl) -amide
2ho Acide {1-[1 -oxo -2 -phényl -4 -(1 -phényl -propylcarbamoyl) -1,2 - dihydro -isoquinolin -3 -ylméthyl] -pipéridin -4 -yl}-carbamique tert -butyl ester
2hp Acide 3 -[1,4']bipipéridinyl -1 ' -ylméthyl -1 -oxo -2 -phényl -1,2 - dihydro -isoquinoline -4 -carboxylique [cyclopropyl-(3 -fluoro-phényl)-méthyl] -amide
2hq Acide 1 -oxo -3 -(4 -phénétyl-pipérazin -1 -ylméthyl) -2 -phényl -1,2 -dihydro-isoquinoline -4 -carboxylique [cyclopropyl-(3 -fluoro-phényl)-méthyl] -amide
2hr Acide 3 -(4 -isopropyl -pipérazin-1 -ylméthyl) -1 -oxo -2 -phényl -1,2 -dihydro -isoquinoline -4 -carboxylique [cyclopropyl -(3 -fluoro-phényl)-méthyl] -amide
2hs Acide 3 -[4 -(2 -morpholin -4 -yl -éthyl) -pipérazin -1 -ylméthyl] -1 - oxo -2 -phényl -1,2 -dihydro -isoquinoline -4 -carboxylique [cyclopropyl - (3 -fluoro -phényl) -méthyl] -amide
2ht Acide 3 -[4 -(1 -méthyl -pipéridin -4 -yl) -pipérazin -1 -ylméthyl] -1 - oxo -2 -phényl -1,2 -dihydro -isoquinoline -4 -carboxylique [cyclopropyl-(3 -fluoro -phényl) -méthyl] -amide
2hu Acide 1 -oxo -2 -phényl -3 -[4 -(2 -pipéridin-1 -yl -éthyl) -pipérazin -1 -ylméthyl] -1,2 -dihydro -isoquinoline -4 -carboxylique [cyclopropyl -(3 - fluoro -phényl) -méthyl] -amide
2hv Acide 1 -oxo -2 -phényl -3 -[4 -(2 -pyrrolidin -1 -yl -éthyl) -pipérazin-1 -ylméthyl] -1,2 -dihydro -isoquinoline -4 -carboxylique [cyclopropyl -(3 - fluoro -phényl) -méthyl] -amide
2hw Acide 1 -oxo -2 -phényl -3 -(4 -pyridin -4 -ylméthyl -pipérazin -1 - ylméthyl) -1,2 -dihydro -isoquinoline -4 -carboxylique [cyclopropyl -(3 - fluoro -phényl) -méthyl] -amide
2hx Acide 3 -(4 -hydroxy -3,4,5,6 -tétrahydro -2h -[4,4']bipyridinyl -1 - ylméthyl) -1 -oxo -2 -phényl -1,2 -dihydro -isoquinoline -4 -carboxylique [cyclopropyl -(3 -fluoro -phényl) -méthyl] -amide
2hy Acide 3 -[4 -(2 -morpholin -4 -yl-éthyl)-pipéridin -1 -ylméthyl] -1 -oxo -2 -phényl -1,2 -dihydro -isoquinoline -4 -carboxylique [cyclopropyl -(3 - fluoro -phényl) -méthyl] -amide
2hz Acide 3 -[4 -hydroxy -4 -(3 -méthoxy -phényl) -pipéridin -1 -ylméthyl] -1 -oxo -2 -phényl -1,2 -dihydro -isoquinoline -4 -carboxylique [cyclopropyl -(3 -fluoro -phényl) -méthyl] -amide
2ia Acide 3 -[4 -(acétyl -méthyl -amino) -4 -phényl -pipéridin -1 -ylméthyl] -1 -oxo -2 -phényl -1,2 -dihydro -isoquinoline -4 -carboxylique [cyclopropyl -(3 -fluoro -phényl) -méthyl] -amide
2ib Acide 2 -(2 -fluoro -phényl) -1 -oxo -3 -(4 -phénétyl -pipérazin -1 - ylméthyl) -1,2 -dihydro -isoquinoline -4 -carboxylique ((S) -1 -phényl - propyl) -amide
2ic Acide 2 -(2 -fluoro -phényl) -3 -(4 -isopropyl -pipérazin -1 -ylméthyl) - 1 -oxo -1,2 -dihydro -isoquinoline -4 -carboxylique ((S) -1 -phényl - propyl) -amide
2id Acide 3 -[1,4']bipipéridinyl -1 ' -ylméthyl -2 -(2 -fluoro -phényl) -1 -oxo -1,2 -dihydro -isoquinoline -4 -carboxylique ((S) -1 -phényl -propyl) - amide
2ie Acide 2 -(2 -fluoro -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2if Acide 2 -(2 -fluoro -phényl) -3 -[4 -(1 -méthyl -pipéridin -4 -yl) - pipérazin -1 -ylméthyl] -1 -oxo -1,2 -dihydro -isoquinoline -4 - carboxylique ((S) -1 -phényl -propyl) -amide
2ig Acide 2 -(2 -fluoro -phényl) -1 -oxo -3 -[4 -(2 -pipéridin -1 -yl -éthyl) - pipérazin -1 -ylméthyl] -1,2 -dihydro -isoquinoline -4 -carboxylique ((S) -1 -phényl -propyl) -amide
2ih Acide 2 -(2 -fluoro -phényl) -1 -oxo -3 -[4 -(2 -pyrrolidin -1 -yl -éthyl) - pipérazin -1 -ylméthyl] -1,2 -dihydro -isoquinoline -4 -carboxylique ((S) -1 -phényl -propyl) -amide
2ii Acide 2 -(2 -fluoro -phényl) -1 -oxo -3 -(4 -pyridin -4 -ylméthyl - pipérazin -1 -ylméthyl) -1,2 -dihydro -isoquinoline -4 -carboxylique ((S) -1 -phényl -propyl) -amide
2ij Acide 2 -(2 -fluoro -phényl) -3 -(4 -hydroxy -3,4,5,6 -tétrahydro -2h - [4,4']bipyridinyl -1 -ylméthyl) -1 -oxo -1,2 -dihydro -isoquinoline -4 - carboxylique ((S) -1 -phényl -propyl) -amide
2ik Acide 2 -(2 -fluoro -phényl) -3 -[4 -(2 -morpholin -4 -yl -éthyl) - pipéridin -1 -ylméthyl] -1 -oxo -1,2 -dihydro -isoquinoline -4 -carboxylique ((S) -1 -phényl -propyl) -amide
2il Acide 2 -(2 -fluoro -phényl) -3 -[4 -hydroxy -4 -(3 -méthoxy -phényl) - pipéridin -1 -ylméthyl] -1 -oxo -1,2 -dihydro -isoquinoline -4 -carboxylique ((S) -1 -phényl -propyl) -amide
2im Acide 3 -[4 -(2 -morpholin -4 -yl -éthyl) -pipérazin -1 -ylméthyl] -1 - oxo -2 -o -tolyl -1,2 -dihydro -isoquinoline -4 -carboxylique ((S) -1 -phényl -propyl) -amide
2in Acide 1 -oxo -3 -[4 -(2 -pipéridin -1 -yl -éthyl) -pipérazin -1 -ylméthyl] -2 -o -tolyl -1,2 -dihydro -isoquinoline -4 -carboxylique ((S) -1 -phényl - propyl) -amide
2io Acide 1 -oxo -3 -(4 -pyridin -4 -ylméthyl -pipérazin -1 -ylméthyl) -2 -o - tolyl -1,2 -dihydro -isoquinoline -4 -carboxylique ((S) -1 -phényl -propyl) - amide
2ip Acide 2 -(3 -fluoro -phényl) -1 -oxo -3 -(4 -phénétyl -pipérazin -1 - ylméthyl) -1,2 -dihydro -isoquinoline -4 -carboxylique ((S) -1 -phényl - propyl) -amide
2iq Acide 2 -(3 -fluoro -phényl) -3 -(4 -isopropyl -pipérazin -1 -ylméthyl) - 1 -oxo -1,2 -dihydro -isoquinoline -4 -carboxylique ((S) -1 -phényl - propyl) -amide
2ir Acide 3 -[1,4']bipipéridinyl -1 ' -ylméthyl -2 -(3 -fluoro -phényl) -1 -oxo -1,2 -dihydro -isoquinoline -4 -carboxylique ((S) -1 -phényl -propyl) - amide
2is Acide 2 -(3 -fluoro -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2it Acide 2 -(3 -fluoro -phényl) -3 -[4 -(1 -méthyl -pipéridin -4 -yl) - pipérazin -1 -ylméthyl] -1 -oxo -1,2 -dihydro -isoquinoline -4 - carboxylique ((S) -1 -phényl -propyl) -amide
2iu Acide 2 -(3 -fluoro -phényl) -1 -oxo -3 -[4 -(2 -pipéridin -1 -yl -éthyl) - pipérazin -1 -ylméthyl] -1,2 -dihydro -isoquinoline -4 -carboxylique ((S) -1 -phényl -propyl) -amide
2iv Acide 2 -(3 -fluoro -phényl) -1 -oxo -3 -[4 -(2 -pyrrolidin -1 -yl -éthyl) - pipérazin -1 -ylméthyl] -1,2 -dihydro -isoquinoline -4 -carboxylique ((S) -1 -phényl -propyl) -amide
2iw Acide 2 -(3 -fluoro -phényl) -1 -oxo -3 -(4 -pyridin -4 -ylméthyl - pipérazin -1 -ylméthyl) -1,2 -dihydro -isoquinoline -4 -carboxylique ((S) -1 -phényl -propyl) -amide
2ix Acide 2 -(3 -fluoro -phényl) -3 -[4 -(2 -morpholin -4 -yl -éthyl) - pipéridin -1 -ylméthyl] -1 -oxo -1,2 -dihydro -isoquinoline -4 -carboxylique ((S) -1 -phényl -propyl) -amide
2iy Acide 2 -(3 -fluoro -phényl) -3 -[4 -hydroxy -4 -(3 -méthoxy -phényl) - pipéridin -1 -ylméthyl] -1 -oxo -1,2 -dihydro -isoquinoline -4 -carboxylique ((S) -1 -phényl -propyl) -amide
2iz Acide 3 -[4 -(acétyl -méthyl -amino) -4 -phényl -pipéridin -1 -ylméthyl] -2 -(3 -fluoro -phényl) -1 -oxo -1,2 -dihydro -isoquinoline -4 -carboxylique ((S) -1 -phényl -propyl) -amide
2ja Acide 2 -(3 -chloro -phényl) -1 -oxo -3 -(4 -phénétyl -pipérazin -1 - ylméthyl) -1,2 -dihydro -isoquinoline -4 -carboxylique ((S) -1 -phényl - propyl) -amide
2jb Acide 2 -(3 -chloro -phényl) -3 -(4 -isopropyl -pipérazin -1 -ylméthyl) - 1 -oxo -1,2 -dihydro -isoquinoline -4 -carboxylique ((S) -1 -phényl - propyl) -amide
2jc Acide 3 -[1,4']bipipéridinyl -1 ' -ylméthyl -2 -(3 -chloro -phényl) -1 -oxo -1,2 -dihydro -isoquinoline -4 -carboxylique ((S) -1 -phényl -propyl) - amide
2jd Acide 2 -(3 -chloro -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2je Acide 2 -(3 -chloro -phényl) -3 -[4 -(1 -méthyl -pipéridin -4 -yl) - pipérazin -1 -ylméthyl] -1 -oxo -1,2 -dihydro -isoquinoline -4 - carboxylique ((S) -1 -phényl -propyl) -amide
2jf Acide 2 -(3 -chloro -phényl) -1 -oxo -3 -[4 -(2 -pipéridin -1 -yl -éthyl) - pipérazin -1 -ylméthyl] -1,2 -dihydro -isoquinoline -4 -carboxylique ((S) -1 -phényl -propyl) -amide
2jg Acide 2 -(3 -chloro -phényl) -1 -oxo -3 -[4 -(2 -pyrrolidin -1 -yl -éthyl) - pipérazin -1 -ylméthyl] -1,2 -dihydro -isoquinoline -4 -carboxylique ((S) -1 -phényl -propyl) -amide
2jh Acide 2 -(3 -chloro -phényl) -1 -oxo -3 -(4 -pyridin -4 -ylméthyl - pipérazin -1 -ylméthyl) -1,2 -dihydro -isoquinoline -4 -carboxylique ((S) -1 -phényl -propyl) -amide
2ji Acide 2 -(3 -chloro -phényl) -3 -[4 -(2 -morpholin -4 -yl -éthyl) - pipéridin -1 -ylméthyl] -1 -oxo -1,2 -dihydro -isoquinoline -4 -carboxylique ((S) -1 -phényl -propyl) -amide
2jj Acide 2 -(3 -chloro -phényl) -3 -[4 -hydroxy -4 -(3 -méthoxy -phényl) - pipéridin -1 -ylméthyl] -1 -oxo -1,2 -dihydro -isoquinoline -4 -carboxylique ((S) -1 -phényl -propyl) -amide
2jk Acide 3 -[4 -(acétyl -méthyl -amino) -4 -phényl -pipéridin -1 -ylméthyl] -2 -(3 -chloro -phényl) -1 -oxo -1,2 -dihydro -isoquinoline -4 -carboxylique ((S) -1 -phényl -propyl) -amide
2jl Acide 1 -oxo -3 -(4 -phénétyl -pipérazin -1 -ylméthyl) -2 -m -tolyl -1,2 - dihydro -isoquinoline -4 -carboxylique ((S) -1 -phényl -propyl) -amide
2jm Acide 3 -[1,4']bipipéridinyl -1 ' -ylméthyl -1 -oxo -2 -m -tolyl -1,2 - dihydro -isoquinoline -4 -carboxylique ((S) -1 -phényl -propyl) -amide
2jn Acide 3 -[4 -(2 -morpholin -4 -yl -éthyl) -pipérazin -1 -ylméthyl] -1 -oxo -2 -m -tolyl -1,2 -dihydro -isoquinoline -4 -carboxylique ((S) -1 -phényl - propyl) -amide
2jo Acide 3 -[4 -(1 -méthyl -pipéridin -4 -yl) -pipérazin -1 -ylméthyl] -1 - oxo -2 -m -tolyl -1,2 -dihydro -isoquinoline -4 -carboxylique ((S) -1 - phényl -propyl) -amide
2jp Acide 1 -oxo -3 -[4 -(2 -pipéridin -1 -yl -éthyl) -pipérazin -1 -ylméthyl] - 2 -m -tolyl -1,2 -dihydro -isoquinoline -4 -carboxylique ((S) -1 -phényl - propyl) -amide
2jq Acide 1 -oxo -3 - [4 -(2 -pyrrolidin -1 -yl -éthyl) -pipérazin -1 -ylméthyl] -2 -m -tolyl -1,2 -dihydro -isoquinoline -4 -carboxylique ((S) -1 -phényl - propyl) -amide
2jr Acide 1 -oxo -3 -(4 -pyridin -4 -ylméthyl -pipérazin -1 -ylméthyl) -2 -m - tolyl -1,2 -dihydro -isoquinoline -4 -carboxylique ((S) -1 -phényl -propyl) - amide
2js Acide 3 -[4 -(2 -morpholin -4 -yl -éthyl) -pipéridin -1 -ylméthyl] -1 -oxo -2 -m -tolyl -1,2 -dihydro -isoquinoline -4 -carboxylique ((S) -1 -phényl - propyl) -amide
2jt Acide 3 -[4 -hydroxy -4 -(3 -méthoxy -phényl) -pipéridin -1 -ylméthyl] - 1 -oxo -2 -m -tolyl -1 ,2 -dihydro -isoquinoline -4 -carboxylique ((S) -1 - phényl -propyl) -amide
2ju Acide 3 -[4 -(acétyl -méthyl -amino) -4 -phényl -pipéridin -1 -ylméthyl] -1 -oxo -2 -m -tolyl -1,2 -dihydro -isoquinoline -4 -carboxylique ((S) -1 - phényl -propyl) -amide
3a Acide 1 -oxo -3 -(2 -oxo -pyrrolidin -1 -ylméthyl) -2 -phényl -1,2 - dihydro -isoquinoline -4 -carboxylique ((S) -1 -phényl -propyl) -amide
3b Acide 8 -chloro -1 -oxo -3 -(2 -oxo -pyrrolidin -1 -ylméthyl) -2 -phényl - 1,2 -dihydro -isoquinoline -4 -carboxylique ((S) -1 -phényl -propyl) -amide
4a Acide 3 -cyanométhyl -1 -oxo -2 -phényl -1,2 -dihydro -isoquinoline -4 -carboxylique ((S) -1 -phényl -propyl) -amide
5a Acide 3 -Méthyl -1 -oxo -2 -phényl -1,2 -dihydro -isoquinoline -4 - carboxylique N,N -diphényl -hydrazide
5b Acide N'-(3-méthyl -1 -oxo -2 -phényl -1,2 -dihydro -isoquinoline -4 - carbonyl) -N -phényl -hydrazine carboxylique méthyl ester
1 aa Acide 2 -(3 -méthoxy -phényl) -3 -méthyl -1 -oxo -1,2 -dihydro - isoquinoline -4 -carboxylique ((S) -1 -phényl -propyl) -amide
1 ab Acide 2 -(4 -méthoxy -phényl) -3 -méthyl -1 -oxo -1,2 -dihydro - isoquinoline -4 -carboxylique ((S) -1 -phényl -propyl) -amide
1ac Acide 2 -(4 -fluoro -phényl) -3 -méthyl -1 -oxo -1,2 -dihydro - isoquinoline -4 -carboxylique ((S) -1 -phényl -propyl) -amide
1ad Acide 2 -(4 -chloro -phényl) -3 -méthyl -1 -oxo -1,2 -dihydro - isoquinoline -4 -carboxylique ((S) -1 -phényl -propyl) -amide
1 ae Acide 3 -méthyl -1 -oxo -2 -p -tolyl -1,2 -dihydro -isoquinoline -4 - carboxylique ((S) -1 -phényl -propyl) -amide
1 af Acide 2 -(3 -méthoxy -phényl) -3 -méthyl -1 -oxo -1,2 -dihydro - isoquinoline -4 -carboxylique [(S) -cyclopropyl -(3 -fluoro -phényl) - méthyl] -amide
1 ag Acide 2 -(4 -méthoxy -phényl) -3 -méthyl -1 -oxo -1,2 -dihydro - isoquinoline -4 -carboxylique [(S) -cyclopropyl -(3 -fluoro -phényl) - méthyl] -amide
1 ah Acide 2 -(4 -fluoro -phényl) -3 -méthyl -1 -oxo -1,2 -dihydro - isoquinoline -4 -carboxylique [(S) -cyclopropyl -(3 -fluoro -phényl) - méthyl] -amide
1ai Acide 2 -(4 -chloro -phényl) -3 -méthyl -1 -oxo -1,2 -dihydro - isoquinoline -4 -carboxylique [(S) -cyclopropyl -(3 -fluoro -phényl) - méthyl] -amide
1aj Acide 3 -méthyl -1 -oxo -2 -p -tolyl -1,2 -dihydro -isoquinoline -4 - carboxylique [(S) -cyclopropyl -(3 -fluoro -phényl) -méthyl] -amide
1 ak Acide 2 -(2 -méthoxy -phényl) -3 -méthyl -1 -oxo -1,2 -dihydro - isoquinoline -4 -carboxylique ((S) -1 -phényl -propyl) -amide
1 al Acide 2 -(2 -méthoxy -phényl) -3 -méthyl -1 -oxo -1,2 -dihydro - isoquinoline -4 -carboxylique [(S) -cyclopropyl -(3 -fluoro -phényl) - méthyl] -amide
1 am Acide 3 -méthyl -8 -nitro -1 -oxo -2 -phényl -1,2 -dihydro - isoquinoline -4 -carboxylique ((S) -1 -phényl -propyl) -amide
1 an Acide 3 -méthyl -8 -nitro -1 -oxo -2 -phényl -1,2 -dihydro - isoquinoline -4 -carboxylique [(S) -cyclopropyl -(3 -fluoro -phényl) - méthyl] -amide
1 ao Acide 8 -méthoxy -3 -méthyl -1 -oxo -2 -phényl -1,2 -dihydro - isoquinoline -4 -carboxylique ((S) -1 -phényl -propyl) -amide
1 ap Acide 8 -méthoxy -3 -méthyl -1 -oxo -2 -phényl -1,2 -dihydro - isoquinoline -4 -carboxylique [(S) -cyclopropyl -(3 -fluoro -phényl) - méthyl] -amide
1aq Acide 8 -Amino -3 -méthyl -1 -oxo -2 -phényl -1,2 -dihydro - isoquinoline -4 -carboxylique ((S) -1 -phényl -propyl) -amide
1 ar Acide 8 -cyano -3 -méthyl -1 -oxo -2 -phényl -1,2 -dihydro - isoquinoline -4 -carboxylique ((S) -1 -phényl -propyl) -amide
1as Acide 8 -chloro -3 -méthyl -1 -oxo -2 -phényl -1,2 -dihydro - isoquinoline -4 -carboxylique [(S) -1 -(4 -chloro -phényl) -propyl] -amide
1 at Acide 8 -chloro -3 -méthyl -1 -oxo -2 -phényl -1,2 -dihydro - isoquinoline -4 -carboxylique [(S) -1 -(4 -fluoro -phényl) -propyl] -amide
1 au Acide 8 -chloro -3 -méthyl -1 -oxo -2 -phényl -1,2 -dihydro - isoquinoline -4 -carboxylique [(S) -1 -(2 -fluoro -phényl) -propyl] -amide
1 av Acide 8 -chloro -3 -méthyl -1 -oxo -2 -phényl -1,2 -dihydro - isoquinoline -4 -carboxylique [(S) -1 -(3 -chloro -phényl) -propyl] -amide
1 aw Acide 8 -chloro -3 -méthyl -1 -oxo -2 -phényl -1,2 -dihydro - isoquinoline -4 -carboxylique [(S) -(3 -chloro -phényl) -cyclopropyl - méthyl] -amide
1 ax Acide 8 -chloro -3 -méthyl -1 -oxo -2 -phényl -1,2 -dihydro - isoquinoline -4 -carboxylique [(S) -(4 -chloro -phényl) -cyclopropyl - méthyl] -amide
1 ay Acide 8 -chloro -3 -méthyl -1 -oxo -2 -phényl -1,2 -dihydro - isoquinoline -4 -carboxylique [(S) -1 -(3 -fluoro -phényl) -propyl] -amide
1 az Acide 8 -chloro -3 -méthyl -1 -oxo -2 -phényl -1,2 -dihydro - isoquinoline -4 -carboxylique ((S) -2 -méthyl -1 -phényl -propyl) -amide
1 ba Acide 8 -chloro -3 -méthyl -1 -oxo -2 -phényl -1,2 -dihydro - isoquinoline -4 -carboxylique [(S) -cyclopropyl -(4 -fluoro -phényl) - méthyl] -amide
1 bb Acide 8 -chloro -3 -méthyl -1 -oxo -2 -phényl -1 ,2 -dihydro - isoquinoline -4 -carboxylique [(S) -1 -(2 -chloro -phényl) -propyl] -amide
1 bc Acide 8 -chloro -3 -méthyl -1 -oxo -2 -phényl -1,2 -dihydro - isoquinoline -4 -carboxylique ((S) -cyclopentyl -phényl -méthyl) -amide
1 bd Acide 8 -chloro -3 -méthyl -1 -oxo -2 -phényl -1 ,2 -dihydro - isoquinoline -4 -carboxylique [(S) -(2 -chloro -phényl) -cyclopropyl - méthyl] -amide
1 be Acide 8 -chloro -3 -méthyl -1 -oxo -2 -phényl -1,2 -dihydro - isoquinoline -4 -carboxylique [(S) -cyclopropyl -(2 -fluoro -phényl) - méthyl] -amide
1bf Acide 8 -chloro -3 -méthyl -1 -oxo -2 -phényl -1 ,2 -dihydro - isoquinoline -4 -carboxylique ((S) -cyclohexyl -phényl -méthyl) -amide
1 bg Acide 8 -chloro -3 -méthyl -1 -oxo -2 -phényl -1,2 -dihydro - isoquinoline -4 -carboxylique ((S) -cyclopropyl -phényl -méthyl) -amide
1 bh Acide 8 -chloro -3 -méthyl -1 -oxo -2 -phényl -1,2 -dihydro - isoquinoline -4 -carboxylique [(S) -cyclobutyl -(3 -fluoro -phényl) -méthyl] -amide
1bi Acide 8 -chloro -3 -méthyl -1 -oxo -2 -phényl -1,2 -dihydro - isoquinoline -4 -carboxylique [(S) -cyclobutyl -(4 -fluoro -phényl) -méthyl] -amide
1bl Acide 8 -chloro -3 -méthyl -1 -oxo -2 -phényl -1,2 -dihydro - isoquinoline -4 -carboxylique [(R) -cyclopropyl -(3 -fluoro -phényl) - méthyl] -amide
1bn Acide 8 -chloro -3 -méthyl -1 -oxo -2 -phényl -1,2 -dihydro - isoquinoline -4 -carboxylique ((S)cyclobutyl -phényl -méthyl) -amide
1bo Acide 8 -chloro -3 -méthyl -1 -oxo -2 -phényl -1,2 -dihydro - isoquinoline -4 -carboxylique [cyclobutyl -(2 -fluoro -phényl) -méthyl] - amide
2ka Acide 3 -(4 -tert -butyl -pipérazin -1 -ylméthyl) -8 -chloro -1 -oxo -2 - phényl -1,2 -dihydro -isoquinoline -4 -carboxylique [(S) -cyclopropyl -(3 - fluoro -phényl) -méthyl] -amide
2kb Acide 8 -chloro -3 -(4 -isopropyl -pipérazin -1 -ylméthyl) -1 -oxo -2 - phényl -1,2 -dihydro -isoquinoline -4 -carboxylique ((S) -1 -phényl - propyl) -amide
2kc Acide 8 -chloro -3 -(4 -isobutyl -pipérazin -1 -ylméthyl) -1 -oxo -2 - phényl -1,2 -dihydro -isoquinoline -4 -carboxylique ((S) -1 -phényl - propyl) -amide
2kd Acide 8 -chloro -3 -(octahydro -pyrido[1,2 -a]pyrazin -2 -ylméthyl) -1 - oxo -2 -phényl -1,2 -dihydro -isoquinoline -4 -carboxylique ((S) -1 -phényl -propyl) -amide
2ke Acide 8 -chloro -3 -(4 -hydroxy -3,4,5,6 -tétrahydro -2h - [4,4']bipyridinyl -1 -ylméthyl) -1 -oxo -2 -phényl -1,2 -dihydro - isoquinoline -4 -carboxylique ((S) -1 -phényl -propyl) -amide
2kf Acide 8 -chloro -3 -(4 -cyclopropylméthyl -pipérazin -1 -ylméthyl) -1 - oxo -2 -phényl -1,2 -dihydro -isoquinoline -4 -carboxylique ((S) -1 -phényl -propyl) -amide
2kg Acide 8 -chloro -3 -[4 -(2 -morpholin -4 -yl -éthyl) -pipéridin -1 - ylméthyl] -1 -oxo -2 -phényl -1,2 -dihydro -isoquinoline -4 -carboxylique ((S) -1 -phényl -propyl) -amide
2kh Acide 8 -chloro -3 -[1,4]diazépan -1 -ylméthyl -1 -oxo -2 -phényl -1,2 -dihydro -isoquinoline -4 -carboxylique ((S) -1 -phényl -propyl) -amide
2ki Acide 8 -chloro -3 -((S) -3 -méthyl -pipérazin -1 -ylméthyl) -1 -oxo -2 - phényl -1,2 -dihydro -isoquinoline -4 -carboxylique ((S) -1 -phényl - propyl) -amide
2kj Acide 8 -chloro -3 -imidazol -1 -ylméthyl -1 -oxo -2 -phényl -1,2 - dihydro -isoquinoline -4 -carboxylique ((S) -1 -phényl -propyl) -amide
2kk Acide 8 -chloro -3 -(4 -méthyl -imidazol - 1 -ylméthyl) -1 -oxo -2 - phényl -1,2 -dihydro -isoquinoline -4 -carboxylique ((S) -1 -phényl - propyl) -amide
2kl Acide 3 -(tert -butylamino -méthyl) -8 -chloro -1 -oxo -2 -phényl -1,2 - dihydro -isoquinoline -4 -carboxylique ((S) -1 -phényl -propyl) -amide
2km Acide 8 -chloro -3 -(isopropylamino -méthyl) -1 -oxo -2 -phényl -1,2 - dihydro -isoquinoline -4 -carboxylique ((S) -1 -phényl -propyl) -amide
2kn Acide 8 -chloro -3 -[4 -hydroxy -4 -(3 -méthoxy -phényl) -pipéridin -1 -ylméthyl] -1 -oxo -2 -phényl -1,2 -dihydro -isoquinoline -4 -carboxylique acid((S) -1 -phényl -propyl) -amide
2ko Acide 3 -(4 -tert -butyl -pipérazin -1 -ylméthyl) -8 -chloro -1 -oxo -2 - phényl -1,2 -dihydro -isoquinoline -4 -carboxylique ((S) -1 -phényl - propyl) -amide
2kp Acide 3 -benzo -imidazol - 1 -ylméthyl -1 -oxo -2 -phényl -1,2 - dihydro -isoquinoline -4 -carboxylique ((S) -1 -phényl -propyl) -amide
2kq Acide 1 -oxo -2 -phényl -3 -pyrazol -1 -ylméthyl -1,2 -dihydro - isoquinoline -4 -carboxylique ((S) -1 -phényl -propyl) -amide
2kr Acide 3 -(4 -méthyl -pyrazol -1 -ylméthyl) -1 -oxo -2 -phényl -1,2 - dihydro -isoquinoline -4 -carboxylique ((S) -1 -phényl -propyl) -amide
2ks Acide 1 -oxo -2 -phényl -3 -[1,2,3]triazol -1 -ylmethyl -1,2 -dihydro - isoquinoline -4 -carboxylique ((S) -1 -phényl -propyl) -amide
2kt Acide 2 -(3 -méthoxy -phényl) -1 -oxo -3 -(4 -pyridin -4 -ylméthyl - pipérazin -1 -ylméthyl) -1,2 -dihydro -isoquinoline -4 -carboxylique ((S) -1 -phényl -propyl) -amide
2ku Acide 2 -(4 -méthoxy -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2kv Acide 2 -(4 -fluoro -phényl) -1 -oxo -3 -(4 -phénétyl -pipérazin -1 - ylméthyl) -1,2 -dihydro -isoquinoline -4 -carboxylique ((S) -1 -phényl - propyl) -amide
2kw Acide 2 -(4 -fluoro -phényl) -3 -(4 -isopropyl -pipérazin -1 -ylméthyl) - 1 -oxo -1,2 -dihydro -isoquinoline -4 -carboxylique ((S) -1 -phényl - propyl) -amide
2kx Acide 3 -[1,4'] bipipéridinyl -1 -ylméthyl -2 -(4 -fluoro -phényl) -1 -oxo -1,2 -dihydro -isoquinoline -4 -carboxylique ((S) -1 -phényl -propyl) - amide
2ky Acide 2 -(4 -fluoro -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2kz Acide 2 -(4 -fluoro -phényl) -3 -[4 -(1 -méthyl -pipéridin -4 -yl) - pipérazin -1 -ylméthyl] -1 -oxo -1,2 -dihydro -isoquinoline -4 - carboxylique ((S) -1 -phényl -propyl) -amide
2la Acide 2 -(4 -fluoro -phényl) -1 -oxo -3 -[4 -(2 -pyrrolidin -1 -yl -éthyl) - pipérazin -1 -ylméthyl] -1,2 -dihydro -isoquinoline -4 -carboxylique ((S) - 1 -phényl -propyl) -amide
2lb Acide 2 -(4 -fluoro -phényl) -3 -[4 -hydroxy -4 -(3 -méthoxy -phényl) - pipéridin -1 -ylméthyl] -1 -oxo -1,2 -dihydro -isoquinoline -4 -carboxylique ((S) -1 -phényl -propyl) -amide
2lc Acide 2 -(4 -chloro -phényl) -1 -oxo -3 -(4 -phénétyl -pipérazin -1 - ylméthyl) -1,2 -dihydro -isoquinoline -4 -carboxylique ((S) -1 -phényl - propyl) -amide
2ld Acide 2 -(4 -chloro -phényl) -3 -(4 -isopropyl -pipérazin -1 -ylméthyl) - 1 -oxo -1,2 -dihydro -isoquinoline -4 -carboxylique ((S) -1 -phényl - propyl) -amide
2le Acide 3 -[1,4']bipipéridinyl -1 ' -ylméthyl -2 -(4 -chloro -phényl) -1 -oxo -1,2 -dihydro -isoquinoline -4 -carboxylique ((S) -1 -phényl -propyl) - amide
2lf Acide 2 -(4 -chloro -phényl) -3 -[4 -(2 -morpholin -4 -yl -éthyl) - pipérazin -1 -ylméthyl] -1 -oxo -1,2 -dihydro -isoquinoline -4 - carboxylique ((S) -1 -phényl -propyl) -amide
2lg Acide 2 -(4 -chloro -phényl) -3 -[4 -(1 -méthyl -pipéridin -4 -yl) - pipérazin -1 -ylméthyl] -1 -oxo -1,2 -dihydro -isoquinoline -4 - carboxylique ((S) -1 -phényl -propyl) -amide
2lh Acide 2 -(4 -chloro -phényl) -1 -oxo -3 -[4 -(2 -pipéridin -1 -yl -éthyl) - pipérazin -1 -ylméthyl] -1,2 -dihydro -isoquinoline -4 -carboxylique ((S) -1 -phényl -propyl) -amide
2li Acide 2 -(4 -chloro -phényl) -1 -oxo -3 -[4 -(2 -pyrrolidin -1 -yl -éthyl) - pipérazin -1 -ylméthyl] -1,2 -dihydro -isoquinoline -4 -carboxylique ((S) -1 -phényl -propyl) -amide
2lj Acide 3 -(4 -tert -butyl -pipérazin -1 -ylméthyl) -8 -fluoro -1 -oxo -2 - phényl -1,2 -dihydro -isoquinoline -4 -carboxylique ((S) -1 -phényl - propyl) -amide
2lk Acide 8 -chloro -3 -cyclopropylaminométhyl -1 -oxo -2 -phényl -1,2 - dihydro -isoquinoline -4 -carboxylique ((S) -1 -phényl -propyl) -amide
2ll Acide 3 -(4 -tert -butyl -pipérazin -1 -ylméthyl) -8 -fluoro -1 -oxo -2 - phényl -1,2 -dihydro -isoquinoline -4 -carboxylique [(S) -cyclopropyl -(3 - fluoro -phényl) -méthyl] -amide
2lm Acide 8 -fluoro -1 -oxo -2 -phényl -3 -pipérazin -1 -ylméthyl -1,2 - dihydro -isoquinoline -4 -carboxylique [(S) -cyclopropyl -(3 -fluoro - phényl) -méthyl] -amide
2ln Acide 8 -fluoro -1 -oxo -3 -(3 -oxo -pyrazolidin -1 -ylméthyl) -2 -phényl -1,2 -dihydro -isoquinoline -4 -carboxylique [(S) -cyclopropyl -(3 -fluoro - phényl) -méthyl] -amide
2lo Acide 8 -fluoro -1 -oxo -3 -(3 -oxo -pipérazin -1 -ylméthyl) -2 -phényl - 1,2 -dihydro -isoquinoline -4 -carboxylique [(S) -cyclopropyl -(3 -fluoro - phényl) -méthyl] -amide
3c Acide 1 -oxo -2 -phényl -3 -(1 h -[1,2,4]triazol -3 -ylsulfanylméthyl) -1,2 -dihydro -isoquinoline -4 -carboxylique ((S) -1 -phényl -propyl) -amide
3d Acide 8 -chloro -1 -oxo -3 -(2 -oxo -pyrrolidin -1 -ylméthyl) -2 -phényl - 1,2 -dihydro -isoquinoline -4 -carboxylique [(S) -cyclopropyl -(3 -fluoro - phényl) -méthyl] -amide
3e Acide 8 -fluoro -1 -oxo -3 -(2 -oxo -pyrrolidin -1 -ylméthyl) -2 -phényl - 1,2 -dihydro -isoquinoline -4 -carboxylique ((S) -1 -phényl -propyl) -amide
3f Acide 8 -fluoro - 1 -oxo -3 -(2 -oxo -pyrrolidin -1 -ylméthyl) -2 -phényl - 1,2 -dihydro -isoquinoline -4 -carboxylique [(S) -cyclopropyl -(3 -fluoro - phényl) -méthyl] -amide
3g Acide 8 -fluoro -1 -oxo -3 -(5 -oxo -pyrazolidin -1 -ylméthyl) -2 -phényl -1,2 -dihydro -isoquinoline -4 -carboxylique [(S) -cyclopropyl -(3 -fluoro - phényl) -méthyl] -amide
3h Acide 8 -fluoro -1 -oxo -3 -(2 -oxo -pipérazin -1 -ylméthyl) -2 -phényl - 1,2 -dihydro -isoquinoline -4 -carboxylique [(S) -cyclopropyl -(3 -fluoro - phényl) -méthyl] -amide
3i Acide 8 -fluoro -1 -oxo -3 -(2 -oxo -pipéridin -1 -ylméthyl) -2 -phényl - 1,2 -dihydro -isoquinoline -4 -carboxylique [(S) -cyclopropyl -(3 -fluoro - phényl) -méthyl] -amide
4b Acide 8 -chloro -3 -cyanométhyl -1 -oxo -2 -phényl -1,2 -dihydro - isoquinoline -4 -carboxylique [(S) -cyclopropyl -(3 -fluoro -phényl) - méthyl] -amide
6a Acide 2 -(3,4 -dichloro -phényl) -3 -méthyl -1 -oxo -1,2 -dihydro - isoquinoline -4 -carboxylique ((S) -1 -phényl -propyl) -amide
6b Acide 8 -fluoro -1 -oxo -3 -(2 -oxo -pyrrolidin -1 -ylméthyl) -2 -phényl - 1,2 -dihydro -isoquinoline -4 -carboxylique ((S) -cyclopropyl -phényl - méthyl) -amide
6c Acide 8 -fluoro -3 -méthyl -1 -oxo -2 -phényl -1,2 -dihydro - isoquinoline -4 -carboxylique ((S) -cyclopropyl -phényl -méthyl) -amide
7a Acide 3 -éthyl -1 -oxo -2 -phényl -1,2 -dihydro -isoquinoline -4 - carboxylique ((S) -1 -phényl -propyl) -amide
7b Acide 8 -fluoro -3 -méthyl -1 -oxo -2 -phényl -1,2 -dihydro - isoquinoline -4 -carboxylique ((S) -1 -phényl -propyl) -amide
7c Acide 8 -fluoro -2 -(3 -fluoro -phényl) -3 -méthyl -1 -oxo -1,2 -dihydro - isoquinoline -4 -carboxylique ((S) -1 -phényl -propyl) -amide
7d Acide 8 -fluoro -2 -(4 -fluoro -phényl) -3 -méthyl -1 -oxo -1,2 -dihydro - isoquinoline -4 -carboxylique ((S) -1 -phényl -propyl) -amide
7f Acide 8 -fluoro -2 -(3 -fluoro -phényl) -1 -oxo -3 -(2 -oxo -pyrrolidin -1 - ylméthyl) -1,2 -dihydro -isoquinoline -4 -carboxylique [(S) -cyclopropyl -(3 -fluoro -phényl) -méthyl] -amide
7g Acide 8 -fluoro -2 -(4 -fluoro -phényl) -1 -oxo -3 -(2 -oxo -pyrrolidin -1 - ylméthyl) -1,2 -dihydro -isoquinoline -4 -carboxylique [(S) -cyclopropyl -(3 -fluoro -phényl) -méthyl] -amide
7i Acide 8 -fluoro -2 -(2 -fluoro -phényl) -3 -méthyl -1 -oxo -1,2 -dihydro - isoquinoline -4 -carboxylique [(S) -cyclopropyl -(3 -fluoro -phényl) - méthyl] -amide
7j Acide 8 -fluoro -2 -(3 -fluoro -phényl) -3 -méthyl -1 -oxo -1,2 -dihydro - isoquinoline -4 -carboxylique [(S) -cyclopropyl -(3 -fluoro -phényl) - méthyl] -amide
7k Acide 8 -fluoro -2 -(4 -fluoro -phényl) -3 -méthyl -1 -oxo -1,2 -dihydro - isoquinoline -4 -carboxylique [(S) -cyclopropyl -(3 -fluoro -phényl) - méthyl] -amide
7l Acide 6,8 -difluoro -3 -méthyl -1 -oxo -2 -phényl -1,2 -dihydro - isoquinoline -4 -carboxylique [(S) -cyclopropyl -(3 -fluoro -phényl) - méthyl] -amide
7m Acide 5,8 -difluoro -3 -méthyl -1 -oxo -2 -phényl -1,2 -dihydro - isoquinoline -4 -carboxylique [(S) -cyclopropyl -(3 -fluoro -phényl) - méthyl] -amide
7n Acide 3 -méthyl -1 -oxo -2 -phényl -8 -trifluorométhyl -1,2 -dihydro - isoquinoline -4 -carboxylique ((S) -1 -phényl -propyl) -amide 8a Acide 3 -(1 -tert -butyl -pipéridin -4 -ylméthyl) -8 -chloro -1 -oxo -2 - phényl -1,2 -dihydro -isoquinoline -4 -carboxylique [(S) -cyclopropyl -(3 - fluoro -phényl) -méthyl] -amide ;
et les sels acceptables sur le plan pharmaceutique de n'importe lesquels de ces composés.

7. Composé selon l'une quelconque des revendications 1 à 6 destiné à être utilisé en thérapie.

8. Composé selon l'une quelconque des revendications 1 à 6 destiné à être utilisé dans le traitement d'une maladie choisie parmi une psychose ; une schizophrénie ; un trouble schizophréniforme ; un trouble schizoaffectif ; un trouble délirant ; un trouble psychotique bref ; un trouble psychotique partagé ; un trouble psychotique lié à une affection médicale générale ; un trouble psychotique induit par une substance ou une drogue (cocaïne, alcool, amphétamine, etc.) ; un trouble de la personnalité schizoïde ; un trouble de la personnalité schizotypique ; une psychose ou schizophrénie associée à une dépression majeure, un trouble bipolaire, la maladie d'Alzheimer ou la maladie de Parkinson ; une dépression majeure ; un trouble de l'anxiété généralisée ; un trouble bipolaire (traitement de substitution, prophylaxie et stabilisation de récidive) ; une manie ; une hypomanie ; une déficience cognitive ; un trouble d'hyperactivité avec déficit d'attention ; une obésité ; une réduction de l'appétit ; la maladie d'Alzheimer ; la maladie de Parkinson ; une douleur ; des convulsions ; la toux ; l'asthme ; une hypersensibilité des voies aériennes ; une hypersensibilité microvasculaire ; une bronchoconstriction ; une maladie pulmonaire obstructive chronique ; une incontinence urinaire ; une inflammation des intestins ; et un syndrome intestinal inflammatoire.

9. Utilisation d'un composé selon l'une quelconque des revendications 1 à 6 pour la fabrication d'un médicament pour le traitement d'une maladie choisie parmi une psychose ; une schizophrénie ; un trouble schizophréniforme ; un trouble schizoaffectif ; un trouble délirant ; un trouble psychotique bref ; un trouble psychotique partagé ; un trouble psychotique lié à une affection médicale générale ; un trouble psychotique induit par une substance ou une drogue (cocaïne, alcool, amphétamine, etc.) ; un trouble de la personnalité schizoïde ; un trouble de la personnalité schizotypique ; une psychose ou schizophrénie associée à une dépression majeure, un trouble bipolaire, la maladie d'Alzheimer ou la maladie de Parkinson ; une dépression majeure ; un trouble de l'anxiété généralisée ; un trouble bipolaire (traitement de substitution, prophylaxie et stabilisation de récidive) ; une manie ; une hypomanie ; une déficience cognitive ; un trouble d'hyperactivité avec déficit d'attention ; une obésité ; une réduction de l'appétit ; la maladie d'Alzheimer ; la maladie de Parkinson ; une douleur ; des convulsions ; la toux ; l'asthme ; une hypersensibilité des voies aériennes ; une hypersensibilité microvasculaire ; une bronchoconstriction ; une maladie pulmonaire obstructive chronique ; une incontinence urinaire ; une inflammation des intestins ; et un syndrome intestinal inflammatoire.

10. Utilisation selon la revendication 9, dans laquelle ladite fabrication comprend en outre l'utilisation d'un composé choisi parmi la liste constituée des antagonistes de D2, agonistes partiels de D2, antagonistes de PDE10, antagonistes de 5-HT_{2A}, antagonistes de 5-HT₆ et antagonistes de KCNQ4.

11. Composition pharmaceutique comprenant un composé selon l'une quelconque des revendications 1-10 et un ou plusieurs supports ou excipients acceptables d'un point de vue pharmaceutique.

12. Composition selon la revendication 11, qui comprend en outre un composé choisi parmi la liste constituée des antagonistes de D2, agonistes partiels de D2, antagonistes de PDE10, antagonistes de 5-HT_{2A}, antagonistes de 5-HT₆ et antagonistes de KCNQ4.

13. Trousse comprenant un composé selon l'une quelconque des revendications 1 à 12 conjointement avec un composé choisi parmi la liste constituée des antagonistes de D2, agonistes partiels de D2, antagonistes de PDE10, antagonistes de 5-HT_{2A}, antagonistes de 5-HT₆ et antagonistes de KCNQ4.
